# EUROPEAN PATENT SPECIFICATION

(11) **EP 2 266 989 B1**
(45) Date of publication and mention of the grant of the patent: **02.09.2015**
(21) Application number: 09754921.6
(22) Date of filing: 31.03.2009
(51) Int. Cl.: C07D 498/04, A61K 31/4375, A61P 3/00, A61P 9/12

(54) **HETEROCYCLIC DERIVATIVES**
HETEROCYCLISCHE DERIVATE
DÉRIVÉS HÉTÉROCYCLIQUES

(30) Priority: 31.03.2008 KR 20080030067
(43) Date of publication of application: 29.12.2010
(73) Proprietor: C&c Research Laboratories, Gyeonggi-do 445-380 (KR)
(72) Inventor: AHN, Sung Oh, Hwaseong-city Gyeonggi-do 445-380 (KR); PARK, Chan Hee, Hwaseong-city Gyeonggi-do 445-380 (KR); IM, Jun Hwan, Hwaseong-city Gyeonggi-do 445-380 (KR); LEE, Soon Ok, Hwaseong-city Gyeonggi-do 445-380 (KR); LEE, Kyoung June, Hwaseong-city Gyeonggi-do 445-380 (KR); CHO, Seong Wook, Hwaseong-city Gyeonggi-do 445-380 (KR); KO, Kwang Seok, Hwaseong-city Gyeonggi-do 445-380 (KR); HAN, Sun Young, Hwaseong-city Gyeonggi-do 445-380 (KR); LEE, Won Il, Hwaseong-city Gyeonggi-do 445-380 (KR)
(74) Representative: Crump, Julian Richard John
(86) International application number: PCT/KR2009/001659
(87) International publication number: WO 2009/145456

(56) References cited:
- WO-A1-2007/089034
- WO-A1-2007/093507
- WO-A2-2009/120660
- US-A1- 2007 010 670
- US-A1- 2007 010 670
- US-A1- 2007 197 512
- US-A1- 2008 064 871
- US-A1- 2008 064 871
- MIURA, YUTAKA ET AL: "Synthesis of 2,3-fused quinolines from 3-substituted quinoline 1-oxides. Part 1", HETEROCYCLES ( 1992 ), 34(5), 1055-63 CODEN: HTCYAM; ISSN: 0385-5414, 1992, - 1992, XP009151642,

## Description

### [Technical field]

The present invention relates to novel heterocycle derivative compounds useful in preparing drugs for treatment of diseases associated with uric acid. More specifically, these novel heterocycle derivatives are useful in preparing drugs for the treatment of diseases which are included in area of diseases treated by using uricosuric agent to target human urate anion transporter 1 (hURAT1). These drugs are useful in treatment of cardiovascular diseases and metabolic syndrome associated with uric acid such as, in particular, hyperuricemia, acute gouty arthritis, chronic gouty arthritis, tophus, gout nephrosis, nephritis, chronic renal failure, nephrolithiasis, uremia, urolithiasis, hyperlipidemia, ischemic heart disease, myocardial infarction, cerebral infarction, cerebrovascular disease, diabetes or hypertension.

### [Background art]

Uric acid is the final oxidation product of purine metabolism and is mainly excreted in urine. It has been reported to have an antioxidative activity and also a function of protecting neuronal cell [Ames, B. N. et al. Proc. Natl. Acad. Sci. USA, 78, 6858-6862 (1981); Becker, B. F., et al., Free Racical Biol. Med. 14, p615-631 (1993); Keller, J. N. et al., J. Neurosci. p687-697 (1998)].

Purine sources, generated from nucleotides existing in cells constituting a living body, and excessively purine-free diet converge at the common intermediate xanthine by *in vivo* biosynthesis procedure, and uric acid is finally produced by enzymatic reaction with xanthine oxidase, the key enzyme in taking the purines all of the way to uric acid, in liver [F. Borges et al. Current Medi. Chem., 9, p195-217 (2002)]. The level of daily uric acid produced in body is approximately 700mg. Of this total, 60~70% (~500mg/day) is excreted through kidney and the residual amount (~200mg/day) is excreted through intestine [Japan Clinic History, Japan Clinic Hyperuricemia Hypouricemia, p161, 166 (2003)].

Hyperuricemia is the abnormally high level of uric acid in blood. In human, it is defined the condition that serum uric acid concentration is higher than normal (7~8mg/dl for male, 6mg/dl for female). It is associated with underexcretion of uric acid in the kidney or overproduction of uric acid in the liver. Gouty patient has a remarkably higher level uric acid in blood than normal (7~8mg/dl for male, 6mg/dl for female). Gout is often associated with hyperuricemia. Gout is a type of inflammatory arthritis that is triggered by the crystallization of uric acid within the joint-mostly peripheral ones like the toes and fingers. Acute gout is typically intermittent, constituting one of the most painful experienced by human. Chronic gout usually develops after years of acute intermittent gout. Nedle-like monosodium urate (MSU) crystals can be deposited on connective soft tissues such as the articular cartilage in the joint, tendon and ligament. These crystals prick muscles or chondrocyte around the joint and then lead to inflammatory arthritis, which causes swelling, redness, heat, pain, and stiffness in the joints. Urate crystals are directly able to initiate, to amplify, and to sustain an intense inflammatory attack because of their ability to stimulate the release of inflammatory mediators. The urate crystals are deposited mainly in metatarsophalangeal joint of big toe, and rarely in lumbar spine [Vervaeck M., et al., Clinical Neurology and Neurosurgery, 93, p233-236 (1991)].

Gout is a very dangerous factor because it may cause a complication of various metabolic diseases such as diabetes, hypertension, heart disease, obesity, nephrolithiasis, urolithiasis or the like. Peak incidence of gout is observed predominantly male in age of 40 to 50's and female patients increase in postmenopausal period. Also, the onset frequency is high in obese persons and those exercising extremely.

Incidence of gouty attack is closely associated with patients who have had hyperuricemia for years. It has been reported that incidence of gouty attack is 4.9% when uric acid level in body is 9mg/dl or higher, 0.5% when uric acid level in body is 7.0~8.9mg/dl and 0.1% when uric acid level in body is 7.0mg/d1 or lower, and accumulated incidence of gouty attack for 5 years is about 22% in patients having uric acid level in body of 9mg/dl or higher [Campion E. W. et al., Am. J. Med., 82, p421-426 (1987)].

As mentioned above, the amount of urate in the body depends on the balance between dietary intake, synthesis, and the excretion rate. For patients having an high level of uric acid in blood, pathogenesis of hyperuricemia and gout result from urate underexcretion (90%), overproduction (10%) or often a combination of the two [Choi et al., Ann. Intern. Med. p499-516 (2005)]. Considering such causes of hyperuricemia and gout, development of uricosuric agent is more effective in hyperuricemia/gout management than those suppressing uric acid production. Urate level in human plasma is higher than those of most other mammals. Human are the only mammals in whom gout is known to develop spontaneously. This is because during evolutionary process, human and primate have lost the gene for uricase in liver which is known as enzyme for degradation of uric acid (Uricase enzyme; catalyzing the conversion of uric acid to more soluble compound allantoin) [Fields, M. et al., Free Radical Biol. Med., 20, p595 (1996); Haliwell, B. Uric acid: an example of antioxidant evaluation, E. Cadenas and L. Packer Editors, Handbook of Antioxidants, Marcel Dekker New York (1996)] and have had a reuptake system of uric acid in kidney by which most of filtered urate from glomerulus is reabsorbed through renal tubule.

A recent literature has reported a gene (SLC22A12) encoding human urate anion transporter 1 (hURAT1), an anion exchange membrane transporter specifically responsible for the function of reabsorption of filtered urate in kidney. The transporter (hURAT1) belongs to organic anion transporter family (OATs) and it has been reported that human urate anion transporters exist in proximal renal tubule through immunochemical experiments and is an important role in urate reuptake through urate absorption experiments by using brush border membrane vesicle (BBMV) of human kidney. Therefore, the human urate anion transporter 1 (hURAT1) has been proved as a useful target molecule for developing treatment agents of diseases associated with uric acid such as hyperuricemia and gout [Enomoto A. et al., Nature, 417, p447-452 (2002)].

In physicochemical properties, uric acid has the acidity (pKa) of 5.75 and exists in acid form (uric acid) or anionic form (urate) depending on pH. Thus, the protein structure of human urate anion transporter 1 (hURAT1) having a functional similarity is expected to have similar structural characteristics with proteins belonging to organic anion transporter family (OATs). Actually, it has been reported that the amino acid sequence of OAT4 (SLC22A12) among organic anion transporter family (OATs) transporting anion present in apical membrane of proximal tubule has a homology of 42% with that of human urate anion transporter 1 (hURAT1) protein [Enomoto A. et al., Nature, 417, p447-452 (2002)].

Up to the present, six (6) of transporters in living body have been identified (OAT1 to 4 and URAT1, and OAT5 of rodents) which are involved in absorption and excretion of anionic substances in kidney that are made from various endogenous substances, xenobiotics and drugs. Their main target substrates are various and different from each other. Meanwhile, as a main substrate for human urate anion transporter 1 (hURAT1), only the uric acid is known [Nahohiko Anzai, et al., J phamacol. Sci., 100, p411-426 (2006)].

As treatment or prophylaxis agents for hyperuricemia and gout, benzbromarone that is an uricosuric agent having the inhibitory activity of hURAT1-mediated urate reabsorption, as well as probenecid and sulfinpyrazone is currently used. However, these drugs do not have sufficient activities to URAT1. In particular, benzbromarone has some demerits in view of adverse effects. Benzbromarone shows a strong inhibitory function to 2C9 protein among cytochrome P450 (CYP450) proteins and thus has a possibility of drug-drug interaction. Formation of reactive metabolite also has been reported from glutathione (GSH) conjugate formation experiments [Dermot F. McGinnity et al., Drug Metabolism and Disposition, 33, p1700-1707 (2005)]. Furthermore, because benzbromarone has a benzofuran backbone similar with drug structures of benziodarone, benzarone and amiodarone which are drugs reported to show hepatotoxicity, it has a problem of death due to hepatotoxicity induction as well as adverse effect of liver injury. Therefore, a liver function of patients who intend to take this drug must be examined before the administration, and even during the administration, it is recommended in therapy to check out for a certain period (six months) on whether the hepatotoxicity is induced or not. For these reason, there still remains an unmet medical need for the treatment of diseases associated with uric acid such as hyperuricemia and gout [Hautekeete M. L., et al., Liver, 15, p25-29 (1995); Makoto Arai, et al., Journal of Gastroenterology and Hepatology 17, p625 -626 (2002); Saitama medical college magazine, 30, 187-194 (2003); Priska Kaufmann, et al., HEPATOLOGY, 41, p925-935 (2005)].

Pharmacokinetic data of benzbromarone are as follows: As for the concentration in blood, when 2 tablets (50 mg/tablet) are administered one time to a healthy and fasting adult, time of maximum drug concentration in blood (Tₘₐₓ) of unmodified benzbromarone is 2.7±1.0 hr, half-life of drug is 5.4±1.9 hr, area under the curve (AUC) is 15.9±3.3 µg· h/Mℓ, and maximum concentration of drug (Cₘₐₓ) is 2.3±0.8 µg/Mℓ. In case of 6-hydroxy benzbromarone which is a metabolite of benzbromarone, time of maximum drug concentration in blood (Tₘₐₓ) is 4.8±1.3 hr, half-life of drug is 18.0±2.9 hr, area under the curve (AUC) is 39.9±4.4 µg· h/Mℓ, and maximum concentration of drug (Cₘₐₓ) is 1.7±0.4 µg/Mℓ. As for the part and amount of drug excretion, the excretion rate of 6-hydroxy benzbromarone in urine was 1.2% of the administered amount until 72 hours after administration. However, the benzbromarone was not detected in urine at all [Urinome international interview (2005); Oikawa Tosihiro et al., New drug and Clinic, 53, p682 (2004)].

Recently, one patent was disclosed and registered in Japan for compounds which inhibit human urate anion transporter 1 (hURAT1) as an uricosuric agent and have a weak inhibitory effect on cytochrome P450 2C9 (CYP2C9) [Japan Tobacco, WO2006/057460 and JP3988832 B2].

### Disclosure of Invention

### [Technical purpose]

The present invention is to solve the problems of prior arts as set forth in the foregoing. Thus, the purpose of the present invention is to provide novel heterocycle derivative compounds having a therapeutic activity to hyperuricemia which show a strong inhibition activity on human urate anion transporter 1 (hURAT1), no drug-drug interaction on cytochrome P450 (CYP450), especially CYP2C9 and a selectivity between organic anion transporters, and can be administered with lower dose and long-term treatment since they have higher solubility and metabolic stability so as to show advantageous pharmacokinetics, as compared with conventional inhibitor of hURAT1 activity; and a pharmaceutical composition comprising the same. Because the novel heterocycle derivative compounds according to the present invention and pharmaceutical composition comprising the same show a strong inhibition activity to human urate anion transporter 1 (hURAT1), they control the uric acid reuptake and thus are useful in treatment or prophylaxis of hyperuricemia, acute gouty arthritis, chronic gouty arthritis, tophus, gout nephrosis, nephritis, chronic renal failure, nephrolithiasis, uremia, urolithiasis and complications reported to be accompanied with uric acid increase in blood such as hyperlipidemia, ischemic heart disease, myocardial infarction, arteriosclerosis, cerebral infarction, cerebrovascular disease, diabetes and hypertension.

### [Technical solution]

According to the present invention, a heterocycle derivative compound having a structure of the following Formula I, or racemate, isomer or pharmaceutically acceptable salt thereof is provided: 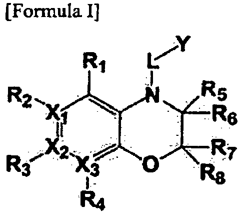 wherein, in Formula I,
each of X₁, X₂ and X₃ is independently carbon or nitrogen, provided that at least one of X₁, X₂ and X₃ is nitrogen,
each of R₁, R₂, R₃ and R₄ may be same or different and is independently selected from the group consisting of hydrogen; hydroxy; C₁-C₆ alkyl; C₂-C₇ alkenyl; C₂-C₇ alkynyl; C₁-C₆ hydroxyalkyl; C₁-C₆ haloalkyl; C₁-C₆ alkoxy, C₁-C₆ haloalkoxy; halogen; phenyl; cyano; nitro; amino; carboxylic acid group; phosphoric acid group; N-oxide; amide; C₁-C₆ alkylamide; aldehyde; hydroxamic acid; C₁-C₆ alkylsulfide; C₁-C₆ alkylthioxo; C₁-C₆ alkylsulfonyl; C₁-C₆ oximealkyl; C₁-C₆ aminoalkyl; C₃-C₈ alkylcarbonylalkyl; C₂-C₇ alkanoyl; C₂-C₇ alkoxycarbonyl; C₂-C₇ alkanoyloxy; C₃-C₁₂ mono or bicycloalkyl; C₄-C₁₂ cycloalkylalkyl; C₆-C₁₂ aryl; , saturated or unsaturated C₃-C₁₂ mono or polycarbocyclyl; and , saturated or unsaturated 3- to 12-membered mono or polyheterocyclyl containing 1 to 3 heteroatoms (preferably, the heteroatom is selected from N, O and S), provided that when X₁ is nitrogen, R₂ does not exist; when X₂ is nitrogen, R₃ does not exist; and when X₃ is nitrogen, R₄ does not exist, or
each of R₁-R₂, R₂-R₃ and R₃-R₄ pairs may be independently fused to form a saturated or
unsaturated 5- to 11-membered carbocycle or heterocycle (preferably, the heterocycle contains 1 to 3 heteroatoms selected from N, O and S),
each of R₅, R₆, R₇ and R₈ may be same or different and is independently selected from the group consisting of hydrogen; hydroxy; C₁-C₆ alkyl; C₂-C₇ alkenyl; C₂-C₇ alkynyl; C₁-C₆ hydroxyalkyl; C₁-C₆ haloalkyl; C₁-C₆ alkoxy; C₁-C₆ haloalkoxy; C₂-C₇ alkanoyl; phosphoric acid group; N-oxide; amide; aldehyde; hydroxamic acid; C₁-C₆ alkylsulfide; C₁-C₆ alkylthioxo; C₁-C₆ alkylsulfonyl; C₁-C₆ oximealkyl; C₁-C₆ aminoalkyl; C₃-C₈ alkylcarbonylalkyl; halogen; phenyl; cyano; nitro; amino; and carboxylic acid group, or R₅ and R₆ together with a carbon atom to which they are attached may form a carbonyl group (C=O) or thioxo group (C=S), or R₇ and R₈ together with a carbon atom to which they are attached may form a carbonyl group (C=O) or thioxo group (C=S),
L may form a carbonyl group (-C(=O)-), sulfonyl group (-S(=O)₂-), C₁-C₆ alkyl carbonyl (for example, -CH₂C(=O)-), carbonyl C₁-C₆ alkyl (for example, -C(=O)CH₂-) or thioxo group (-C(=S)-), and
Y is selected from the group consisting of saturated or unsaturated C₃-C₁₂ mono or polycarbocyclyl substituted with R₉, R₁₀ and R₁₁; and saturated or unsaturated 3- to 12-membered mono or polyheterocyclyl containing 1 to 3 heteroatoms (preferably, the heterocycle contains 1 to 3 heteroatoms selected from N, O and S) and being substituted with R₉, R₁₀ and R₁₁,
wherein each of R₉, R₁₀ and R₁₁ is independently selected from the group consisting of hydrogen; hydroxy; C₁-C₆ alkyl; C₂-C₇ alkenyl; C₂-C₇ alkynyl; C₁-C₆ hydroxyalkyl; C₁-C₆ haloalkyl; C₁-C₆ alkoxy; C₁-C₆ haloalkoxy; halogen; C₁-C₆ alkylsulfide; C₁-C₆ alkylthioxo; hydroxamic acid; phenyl; cyano; nitro; amino; carboxylic acid group; amide; C₁-C₆ alkylamide; C₂-C₇ alkanoyl; aldehyde; C₃-C₈ ester; C₃-C₈ esteroxy; C₁-C₆ alkylsulfonyl; C₁-C₆ oximealkyl; C₁-C₆ aminoalkyl; C₃-C₈ alkylcarbonylalkyl; phosphoric acid group; and N-oxide,
provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen.

Preferably, in the above Formula I, L may form a carbonyl group (-C(=O)-), sulfonyl group (-S(=O)₂-) or thioxo group (-C(=S)-).

Preferably, in the above Formula I, each of R₅, R₆, R₇ and R₈ may be same or different and is independently selected from the group consisting of hydrogen; hydroxy; C₁-C₄ alkyl; C₁-C₄ haloalkyl; C₁-C₄ alkoxy; C₁-C₄ haloalkoxy; C₂-C₅ alkanoyl; halogen; phenyl; cyano; nitro; amino; and carboxylic acid group; or R₅ and R₆ together with a carbon atom to which they are attached may form a carbonyl group (C=O) or thioxo group (C=S), or R₇ and R₈ together with a carbon atom to which they are attached may form a carbonyl group (C=O) or thioxo group (C=S).

Preferably, in the above Formula I, Y is selected from the group consisting of saturated or unsaturated C₅-C₆ carbocyclyl substituted with R₉, R₁₀ and R₁₁; and saturated or unsaturated 5-to 6-membered heterocyclyl containing 1 to 3 heteroatoms (preferably, the heterocycle contains 1 to 3 heteroatoms selected from N, O and S) and being substituted with R₉, R₁₀ and R₁₁, wherein R₉, R₁₀ and R₁₁ are the same as defined in the above Formula I.

Preferably, in the above Formula I, each of R₉, R₁₀ and R₁₁ is independently selected from the group consisting of hydrogen; hydroxy; C₁-C₄ alkyl; C₁-C₄ haloalkyl; C₁-C₄ alkoxy; C₁-C₄ haloalkoxy; halogen; phenyl; cyano; nitro; amino; carboxylic acid group; amide; C₁-C₆ alkylamide; C₂-C₅ alkanoyl; aldehyde; C₃-C₇ ester, C₃-C₇ esteroxy; C₁-C₄ alkylsulfonyl; C₁-C₄ oximealkyl; C₁-C₄ aminoalkyl; C₃-C₇ alkylcarbonylalkyl; phosphoric acid group; and N-oxide, provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen.

Preferably, in the above Formula I, each of R₁-R₂, R₂-R₃ and R₃-R₄ pairs may be independently fused to form a saturated or unsaturated 5- to 6-membered carbocycle or heterocycle, wherein the heterocycle preferably contains 1 to 3 heteroatoms selected from N, O and S.

Preferably, in the above Formula I, each of R₁, R₂, R₃ and R₄ may be same or different and is independently selected from the group consisting of hydrogen; hydroxy; C₁-C₄ alkyl; C₁-C₄ haloalkyl; C₁-C₄ alkoxy; C₁-C₄ haloalkoxy; halogen; phenyl; cyano; nitro; amino; carboxylic acid group; C₂-C₅ alkanoyl; C₂-C₅ alkoxycarbonyl; C₂-C₅ alkanoyloxy; C₃-C₁₀ mono or bicycloalkyl; C₄-C₁₁ cycloalkylalkyl; C₆-C₁₀ aryl; , saturated or unsaturated C₃-C₁₀ mono or polycarbocyclyl; and , saturated or unsaturated 3- to 10-membered mono or polyheterocyclyl containing 1 to 3 heteroatoms (preferably, the heteroatom is selected from N, O and S), provided that when X₁ is nitrogen, R₂ does not exist; when X₂ is nitrogen, R₃ does not exist; and when X₃ is nitrogen, R₄ does not exist.

In the above Formula I, a preferable example of "C₃-C₁₂ mono or bicycloalkyl" can be a monocycloalkyl such as cyclopropyl, cyclobutyl, cyclopentyl, cyclohexyl, cycloheptyl, cyclooctyl and cyclononyl, or a bicycloalkyl obtained by the fusion of the same or different two of said monocycloalkyls, but not limited thereto.

In the above Formula I, a preferable example of "C₃-C₁₂ mono or polycarbocyclyl" can be a monocycloalkyl such as cyclopropyl, cyclobutyl, cyclopentyl, cyclohexyl, cycloheptyl, cyclooctyl and cyclononyl, or a polycycloalkyl obtained by the fusion of the same or different two or more of said monocycloalkyls, or carboaryl (for example, phenyl or naphthyl), but not limited thereto.

In the above Formula I, a preferable example of "saturated or unsaturated 3- to 12-membered mono or polyheterocyclyl containing 1 to 3 heteroatoms" can be thienyl, thiazolyl, imidazolyl, benzimidazolyl, triazolyl, tetrahydropyranyl, pyridinyl, furanyl, pyranyl, pyrrolyl, pyrazolyl, pyrazinyl, pyrimidinyl, isothiazolyl, isoxazolyl, pyridazinyl, isobenzopyranyl, chromenyl, indolyl, indazolyl, quinolinyl, purinyl, pyrrolinyl, chromanyl, pyrazolidinyl, piperidinyl, piperazinyl or the like, but not limited thereto.

In the above Formula I, a preferable example of "C₄-C₁₂ cycloalkylalkyl" can be cyclopropylmethyl, cyclobutylmethyl, cyclopentylmethyl, cyclohexylmethyl, cyclopentylethyl, cyclohexylethyl, cycloheptylmethyl, cycloheptylethyl, cyclooctylmethyl or the like, but not limited thereto.

Preferably, in the above Formula I, X₂ is carbon, and each of X₁ and X₃ is independently carbon or nitrogen, provided that at least one of X₁ and X₃ is nitrogen.

Preferably, in the above Formula I, each of R₁, R₂, R₃ and R₄ may be same or different and is independently selected from the group consisting of hydrogen; C₁-C₄ alkyl; C₁-C₄ haloalkyl; halogen; phenyl; cyano; and , saturated or unsaturated 3- to 10-membered mono or polyheterocyclyl containing 1 to 3 heteroatoms (preferably, the heteroatom is selected from N, O and S); or each of R₁-R₂, R₂-R₃ and R₃-R₄ pairs may be independently fused to form a saturated or unsaturated 5- to 6-membered carbocycle; provided that when X₁ is nitrogen, R₂ does not exist; when X₂ is nitrogen, R₃ does not exist; and when X₃ is nitrogen, R₄ does not exist.

Preferably, in the above Formula I, Y is an aromatic 5- to 6-membered carbocycle or heterocycle (preferably, the heterocycle contains 1 to 3 heteroatoms selected from N, O and S) substituted with R₉, R₁₀ and R₁₁, wherein R₉, R₁₀ and R₁₁ are the same as defined in the above Formula I.

Preferably, in the above Formula I, each of R₉, R₁₀, and R₁₁ is independently selected from the group consisting of hydrogen; hydroxy; C₁-C₄ alkyl; C₁-C₄ haloalkyl; C₁-C₄ alkoxy; C₁-C₄ haloalkoxy; halogen; nitro; carboxylic acid group; and C₃-C₇ esteroxy, provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen.

According to a preferred embodiment of the present invention, in the above Formula I,
each of X₁, X₂ and X₃ is independently carbon or nitrogen, provided that at least one of X₁, X₂ and X₃ is nitrogen,
each of R₁, R₂, R₃ and R₄ may be same or different and is independently selected from the group consisting of hydrogen; hydroxy; C₁-C₄ alkyl; C₁-C₄ haloalkyl; C₁-C₄ alkoxy; C₁-C₄ haloalkoxy; halogen; cyano; nitro; amino; carboxylic acid group; C₂-C₅ alkanoyl; C₂-C₅ alkoxycarbonyl; C₂-C₅ alkanoyloxy; C₆-C₁₂ aryl; and , saturated or unsaturated C₃-C₁₂ mono or polycarbocyclyl or heterocyclyl, provided that when X₁ is nitrogen, R₂ does not exist; when X₂ is nitrogen, R₃ does not exist; and when X₃ is nitrogen, R₄ does not exist, or each of R₁-R₂, R₂-R₃ and R₃-R₄ pairs may be independently fused to form a saturated or unsaturated 5-to 6-membered carbocycle or heterocycle,
each of R₅, R₆, R₇ and R₈ may be same or different and is independently selected from the group consisting of hydrogen; hydroxy; C₁-C₄ alkyl; C₁-C₄ haloalkyl; C₁-C₄ alkoxy; C₁-C₄ haloalkoxy; C₂-C₅ alkanoyl; halogen; cyano; nitro; amino; and carboxylic acid group, or R₅ and R₆ together with a carbon atom to which they are attached may form a carbonyl group (C=O), or R₇ and R₈ together with a carbon atom to which they are attached may form a carbonyl group (C=O),
L may form a carbonyl group (-C(=O)-), sulfonyl group (-S(=O)₂-) or thioxo group (-C(=S)-), and
Y is selected from the group consisting of saturated or unsaturated C₃-C₁₂ mono or
polycarbocyclyl substituted with R₉, R₁₀ and R₁₁; and saturated or unsaturated 3- to 12-membered mono or polyheterocyclyl containing 1 to 3 heteroatoms and being substituted with R₉, R₁₀ and R₁₁, wherein the heterocycle preferably contains 1 to 3 heteroatoms selected from N, O and S,
wherein each of R₉, R₁₀ and R₁₁ is independently selected from the group consisting of hydrogen; hydroxy; C₁-C₄ alkyl; C₁-C₄ haloalkyl; C₁-C₄ alkoxy; C₁-C₄ haloalkoxy; halogen; cyano; nitro; amino; carboxylic acid group; and C₃-C₇ esteroxy,
provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen.

According to another preferred embodiment of the present invention, in the above Formula I,
each of X₁, X₂ and X₃ is independently carbon or nitrogen, provided that at least one of X₁, X₂ and X₃ is nitrogen,
each of R₁, R₂, R₃ and R₄ may be same or different and is independently selected from the group consisting of hydrogen; C₁-C₄ alkyl; C₁-C₄ haloalkyl; C₁-C₄ alkoxy; halogen; cyano; nitro; amino; and , saturated or unsaturated C₅-C₆ carbocyclyl or heterocyclyl, provided that when X₁ is nitrogen, R₂ does not exist; when X₂ is nitrogen, R₃ does not exist; and when X₃ is nitrogen, R₄ does not exist, or each of R₁-R₂, R₂-R₃ and R₃-R₄ pairs may be independently fused to form a phenyl or a 6-membered heterocycle containing 1 to 2 atoms of nitrogen or oxygen,
each of R₅, R₆, R₇ and R₈ may be same or different and is independently selected from the group consisting of hydrogen; C₁-C₄ alkyl; C₁-C₄ alkoxy; halogen; cyano; nitro; and amino,
L is a carbonyl group (-C(=O)-) or thioxo group (-C(=S)-), and
Y is a phenyl which has a hydroxy group in para-position to the attachment position of L and is further substituted with 1 to 3 substituents independently selected from halogen and nitro.

As representative examples of the compound of Formula I according to the present inventon, the following compounds may be mentioned:
(3,5-dibromo-4-hydroxy-phenyl)-(2,3-dihydro-pyrido[2,3-b][1,4]oxazin-1-yl)-methanone (compound 1);
(3,5-dibromo-4-methoxy-phenyl)-(2,3-dihydro-pyrido[4,3-b][1,4]oxazin-4-yl)-methanone (compound 2);
(3,5-dibromo-4-hydroxy-phenyl)-(2,3-dihydro-pyrido[4,3-b][1,4]oxazin-4-yl)-methanone hydrobromic acid salt (compound 3);
(3,5-dibromo-4-hydroxy-phenyl)-(2,3-dihydro-pyrido[4,3-b][1,4]oxazin-4-yl)-methanone (compound 4);
(3,5-dibromo-4-methoxy-phenyl)-(2,3-dihydro-pyrido[3,4-b][1,4]oxazin-1-yl)-methanone (compound 5);
(3,5-dibromo-4-hydroxy-phenyl)-(2,3-dihydro-pyrido[3,4-b][1,4]oxazin-1-yl)-methanone (compound 6);
(3,5-dibromo-4-hydroxy-phenyl)-(6-methyl-2,3-dihydro-pyrido[2,3-b][1,4]oxazin-1-yl)-methanone (compound 7);
(3,5-dibromo-4-hydroxy-phenyl)-(2,2-dimethyl-2,3-dihydro-pyrido[4,3-b][1,4]oxazin-4-yl)-methanone (compound 8);
(3,5-dibromo-4-hydroxy-phenyl)-(7-cyclopropyl-2,3-dihydro-pyrido[2,3-b][1,4]oxazin-1-yl)-methanone (compound 9);
(3-chloro-4-hydroxy-phenyl)-(2,3-dihydro-pyrido[4,3-b][1,4]oxazin-4-yl)-methanone hydrobromic acid salt (compound 10);
(3-bromo-4-hydroxy-phenyl)-(2,3-dihydro-pyrido[4,3-b][1,4]oxazin-4-yl)-methanone hydrobromic acid salt (compound 11);
(2,3-dihydro-pyrido[4,3-b][1,4]oxazin-4-yl)-(4-hydroxy-3-trifluoromethyl-phenyl)-methanone (compound 12);
(3,5-dichloro-4-hydroxy-phenyl)-(2,3-dihydro-pyrido[4,3-b][1,4]oxazin-4-yl)-methanone hydrobromic acid salt (compound 13);
(3-chloro-4-hydroxy-5-nitro-phenyl)-(2,3-dihydro-pyrido[4,3-b][1,4]oxazin-4-yl)-methanone (compound 14);
(3,5-dichloro-4-hydroxy-phenyl)-(2,3-dihydro-pyrido[3,4-b][1,4]oxazin-1-yl)-methanone hydrobromic acid salt (compound 15);
(3-bromo-4-hydroxy-phenyl)-(2,3-dihydro-pyrido[3,4-b][1,4]oxazin-1-yl)-methanone hydrobromic acid salt (compound 16);
(3-chloro-4-hydroxy-5-nitro-phenyl)-(2,3-dihydro-pyrido[3,4-b][1,4]oxazin-1-yl)-methanone hydrobromic acid salt (compound 17);
(3-chloro-4-hydroxy-phenyl)-(2,3-dihydro-pyrido[3,4-b][1,4]oxazin-1-yl)-methanone hydrobromic acid salt (compound 18);
(2,3-dihydro-pyrido[3,4-b][1,4]oxazin-1-yl)-(4-hydroxy-3-trifluoromethyl-phenyl)-methanone (compound 19);
(3,5-dibromo-4-hydroxy-phenyl)-(7-phenyl-2,3-dihydro-pyrido[2,3-b][1,4]oxazin-1-yl)-methanone (compound 20);
2,6-dichloro-4-(2,3-dihydro-pyrido[4,3-b][1,4]oxazin-4-sulfonyl)-phenol (compound 21);
(3,5-dibromo-4-methoxy-phenyl)-(7-trifluoromethyl-2,3-dihydro-pyrido[2,3-b][1,4]oxazin-1-yl)-methanone (compound 22-1);
(3,5-dibromo-4-hydroxy-phenyl)-(7-trifluoromethyl-2,3-dihydro-pyrido[2,3-b][1,4]oxazin-1-yl)-methanone (compound 22-2);
2,5-dibromo-4-(2,3-dihydro-pyrido[4,3-b][1,4]oxazin-4-carbonyl)-benzoic acid (compound 23);
[2,6-dibromo-4-(2,3-dihydro-pyrido[4,3-b][1,4]oxazin-4-carbonyl)-phenoxy]-acetic acid methyl ester (compound 24);
(7-bromo-2,3-dihydro-pyrido[2,3-b][1,4]oxazin-1-yl)-(3,5-dibromo-4-hydroxy-phenyl)-methanone (compound 25);
(2,3-dihydro-pyrido[4,3-b][1,4]oxazin-4-yl)-(3-fluoro-4-hydroxy-phenyl)-methanone (compound 26);
(3,5-dibromo-4-methoxy-phenyl)-(7-methyl-2,3-dihydro-pyrido[2,3-b][1,4]oxazin-1-yl)-methanone (compound 27-1);
(3,5-dibromo-4-hydroxy-phenyl)-(7-methyl-2,3-dihydro-pyrido[2,3-b][1,4]oxazin-1-yl)-methanone (compound 27-2);
(3,5-difluoro-4-methoxy-phenyl)-(2,3-dihydro-pyrido[4,3-b][1,4]oxazin-4-yl)-methanone (compound 28);
(3,5-difluoro-4-hydroxy-phenyl)-(2,3-dihydro-pyrido[4,3-b][1,4]oxazin-4-yl)-methanone (compound 29);
(5-chloro-2,3-dihydro-pyrido[4,3-b][1,4]oxazin-4-yl)-(3,5-dibromo-4-hydroxy-phenyl)-methanone (compound 30);
(2,6-dichloro-pyridin-4-yl)-(2,3-dihydro-pyrido[4,3-b][1,4]oxazin-4-yl)-methanone (compound 31);
(2,3-dihydro-pyrido[4,3-b][1,4]oxazin-4-yl)-(6-hydroxy-pyridin-3-yl)-methanone (compound 32);
(3,5-dibromo-4-hydroxy-phenyl)-(2,3-dihydro-pyrido[4,3-b][1,4]oxazin-4-yl)-methanone hydrochloric acid salt (compound 33);
(3-chloro-4-hydroxy-phenyl)-(2,3-dihydro-pyrido[3,4-b][1,4]oxazin-1-yl)-methanone (compound 34);
4-(2,3-dihydro-pyrido[4,3-b][1,4]oxazin-4-sulfonyl)-phenol (compound 35-1);
2,6-dibromo-4-(2,3-dihydro-pyrido[4,3-b][1,4]oxazin-4-sulfonyl)-phenol (compound 35-2);
(3-chloro-4-hydroxy-5-nitro-phenyl)-(2,3-dihydro-pyrido[3,4-b][1,4]oxazin-1-yl)-methanone (compound 36);
(3-chloro-4-hydroxy-phenyl)-(2,3-dihydro-pyrido[4,3-b][1,4]oxazin-4-yl)-methanone (compound 37);
(3-bromo-4-hydroxy-phenyl)-(2,3-dihydro-pyrido[4,3-b][1,4]oxazin-4-yl)-methanone (compound 38);
(3,5-dichloro-4-hydroxy-phenyl)-(2,3-dihydro-pyrido[4,3-b][1,4]oxazin-4-yl)-methanone (compound 39);
(3-bromo-4-hydroxy-phenyl)-(2,3-dihydro-pyrido[3,4-b][1,4]oxazin-1-yl)-methanone (compound 40);
(3,5-dichloro-4-hydroxy-phenyl)-(2,3-dihydro-pyrido[3,4-b][1,4]oxazin-1-yl)-methanone (compound 41);
2-(3,5-dibromo-4-hydroxy-phenyl)-1-(2,3-dihydro-pyrido[4,3-b][1,4]oxazin-4-yl)-ethanone (compound 42);
(2,3-dihydro-pyrido[4,3-b][1,4]oxazin-4-yl)-(3-methoxy-isoxazol-5-yl)-methanone (compound 43);
(2,3-dihydro-pyrido[4,3-b][1,4]oxazin-4-yl)-(3-hydroxy-isoxazol-5-yl)-methanone (compound 44);
(3,5-dibromo-4-hydroxy-phenyl)-[7-(4-trifluoromethyl-phenyl)-2,3-dihydro-pyrido[2,3-b][1,4]oxazin-1-yl]-methanone (compound 45);
(3,5-dibromo-4-hydroxy-phenyl)-[7-(2-trifluoromethyl-phenyl)-2,3-dihydro-pyrido[2,3-b][1,4]oxazin-1-yl]-methanone (compound 46);
1-(3,5-dibromo-4-methoxy-benzoyl)-2,3-dihydro-1H-pyrido[2,3-b][1,4]oxazin-7-carbonitrile (compound 47-1);
1-(3,5-dibromo-4-hydroxy-benzoyl)-2,3-dihydro-1H-pyrido[2,3-b][1,4]oxazin-7-carbonitrile (compound 47-2);
(3,5-dibromo-4-methoxy-phenyl)-[7-(3-nitro-phenyl)-2,3-dihydro-pyrido[2,3-b][1,4]oxazin-1-yl]-methanone (compound 48);
(3,5-dibromo-4-methoxy-phenyl)-[7-(3-nitro-phenyl)-2,3-dihydro-pyrido[2,3-b][1,4]oxazin-1-yl]-methanone (compound 49);
(3,5-dibromo-4-hydroxy-phenyl)-[7-(3-dimethylamino-phenyl)-2,3-dihydro-pyrido[2,3-b][1,4]oxazin-1-yl]-methanone (compound 50);
(3,5-dibromo-4-hydroxy-phenyl)-(2,3-dihydro-pyrido[4,3-b][1,4]oxazin-4-yl)-methanethione (compound 51);
(3,5-dibromo-4-hydroxy-phenyl)-(7-pyridin-3-yl-2,3-dihydro-pyrido[2,3-b][1,4]oxazin-1-yl]-methanone (compound 52);
(3,5-dibromo-4-hydroxy-phenyl)-(7-furan-3-yl-2,3-dihydro-pyrido[2,3-b][1,4]oxazin-1-yl]-methanone (compound 53);
1-(3,5-dibromo-4-hydroxy-phenyl)-2-(2,3-dihydro-pyrido[4,3-b][1,4]oxazin-4-yl)-ethanone (compound 54);
(3,5-dibromo-4-hydroxy-phenyl)-(2,3-dihydro-4-oxa-1,9-diaza-phenanthren-1-yl)-methanone (compound 55);
4-[2-(3,5-dibromo-4-hydroxy-phenyl)-2-oxo-ethyl)]-4H-pyrido[4,3-b][1,4]oxazin-3-one (compound 56);
4-(3,5-dibromo-4-methoxy-benzoyl)-4H-pyrido[4,3-b][1,4]oxazin-3-one (compound 57);
(3,5-dibromo-4-methoxy-phenyl)-(6-methyl-2,3-dihydro-pyrido[2,3-b][1,4]oxazin-1-yl)-methanone (compound 58);
(2,3-dihydro-pyrido[4,3-b][1,4]oxazin-4-yl)-(2,4-dihydroxy-pyrimidin-5-yl)-methanone (compound 59);
(2,3-dihydro-pyrido[4,3-b][1,4]oxazin-4-yl)-(2,6-dihydroxy-pyrimidin-4-yl)-methanone (compound 60);
(3,5-dibromo-4-hydroxy-phenyl)-(7-isoquinolin-4-yl-2,3-dihydro-pyrido[2,3-b][1,4]oxazin-1-yl)-methanone (compound 61);
(3,5-dibromo-4-hydroxy-phenyl)-(6,7-dihydro-pyrimido[4,5-b][1,4]oxazin-5-yl)-methanone (compound 62);
(3,5-dibromo-4-hydroxy-phenyl)-[7-(3-trifluoromethyl-phenyl)-2,3-dihydro-pyrido[2,3-b][1,4]oxazin-1-yl]-methanone (compound 63);
(3,5-dibromo-4-hydroxy-phenyl)-[7-(3-fluoromethyl-phenyl)-2,3-dihydro-pyrido[2,3-b][1,4]oxazin-1-yl]-methanone (compound 64);
4-[1-(3,5-dibromo-4-hydroxy-benzoyl)-2,3-dihydro-1H-pyrido[2,3-b][1,4]oxazin-7-yl]-benzonitrile (compound 65);
(3,5-dibromo-4-hydroxy-phenyl)-[7-(4-trifluoromethoxy-phenyl)-2,3-dihydro-pyrido[2,3-b][1,4]oxazin-1-yl]-methanone (compound 66);
1-{4-[1-(3,5-dibromo-4-hydroxy-benzoyl)-2,3-dihydro-1H-pyrido[2,3-b][1,4]oxazin-7-yl]-phenyl}-ethanone (compound 67);
(3,5-dibromo-4-hydroxy-phenyl)-[7-(5-methoxy-pyridin-3-yl)-2,3-dihydro-pyrido[2,3-b][1,4]oxazin-1-yl]-methanone (compound 68);
(4-hydroxy-3-trifluoromethyl-phenyl)-(7-methyl-2,3-dihydro-pyrido[2,3-b][1,4]oxazin-1-yl)-methanone (compound 69);
(3,5-dibromo-4-hydroxy-phenyl)-[7-(1H-indol-4-yl)-2,3-dihydro-pyrido[2,3-b][1,4]oxazin-1-yl]-methanone (compound 70);
(3,5-dibromo-4-hydroxy-phenyl)-(2,3-dihydro-pyrido[4,3-b][1,4]oxazin-4-yl)-methanone sulfuric acid salt (compound 71);
(2,6-dibromo-4-(2,3-dihydro-pyrido[4,3-b][1,4]oxazin-4-carbonyl)-phenolate sodium salt (compound 72);
(2,6-dibromo-4-{2,3-dihydro-pyrido[4,3-b][1,4]oxazin-4-carbonyl)-phenolate potassium salt (compound 73);
(3,5-dibromo-4-hydroxy-phenyl)-(2,3-dihydro-pyrido[4,3-b][1,4]oxazin-4-yl)-methanethione trifluoroacetic acid salt (compound 74); and
1-[1-(3,5-dibromo-4-hydroxy-benzoyl)-2,3-dihydro-1H-pyrido[2,3-b][1,4]oxazin-7-yl]-pyrrolidin-2-one (compound 75).

The names of the compounds described in the above are in accordance with the nomenclature method (AutoNom version 2.1) using the structures provided by CS ChemDraw Ultra software of CambridgeSoft.

As used herein, the term "" refers to unsubstituted condition, or substituted condition with one or more of proper substituents, for example, such as hydroxy; oxo (=O); C₁-C₆ alkyl or haloalkyl; C₁-C₆ alkoxy or haloalkoxy; C₁-C₆ alkanoyl or haloalkanoyl; halogen; cyano; nitro; amino; mono- or di-(C₁-C₆)alkylamino; carboxylic acid group; C₂-C₇ alkenyl; C₂-C₇ alkynyl; C₁-C₆ hydroxyalkyl; phenyl; phosphoric acid group; N-oxide; amide; C₁-C₆ alkylamide; aldehyde; hydroxamic acid; C₁-C₆ alkylsulfide; C₁-C₆ alkylthioxo; C₁-C₆ alkylsulfonyl; C₁-C₆ oximealkyl; C₁-C₆ aminoalkyl; C₃-C₈ alkylcarbonylalkyl; C₂-C₇ alkoxycarbonyl; C₂-C₇ alkanoyloxy or the like.

Unless mentioned otherwise, alkyl substituent as referred herein and alkyl residue in other substituents (for example, alkoxy) as referred herein may be linear or branched. Also, halogen includes fluorine (F), chlorine (C1), bromine (Br) and iodine (I).

The compound of Formula I according to the present invention may be a racemate. The racemate can be separated into each isomer by conventional separation methods, for example, a method using a general column chromatography filled with normal phase silica gel (Merk, 0.040-0.063 mm and 0.063-0.200 mm), a general column chromatography filled with amine silica gel (chromatorex, 100~200 mesh) or a column chromatography for pressurized fractionation filled in reverse phase (Young-rin, SDV 30 plus) with corresponding solvent, preferably mixed solvent of hexane, ethyl acetate, dichloromethane and methanol for normal phase and mixture of water and acetonitrile for reverse phase.

The compound of Formula I according to the present invention may also form pharmaceutically acceptable salts. The pharmaceutically acceptable salts includes acid addition salts prepared by acids forming non-toxic acid addition salts containing pharmaceutically acceptable anion, for example, inorganic acids such as hydrochloric acid, sulfuric acid, nitric acid, phosphoric acid, hydrobromic acid, hydroiodic acid, etc.; organic carbon acids such as tartaric acid, formic acid, citric acid, acetic acid, trichloroacetic acid or trifluoroacetic acid, gluconic acid, benzoic acid, lactic acid, fumaric acid, maleic acid, etc.; and sulfonic acids such as methanesulfonic acid, benzenesulfonic acid, p-toluenesulfonic acid or naphthalsulfonic acid. Salts with alkali metal such as sodium, potassium or the like are also included. In addition, salts with other acids or bases which are conventionally used in a field of art relating to aromatic amidine derivative or lactam derivative, may be included. They are prepared according to conventionally known processes.

The compound of the present invention is useful as an inhibitor, specifically a selective inhibitor, of uric acid reuptake through human urate anion transporter 1 (hURAT1).

The compounds of the present invention having the above Formula I structure can be prepared according to the methods described below. Therefore, it is another object of the present invention to provide the methods for preparing the Formula I compound.

More concretely, the compound of Formula I can be obtained by each of the Preparation Methods 1 to 5 explained below, but not limited thereto.

### Preparation Method 1

The following Formula II compound which is a compound of Formula I wherein L is -C(=O)-, can be prepared by a method comprising the steps of: reducing Formula VII compound to obtain Formula VI compound; cyclizing the obtained Formula VI compound with Formula V compound to obtain Formula IV compound; and peptide-bonding the obtained Formula IV compound with Formula III compound.

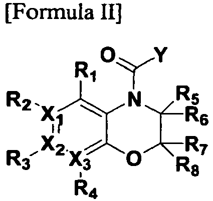

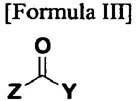

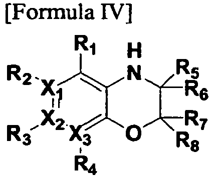

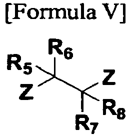

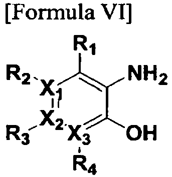

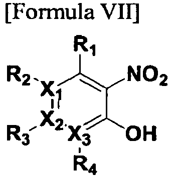

In Formulae II to VII above, X₁, X₂, X₃, R₁, R₂, R₃, R₄, R₅, R₆, R₇, R₈ and Y are the same as mentioned Formula I above, and Z represents a reactive leaving group, preferably hydroxy or halogen.

Preparation Method 1 is explained in detail below.

Compounds of Formulae VI and VII used as starting materials can be prepared according to the method described in JOC. 60, 1995, 5721-5725, or are commercially available from chemical reagent companies such as Sigma Aldrich, Merck, etc.

The reduction of Formula VII compound to obtain Formula VI compound is carried out according to known synthetic methods in the presence of suitable solvent, reduction catalyst and hydrogen gas. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, N,N-dimethyl formamide, N,N-dimethyl acetamide, dimethyl sulfoxide, acetonitrile, etc. A mixture solvent of two or more of solvents as described above also can be used. Methanol, ethyl acetate and tetrahydrofuran are particularly preferable as the solvent for this reaction. The reducing agents used in this reaction include general reduction catalysts such as palladium on active carbon (5w/w%), palladium on active carbon (10w/w%), palladium hydroxide on active carbon (10w/w%), and Raney-nickel, etc. Palladium on active carbon (10w/w%) is preferable for this reaction. The reaction can be carried out for the time between 1 hour and 24 hours, preferably for about 18 hours, at the temperature between 0°C and room temperature, preferably at room temperature, under hydrogen pressure between atmospheric pressure and 3 atm, preferably under atmospheric hydrogen pressure, but not limited thereto.

In the next step, the cyclization reaction of the Formula VI compound obtained according to the above method is carried out by known synthetic methods in the presence of suitable solvent, Formula V compound and base. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, N,N-dimethyl formamide, N,N-dimethyl acetamide, dimethyl sulfoxide, acetonitrile, etc. A mixture solvent of two or more of solvents as described above also can be used. Acetonitrile and N,N-dimethyl formamide are particularly preferable as the solvent for this reaction. Examples of the base used in the reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; and alkali metal hydrogen carbonates such as sodium hydrogen carbonate, potassium hydrogen carbonate, etc. Potassium carbonate is preferable for this reaction. The cyclization reaction is carried out for the time between 1 hour and 24 hours, preferably for about 18 hours, at room temperature or reflux condition depending on the used solvent, preferably between room temperature and 150°C. More preferably 100°C condition in N,N-dimethyl formamide solvent.

Also Formula IV compounds can be prepared from another intermediate depending on the structure of Formula V compound. For example, in the case of ketone structure wherein R₇ and R₈ form carbonyl(-C(=O)-), Formula IV compounds can be prepared by a reduction reaction using proper solvent and reducing agent. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, N,N-dimethyl formamide, N,N-dimethyl acetamide, dimethyl sulfoxide, acetonitrile, etc. A mixture solvent of two or more of solvents as described above also can be used. Tetrahydrofuran, dichloromethane, dimethyl ether and toluene are particularly preferable as the solvent for this reaction. Examples of the reducing agent include lithium aluminum hydride, sodium borohydride, diborane, diisobutyl aluminum hydride, borane-tetrahydrofuran complex, sodium bis(2-methoxyethoxy)aluminum hydride (Red-Al), tetra-n-butyl ammonium borohydride, etc. Preferably, lithium aluminum hydride or tetra-n-butyl ammonium borohydride is used. The reaction starts at 0°C, and is further carried out at the temperature between 0°C and 150°C, preferably between room temperature and 50°C, for the time between 10 minutes and 10 hours, preferably for about 2 hours to obtain a compound of Formula IV wherein R₇ and R₈ are reduced.

Also, Formula IV compounds, which are substituted by R₁, R₂, R₃ and R₄, can be prepared from the coupling reaction of commercially available arylboronic acid or vinylboronic acid from chemical reagent companies such as Sigma Aldrich, Merck, etc. in the presence of metal catalyst. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, N,N-dimethyl formamide, N,N-dimethyl acetamide, dimethyl sulfoxide, acetonitrile, etc. A mixture solvent of two or more of solvents as described above also can be used. A mixture of acetonitrile and water is particularly preferable as the solvent for this reaction. Examples of the metal catalyst used in this reaction include palladium acetate, tetrakis(triphenylphosphine) palladium, tris(dibenzylidene acetone) dipalladium, palladium dichloride, (1,1-bis(diphenylphophino)ferrocene) palladium dichloride, bis(triphenylphosphine) palladium chloride, etc. Palladium acetate is particularly preferable for this reaction. Examples of the base for used in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; alkali metal hydrogen carbonates such as sodium hydrogen carbonate, potassium hydrogen carbonate, etc.; and potassium phosphate. Potassium carbonate and potassium phosphate are preferable for this reaction. The reaction can be further carried out at the temperature between room temperature and 180°C, preferably between room temperature and 100°C, for the time between 1 hour and 24 hours, preferably for about 18 hours to obtain another compound within Formula IV wherein the substituent as mentioned above is introduced in R₁, R₂, R₃, R₄.

In the next step, Formula II compound is obtained by the condensation reaction of the Formula IV compound with the intermediate formed from Formula III compound using general halogenating agent, preferably oxalyl chloride or thionyl chloride thereto or by the peptide bond formation of the Formula IV compound with Formula III compound using peptide coupling reagents such as HATU, HBTU, BOP, PyBOP, EDC hydrochloric acid salt, TBTU, HOBt, DEPBT, CDI, etc. in a solvent such as N,N-dimethyl acetamide, dichloromethane, tetrahydrofuran. In the reaction with the reaction product formed from Formula III compound by adding general halogenating agent thereto, organic base can be selected according to Formula III compound and in this case, the reaction is carried out under basic condition by using triethylamine preferably. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, N,N-dimethyl formamide, N,N-dimethyl acetamide, dimethyl sulfoxide, acetonitrile, etc. A mixture solvent of two or more of solvents as described above also can be used. Dichloromethane and N,N-dimethyl acetamide are particularly preferable as the solvent for this reaction. Examples of the base used in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, etc.; and alkali metal hydrogen carbonates such as sodium hydrogen carbonate, potassium hydrogen carbonate, etc. Triethylamine is preferable for this reaction. The reaction under basic condition is carried out for the time between 10 minutes and 10 hours, preferably for about 2 hours, at the temperature between 0°C and 60°C, preferably at room temperature. The reaction without basic condition is carried out for the time between 1 hour and 24 hours, preferably for about 18 hours. The reaction starts at 0°C and is further carried out at the temperature between room temperature and 60°C, preferably at room temperature.

The preparation method of Formula II compound as mentioned above will be explained in detail in the Examples.

### Preparation Method 2

The Formula II compound also can be prepared by a method comprising the steps of: halogenating Formula VII compound to obtain Formula X compound and then reacting the obtained Formula X compound with Formula IX compound to obtain Formula VIII compound, or carring out a Mitsunobu reaction of Formula VII compound and Formula IX compound to obtain Formula VIII compound; cyclizing the obtained Formula VIII compound to obtain Formula IV compound; and carring out peptide coupling reaction of the obtained Formula IV compound with Formula III compound.

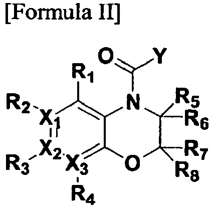

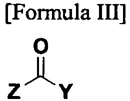

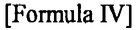

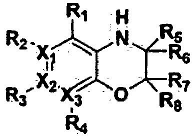

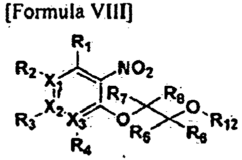

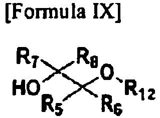

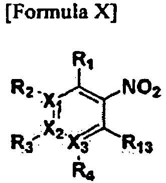

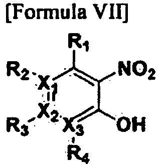

In Formulae II to X above, X₁, X₂, X₃, R₁, R₂, R₃, R₄, R₅, R₆, R₇, R₈ and Y are the same as defined as mentioned Formula I above; R₁₂ is a non-hydrogen substituent, preferably C₁-C₄ alkyl, more preferably methyl or ethyl; and R₁₃ represents a leaving group, preferably halogen.

Preparation Method 2 is explained in detail below.

Compounds of Formulae VII and IX used as starting materials are commercially available from chemical reagent companies such as Sigma Aldrich, Merck, etc.

The reaction of halogenating Formula VII compound to obtain Formula X compound is carried out according to known synthetic methods in the presence of suitable solvent, and halogenating agent. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, N,N-dimethyl formamide, N,N-dimethyl acetamide, dimethyl sulfoxide, acetonitrile, etc. A mixture solvent of two or more of solvents as described above also can be used. Acetonitrile is particularly preferable as the solvent for this reaction. Examples of the halogenating agent used in this reaction include general halogenating agents such as phosphorus oxychloride, phosphorus oxybromide, phosphorus pentachloride or phosphorus pentabromide, etc. Phosphorus oxychloride is preferable for this reaction. The reaction can be carried out for the time between about 2 hours and about 6 hours under heating condition at the temperature between 80°C and 100°C, but not limited thereto.

In the next step, the Formula X compound is carried out a substitution reaction with Formula IX compound according to known synthetic methods in the presence of suitable solvent and base. The solvent is not particularly limited but examples thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Tetrahydrofuran and diethyl ether are particularly preferable as the solvent for this reaction. Examples of the base used in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; and alkali metal hydrogen carbonates such as sodium hydrogen carbonate, potassium hydrogen carbonate, etc. Sodium hydride is preferable for this reaction. The substitution reaction is carried out for the time between about 1 hour and about 6 hours at room temperature, preferably for about 1 hour at room temperature, to obtain Formula VIII compound substituted with Formula IX compound.

In a different manner, the Formula VIII compound can be prepared by carring out a generally-known Mitsunobu condensation reaction between Formula VII compound and Formula IX compound, not undergoing Formula X compound. As the condensation reagent, triphenylphosphine and diethyl azodicarboxylate (DEAD) can be used conventionally, but not limited thereto. Reagents modified from them also can be used. The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Tetrahydrofuran and diethyl ether are particularly preferable as the solvent for this reaction. The condensation reaction is carried out for the time between about 6 hours and about 18 hours at 0°C or room temperature, preferably for about 6 hours at room temperature.

In the next step, for the Formula VIII compound obtained according to the above method, a cyclization reaction is carried out according to known synthetic methods in the presence of suitable solvent, nitro group reducing agent and acid. The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Acidic solution of concentrated hydrochloric acid, acetic acid or other acids, aqueous ammonium solution, methanol and toluene are particularly preferable as the solvent for this reaction. Examples of the reducing agent include general reduction catalysts such as tin tetrachloride, iron, zinc, palladium on active carbon (5w/w%), palladium on active carbon (10w/w%), palladium hydroxide on active carbon (10w/w%), Raney-nickel, etc. Tin tetrachloride is particularly preferable for this reaction. The cyclization reaction is carried out for the time between about 1 hour and about 6 hours under heating condition of the temperature between 80°C and 100°C, preferably for about 1 hour under heating condition of 80°C, to obtain the cyclized Formula IV compound.

From the obtained Formula IV compound, another compound within Formula IV can be prepared by carring our a proper reaction, for example, a reduction reaction using proper solvent and reducing agent in case of ketone structure wherein R₇ and R₈, together with a carbon atom to which they are attached, form carbonyl(-C(=O)-). The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Tetrahydrofuran, dichloromethane, dimethyl ether and toluene are particularly preferable as the solvent for this reaction. Examples of the reducing agent include lithium aluminum hydride, sodium borohydride, diborane, diisobutyl aluminum hydride, borane-tetrahydrofuran complex, sodium bis(2-methoxyethoxy)aluminum hydride (Red-Al), tetra-n-butyl ammonium borohydride, etc. Preferably, lithium aluminum hydride or tetra-n-butyl ammonium borohydride is used. The reaction starts at 0°C, and is further carried out at the temperature between 0°C and 150°C, preferably between room temperature and 50°C, for the time between 10 minutes and 10 hours, preferably for about 2 hours to obtain a compound of formula IV wherein R₇ and R₈ are reduced.

If needed, Formula IV compounds, which are substituted by R₁, R₂, R₃ and R₄, can be prepared from the coupling reaction of commercially available arylboronic acid or vinylboronic acid from chemical reagent companies such as Sigma Aldrich, Merck, etc. in the presence of metal catalyst. The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. A mixture of acetonitrile and water is particularly preferable as the solvent for this reaction. Examples of the metal catalyst used in this reaction include palladium acetate, tetrakis(triphenylphosphine) palladium, tris(dibenzylidene acetone) dipalladium, palladium dichloride, (1,1-bis(diphenylphophino)ferrocene) palladium dichloride, bis(triphenylphosphine) palladium chloride, etc. Palladium acetate is particularly preferable for this reaction. Examples of the base used in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; alkali metal hydrogen carbonates such as sodium hydrogen carbonate, potassium hydrogen carbonate, etc.; and potassium phosphate. Potassium carbonate and potassium phosphate are preferable for this reaction. The reaction can be further carried out at the temperature between room temperature and 180°C, preferably between room temperature and 100°C, for the time between 1 hour and 24 hours, preferably for about 18 hours to obtain another compound within Formula IV wherein the substituent as mentioned above is introduced in R₁, R₂, R₃, R₄.

In the next step, Formula II compound is obtained by the condensation reaction of the Formula IV compound with the intermediate formed from Formula III compound using general halogenating agent, preferably oxalyl chloride or thionyl chloride thereto or by the peptide bond formation of the Formula IV compound with Formula III compound using peptide coupling reagents such as HATU, HBTU, BOP, PyBOP, EDC hydrochloric acid salt, TBTU, HOBt, DEPBT, CDI, etc. in a solvent such as N,N-dimethyl acetamide, dichloromethane, tetrahydrofuran. In the reaction with the reaction product formed from Formula III compound by adding general halogenating agent thereto, organic base can be selected according to Formula III compound and in this case, the reaction is carried out under basic condition by using triethylamine preferably. The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Dichloromethane and N,N-dimethyl acetamide are particularly preferable as the solvent for this reaction. Examples of the base usde in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; and alkali metal hydrogen carbonates such as sodium hydrogen carbonate, potassium hydrogen carbonate, etc. Triethylamine is preferable for this reaction. The reaction under basic condition is carried out for the time between 10 minutes and 10 hours, preferably for about 2 hours, at the temperature between 0°C and 60°C, preferably at room temperature. The reaction without basic condition is carried out for the time between 1 hour and 24 hours, preferably for about 18 hours. The reaction starts at 0°C and is further conducted at the temperature between room temperature and 60°C, preferably at room temperature.

The preparation method of Formula II compound as mentioned above will be explained in detail in the Examples below.

### Preparation Method 3

The following Formula XI compound which is a compound of Formula I wherein L is -C(=S)-, can be prepared from Formula II compound by reacting with Lawesson's reagent.

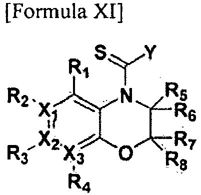

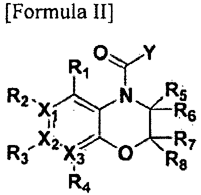

In Formulae II and XI above, X₁, X₂, X₃, R₁, R₂, R₃, R₄, R₅, R₆, R₇, R₈ and Y are the same as defined as mentioned Formula I above.

Preparation Method 3 is explained in detail below.

Compound of Formula II used as starting material can be prepared by the same method as described above, and the obtained Formula II compound can be reacted in a solvent with commercially available Lawesson's reagent from Sigma Aldrich, Merck, etc. to prepare Formula XI compound.

The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Tetrahydrofuran and toluene are particularly preferable as the solvent for this reaction. The reaction is carried out, for example, for the time between 1 hour and 24 hours at the temperature between 0°C and 150°C, preferably for about 18 hours at about 120°C, to obtain Formula XI compound.

The preparation method of Formula XI compound as mentioned above will be explained in detail in the Examples.

### Preparation Method 4

The following Formula XII compound which is a compound of Formula I wherein L is -S(=O)₂-, can be prepared from sulfonamide formation reaction of Formula IV compound with Formula XIII compound in the presence of base.

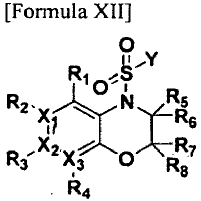

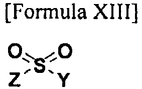

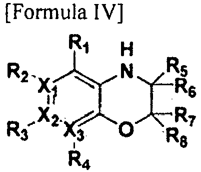

In Formulae IV, XII and XIII above, X₁, X₂, X₃, R₁, R₂, R₃, R₄, R₅, R₆, R₇, R₈ and Y are the same as mentioned Formula I above; and Z represents a leaving group, preferably halogen.

Preparation Method 4 is explained in detail below.

Compound of Formula IV used as starting material can be prepared by the same method as described above, and the obtained Formula IV compound can be used in sulfonamide formation reaction with commercially available Formula XIII compound from Sigma Aldrich, Merck, etc. in a solvent in the presence of base, to prepare Formula XII compound.

The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Dichloromethane and tetrahydrofuran are particularly preferable as the solvent for this reaction. Examples of the base used in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; and alkali metal hydrogen carbonates such as sodium hydrogen carbonate, potassium hydrogen carbonate, etc. Triethylamine is preferable for this reaction. The sulfonamide formation reaction is carried out for the time between about 10 minutes and about 8 hours at the temperature between 0°C and 60°C, preferably for about 2 hours at room temperature, to obtain Formula XII compound.

The preparation method of Formula XII compound as mentioned above will be explained in detail in the Examples.

### Preparation Method 5

The following Formula XIV compound which is a compound of Formula I wherein L is alkylcarbonyl or carbonylalkyl, can be prepared from Formula IV compound by alkylating Formula with Formula XV compound in the presence of base.

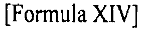

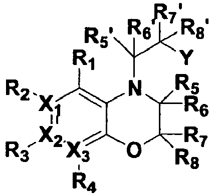

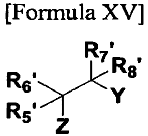

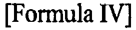

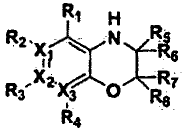

In Formulae IV to XV above, X₁, X₂, X₃, R₁, R₂, R₃, R₄, R₅, R₆, R₇, R₈ and Y are the same as mentioned Formula I above; Z represents a leaving group, preferably halogen; and each of R₅', R₆, R₇ and R₈' may be same or different and is independently selected from the group consisting of hydrogen; hydroxy; C₁-C₆ alkyl; C₁-C₆ haloalkyl; C₁-C₆ alkoxy; C₁-C₆ haloalkoxy; C₂-C₇ alkanoyl; halogen; phenyl; cyano; nitro; amino; and carboxylic acid group; or R₅ and R₆ together with a carbon atom to which they are attached may form a carbonyl group (C=O) or thioxo group (C=S), or R₇' and R₈' together with a carbon atom to which they are attached may form a carbonyl group (C=O) or thioxo group (C=S).

Preparation Method 5 is explained in detail below.

Compound of Formula IV used as starting material can be prepared by the same method as described above, and the obtained Formula IV compound can be subjected to an alkylation reaction in a solvent in the presence of base with Formula XV compound which is prepared by known synthetic methods or purchased from Sigma Aldrich, Merck, etc. to prepare Formula XIV compound.

The solvent is not particularly limited but example thereof preferably includes ether solvents such as diethyl ether, tetrahydrofuran, dioxane, 1,2-dimethoxyethane, diglyme, etc.; hydrocarbon solvents such as benzene, toluene, hexane, xylene, etc.; halogenated hydrocarbon solvents such as dichloromethane, chloroform, carbon tetrachloride, 1.2-dichloroethane, etc.; alcohol solvents such as methanol, ethanol, isopropyl alcohol, tert-butyl alcohol, etc.; ester solvents such as ethyl acetate, methyl acetate, butyl acetate, etc.; and polar solvents such as acetone, acetonitrile, N,N-dimethyl formamide, N,N-dimethyl acetamide, dimethyl sulfoxide, etc. A mixture solvent of two or more of solvents as described above also can be used. Dichloromethane and tetrahydrofuran are particularly preferable as the solvent for this reaction. Examples of the base used in this reaction include organic bases such as triethylamine, pyridine, 4-methylaminopyridine, 4-methylmorpholine, piperazine, N-methylpiperazine, etc.; alkali metal hydroxides such as lithium hydroxide, sodium hydroxide, potassium hydroxide, calcium hydroxide, etc.; alkali metal hydrides such as sodium hydride, potassium hydride, etc.; alkali metal carbonates such as sodium carbonate, potassium carbonate, cesium carbonate, etc.; and alkali metal hydrogen carbonates such as sodium hydrogen carbonate, potassium hydrogen carbonate, etc. Triethylamine is preferable for this reaction. The alkylation reaction is carried out for the time between about 10 minutes and about 8 hours at the temperature between 0°C and 60°C, preferably for about 2 hours at room temperature, to obtain Formula XIV compound.

The preparation method of Formula XIV compound as mentioned above will be explained in detail in the Examples.

Formula I compound of the invention as explained above has a strong inhibitory activity on human urate anion transporter 1 (hURAT1). The pharmaceutical composition containing the Formula I compound or racemate, stereochemical isomer or pharmaceutically acceptable salt thereof as an active ingredient may be prepared using a suspension in combination with pharmaceutically acceptable carrier, excipient, receptor, binder, stabilizer, and other additives generally used in formulation.

Also, when the pharmaceutical composition of the invention is prepared in an injection form, a pharmaceutically acceptable buffer, dissolution aid, and/or isotoner can be mixed with the Formula I compound of the invention or racemate, stereochemical isomer or pharmaceutically acceptable salt thereof.

The pharmaceutical composition of the invention has a strong activity on inhibiting human urate anion transporter 1 (hURAT1) and thus is useful for the treatment or prophylaxis of hyperuricemia; gout disease such as acute gouty arthritis, chronic gouty arthritis, tophus or gout nephrosis; nephritis; chronic renal failure; nephrolithiasis; uremia; urolithiasis; disease associated with uric acid such as hyperlipidemia, ischemic heart disease, myocardial infarction, cerebral infarction, cerebrovascular disease, diabetes or hypertension; and the like.

The pharmaceutical composition of the invention can be prepared in forms of pharmaceutical formulation suitable for oral or parenteral administration. The pharmaceutical formulation can be administered orally in form of powder, granule, tablet, pill, capsule, sylup or suspension, or parenteral administration by means of injection such as intravaneous or intramuscular injection by using solution, emulsion or suspension thereof.

Adminstration time and dosage may be suitably determined, depending on individual cases in consideration of the symptom, age, sex, body weight, and dosage form. For adults, the pharmaceutical composition of the present invention may be administered in amount of 0.1~2,000mg, preferably 1~200mg per day, in a single dose or multiple doses, but not limited thereto.

### [Advantageous effect]

The novel heterocycle derivative compounds of the invention has a strong inhibition activity to human urate anion transporter 1 (hURAT1), no drug-drug interaction to cytochrome P450 (CYP450) and a selectivity between organic anion transporters, and can be administered with lower dosage and fewer administration time since they have higher solubility and metabolic stability so as to show advantageous pharmacokinetics, as compared with conventional inhibitor of hURAT1 activity. As a result of pharmacokinetic analysis and comparison with a cononventional drug of benzbromarone and a compound disclosed in WO2006/057460 by using animal model such as mouse, rat and monkey, the heterocycle derivative compounds of the invention showed superior effects. In addition, in experiments of urinary drug excretion, the heterocycle derivative compounds of the invention showed superior results, by which it was confirmed that the drug delivery to kidney is easy.

Therefore, the novel heterocycle derivative compound of the invention and a pharmaceutical composition containing the Formula I can show superior effects to conventional drugs in the treatment or prophylaxis of hyperuricemia, acute gouty arthritis, chronic gouty arthritis, tophus, gout nephrosis, nephritis, chronic renal failure, nephrolithiasis, uremia, urolithiasis and complications reported to be accompanied with uric acid increase in blood such as hyperlipidemia, ischemic heart disease, myocardial infarction, arteriosclerosis, cerebral infarction, cerebrovascular disease, diabetes, hypertension or the like.

### [Embodiments for carrying out the invention]

The following will explain the preparation methods of Formula I of the invention in further detail with reference to following examples and experiments. However, the invention is not limited to the invention of the compounds described in following examples. Also, preparation methods of intermediates are shown as Intermediate Synthesis Examples.

The names of the compounds described in the following examples are in accordance with the nomenclature method (AutoNom version 2.1) using the structures provided by CS ChemDraw Ultra software of CambridgeSoft.

### Example 1)

### Synthesis of (3,5-dibromo-4-hydroxy-phenyl)-(2,3-dihydro-pyrido[2,3-b][1,4]oxazin-1-yl)-methanone (compound 1)

### a) Synthesis of 3-amino-pyridin-2-ol

In a 500ml flask, 3-nitro-pyridin-2-ol (1g, 71.4mmol) was dissolved in methanol (200ml), and then added 10% palladium on active carbon (100mg, 10w/w%). The reaction mixture was stirred for 3 hours under hydrogen atmosphere at room temperature. The resulting reaction solution was filtered on celite and then evaporated under reduced pressure to obtain white solid (800mg, quantitative yield).
¹H-NMR(CD₃OD, 300MHz); δ = 6.78-6.73 (m, 2H), 6.23 (t, *J*=6.5Hz, 1H).
MS(ESI); 111.1(M⁺+1).

### b) Synthesis of 1H-pyrido[2,3-b][1,4]oxazin-2-one

In a 20ml flask equipped with a reflux apparatus, 3-amino-pyridin-2-ol (300mg, 2.72mmol) was dissolved in acetonitrile (27ml). To the reaction solution was added chloroacetyl chloride (0.24ml, 3.0mmol) dropwise at room temperature, followed by Potassium carbonate (940mg, 6.80mmol). The mixture was heated at 100°C for 15 hours. After completion of the reaction, acetonitrile was evaporated under the reduced pressure. To the residue was added water and then the mixture was extracted with ethyl acetate. The combined organic layer was dried over anhydrous sodium sulfate (Na₂SO₄), filtered and evaporated under reduced pressure to obtain white solid (113mg, 28%).
¹H-NMR(CD₃0D, 300MHz); δ = 7.79 (dd, *J*=5.0Hz, 1.5Hz, 1H), 7.29 (dd, *J*=7.6Hz, 1.9Hz, 1H), 7.03 (dd, *J*=7.6Hz, 5.0Hz, 1H), 4.82(s, 2H).
MS(ESI); 151.1(M⁺+1).

### c) Synthesis of 2,3-dihydro-1H-pyrido[2,3-b][1,4]oxazine

1H-pyrido[2,3-b][1,4]oxazin-2-one (113mg, 0.75mmol) was dissolved in tetrahydrofuran (3.5ml) and then cooled to 0°C. 1.0M Lithium aluminum hydride dissolved in tetrahydrofuran (1.5ml, 1.5mmol) was added thereto dropwise. After stirring for 2 hours at room temperature, 0.1ml of water, 0.2ml of 10% aqueous solution of sodium hydroxide, and 0.3ml of water were added in this order under stirring. After filtering precipitates, the filtrate was extracted with ethyl acetate. The combined organic layer was dried over anhydrous sodium sulfate (Na₂SO₄), filtered and evaporated under reduced pressure to obtain colorless liquid (90mg, 88%).
¹H-NMR(DMSO-d₆, 300MHz); δ = 7.35 (dd, *J*=5.0Hz, 1.9Hz, 1H), 6.86 (dd, *J*=7.6Hz, 1.5Hz, 1H), 6.72 (dd, *J*=7.6Hz, 4.6Hz, 1H), 6.00 (s,1H), 4.23 (m, 2H), 3.25 (m, 2H).
MS(ESI); 136.9(M⁺+1).

### d) Synthesis of (3,5-dibromo-4-hydroxy-phenyl)-(2,3-dihydro-pyrido[2,3-b][1,4]oxazin-1-yl)-methanone

3,5-dibromo-4-hydroxy-benzoic acid (65mg, 0.22mmol) was dissolved in N,N-dimethyl acetamide (1.5ml) and then cooled to -5°C. Thionyl chloride (0.024ml, 0.33mmol) was added thereto and stirred at -5°C for 30 minutes. 2,3-dihydro-1H-pyrido[2,3-b][1,4]oxazine dissolved in N,N-dimethyl acetamide (0.7ml) was added thereto and then stirred at -5°C for 20 minutes, and subsequently stirred at room temperature for 15 hours. Water was added and the mixture was extracted with dichloromethane, and then the (extracted) combined organic layer was dried over anhydrous sodium sulfate (Na₂SO₄), filtered and evaporated under reduced pressure. The residue was purified by column chromatography on silica eluting with a solvent of n-hexane:ethyl acetate=1:1. The fractions containing the product were collected and evaporated to obtain pale-yellow solid (28mg, 31%).
¹H-NMR(CDCl₃, 300MHz); 8 = 10.58 (s, 1H), 7.93 (dd, *J*=4.6Hz, 1.5Hz, 1H), 7.81-7.55 (m, 3H), 6.92 (dd, *J*=8.0Hz, 4.6Hz, 1H), 4.41 (m, 2H), 3.87 (m, 2H).
MS(ESI); 412.9(M⁺+1).

### Example 2)

### Synthesis of (3,5-dibromo-4-methoxy-phenyl)-(2,3-dihydro-pyrido[4,3-b][1,4]oxazin-4-yl)-methanone (compound 2)

### a) Synthesis of 3-amino-pyridin-4-ol

In a 1000ml flask, 3-nitro-pyridin-4-ol (11.6g, 82.8mmol) was dissolved in methanol (800ml), and then added 10% palladium on active carbon (1.2g, 10w/w%). The reaction mixture was stirred for 3 hours under hydrogen atmosphere at room temperature. The resulting reaction solution was filtered on celite and then evaporated under reduced pressure to obtain white solid (9.4g, quantitative yield).
¹H-NMR(DMSO-d₆, 300MHz); δ = 7.34 (dd, *J*=6.5Hz, 1.5Hz, 1H), 7.13 (d, *J*=1.5Hz, 1H), 5.99 (d, *J*=6.5Hz, 1H), 4.53 (s, 2H), 3.90-2.90 (br s, 1H).
MS(ESI); 111.1(M⁺+1).

### b) Synthesis of 4H-pyrido[4,3-b][1,4]oxazin-3-one

In a 500ml flask equipped with a reflux apparatus, 3-amino-pyridin-4-ol (7.8g, 71mmol) was dissolved in N,N-dimethyl formamide (350ml). To the reaction solution was added chloroacetyl chloride (6.2ml, 78mmol) dropwise at room temperature and then stirred at room temperature for 30 minutes. Potassium carbonate (24g, 177mmol) was added thereto and the mixture was heated at 100°C for 40 hours. After completion of the reaction, water was added thereto. The mixture was extracted with ethyl acetate. The combined organic layer was dried over anhydrous sodium sulfate (Na₂SO₄), filtered and evaporated under reduced pressure. The residue was purified by recrystallizing from solvents of ethyl acetate and n-hexane to obtain white solid (5.6g, 53%).
¹H-NMR(DMSO-d₆, 300MHz); δ = 10.90 (s, 1H), 8.06-8.04 (m, 2H), 6.96 (d, *J*=5.3Hz, 1H), 4.71 (s, 2H).
MS(ESI); 150.8(M⁺+1).

### c) Synthesis of 3,4-dihydro-2H-pyrido[4,3-b][1,4]oxazine

4H-pyrido[4,3-b][1,4]oxazin-3-one (4.3g, 28mmol) was dissolved in tetrahydrofuran (140ml) and then cooled to 0°C. 1.0M Lithium aluminum hydride dissolved in tetrahydrofuran (57ml, 57mmol) was added thereto dropwise. After stirring for 2 hours at room temperature, 1.4ml of water, 2.8ml of 10% aqueous solution of sodium hydroxide, and 4.2ml of water were added in this order under stirring. After filtering precipitates, the filtrate was extracted with ethyl acetate. The combined organic layer was dried over anhydrous sodium sulfate (Na₂SO₄), filtered and evaporated under reduced pressure. The residue was purified by column chromatography on amine silica eluting with a solvent of dichloromethane. The fractions containing the product were collected and evaporated to obtain colorless liquid (3.5g, 91 %).
¹H-NMR(CDCl₃, 300MHz); δ = 7.89 (s, 1H), 7.83 (d, *J*=5.3Hz, 1H), 6.67 (d, *J*=5.3Hz, 1H), 4.30 (m, 2H), 4.25-3.80 (br s, 1H), 3.42 (m, 2H).
MS(ESI); 136.8(M⁺+1).

### d) Synthesis of (3,5-dibromo-4-methoxy-phenyl)-(2,3-dihydro-pyrido[4,3-b][1,4]oxazin-4-yl)-methanone

To a 10ml flask, 3,4-dihydro-2H-pyrido[4,3-b][1,4]oxazine (100mg, 0.73mmol) and 3,5-dibromo-4-methoxy-benzoyl chloride (240mg, 0.73mmol) were dissolved in dichloromethane. Triethylamine (0.51ml, 3.7mmol) was added thereto and then stirred at room temperature for 5 hours. After neutralizing with 1N hydrochloric acid solution, the mixture was extracted with dichloromethane and the combined organic layer was dried over anhydrous sodium sulfate (Na₂SO₄), filtered and evaporated under reduced pressure. The residue was purified by column chromatography on silica eluting with a solvent of dichloromethane:methanol=20:1. The fractions containing the product were collected and evaporated to obtain the target compound 2 as white solid (190mg, 61 %).
¹H-NMR(CDCl₃, 300MHz); δ = 8.30 (s, 1H), 8.16 (d, *J*=5.7Hz, 1H), 7.66 (s, 2H), 6.87 (d, *J*=5.3 Hz, 1H), 4.44 (m, 2H), 4.01 (m, 2H), 3.93 (s, 3H).
MS(ESI); 426.8(M⁺+1).

### Example 3)

### Synthesis of (3,5-dibromo-4-hydroxy-phenyl)-(2,3-dihydro-pyrido[4,3-b][1,4]oxazin-4-yl)-methanone hydrobromic acid salt (compound 3)

To a 20ml flask, (3,5-dibromo-4-methoxy-phenyl)-(2,3-dihydro-pyrido[4,3-b][1,4]oxazin-4-yl)-methanone (310mg, 0.72mmol) obtained in the example 2 was dissolved in dichloromethane, and then cooled to 0°C. 1.0M Boron tribromide dissolved in dichloromethane (4.3ml, 4.3mmol) was added thereto and then stirred at 0°C for 10 hours. The resulting solid by addition of n-hexane was filtered and collected, and purified by recrystallizing from methanol to obtain the target compound 3 as white solid (130mg, 43%).
¹H-NMR(DMSO-d₆, 300MHz); δ = 9.02 (s, 1H), 8.45 (d, 1H, *J*=6.5Hz), 7.82 (s, 2H), 7.53 (d, 1H, *J*=6.5Hz), 4.57 (m, 2H), 3.99 (m, 2H).
MS(ESI); 412.8(M⁺+1).
Elementary Analysis: C14H10Br2N2O3 •HBr C, 34.47; H, 2.26; N, 5.63

### Example 4)

### Synthesis of (3,5-dibromo-4-hydroxy-phenyl)-(2,3-dihydro-pyrido[4,3-b][1,4]oxazin-4-yl)-methanone (compound 4)

(3,5-dibromo-4-hydroxy-phenyl)-(2,3-dihydro-pyrido[4,3-b][1,4]oxazin-4-yl)-methanone hydrobromic acid salt (45mg, 0.091mmol) obtained in the example 3 was added to water (5ml) and neutralized (pH=7) by using saturated solution of sodium hydrogen carbonate, and then extracted with ethyl acetate. The combined organic layer was dried over anhydrous sodium sulfate (Na₂SO₄), filtered and evaporated under reduced pressure. The resulting solid was recrystallized from dichloromethane and n-hexane to obtain the target compound 4 as white solid (20mg, 53%).
¹H-NMR(DMSO-d₆, 300MHz); δ = 10.63 (br s,1H) 8.44 (br s, 1H), 8.74 (d, *J*=5.1Hz, 1H), 7.74 (s, 2H), 6.95 (d, *J*=5.7Hz, 1H), 4.37-4.40 (m, 2H), 3.90-3.92 (m, 2H).
MS(ESI): 412.8(M⁺+1).
Elementary Analysis: C14H10Br2N2O3 C, 40.83; H, 2.46; N, 6.52

### Example 5)

### Synthesis of (3,5-dibromo-4-methoxy-phenyl)-(2,3-dihydro-pyrido[3,4-b][1,4]oxazin-1-yl)-methanone (compound 5)

### a) Synthesis of 2,2-dimethyl-N-pyridin-4-yl-propionamide

4-pyridylamiine (2g, 21.3mmol) was dissolved in dichloromethane (20ml), and pyvaloyl chloride (3.1ml, 25.6mmol) and triethylamine (8.9mg, 63.9mmol) were added thereto in this order at room temperature dropwise. The reaction solution was stirred at room temperature for 15 hours and the reaction was quenched by adding water. The organic layer was extracted with ethyl acetate, and then the combined organic layer was washed with saturated saline solution (50ml), dried over anhydrous sodium sulfate (Na₂SO₄), filtered and evaporated under reduced pressure. The residue was purified by column chromatography on silica eluting with a solvent of dichloromethane: methanol=20:1. The fractions containing the product were collected and evaporated to obtain the target compound as white solid (3.6g, 95%).
¹H-NMR(CDCl₃, 300MHz); δ = 8.47 (d, *J*=6.1Hz, 2H), 7.79 (br s, 1H), 7.52 (d, *J*=6.0Hz, 2H), 1.32 (s, 9H).

### b) Synthesis of N-(3-hydroxy-4-pyridinyl)-2,2-dimethyl-propionamide

2,2-dimethyl-N-pyridin-4-yl-propionamide (1.6g, 8.977mol) was dissolved in anhydrous tetrahydrofuran (20ml) under nitrogen atmosphere and then cooled to -78°C, and then 2.5M solution of n-butyl lithium (n-BuLi) in n-hexane (9ml, 22.443mmol) was added thereto dropwise and then stirred at 0°C for 2.5 hours until yellow crystals were formed. After cooling the reaction mixture to -78°C, trimethylboron (2.5ml, 22.443mmol) was added thereto dropwise for 10 minutes, and the temperature was raised slowly up to 0°C. After stirring for 2 hours, acetic acid (1.9ml) and 30%w/w aqueous solution of hydrogen peroxide were added dropwise to the reaction solution at 0°C, and then after stirring for 30 minutes, water (1ml) was added thereto dropwise and then stirred at room temperature for 18 hours. Water was added and the mixture was evaporated under reduced pressure, and the residue was extracted by using water and 10% isopropanol/chloroform. The combined organic layer was treated with active carbon and the filtrate was washed with saturated saline solution, dried over anhydrous sodium sulfate (Na₂SO₄), filtered and evaporated under reduced pressure. The residue was purified by column chromatography on silica eluting with a solvent of dichloromethane:methanol=20:1. The fractions containing the product were collected and evaporated to obtain white solid (1.16g, 67%).
¹H-NMR(CDCl₃, 300MHz); δ = 8.83 (s, 1H), 8.39 (d, *J*=6.0Hz, 1H), 7.83 (s, 1H), 7.80 (d, *J*=6.0Hz, 1H), 5.30 (br s, 1H), 1.35 (s, 9H).

### c) Synthesis of 4-amino-3-pyridinol

At room temperature, N-(3-hydroxy-4-pyridinyl)-2,2-dimethyl-propionamide (1.15g, 5.921mmol) was suspended by dropwise addition to 3N hydrochloric acid solution. The heterogenous solution was heated at 90 °C for 18 hours. The reaction mixture was cooled to 0°C and neutralized by adding 6N sodium hydroxide solution dropwise, and evaporated under reduced pressure. The residue was dissolved in methanol and then filtered to remove the byproduct. The filtrate was evaporated under reduced pressure to obtain solid. Again, the resulting solid was dissolved in ethanol solution, filtered and evaporated under reduced pressure to obtain white solid (660mg, quantitative yield).
¹H-NMR(CD₃OD, 300MHz); δ = 7.42 (d, *J*=6.1Hz, 1H), 7.24 (s, 1H), 6.67 (d, *J*=6.1Hz, 1H).

### d) Synthesis of 1H-pyrido[3,4-b][1,4]-oxazin-2-one

4-amino-3-pyridinol (650mg, 5.904mmol) was dissolved in anhydrous N,N-dimethyl formamide (15ml) under nitrogen atmosphere, and chloroacetyl chloride (0.52ml, 6.494mmol) solution was added thereto dropwise at room temperature and then stirred for 30 minutes. Then, potassium carbonate (2.0g, 14.760mmol) was added thereto dropwise at room temperature, and the reaction mixture was heated at 100°C for 18 hours. The reaction mixture was cooled to room temperature and water was added to quench the reaction. The organic layer was extracted with ethyl acetate and the combined organic layer was washed with water and saturated saline solution, dried over anhydrous sodium sulfate (Na₂SO₄), filtered and evaporated under reduced pressure to obtain white solid. The obtained white solid was recrystallized from ethyl acetate and n-hexane to obtain white solid (520mg, 59%).
¹H-NMR(DMSO-d₆, 300MHz); δ = 11.05 (br s, 1H), 8.16 (s, 1H), 8.07 (d, *J*=5.8Hz, 1H), 6.87 (d, *J*=5.8Hz, 1H), 4.68 (s, 2H).
MS(ESI); 150.9(M⁺+1).

### e) Synthesis of 2,3-dihydro-1H-pyrido[3,4-b][1,4]-oxazine

1H-pyrido[3,4-b][1,4]-oxazin-2-one (510mg, 3.397mmol) was dissolved in anhydrous tetrahydrofuran (20ml) under nitrogen atmosphere and then cooled to 0°C. 1.0M Lithium aluminum hydride dissolved in tetrahydrofuran (6.8ml, 6.794mmol) was added thereto dropwise. The mixture was stirred for 30 minutes, and the temperature was raised to room temperature, and then the mixture was stirred for 1 hour. After completion of the reaction, the mixture was cooled to 0°C again, and then water (0.25ml), 10% aqueous solution of sodium hydroxide (0.5ml) and water (0.8ml) were added thereto dropwise in this order, and the resulting solution was vigorously stirred at room temperature for 1 hour. The produced solid was filtered and washed with excessive ethyl acetate, and then the filtrate was washed with water and saturated saline solution, dried over anhydrous sodium sulfate (Na₂SO₄), filtered and evaporated under reduced pressure to obtain white solid (300mg, 65%).
¹H-NMR(DMSO-d₆, 300MHz); δ = 7.74 (s, 1H), 7.67 (d, *J*=5.8Hz, 1H), 6.67 (br s, 1H), 6.46 (d, *J*=5.8Hz, 1H), 4.10 (m, 2H), 3.34 (m, 2H).
MS(ESI); 136.7(M⁺+1)

### f) Synthesis of (3,5-dibromo-4-methoxy-phenyl)-(2,3-dihydro-pyrido[3,4-b][1,4]oxazin-1-yl)-methanone

By the same method as in the step d) of Example 2, 2,3-dihydro-1H-pyrido[3,4-b][1,4]-oxazine (180mg, 0.587mmol) and 3,5-dibromo-4-methoxy-benzoyl chloride (193mg, 0.587mmol) were reacted to obtain the target compound 5, (3,5-dibromo-4-methoxy-phenyl)-(2,3-dihydro-pyrido[3,4-b][1,4]oxazin-1-yl)-methanone, as white solid (85mg, 34%).
¹H-NMR(DMSO-d₆, 300MHz); δ =8.23 (s, 1H), 7.99 (d, *J*=5.7Hz, 1H), 7.92 (s, 2H), 7.56 (m, 1H), 4.33 (m, 2H), 3.86 (m, 5H).
MS(ESI); 426.9(M⁺+1).

### Example 6)

### Synthesis of (3,5-dibromo-4-hydroxy-phenyl)-(2,3-dihydro-pyrido[3,4-b][1,4]oxazin-1-yl)-methanone (compound 6)

By the same method as in Example 3, (3,5-dibromo-4-methoxy-phenyl)-(2,3-dihydro-pyrido[3,4-b][1,4]oxazin-1-yl)-methanone (60mg, 0.140mmol) and 1M solution of boron tribromide (0.84ml, 0.840mmol) were reacted, and then the reaction mixture was neutralized (pH=7) by using saturated solution of sodium hydrogen carbonate and extracted with ethyl acetate. The combined organic layer was dried over anhydrous sodium sulfate (Na₂SO₄), filtered and evaporated under reduced pressure to obtain solid. The obtained solid was recrystallized from dichloromethane and n-hexane to obtain the target compound 6, (3,5-dibromo-4-hydroxy-phenyl)-(2,3-dihydro-pyrido[3,4-b][1,4]oxazin-1-yl)-methanone, as white solid (28mg, 48%).
¹H-NMR(DMSO-d₆, 300MHz); δ = 10.74 (br s, 1H), 8.23 (s, 1H), 7.96 (d, *J*=5.7Hz, 1H), 7.80 (s, 2H), 7.41 (d, *J*=5.7Hz, 1H), 4.34 (m, 2H), 3.89 (m, 2H).
MS(ESI); 412.9(M⁺+1).

### Example 7)

### Synthesis of (3,5-dibromo-4-hydroxy-phenyl)-(6-methyl-2,3-dihydro-pyrido[2,3-b][1,4]oxazin-1-yl)-methanone (compound 7)

### a) Synthesis of 3-amino-6-methyl-pyridin-2-ol

6-hydroxy-5-nitro-2-picoline (500mg, 3.247mmol) was dissolved in methanol: dichloromethane (5:1, 60ml) and 10% palladium on active carbon was added thereto slowly, and then stirred for 2 hours with adding hydrogen gas at room temperature. The reaction mixture was filtered by using celite and evaporated under reduced pressure to quantitatively obtain white solid (400mg).
¹H-NMR(CDCl₃, 300MHz); 8 = 11.67 (br s, 1H), 6.57 (d, *J*=7.2Hz, 1H), 5.88 (d, *J*=7.2Hz, 1H), 3.20 (br s, 2H), 2.25 (s, 3H).
MS(ESI); 125.1(M⁺+1).

### b) Synthesis of 6-methyl-1H-pyrido[2,3-b][1,4]oxazin-2-one

By the same method as in the step d) of Example 5, 3-amino-6-methyl-pyridin-2-ol (400mg, 3.222mmol) was reacted with chloroacetyl chloride (0.26ml, 3.222mmol) to obtain the target compound, 6-methyl-1H-pyrido[2,3-b][1,4]oxazin-2-one (230mg, 44%).
¹H-NMR(CDCl₃, 300MHz); δ = 8.37 (s, 1H), 7.10 (d, *J*=5.2Hz, 1H), 6.81 (d, *J*=5.2Hz, 1H), 4.80 (s, 2H), 2.44 (s, 3H).
MS(ESI); 165.1(M⁺+1).

### c) Synthesis of 6-methyl-2,3-dihydro-1H-pyrido[2,3-b][1,4]oxazine

By the same method as in the step e) of Example 5, 6-methyl-1H-pyrido[2,3-b][1,4]oxazin-2-one (230mg, 1.401mmol) was reacted with 1.0M Lithium aluminum hydride dissolved in tetrahydrofuran (3.0ml, 3.082mmol) to obtain the target compound, 6-methyl-2,3-dihydro-1H-pyrido[2,3-b][1,4]oxazine, as white solid (160mg, 76%).
¹H-NMR(CDCl₃, 300MHz); δ = 6.79 (d, *J*=7.8Hz, 1H), 6.59 (d, *J*=7.5Hz, 1H), 4.39 (m, 2H), 3.65 (m, 2H), 2.35 (s, 3H).
MS(ESI); 151.1(M⁺+1).

### d) Synthesis of (3,5-dibromo-4-hydroxy-phenyl)-(6-methyl-2,3-dihydro-pyrido[2,3-b][1,4]oxazin-1-yl)-methanone

By the same method as in the step d) of Example 1, 6-methyl-2,3-dihydro-1H-pyrido[2,3-b][1,4]oxazine (50mg, 0.333mmol), thionyl chloride (53µl, 0.733mmol) and 3,5-dibromo-4-hydroxy-benzoyl chloride (197mg, 0.666mmol) were reacted to obtain the target compound 8, (3,5-dibromo-4-hydroxy-phenyl)-(6-methyl-2,3-dihydro-1H-pyrido[2,3-b][1,4]oxazin-1-yl)-methanone, as white solid (40mg, 28%).
¹H-NMR(DMSO-d₆, 300MHz); δ = 10.42(br s, 1H), 7.58 (s, 2H), 7.42 (m, 1H), 6.63 (br s, 1H), 4.21 (m, 2H), 3.68 (m, 2H), 2.15(s, 3H)
MS(ESI); 426.9(M⁺+1).

### Example 8)

### Synthesis of (3,5-dibromo-4-hydroxy-phenyl)-(2,2-dimethyl-2,3-dihydro-pyrido[4,3-b][1,4]oxazin-4-yl)-methanone (compound 8)

### a) Synthesis of 3-aminopyridin-4-ol

By the same method as in the step a) of Example 2, 3-nitropyridin-4-ol (3.0g, 21mmol) was reduced with palladium on active carbon to obtain 3-aminopyridin-4-ol (2.4g, quantitative yield).
¹H-NMR(DMSO-d₆, 300MHz); δ = 7.35(dd, *J*=6.9Hz, 1.5Hz, 1H), 7.13 (d, *J*=1.5Hz, 1H), 6.00 (d, *J*=6.9Hz, 1H), 4.53 (br s, 3H).
MS(ESI); 111.1(M⁺+1).

### b) Synthesis of 2,2-dimethyl-4H-pyrido[4,3-b][1,4]oxazin-3-one

By the same method as in the step b) of Example 2, 3-aminopyridin-4-ol (300mg, 2.7mmol) was reacted with 2-bromo-2-methyl-propionyl bromide (0.37ml, 3.0mmol) to obtain 2,2-dimethyl-4H-pyrido[4,3-b][1,4]oxazin-3-one (122mg, 25%).
¹H-NMR(DMSO-d₆, 300MHz); δ = 10.84 (s, 1H), 8.05 (m, 2H), 8.00 (br s, 1H), 6.96 (d, *J*=5.4Hz, 1H), 1.44 (s, 6H).
MS(ESI); 179.0(M⁺+1).

### c) Synthesis of 2,2-dimethyl-3,4-dihydro-2H-pyrido[4,3-b][1,4]oxazine

By the same method as in the step c) of Example 2, 2,2-dimethyl-4H-pyrido[4,3-b][1,4]oxazin-3-one (120mg, 0.67mmol) was reacted with 1.0M Lithium aluminum hydride dissolved in tetrahydrofuran (1.4ml, 1.4mmol) to obtain 2,2-dimethyl-3,4-dihydro-2H-pyrido[4,3-b][1,4]oxazine (110mg, quantitative yield).
¹H-NMR(CDCl₃, 300MHz); δ = 7.95 (s, 1H), 7.86 (d, *J*=5.4Hz, 1H), 6.67 (d, *J*=5.7Hz, 1H), 3.88 (br s, 1H), 3.12 (s, 2H), 1.36 (s, 6H).
MS(ESI); 165.0(M⁺+1).

### d) Synthesis of (3,5-dibromo-4-methoxy-phenyl)-(2,2-dimethyl-2,3-dihydro-pyrido[4,3-b][1,4]oxazin-4-yl)-methanone

By the same method as in the step d) of Example 2, 2,2-dimethyl-3,4-dihydro-2H-pyrido[4,3-b][1,4]oxazine (60mg, 0.36mmol) was reacted with 3,5-dibromo-4-methoxy-benzoyl chloride (120mg, 0.36mmol) to obtain the target compound, (3,5-dibromo-4-methoxy-phenyl)-(2,2-dimethyl-2,3-dihydro-pyrido[4,3-b][1,4]oxazin-4-yl)-methanone (73mg, 44%).
¹H-NMR(CDCl₃, 300MHz); δ = 8.36 (br s, 1H), 8.17 (d, *J*=5.4Hz, 1H), 7.66 (s, 2H), 6.83 (d, *J*=5.4Hz, 1H), 3.9 (s, 3H), 3.7 (br s, 2H), 1.4 (s, 6H).
MS(ESI); 454.9(M⁺+1).

### e) Synthesis of (3,5-dibromo-4-hydroxy-phenyl)-(2,2-dimethyl-2,3-dihydro-pyrido[4,3-b][1,4]oxazin-4-yl)-methanone

By the same method as in Example 6, (3,5-dibromo-4-methoxy-phenyl)-(2,2-dimethyl-2,3-dihydro-pyrido[4,3-b][1,4]oxazin-4-yl)-methanone (70mg, 0.15mmol) was reacted with 1M solution of boron tribromide (0.9ml, excess amount) to obtain the target compound 8, (3,5-dibromo-4-hydroxy-phenyl)-(2,2-dimethyl-2,3-dihydro-pyrido[4,3-b][1,4]oxazin-4-yl)-methanone (33mg, 49%).
¹H-NMR(DMSO-d₆, 300MHz); 8 = 10.71 (br s, 1H), 8.54 (br s, 1H), 8.11 (d, *J*=5.7Hz, 1H), 7.72 (s, 2H), 6.92 (d, *J*=5.7Hz, 1H), 3.73 (br s, 2H) 1.26 (s, 6H).
MS(ESI); 440.9(M⁺+1).

### Example 9)

### Synthesis of (7-cyclopropyl-2,3-dihydro-pyrido[2,3-b][1,4]oxazin-1-yl)-(3,5-dibromo-4-hydroxy-phenyl)-methanone (compound 9)

### a) Synthesis of 7-cyclopropyl-1H-pyrido[2,3-b][1,4]oxazin-2-one

To a 10ml flask, 7-bromo-1H-pyrido[2,3-b][1,4]oxazin-2-one (400mg, 1.746mmol), cyclopropyl boronic acid (165mg, 1.921mmol), palladium acetate (39.1mg, 0.174mmol), triphenylphosphine (91.5mg, 0.349mmol) and potassium carbonate (482.6mg, 3.492mmol) were dissolved in a solvent of acetonitrile/water (6/1, 4.2ml/0.9ml). The reaction mixture was stirred for 60 minutes at 100°C using microwave (100W) apparatus. After cooling to room temperature, the reaction mixture was extracted with ethyl acetate and the organic layer was washed with saturated saline solution. The combined organic layer was dried over anhydrous magnesium sulfate (MgSO₄), filtered and evaporated under reduced pressure. The residue was purified by column chromatography on silica eluting with a solvent of dichloromethane and ethyl acetate. The fractions containing the product were collected and evaporated to obtain a mixture (40mg, 0.8%) containing triphenyl phosphoxide.
¹H-NMR(CDCl₃, 300MHz); δ = 8.95 (s, 1H), 7.72 (d, *J*=1.8Hz, 1H), 6.82 (d, *J*=2.1Hz, 1H), 4.77 (s, 2H), 1.80-1.87 (m, 1H), 0.64-0.99 (m, 2H), 0.57-0.63 (m, 2H).

### b) Synthesis of 7-cyclopropyl -2,3-dihydro-1H-pyrido[2,3-b][1,4]oxazine

7-cyclopropyl-1H-pyrido[2,3-b][1,4]oxazin-2-one (40mg, 0.052mmol) was dissolved in 1ml anhydrous tetrahydrofuran under nitrogen atmosphere and then cooled to 0°C. 1.0M Lithium aluminum hydride dissolved in tetrahydrofuran (0.1ml, 0.104mmol) was added thereto dropwise and then stirred at room temperature for 5 hours. Water (0.3ml) was added thereto dropwise. The resulting solution was evaporated under reduced pressure to obtain a mixture containing 7-cyclopropyl-2,3-dihydro-1H-pyrido[2,3-b][1,4]oxazine and triphenyl phosphoxide as white solid (10mg).

### c) Synthesis of (7-cyclopropyl-2,3-dihydro-pyrido[2,3-b][1,4]oxazin-1-yl)-(3,5-dibromo-4-methoxy-phenyl)-methanone

To a 10ml flask, 7-cyclopropyl-2,3-dihydro-1H-pyrido[2,3-b][1,4]oxazine (10mg, 0.057mmol) and 3,5-dibromo-4-methoxy-benzoyl chloride (28mg, 0.085mmol) were dissolved in dichloromethane (1.2ml). After cooling to 0°C, triethylamine (0.04ml, 0.285mmol) was added thereto and then stirred at room temperature for 3 hours. The reaction mixture was neutralized with 1N hydrochloric acid solution and extracted with dichloromethane, and then the combined organic layer was dried over anhydrous magnesium sulfate (MgSO₄), filtered and evaporated under reduced pressure. The residue was purified by column chromatography on silica eluting with a solvent of ethyl acetate and n-hexane (1:1). The fractions containing the product were collected and evaporated to obtain the target compound as white solid (9.0mg, 33%).
¹H-NMR(CDCl₃, 300MHz); δ = 7.86 (d, *J*=2.4Hz, 1H), 7.45 (s, 2H), 7.21 (s, 1H), 4.43-4.47 (m, 2H), 3.95-3.97 (m, 2H), 3.94 (s, 3H), 1.73-1.82 (m, 1H), 0.85-0.91 (m, 2H), 0.39-0.45 (m, 2H).

### d) Synthesis of (7-cyclopropyl-2,3-dihydro-pyrido[2,3-b][1,4]oxazin-1-yl)(3,5-dibromo-4-hydroxy-phenyl)-methanone

To a 10ml flask, (7-cyclopropyl-2,3-dihydro-pyrido[2,3-b][1,4]oxazin-1-yl)-(3,5-dibromo-4-methoxy-phenyl)-methanone (9.0mg, 0.019mmol) was dissolved in dichloromethane, and then cooled to 0°C. 1.0M Boron tribromide dissolved in dichloromethane (0.2ml*2, 0.203mmol) was added thereto and then stirred at room temperature for 16 hours. The pH was adjusted to 6 with 1N sodium hydroxide solution and the reaction mixture was extracted with dichloromethane, and the combined organic layer was dried over anhydrous sodium sulfate (Na₂SO₄), filtered and evaporated under reduced pressure to obtain solid. The resulting solid was recrystallized from dichloromethane to obtain white solid (6.7mg, 84%).
¹H-NMR(DMSO-d₆, 300MHz); δ = 10.59 (s, 1H), 7.78 (d, *J*=2.1Hz, 1H), 7.72 (s, 2H), 7.25 (s, 1H), 4.35-4.38 (m, 2H), 3.82-3.84 (m, 2H), 1.79-1.81 (m, 1H), 0.80-0.86 (m, 2H), 0.28-0.39 (m, 2H).
MS(ESI); 452.8 (M⁺+1).

### Example 10)

### Synthesis of (3-chloro-4-hydroxy-phenyl)-(2,3-dihydro-pyrido[43-b][1,4]oxazin-4-yl)-methanone hydrobromic acid salt (compound 10)

### a) Synthesis of (3-chloro-4-methoxy-phenyl)-(2,3-dihydro-pyrido[4,3-b][1,4]oxazin-4-yl)-methanone

To a 10ml flask, 3,4-dihydro-2H-pyrido[4,3-b][1,4]oxazine (150mg, 1.102mmol) and 3-chloro-4-methoxy-benzoyl chloride (249mg, 1.212mmol) were dissolved in dichloromethane. Triethylamine (0.77ml, 5.510mmol) was added thereto and then stirred at room temperature for 2 hours. The reaction mixture was neutralized with 1N hydrochloric acid solution and extracted with dichloromethane. And the combined organic layer was dried over anhydrous magnesium sulfate (MgSO₄), filtered and evaporated under reduced pressure. The residue was purified by column chromatography on silica eluting with a solvent of ethyl acetate. The fractions containing the product were collected and evaporated to obtain ivory-colored solid (238mg, 71%).
¹H-NMR(DMSO-d₆); δ = 8.39(br, 1H), 8.07(d, *J*=5.7Hz, 1H), 7.66(d, *J*=2.3Hz, 1H), 7.55(dd, *J*=1.9Hz, 8.4Hz, 1H), 7.23(d, *J*=8.4Hz, 1H), 6.95(d, *J*=5.7Hz, 1H), 4.40(m, 2H), 3.92(s, 5H). MS(ESI); 305(M⁺+1).

### b) Synthesis of (3-chloro-4-hydroxy-phenyl)-(2,3-dihydro-pyrido[4,3-b][1,4]oxazin-4-yl)-methanone hydrobromic acid salt

To a 10ml flask, (3-chloro-4-methoxy-phenyl)-(2,3-dihydro-pyrido[4,3-b][1,4]oxazin-4-yl)-methanone (100mg, 0.328mmol) was dissolved in dichloromethane, and then cooled to 0°C. 1.0M Boron tribromide dissolved in dichloromethane (2.0ml, 1.969mmol) was added thereto and then stirred at room temperature for 16 hours. The solid formed by adding ethyl acetate solvent was filtered and collected, and purified by recrystallizing from dichloromethane to obtain white solid (45mg, 47%).
¹H-NMR(DMSO-d₆, 300MHz); δ = 11.03 (br s, 1H), 9.04 (s, 1H), 8.48 (d, *J*=6.9Hz, 1H), 7.65 (d, *J*=1.9Hz, 1H), 7.58 (d, *J*=6.5Hz, 1H), 7.46 (dd, *J*=1.5Hz, 8.4Hz, 1H), 7.09 (d, *J*=8.4Hz, 1H), 4.58 (m, 2H), 4.01 (m, 2H).
MS(ESI); 291.2(M⁺+1).

### Example 11)

### Synthesis of (3-bromo-4-hydroxy-phenyl)-(2,3-dihydro-pyrido[4,3-b][1,4]oxazin-4-yl)-methanone hydrobromic acid salt (compound 11)

### a) Synthesis of (3-bromo-4-methoxy-phenyl)-(2,3-dihydro-pyrido[4,3-b][1,4]oxazin-4-yl)-methanone

To a mixture of 3,4-dihydro-2H-pyrido[4,3-b][1,4]oxazine (50mg, 0.37mmol) and 3-bromo-4-methoxy-benzoic acid (102mg, 0.44mmol), phosphorus oxychloride (2ml) was added and stirred at 100°C for 12 hours. The reaction mixture was cooled to 0°C, neutralized with saturated solution of sodium hydrogen carbonate to pH of 8-9 and extracted with ethyl acetate. And then the combined organic layer was washed with saturated saline solution, dried over anhydrous sodium sulfate (Na₂SO₄), filtered and evaporated under reduced pressure. The residue was purified by column chromatography on silica eluting with a solvent of n-hexane:dichloromethane=1:1. The fractions containing the product were collected and evaporated to obtain white solid (38mg, 51%).
¹H-NMR(CDCl₃, 300MHz); δ = 8.19 (s, 1H), 8.12 (d, *J*=5.7Hz, 1H), 7.77 (d, *J*=1.9Hz, 1H), 7.44 (dd, *J*=8.6Hz, 1.9Hz, 1H), 6.91-6.81 (m, 2H), 4.44 (m, 2H), 4.03 (m, 2H), 3.93 (s, 3H).

### b) Synthesis of (3-bromo-4-hydroxy-phenyl)-(2,3-dihydro-pyrido[4,3-b][1,4]oxazin-4-yl)-methanone hydrobromic acid salt

By the same method as in Example 3, (3-bromo-4-methoxy-phenyl)-(2,3-dihydro-pyrido[4,3-b][1,4]oxazin-4-yl)-methanone (30mg, 0.086mmol) and 1M solution of boron tribromide (0.86ml, 0.86mmol) were reacted to obtain the target compound, (3-bromo-4-hydroxy-phenyl)-(2,3-dihydro-pyrido[4,3-b][1,4]oxazin-4-yl)-methanone hydrobromic acid salt (26mg, 72%).
¹H-NMR(DMSO-d₆, 300MHz); δ = 11.1 (br s, 1H), 8.97 (s, 1H), 8.42 (d, *J*=6.5Hz, 1H), 7.78 (d, *J*=1.9Hz, 1H), 7.54-7.44 (m, 2H), 7.05 (d, *J*=8.4Hz, 1H), 4.56 (m, 2H), 4.01 (m, 2H).
MS(ESI); 335.1(M⁺+1).

### Example 12)

### Synthesis of (2,3-dihydro-pyrido[4,3-b][1,4]oxazin-4-yl)-(4-hydroxy-3-trifluoromethyl-phenyl)-methanone (compound 12)

### a) Synthesis of (2,3-dihydro-pyrido[4,3-b][1,4]oxazin-4-yl)-(4-methoxy-3-trifluoromethyl-phenyl)-methanone

To a 50ml flask, 3,4-dihydro-2H-pyrido[4,3-b][1,4]oxazine (1.0g, 7.34mmol) and 3-trifluoromethyl-4-methoxy-benzoyl chloride (2.1g, 8.80mmol) were dissolved in dichloromethane. Triethylamine (2.0ml, 14.68mmol) was added thereto and then stirred at room temperature for 4 hours. To the reaction mixture, water was added to quench the reaction and the mixture was extracted with dichloromethane, and the combined organic layer was washed with water and saturated saline solution, dried over anhydrous sodium sulfate (Na₂SO₄), filtered and evaporated under reduced pressure.

The residue was purified by column chromatography on silica eluting with a solvent of ethyl acetate:n-hexane=2:1. The fractions containing the product were collected and evaporated to obtain yellow solid (1.19g, 80%).
¹H-NMR(CDCl₃, 300MHz); δ = 8.41 (s, 1H), 8.08 (d, *J*=5.7Hz, 1H), 7.88 (dd, *J*=8.7Hz, 2.1Hz, 1H), 7.82 (s, 1H), 7.36 (d, *J*=8.7Hz, 1H), 6.96 (d, *J*=5.7Hz, 1H), 4.42 (m, 2H), 3.96 (s, 3H), 3.93 (m, 2H).
MS(ESI); 339.0(M⁺+1).

### b) Synthesis of (2,3-dihydro-pyrido[4,3-b][1,4]oxazin-4-yl)-(4-hydroxy-3-trifluoromethylphenyl)-methanone

To a 100ml flask, 2,3-dihydro-pyrido[4,3-b][1,4]oxazin-4-yl)-(4-methoxy-3-trifluoromethylphenyl)-methanone (1.19g, 3.52mmol) was dissolved in dichloromethane. After cooling to 0°C, 1.0M Boron tribromide dissolved in dichloromethane (35.2ml, 35.2mmol) was added thereto and then stirred at room temperature for 10 hours. The solid formed by adding a solvent of n-hexane and water was filtered. The crude was purified by recrystallizing from dichloromethane to obtain white solid. The obtained solid was further purified by preparative high pressure chromatography using Waters LC/MS apparatus eluting a solvent of acetonitrile and distilled water containing 0.1 % trifluoroacetic acid (TFA) to obtain the compound as salt form of trifluoroacetic acid (TFA). The obtained compound was washed with 10% aqueous solution of sodium hydrogen carbonate (NaHCO₃) and extracted with dichloromethane. The combined organic layer was washed with water and saturated saline solution, dried over anhydrous sodium sulfate (Na₂SO₄), filtered and evaporated under reduced pressure to obtain the target compound 12 as white solid (13.52mg, 1.2%).
¹H-NMR(CDCl₃, 300MHz); δ = 11.34 (s, 1H), 8.39 (s, 1H), 8.07 (d, *J*=5.7Hz, 1H), 7.73 (s, 1H), 7.68 (dd, *J*=8.7Hz, 1H), 7.06 (d, *J*=8.7Hz, 1H), 6.95 (d, *J*=5.4Hz, 1H), 4.40 (m, 2H), 3.93 (m, 2H).
MS(ESI); 325.0(M⁺+1).

### Example 13)

### Synthesis of (3,5-dichloro-4-hydroxy-phenyl)-(2,3-dihydro-pyrido[4,3-b][1,4]oxazin-4-yl)-methanone hydrobromic acid salt (compound 13)

### a) Synthesis of (3,5-dichloro-4-methoxyphenyl)-(2,3-dihydro-pyrido[4,3-b][1,4]oxazin-4-yl)-methanone

To a mixture of 3,4-dihydro-2H-pyrido[4,3-b][1,4]oxazine (80mg, 0.58mmol) and 3,5-dichloro-4-methoxy-benzoic acid (141mg, 0.64mmol), phosphorus oxychloride (4ml) was added and then stirred at 100°C for 12 hours. The reaction mixture was cooled to 0°C and adjusted to pH of 8-9 by using saturated solution of sodium hydrogen carbonate and extracted with ethyl acetate. The combined organic layer was washed with saturated saline solution, dried over anhydrous sodium sulfate (Na₂SO₄), filtered and evaporated under reduced pressure. The residue was purified by column chromatography on silica eluting with a solvent of n-hexane:ethyl acetate=4:1. The fractions containing the product were collected and evaporated to obtain white solid (107mg, 54%).
¹H-NMR(CDCl₃, 300MHz); δ = 7.67 (s, 2H), 7.12-6.96 (m, 1H), 6.87 (dd, *J*=5.3Hz, 9.2Hz, 1H), 6.78 (td, *J*=3.1Hz, 8.8Hz, 1H), 4.31 (m, 2H), 3.97-3.88 (m, 5H).
MS(ESI); 339.0(M⁺+1).

### b) Synthesis of (3,5-dichloro-4-hydroxy-phenyl)-(2,3-dihydro-pyrido[4,3-b][1,4]oxazin-4-yl)-methanone hydrobromic acid salt

By the same method as in Example 3, (3,5-dichloro-4-methoxy-phenyl)-(2,3-dihydro-pyrido[4,3-b][1,4]oxazin-4-yl)-methanone (116mg, 0.342mmol) was reacted with 1M solution of boron tribromide (3.42ml, 3.42mmol) to obtain the target compound, (3,5-dichloro-4-hydroxy-phenyl)-(2,3-dihydro-pyrido[4,3-b][1,4]oxazin-4-yl)-methanone hydrobromic acid salt (65mg, 67%).
¹H-NMR(DMSO-d₆, 300MHz); δ = 11.0 (br s, 1H), 9.04 (s, 1H), 8.46 (d, *J*=6.5Hz, 1H), 7.66 (s, 2H), 7.55 (d, *J*=6.5Hz, 1H), 4.58 (m, 2H), 4.00 (m, 2H).
MS(ESI); 325.0(M⁺+1).

### Example 14)

### Synthesis of (3-chloro-4-hydroxy-5-nitro-phenyl)-(2,3-dihydro-byciclo[4,3-b][1,4]oxazin-4-yl) methanone (compound 14)

### a) Synthesis of (3-chloro-4-methoxy-5-nitro-phenyl)-(2,3-dihydro-pyrido[4,3-b][1,4]oxazin-4-yl)-methanone

To phosphorus oxychloride (3.00ml), 3,4-dihydro-2H-pyrido[4,3-b][1,4]oxazine (177mg, 1.30mmol) and 3-chloro-4-methoxy-5-nitro benzoic acid (200mg, 0.87mmol) were added and then stirred at 95 °C for 16 hours. After completion of the reaction, the reaction mixture was poured into ice water and neutralized with saturated solution of sodium hydroxide, and extracted with ethyl acetate. The combined organic layer was dried over anhydrous sodium sulfate

(Na₂SO₄), filtered and evaporated under reduced pressure. The residue was purified by column chromatography on silica eluting with a solvent of dichloromethane:methanol=20:1. The fractions containing the product were collected and evaporated to obtain yellow solid (132mg, 29%).
¹H-NMR(DMSO-d₆, 300MHz); δ = 8.70 (br s, 1H), 8.16 (d, *J*=1.9Hz, 1H), 8.14-8.09 (m, 2H), 6.98 (d, *J*=5.3Hz, 1H), 4.40 (m, 2H), 4.00 (s, 3H), 3.90 (m, 2H).
MS(ESI); 350.1(M⁺+1).

### b) Synthesis of (3-chloro-4-hydroxy-5-nitro-phenyl)-(2,3-dihydro-pyrido[4,3-b][1,4]oxazin-4-yl)-methanone

To a 20ml flask, (3-chloro-4-methoxy-5-nitro-phenyl)-(2,3-dihydro-pyrido[4,3-b][1,4]oxazin-4-yl)-methanone(132mg, 0.38mmol) was dissolved in dichloromethane and then cooled to 0°C. 1.0M Boron tribromide dissolved in dichloromethane (3ml, 3.00mmol) was added thereto and then stirred at 0°C for 10 hours. After completion of the reaction, the concentrated liquid was purified by HPLC. The resulting solid was further purified by recrystallizing from ethanol to obtain the target compound 14, (3-chloro-4-hydroxy-5-nitro-phenyl)-(2,3-dihydro-pyrido[4,3-b][1,4]oxazin-1-yl)-methanone, as white solid (80mg, 63%).
¹H-NMR(DMSO-d₆, 300MHz); δ = 8.45 (br s, 1H), 8.10 (br s, 1H), 7.98 (d, *J*=2.7Hz, 1H), 6.58 (d, *J*=2.7Hz, 1H), 6.98 (d, 1H), 4.38 (m, 2H), 3.96 (m, 2H).
MS(ESI); 336.1(M⁺+1).

### Example 15)

### Synthesis of (3,5-dichloro-4-hydroxy-phenyl)-(2,3-dihydro-pyrido[3,4-b][1,4]ozazin-1-yl)-methanone hydrobromic acid salt (compound 15)

By the same method as in Example 3, (3,5-dichloro-4-methoxy-phenyl)-(2,3-dihydro-pyrido[3,4-b][1,4]oxazin-1-yl)-methanone (114mg, 0.33mmol) was reacted with 1M solution of boron tribromide (3.3ml, 3.3mmol). The target compound, (3,5-dichloro-4-hydroxy-phenyl)-(2,3-dihydro-pyrido[3,4-b][1,4]oxazin-1-yl)-methanone hydrobromic acid salt, was purified by recrystallizing from ethanol to obtain brown solid (19.7mg, 14%).
¹H-NMR(DMSO-d₆, 300MHz); δ = 8.63 (s, 1H), 8.25 (d, *J*=6.5Hz, 1H), 7.89 (d, *J*=6.5Hz, 1H), 7.74 (s, 2H), 4.44 (m, 2H), 3.98 (m, 2H).
MS(ESI); 325.1(M⁺+1).

### Example 16)

### Synthesis of (3-bromo-4-hydroxy-phenyl)-(2,3-dihydro-pyrido[3,4-b][1,4]oxazin-1-yl)-methanone hydrobromic acid salt (compound 16)

By the same method as in Example 3, (3-bromo-4-methoxy-phenyl)-(2,3-dihydro-pyrido[3,4-b][1,4]oxazin-1-yl)-methanone (68mg, 0.19mmol) was reacted with 1M solution of boron tribromide (1.9ml, 1.9mmol). The target compound, (3-bromo-4-hydroxy-phenyl)-(2,3-dihydro-pyrido[3,4-b][1,4]oxazin-1-yl)-methanone hydrobromic acid salt, was purified by recrystallizing from ethanol to obtain brown solid (48.6mg, 60%).
¹H-NMR(DMSO-d₆, 300MHz); δ = 11.2 (s, 1H), 8.63 (s, 1H), 8.30 (d, *J*=6.9Hz, 1H), 7.88 (d, *J*=1.8Hz, 1H), 7.83 (d, *J*=6.6Hz, 1H), 7.58 (dd, *J*=8.3, 1.8Hz, 1H), 7.05 (d, *J*=8.3Hz, 1H), 4.44 (m, 2H), 3.98 (m, 2H).
MS(ESI); 335.1(M⁺+1).

### Example 17)

### Synthesis of (3-chloro-4-hydroxy-5-nitro-phenyl)-(2,3-dihydro-pyrido[3,4-b][1,4]oxazin-1-yl)-methanone hydrobromic acid salt (compound 17)

### a) Synthesis of (3-chloro-4-methoxy-5-nitro-phenyl)-(2,3-dihydro-pyrido[3,4-b][1,4]oxazin-1-yl)-methanone

To a 10ml flask, 2,3-dihydro-1H-pyrido[3,4-b][1,4]oxazine (57mg, 0.42mmol) and 3-chloro-4-methoxy-5-nitro-benzoyl chloride (117mg, 0.47mmol) were dissolved in dichloromethane. Triethylamine (0.29ml, 2.1mmol) was added thereto and then stirred at room temperature for 2 hours. After neutralizing with 1N hydrochloric acid solution, the reaction mixture was extracted with dichloromethane. The combined organic layer was dried over anhydrous sodium sulfate (Na₂SO₄) and filtered and the solvent was removed under reduced pressure. The residue was purified by column chromatography on silica with a solvent of ethyl acetate:n-hexane=1:2. The fractions containing the product were collected and evaporated to obtain yellow solid (118mg, 81%).
¹H-NMR(DMSO-d₆, 300MHz); δ = 8.24 (s, 1H), 8.19 (d, *J*=2.1Hz,1H), 8.14 (d, *J*=1.8Hz, 1H), 8.00 (d, *J*=6.0Hz, 1H), 7.61 (d, *J*=5.1Hz, 1H), 4.34 (m, 2H), 3.99 (s, 3H), 3.88 (m, 2H).

### b) Synthesis of (3-chloro-4-hydroxy-5-nitro-phenyl-(2,3-dihydro-pyrido-3,4-b][1,4]oxazin-1-yl)-methanone hydrobromic acid salt

To a 20ml flask, (3-chloro-4-methoxy-5-nitro-phenyl)-(2,3-dihydro-pyrido[3,4-b][1,4]oxazin-1-yl)-methanone (109mg, 0.31mmol) was dissolved in dichloromethane and then cooled to 0 °C. 1.0M Boron tribromide dissolved in dichloromethane (3.1ml, 3.1mmol) was added thereto and then stirred at 0 °C for 18 hours. The reaction was quenched by using a small amount of water and then evaporated under reduced pressure. The resulting residue was purified by recrystallizing from methanol to obtain yellow solid (67mg, 52%).
¹H-NMR(DMSO-d₆, 300MHz); δ = 8.67 (s, 1H), 8.27 (d, *J*=6.9Hz, 1H), 8.24 (d, *J*=2.1Hz, 1H), 8.08 (d, *J*=1.8Hz, 1H), 7.98 (d, *J*=6.6Hz, 1H), 4.45 (m, 2H), 3.99 (m, 2H).
MS(ESI); 336.0(M⁺+1).

### Example 18)

### Synthesis of (3-chloro-4-hydroxy-phenyl)-(2,3-dihydro-pyrido[3,4-b][1,4]ozazin-1-yl) methanone hydrobromic acid salt (compound 18)

By the same method as in Example 3, (3-chloro-4-methoxy-phenyl)-(2,3-dihydro-pyrido[3,4-b][1,4]oxazin-1-yl)-methanone(100mg, 0.328mmol) was reacted with 1M solution of boron tribromide (3.28ml, 3.28mmol) to obtain the target compound, (3-chloro-4-hydroxy-phenyl)-(2,3-dihydro-pyrido[3,4-b][1,4]oxazin-1-yl)-methanone hydrobromic acid salt, as white solid (45mg, 37%).
¹H-NMR(DMSO-d₆, 300MHz); δ = 11.17 (s, 1H), 8.65 (s, 1H), 8.25 (d, *J*=6.4Hz, 1H), 7.85 (d, *J*=6.4Hz, 1H), 7.75 (d, *J*=2.1Hz 1H), 7.55 (dd, *J*=8.4, 2.1 Hz, 1H), 7.06 (d, *J*=8.4Hz, 1H), 4.44 (m, 2H), 3.99 (m, 2H).
MS(ESI); 291.1(M⁺+1).

### Example 19)

### Synthesis of (2,3-dihydro-pyrido[3,4-b][1,4]oxazin-1-yl)-(4-hydroxy-3-trifluoromethyl-phenyl)-methanone (compound 19)

By the same method as in the step b) of Example 12, (2,3-dihydro-pyrido[3,4-b][1,4]oxazin-1-yl)-(4-methoxy-3-trifluoromethyl-phenyl)-methanone (110mg, 0.325mmol) was reacted with 1M solution of boron tribromide (3.25ml, 3.25mmol) to obtain the target compound, (2,3-dihydro-pyrido[3,4-b][1,4]oxazin-1-yl)-(4-hydroxy-3-trifluoromethyl-phenyl)-methanone, as white solid (11mg, 10%).
¹H-NMR(CD₃OD, 300MHz); δ = 8.16 (s, H), 7.85 (d, *J*=5.7Hz, 1H), 7.81(d, *J*=1.8Hz, 1H), 7.66 (dd, *J*=8.4, 1.8Hz, 1H), 7.32 (d, *J*=5.7Hz, 1H), 7.00 (d, *J*=8.4Hz, 1H), 4.37 (m, 2H), 3.98 (m, 2H).
MS(ESI); 325.1(M⁺+1).

### Example 20)

### Synthesis of (3,5-dibromo-4-hydroxy-phenyl)-(7-phenyl-2,3-dihydro-pyrido[2,3-b][1,4]oxazin-1-yl)-methanone (compound 20)

### a) Synthesis of 7-phenyl-1H-pyrido[2,3-b][1.4]oxazin-2-one

To a 10ml flask equipped with refluxing apparatus, 7-bromo-1H-pyrido[2,3-b][1,4]oxazin-2-one (48mg, 0.21mmol), phenyl boronic acid (PhB(OH)₂, 28mg, 0.23mmol), triphenylphosphine (PPh₃, 11mg, 0.04mmol), palladium acetate (Pd(OAc)₂, 5mg, 0.02mmol), potassium carbonate (44mg, 0.31mmol) were dissolved in acetonitrile (1.6ml) and water (0.4ml). The mixture was heated to 90°C and then refluxed for 15 hours. The mixture was evaporated and water was added, and then the mixture was extracted with ethyl acetate. The combined organic layer was dried over anhydrous sodium sulfate (Na₂SO₄), filtered and evaporated under reduced pressure. The resulting residue was purified by column chromatography on silica eluting with a solvent of dichloromethane:methanol=30:1. The fractions containing the product were collected and evaporated to obtain pale-brown solid (21mg, 44%).
MS(ESI); 227.1(M⁺+1).

### b) Synthesis of 7-phenyl-2,3-dihydro-1H-pyrido[2.3-b][1,4]oxazine

7-phenyl-1H-pyrido[2,3-b][1,4]oxazin-2-one (21mg, 0.09mmol) was dissolved in tetrahydrofuran (0.5ml) and then cooled to 0°C. 1.0M Lithium aluminum hydride dissolved in tetrahydrofuran (0.19ml, 0.19mmol) was added thereto dropwise. After stirring for 5 hours at room temperature, 0.1ml of water, 0.2ml of 10% aqueous solution of sodium hydroxide, and 0.3ml of water were added in this order under stirring. After filtering precipitates, the filtrate was extracted with ethyl acetate. The combined organic layer was dried over anhydrous sodium sulfate (Na₂SO₄), filtered and evaporated under reduced pressure. The residue was purified by column chromatography on silica eluting with a solvent of dichloromethane:methanol=40:1. The fractions containing the product were collected and evaporated to obtain pale-grey solid (10mg, 50%).
MS(ESI); 213.0(M⁺+1).

### c) Synthesis of (3,5-dibromo-4-methoxy-phenyl)-(7-phenyl-2,3-dihydro-pyrido[2,3-b][1,4]oxazin-1-yl)-methanone

To a 5ml flask, 7-phenyl-2,3-dihydro-1H-pyrido[2,3-b][1,4]oxazine (10mg 0.05mmol) and 3,5-dibromo-4-methoxy-benzoyl chloride (17mg, 0.05mmol) were dissolved in dichloromethane. Triethylamine (0.02ml, 0.14mmol) was added thereto and then stirred at room temperature for 1 hour. After neutralizing with 1N hydrochloric acid solution, the mixture was extracted with dichloromethane and the combined organic layer was dried over anhydrous sodium sulfate (Na₂SO₄), filtered and evaporated under reduced pressure. The residue was purified by column chromatography on silica eluting with a solvent of dichloromethane:methanol=40:1. The fractions containing the product were collected and evaporated to obtain white solid (14mg, 59%).
MS(ESI); 503.0(M⁺+1).

### d) Synthesis of (3,5-dibromo4-hydroxy-phenyl)-(7-phenyl-2,3-dihydro-pyrido[2,3-b][1,4]oxazin-1-yl)-methanone

To a 5ml flask, (3,5-dibromo-4-methoxy-phenyl)-(7-phenyl-2,3-dihydro-pyrido[2,3-b][1,4]oxazin-1-yl)-methanone (14mg, 0.03mmol) was dissolved in dichloromethane and then cooled to 0°C. 1.0M Boron tribromide dissolved in dichloromethane (0.17ml, 0.17mmol) was added and then stirred at 0°C for 15 hours. After completion of the reaction, water was added thereto and the mixture was extracted with ethyl acetate and the combined organic layer was dried over anhydrous sodium sulfate (Na₂SO₄), filtered and evaporated under reduced pressure. The residue was purified by column chromatography on silica eluting with a solvent of dichloromethane:methanol=40:1. The fractions containing the product were collected and evaporated to obtain the target compound 20 as white solid (8.7mg, 64%).
¹H-NMR(DMSO-d₆, 300MHz); δ = 10.53 (br s, 1H), 8.23 (d, 1H, *J*=2.28Hz), 8.02 (s, 1H), 7.79 (s, 2H), 7.43-7.32 (m, 5H), 4.45 (m, 2H), 3.92 (m, 2H).
MS(ESI); 488.9(M⁺+1).

### Example 21)

### Synthesis of 2,6-dichloro-4-(2,3-dihydro-pyrido[4,3-b][1,4]oxazin-4-sulfonyl)-phenol (compound 21)

To a 5ml flask, 3,4-dihydro-2H-pyrido[4,3-b][1,4]oxazine(30mg, 0.220mmol) was dissolved in tetrahydrofuran (3ml). Diisopropylethylamine (0.153ml, 0.878mmol) was added and then stirred at 0°C for 10 minutes, and then 3,5-dichloro-4-hydroxy-benzenesulfonyl chloride (69mg, 0.264mmol) was added thereto and then stirred at 0°C for 1 hour. The reaction mixture was evaporated under reduced pressure. The residue was purified by column chromatography on silica eluting with a solvent of dichloromethane:ethyl acetate=1:2. The fractions containing the product were collected and evaporated to obtain the target compound 21 as pale-yellow solid (2.7mg, 3.4%).
¹H-NMR(CO₃OD, 300MHz); δ = 8.80 (s, 1H), 8.12 (d, *J*=5.3Hz, 1H), 7.38 (s, 2H), 6.90 (d, *J*=5.3Hz, 1H), 3.86-3.84 (br s, 4H).
MS(ESI); 361.0(M⁺+1).

### Example 22)

### Synthesis of (3,5-dibromo-4-hydroxy-phenyl)-(7-trifluoromethyl-2,3-dihydro-pyrido[2,3-b][1,4]oxazin-1-yl)-methanone (compound 22-2)

### a) Synthesis of 2-chloro-3-nitro-5-trifluoromethyl-pyridine

In a 25ml flask, 3-nitro-5-trifluoromethyl-pyridin-2-ol (300mg, 1.44mmol) was dissolved in acetonitrile (4.5ml), and then phosphorus oxychloride (0.4ml, 4.33mmol) and benzyltrimethyl ammonium chloride (164mg, 0.721mmol) were added thereto. The mixture was stirred for 3 hours at 80°C. After completion of the reaction, water was added thereto and the mixture was extracted with ethyl acetate. The combined organic layer was dried over anhydrous sodium sulfate (Na₂SO₄), filtered and evaporated under reduced pressure to obtain white solid (300mg, 92%).
¹H-NMR(DMSO-d₆, 300MHz); δ = 9.21 (s, 1H), 9.08 (s, 1H).

### b) Synthesis of (3-nitro-5-trifluoromethyl-pyridin-2-yloxy)-acetic acid methyl ester

In a 10ml flask, hydroxy-acetic acid methyl ester (0.4ml, 0.53mmol) was dissolved in tetrahydrofuran (1.5ml), and then sodium hydride (23mg, 0.57mmol) was added thereto. After 40 minutes, 2-chloro-3-nitro-5-trifluoromethyl-pyridine (100mg, 0.44mmol) was added and then stirred for 2 hours at room temperature. After completion of the reaction, water was added thereto and the mixture was extracted with ethyl acetate. The combined organic layer was dried over anhydrous sodium sulfate (Na₂SO₄), filtered and evaporated under reduced pressure. The residue was purified by column chromatography on silica eluting with a solvent of n-hexane:ethyl acetate=9:1. The fractions containing the product were collected and evaporated to obtain white solid (84mg, 68%).
¹H-NMR(DMSO-d₆, 300MHz); δ = 8.95 (s, 1H), 8.92 (s, 1H), 5.23 (s, 2H), 3.69 (s, 3H).

### c) Synthesis of 7-trifluoromethyl-1H-pyrido[2.3-b][1,4]oxazin-2-one

In a 10ml flask, (3-nitro-5-trifluoromethyl-pyridin-2-yloxy)-acetic acid methyl ester (84mg, 0.30mmol) was dissolved in concentrated hydrochloric acid (1.5ml) and then tin tetrachloride (227mg, 1.2mmol) was added thereto. The mixture was stirred for 30 minutes at 80°C. After completion of the reaction, water was added thereto at room temperature and the mixture was extracted with a solvent of methanol: dichloromethane=1:9. The combined organic layer was dried over anhydrous sodium sulfate (Na₂SO₄), filtered and evaporated under reduced pressure. The residue was purified by column chromatography on silica eluting with a solvent of methanol:dichloromethane=1:50. The fractions containing the product were collected and evaporated to obtain white solid (40mg, 62%).
¹H-NMR(DMSO-d₆, 300MHz); δ = 11.1 (s, 1H), 8.19 (s, 1H), 7.40 (s, 1H), 4.91 (s, 2H), MS(ESI); 219(M⁺+1).

### d) Synthesis of 7-trifluoromethyl-2,3-dihydro-1H-pyrido[2,3-b][1,4]oxazine

In a 5ml flask, 7-trifluoromethyl-1H-pyrido[2,3-b][1,4]oxazin-2-one (60mg, 0.28mmol) was dissolved in tetrahydrofuran (1.0ml), and then 1.0M Lithium aluminum hydride dissolved in tetrahydrofuran (0.33ml, 0.33mmol) was added thereto dropwise and stirred for 1 hour at 0°C. Water and saturated solution of sodium hydrogen carbonate were added and the mixture was extracted with ethyl acetate. The combined organic layer was dried over anhydrous sodium sulfate (Na₂SO₄), filtered and evaporated under reduced pressure. The residue was purified by column chromatography on silica eluting with a solvent of methanol:dichloromethane=1:50. The fractions containing the product were collected and evaporated to obtain white solid (29mg, 52%).
¹H-NMR(DMSO-d₆, 300MHz); δ = 7.69 (br s, *J*=2.29Hz, 1H), 7.10 (br s, *J=*2.29Hz, 1H), 6.48-6.54 (m, 1H), 4.31-4.37 (m, 2H), 3.33-3.39 (m, 2H).
MS(ESI); 205(M⁺+1).

### e) Synthesis of (3,5-dibromo-4-methoxy-phenyl)-(7-trifluoromethyl-2,3-dihydro-pyrido[2,3-b][1,4]oxazin-1-yl)-methanone (compound 22-1)

In a 5ml flask, 7-trifluoromethyl-2,3-dihydro-1H-pyrido[2,3-b][1,4]oxazine (27mg, 0.23mmol) and 3,5-dibromo-4-methoxy-benzoyl chloride (65mg, 0.20mmol) were dissolved in dichloromethane (0.6ml), and then triethylamine (0.55ml, 0.33mmol) was added thereto dropwise at 0°C. After stirring for 4 hours at room temperature, water was added and the mixture was extracted with ethyl acetate. The combined organic layer was dried over anhydrous sodium sulfate (Na₂SO₄), filtered and evaporated under reduced pressure. The residue was purified by column chromatography on silica eluting with a solvent of methanol:dichloromethane=1:100. The fractions containing the product were collected and evaporated to obtain white solid (64mg, 98%).
H-NMR(DMSO-d₆, 300MHz); δ = 8.37 (s, 2H), 7.91 (s, 2H), 4.46-4.53 (m, 2H), 3.86-3.92 (m, 2H), 3.84 (s, 3H).
MS(ESI); 494.8(M⁺+1).

### f) Synthesis of (3,5-dibromo-4-hydroxy-phenyl)-(7-trifluoromethyl-2,3-dihydro-pyrido[2,3-b][1,4]oxazin-1-yl)-methanone (compound 22-2).

In a 5ml flask, (3,5-dibromo-4-methoxy-phenyl)-(7-trifluoromethyl-2,3-dihydro-pyrido[2,3-b][1,4]oxazin-1-yl)-methanone (30mg, 0.061mmol) was dissolved in N,N-dimethyl formamide (0.3ml), and then lithium bromide (10mg, 0.12mmol) and piperazine (7.8mg, 0.091mmol) were added thereto. After stirring for 1 hour, the solvent was removed by evaporation under reduced pressure. The residue was neutralized with water and 1N hydrochloric acid. The resulting solid was filtered to obtain the target compound 22-2 as white solid (25mg, 86%).
¹H-NMR(DMSO-d₆, 300MHz); δ = 10.7 (s, 1H), 8.34 (s, 1H), 8.29 (s, 1H), 7.79 (s, 2H), 4.45-4.50 (m, 2H), 3.86-3.93 (m, 2H).
MS(ESI); 480.8(M⁺+1).

### Example 23)

### Synthesis of 2,5-dibromo-4-(2,3-dihydro-pyrido[4,3-b][1,4]oxazin-4-carbonyl)-benzoic acid (compound 23)

In a 20ml flask, 2,5-dibromo-terephthalic acid (100mg, 0.31mmol) and N,N-dimethyl formamide (3 drops) were dissolved in thionyl chloride (2ml). After stirring for 2 hours at 80°C, the reaction mixture was cooled to room temperature and concentrated. Crude 2,5-dibromo-terephthaloic dichloride (112mg, 0.31mmol) was dissolved in dichloromethane (6ml) and was added to 3,4-dihydro-2H-pyrido[4,3-b][1,4]oxazine (20.9mg, 0.15mmol) dissolved in dichloromethane (1ml). Triethylamine (3 drops) were slowly added thereto and stirred at room temperature for 16 hours. After completion of the reaction, the residue was purified by preparative LC/Mass to obtain the target compound 23, i.e., 2,5-dibromo-4-(2,3-dihydro-pyrido[4,3-b][1,4]oxazin-4-carbonyl)-benzoic acid, as white solid (23mg, 5%).
¹H-NMR(CD₃OD, 300MHz); δ = 9.70 (s, 1H), 8.47 (d, *J*=7.2Hz, 1H), 7.83 (d, *J*=12.6Hz, 2H), 7.58 (d, *J*=6.9Hz, 1H), 4.72 (m, 2H), 4.12-3.88 (m, 2H).
MS(ESI); 440.9(M⁺+1).

### Example 24)

### Synthesis of [2,6-dibromo-4-(2,3-dihydro-pyrido[4,3-b][1,41oxazin4-carbonyl)-phenoxyl-acetic acid methyl ester (compound 24)

In a 25ml flask, (3,5-dibromo-4-hydroxy-phenyl)-(2,3-dihydro-pyrido[4,3-b][1,4]oxazin-4-yl)-methanone (100mg, 0.24mmol) was dissolved in N,N-dimethyl formamide (2.4ml), and potassium carbonate (37mg, 0.266mmol) was added thereto dropwise at room temperature. After stirring at room temperature for 10 minutes, bromo-methyl acetate was added dropwise and then stirred for 40 minutes at room temperature. The reaction mixture was filtered on celite and evaporated under reduced pressure. The residue was purified by column chromatography on silica eluting with a solvent of dichloromethane:methanol=20:1 to obtain the target compound 24 as white foam (48mg, 41.0%).
¹H-NMR(DMSO-d₆, 300MHz); δ = 8.63 (br s, 1H), 8.11(d, *J*=5.7Hz, 1H), 7.90 (s, 2H), 6.97 (d, *J*=5.7Hz, 1H), 4.70 (s, 2H), 4.42-4.37 (m, 2H), 3.90-3.85 (m, 2H), 3.75 (s, 3H).
MS(ESI); 484.9 (M⁺+1).

### Example 25)

### Synthesis of (7-bromo-23-dihydro-pyrido[2,3-b][1,4]oxazin-1-yl)-(3,5-dibromo-4-hydroxyphenyl)-methanone (compound 25)

### a) Synthesis of 7-bromo-2,3-dihydro-1H-pyrido[2,3-b][1.41oxazine

To a 10ml flask, 7-bromo-2,3-dihydro-1H-pyrido[2,3-b][1,4]oxazin-2-one (100mg, 0.436mmol) was dissolved in tetrahydrofuran (1.0ml). To the reaction mixture, 1.0M Lithium aluminum hydride dissolved in tetrahydrofuran (0.524ml, 0.524mmol) was added dropwise at 0°C, and then stirred for 20 minutes. After completion of the reaction by adding water to the reaction mixture, the organic layer was extracted with ethyl acetate and the combined organic layer was washed with water and saturated saline solution, dried over anhydrous sodium sulfate (Na₂SO₄), filtered and evaporated under reduced pressure. The residue was recrystallized from dichloromethane and diisopropyl ether to obtain white solid (63mg, 67%).
¹H-NMR(CDCl₃, 300MHz); δ = 7.38 (d, *J*=2.4Hz, 1H), 7.01 (d, *J*=2.1Hz, 1H), 6.37 (s, 1H), 4.22 (m, 2H), 3.25 (m, 2H).
MS(ESI); 215(M⁺+1).

### b) Synthesis of (7-bromo-2,3-dihydro-pyrido[2,3-b][1,4]oxazin-1-yl)-(3,5-dibromo-4-methoxy-phenyl)-methanone

To a 10ml flask, 7-bromo-2,3-dihydro-1H-pyrido[2,3-b][1,4]oxazine (37mg, 0.172mmol) and 3,5-dibromo-4-methoxy-benzoyl chloride (113mg, 0.344mmol) were dissolved in dichloromethane (0.5ml). Triethylamine (0.120ml, 0.860mmol) was added thereto and then stirred at room temperature for 1 hour. After completion of the reaction by adding water to the reaction mixture, the organic layer was extracted with dichloromethane and the combined organic layer was washed with water and saturated saline solution, dried over anhydrous sodium sulfate (Na₂SO₄), filtered and evaporated under reduced pressure. The residue was purified by column chromatography on silica eluting with a solvent of dichloromethane:ethyl acetate:n-hexane=1:2:5. The fractions containing the product were collected and evaporated to obtain white solid (55mg, 63%).
¹H-NMR(CDCl₃, 300MHz); δ = 8.14 (s, 1H), 8.08 (d, *J*=2.1Hz, 1H), 7.82 (s, 2H), 4.41 (m, 2H), 3.95 (s, 3H), 3.90 (m, 2H).
MS(ESI); 504.8(M⁺+1).

### c) Synthesis of (7-bromo-2,3-dihydro-pyrido[2,3-b][1,4]oxazin-1-yl)-(3.5-dibromo-4-hydroxy-phenyl)-methanone

To a 100ml flask, (7-bromo-2,3-dihydro-pyrido[2,3-b][1,4]oxazin-1-yl)-(3,5-dibromo-4-methoxy-phenyl)-methanone (55mg, 0.108mmol) was dissolved in dichloromethane and then cooled to 0°C. 1.0M Boron tribromide dissolved in dichloromethane (1.63ml, 1.63mmol) was added thereto and stirred at room temperature for 10 hours. n-hexane and water were added and the resulting solid was filetered. And then the crude was purified by recrystallizing from dichloromethane to obtain white solid as TFA salt. The resulting solid was dissolved in dichloromethane and then washed with 10% aqueous solution of sodium hydrogen carbonate, water and saturated saline solution. The combined organic layer was dried over anhydrous sodium sulfate (Na₂SO₄), filtered and evaporated under reduced pressure to obtain the target compound 25 as white solid (16.6mg, 31 %).
¹H-NMR(CDCl₃, 300MHz); δ = 10.66 (s, 1H), 8.19 (s, 1H), 8.05 (d, *J*=2.4Hz, 1H), 7.78 (s, 2H), 4.36 (m, 2H), 3.83 (m, 2H).
MS(ESI); 490.7(M⁺+1).

### Example 26)

### Synthesis of (2,3-dihydro-pyrido[4,3-b][1,4]oxazin-4-yl)-(3-fluoro-4-hydroxy-phenyl)-methanone (compound 26)

### a) Synthesis of (2,3-dihydro-pyrido[4,3-b][1,4]oxazin-4-yl)-(3-fluoro-4-methoxy-phenyl)-methanone

In a 10ml flask, 3,4-dihydro-2H-pyrido[4,3-b][1,4]oxazine (50mg, 0.441mmol) and 3-fluoro-4-methoxy-benzoyl chloride (125mg, 0.661mmol) were dissolved in dichloromethane. Triethylamine (0.77ml, 1.288mmol) was added thereto and then stirred at room temperature for 2 hours. After neuralizing with 1N hydrochloric acid solution, the mixture was extracted with dichloromethane and the combined organic layer was dried over anhydrous sodium sulfate (Na₂SO₄), filtered and evaporated under reduced pressure. The residue was purified by column chromatography on silica eluting with a solvent of ethyl acetate. The fractions containing the product were collected and evaporated to obtain white solid (42.1mg, 33%).
¹H-NMR(CDCl₃, 300MHz); δ = 8.15 (s, 1H), 8.11 (d, *J*=5.7Hz, 1H), 7.31 (m, 1H), 7.27 (d, *J*=3.8Hz, 1H), 6.92-6.98 (m, 1H), 6.85 (d, *J*=5.7Hz, 1H), 4.43-4.46 (m, 2H), 4.02-4.05 (m, 2H), 3.92 (s, 3H).
MS(ESI); 289.1(M⁺+1).

### b) Synthesis of (2,3-dihydro-pyrido[4,3-b][1,4]oxazin-4-yl)-(3-fluoro-4-hydroxy-phenyl)-methanone

In a 10ml flask, (2,3-dihydro-pyrido[4,3-b][1,4]oxazin-4-yl)-(3-fluoro-4-methoxy-phenyl)-methanone (30mg, 0.104mmol) was dissolved in dichloromethane and the mixture was cooled to 0°C. 1.0M Boron tribromide dissolved in dichloromethane (1.25ml, 1.25mmol) was added thereto and stirred at room temperature for 10 hours. n-hexane and water were added and then filetered to obtain solid. The resulting solid was purified by preparative LC-Mass using 0.05% TFA in acetonitrile and 0.05% TFA in water solvent. The fractions containing the product were collected and adjusted to pH 6 to 7 with saturated solution of sodium hydrogen carbonate. The organic layer was extracted with ethyl acetate. The combined organic layer was evaporated under reduced pressure to obtain the target compound 26 as white solid (9mg, 28%).
¹H-NMR(DMSO-d₆, 300MHz); δ = 8.37 (s, 1H), 8.05 (d, *J*=5.3Hz, 1H), 7.39 (d, *J*=11.8Hz, 1H), 7.25 (d, *J*=8.3Hz, 1H), 6.97 (m, 2H), 4.36-4.39 (m, 2H), 3.93-3.90 (m, 2H).
MS(ESI); 275.2(M⁺+1).

### Example 27)

### Synthesis of (3,5-dibromo-4-hydroxy-phenyl)-(7-methyl-2,3-dihydro-pyrido[2,3-b][1,4]oxazin-1-yl)-methanone (compound 27-2)

### a) Synthesis of (3,5-dibromo-4-methoxy-pheny)-(7-methyl-2,3-dihydro-pyrido[2,3-b][1,4]oxazin-1-yl)-methanone (compound 27-1)

In a 10ml flask, 7-methyl-2,3-dihydro-1H-pyrido[2,3-b][1,4]oxazine (79mg, 0.52mmol) and 3,5-dibromo-4-methoxy-benzoyl chloride (259mg, 0.79mmol) weres dissolved in dichloromethane (2ml). Triethylamine (0.22ml, 1.58mmol) was added thereto and then stirred at room temperature for 2 hours. After neuralizing with 1N hydrochloric acid solution, the mixture was extracted with dichloromethane and the combined organic layer was dried over anhydrous sodium sulfate (Na₂SO₄), filtered and evaporated under reduced pressure. The residue was purified by column chromatography on silica eluting with a solvent of methanol:dichloromethane=1:30. The fractions containing the product were collected and evaporated to obtain white solid (210mg, 90%).
¹H-NMR(CDCl₃, 300MHz); δ = 8.25 (s, 1H), 7.87 (d, *J*=1.5Hz, 1H), 7.68 (s, 1H), 4.45-4.42 (m, 2H), 3.95-3.91 (m, 2H), 3.94 (s, 3H), 2.21 (s, 3H).
MS(ESI); 439.9(M⁺+1).

### b) Synthesis of (3,5-dibromo-4-hydroxy-phenyl)-(7-methyl-2,3-dihydro-pyrido[2,3-b][1,4]oxazin-1-yl)-methanone (compound 27-2)

In a 5ml flask, (3,5-dibromo-4-methoxy-phenyl)-(7-methyl-2,3-dihydro-pyrido[2,3-b][1,4]oxazin-1-yl)-methanone (100mg, 0.23mmol), lithium bromide (39mg, 0.45mmol), piperazine (30mg, 0.35mmol) and N,N-dimethyl formamide (1ml) were added and then stirred at 100°C for 30 minutes. Lithium bromide (39mg, 0.45mmol) was further added thereto and then stirred at 100°C for 1 hour. Lithium bromide (39mg, 0.45mmol) was further added thereto and then stirred for 1 hour. After neuralizing with saturated solution of sodium hydrogen carbonate to pH 8 to 9, the mixture was adjusted to pH 6 again by using 1N hydrochloric acid. The formed solid was filtered. The resulting solid was purified by column chromatography on silica eluting with a solvent of methanol:dichloromethane=1:30. The fractions containing the product were collected and evaporated to obtain the target compound 27-2 as white solid (74mg, 76%).
¹H-NMR(DMSO-d₆, 300MHz); δ = 10.61 (br s, 1H), 7.77-7.75 (m, 4H), 4.35-4.32 (m, 2H), 3.85-3.82 (m, 2H), 2.15 (s, 3H).
MS(ESI); 426.8(M⁺+1).

### Example 28)

### Synthesis of (3,5-difluoro-4-methoxy-phenyl)-(2,3-dihydro-pyrido[4,3-b][1,4]oxazin-4-yl)-methanone (compound 28)

In a 10ml flask, 3,4-dihydro-2H-pyrido[4,3-b][1,4]oxazine (60mg, 0.44mmol) and 3,5-difluoro-4-methoxy-benzoyl chloride (120mg, 0.572mmol) were dissolved in dichloromethane (1ml). Triethylamine (0.080ml, 0.57mmol) was added thereto and stirred at room temperature for 1 hour. After completion of the reaction by adding water to the reaction mixture, the organic layer was extracted with dichloromethane, and the combined organic layer was washed with water and saturated saline solution, dried over anhydrous sodium sulfate (Na₂SO₄), filtered and evaporated under reduced pressure. The residue was purified by column chromatography on silica eluting with a solvent of dichloromethane:ethyl acetate:n-hexane=1:2:5. The fractions containing the product were collected and evaporated to obtain the target compound 28 as white solid (102mg, 76%).
¹H-NMR(CDCl₃, 300MHz); δ = 8.17 (br s, 1H), 8.14 (d, *J*=5.7Hz, 1H), 7.08 (d, *J*=7.2Hz, 2H), 6.85(d, *J*=5.7Hz, 1H), 4.43 (m, 2H), 4.05 (s, 3H), 4.01 (m, 2H).
MS(ESI); 307.2(M⁺+1).

### Example 29)

### Synthesis of (3,5-difluoro-4-hydroxy-phenyl)-(2,3-dihydro-pyrido[4,3-b][1,4]oxazin-4-yl)-methanone (compound 29)

In a 10ml flask, (3,5-difluoro-4-methoxy-phenyl)-(2,3-dihydro-pyrido[4,3-b][1,4]oxazin-4-yl)-methanone (50g, 0.163mmol), lithium bromide (57mg, 0.65mmol) and piperazine (21mg, 0.24mmol) were dissolved with N,N-dimethyl formamide, and stirred at 100°C for 3 hours. After completion of the reaction by adding water dropwise, the mixture was adjusted to weak acidic condition (pH=6) by using 1N hydrochloric acid, and the formed solid was filtered and washed with distilled water and ethyl acetate. The resulting solid was recrystallized from methanol to obtain the target compound 29, i.e., 3,5-difluoro-4-hydroxy-phenyl)-(2,3-dihydro-pyrido[4,3-b][1,4]oxazin-4-yl)-methanone (31mg, 65%).
¹H-NMR(DMSO-d₆, 300MHz); δ = 10.9 (br s, 1H), 8.45 (br s, 1H), 8.01 (m, 1H), 7.81 (d, *J*=7.2Hz, 2H), 6.95 (d, *J*=5.34 Hz, 1H), 4.38 (m, 2H), 3.91 (m, 2H).
MS(ESI); 293.1 (M⁺+1).

### Example 30)

### Synthesis of (5-chloro-2,3-dihydro-pyrido[4,3-b][1,4]oxazin-4-yl)-(3,5-dibromo-4-hydroxyphenyl)-methanone (compound 30)

### a) Synthesis of (5-chloro-2,3-dihydro-pyrido[4,3-b][1,4]oxazin-4-yl)-(3,5-dibromo-4-methoxyphenyl)-methanone

In a 50ml flask equipped with a reflux apparatus, 5-chloro-3,4-dihydro-2H-pyrido[4,3-b][1,4]oxazine (890mg, 5.22mmol) was dissolved in butyl ether (20ml), and 60% sodium hydride in mineral oil (250mg, 6.26mmol) was added and stirred at 125°C for 3.5 hours. The mixture was cooled to room temperature and 3,5-dibromo-4-methoxy-benzoyl chloride (2.06g, 6.26mmol) was added thereto and then stirred at 125°C for 24 hours. The mixture was cooled to room temperature and filtered and evaporated under reduced pressure. The residue was washed with methanol and dried under reduced pressure to obtain white solid (1.2g, 50%).
¹H-NMR(CDCl₃, 300MHz); δ = 8.09 (d, *J*=5.7Hz, 1H), 7.78 (s, 2H), 6.86 (d, *J*=5.7Hz, 1H), 4.47-4.31 (m, 2H), 4.02-3.82 (m, 2H), 3.93 (s, 3H).
MS(ESI); 461(M⁺+1).

### b) Synthesis of (5-chloro-2,3-dihydro-pyrido[4,3-b][1,4]oxazin-4-yl)-(3,5-dibromo-4-hydroxy-phenyl)-methanone

In a 5ml flask equipped with a reflux apparatus, (5-chloro-2,3-dihydro-pyrido[4,3-b][1,4]oxazin-4-yl)-(3,5-dibromo-4-methoxy-phenyl)-methanone (48mg, 0.1mmol) was dissolved in dichloroethane (0.5ml) and chloroform (0.5ml). 1.0M Boron tribromide dissolved in dichloromethane (1ml, 1mmol) was added thereto dropwise at room temperature. The reaction mixture was stirred at 45°C for 18 hours. After cooling to room temperature, methanol (1ml) was added and then stirred at room temperature for 10 minutes. After evaporating under reduced pressure, dichloromethane was added and then stirred. The reaction mixture was filtered to obtain the target compound 30 as white solid (24mg, 54%).
¹H-NMR(DMSO-d₆, 300MHz); δ = 10.80 (br s, 1H), 8.06 (d, *J*=5.7Hz, 1H), 7.78 (s, 2H), 7.04 (d, *J*=5.7Hz, 1H), 4.55-4.16 (m, 2H), 4.04-3.91 (m, 2H).
MS(ESI); 447.0(M⁺+1).

### Example 31)

### Synthesis of (2,6-dichloro-pyridin-4-yl)-(2,3-dihydro-pyrido[4,3-b][1,4]oxazin-4-yl) methanone (compound 31)

In a 25ml flask, 2,6-dichloro-isonicotinic acid (100mg, 0.52 mmol) was dissolved in thionylchloride (0.76ml), and a catalytic amount of N,N-dimethyl formamide was added thereto and then stirred for 4 hours under reflux. Thionyl chloride was removed under reduced pressure, and 3,4-dihydro-2H-pyrido[4,3-b][1,4]oxazine (78mg, 0.57mmol) and pyridine (0.13ml) were added to the reaction flask and dissolved in N,N-dimethyl acetamide (2.6ml) and then stirred at room temperature for 18 hours. The reaction mixture was extracted with dichloromethane, and the combined organic layer was dried over anhydrous magnesium sulfate (MgSO₄) and evaporated under reduced pressure. The residue was purified by column chromatography on silica eluting with a solvent of n-hexane:ethyl acetate=2:1. The fractions containing the product were collected and evaporated to obtain the target compound 31 as yellow solid (116mg, 72%).
¹H-NMR(DMSO-d₆, 300MHz); 8 = 9.18 (br s, 1H), 8.15 (d, *J*=5.7Hz, 1H), 7.81 (s, 2H), 6.99 (d, *J*=5.7Hz, 1H), 4.55-4.25 (m, 2H), 3.94-3.86 (m, 2H).
MS(ESI); 310.1 (M⁺+1).

### Example 32)

### Synthesis of (2,3-dihydro-pyrido[4,3-b][1,4]oxazin-4-yl)-(6-hydroxy-pyridin-3-yl)-methanone (compound 32)

In a 25ml flask, 6-hydroxy-nicotinic acid (102mg, 0.73mmol) was dissolved in N,N-dimethyl acetamide (2.7ml) and then cooled to -15°C. Thionyl chloride (0.06ml) was added thereto and the temperature was slowly raised up to -5°C and then stirred at -5°C for 30 minutes. The mixture was cooled again to -15°C and then 3,4-dihydro-2H-pyrido[4,3-b][1,4]oxazine (100mg, 0.73mmol) and potassium carbonate (127mg, 0.82 mmol) were added thereto and stirred for 10 minutes. The temperature was raised up to room temperature and then stirred for 14 hours. Ethyl acetate was added to the reaction mixture, and the mixture was neutralized with saturated solution of sodium hydrogen carbonate and evaporated under reduced pressure. The residue was purified by column chromatography on amine silica eluting with a solvent of dichloromethane:methanol=50:1. The fractions containing the product were collected and evaporated to obtain the target compound 32 as white solid (86mg, 45%).
¹H-NMR(DMSO-d₆, 300MHz); δ = 12.0 (br s, 1H), 8.43 (s, 1H), 8.06 (d, *J*=5.7Hz, 1H), 7.77 (d, *J*=2.1Hz, 1H), 7.56 (dd, *J*=2.7Hz, 9.3Hz, 1H), 6.94 (d, *J*=5.7Hz, 1H), 6.34 (d, *J*=9.3Hz, 1H), 4.45-4.25 (m, 2H), 4.00-3.92 (m, 2H).
MS(ESI); 258.1 (M⁺+1).

### Example 33)

### Synthesis of (3,5-dibromo-4-hydroxy-phenyl)-(2,3-dihydro-pyrido[4,3-b][1,4]oxazin-4-yl)-methanone hydrochloric acid salt (compound 33)

In a 25ml flask, (3,5-dibromo-4-hydroxy-phenyl)-(2,3-dihydro-pyrido[4,3-b][1,4]oxazin-4-yl)-methanone (510mg, 1.23mmol) and tetrahydrofuran (40ml) were added and stirred at 75°C for 20 minutes. 4.0M Hydrochloric acid dissolved in 1,4-dioxane (0.34ml, 1.36mmol) was added thereto dropwise and then stirred at 75°C for 10 minutes. The reaction mixture was filtered in hot state without cooling and washed with ether, and the resulting solid was dried under vacuum at 60°C for 12 hours to obtain the target compound 33 as white solid (520mg, 93.7%).
¹H-NMR(DMSO-d₆, 300MHz); δ = 8.88 (s, 1H), 8.36 (d, *J*=6.4Hz, 1H), 7.80 (s, 2H), 7.39 (d, *J*=6.3Hz, 1H), 4.54-4.51 (m, 2H), 3.98-3.95 (m, 2H).
MS(ESI); 413.1 (M⁺+1).

### Example 34)

### Synthesis of (3-chloro-4-hydroxy-phenyl)-(2,3-dihydro-pyrido[3,4-b][1,4]oxazin-1-yl)-methanone (compound 34)

### a) Synthesis of (3-chloro-4-methoxy-phenyl)-(2,3-dihydro-pyrido[3,4-b][1,4]oxazin-1-yl)-methanone

In a 10ml flask, 2,3-dihydro-1H-pyrido[3,4-b][1,4]oxazine (60mg, 0.441mmol) and 3-chloro-4-methoxy-benzoyl chloride (108mg, 0.529mmol) were dissolved in dichloromethane (3ml). Triethylamine (0.12ml, 0.881mmol) was added thereto and then stirred at room temperature for 2 hours. After neutralizing with 1N hydrochloric acid solution, the mixture was extracted with dichloromethane, and the combined organic layer was dried over anhydrous sodium sulfate (Na₂SO₄), filtered and evaporated under reduced pressure. The residue was purified by column chromatography on silica eluting with a solvent of ethyl acetate:n-hexane=1:1. The fractions containing the product were collected and evaporated to obtain white solid (134mg, 99.8%).
¹H-NMR(DMSO-d₆, 300MHz); δ = 8.28 (s, 1H), 7.94 (d, *J*=5.7Hz, 1H), 7.64 (d, *J*=2.3Hz, 1H), 7.45 (dd, *J*=2.3Hz, 8.8Hz, 1H), 6.98 (d, *J*=5.3Hz, 1H), 6.94 (d, *J*=8.8Hz, 1H), 4.41-4.38 (m, 2H), 4.01-3.98 (m, 2H), 3.96 (s, 3H).
MS(ESI); 305.1(M⁺+1).

### b) Synthesis of (3-chloro-4-hydroxy-phenyl)-(2,3-dihydro-pyrido[3,4-b][1,4]oxazin-1-yl)-methanone

In a 10ml flask, (3-chloro-4-methoxy-phenyl)-(2,3-dihydro-pyrido[3,4-b][1,4]oxazin-1-yl)-methanone (134mg, 0.441mmol) was dissolved in dichloromethane (5ml), and the mixture was cooled to 0°C. 1.0M Boron tribromide dissolved in dichloromethane (2.2ml, 2.2mmol) was added thereto and stirred at room temperature for 16 hours. After neutralizing with saturated solution of sodium hydrogen carbonate to pH 8 to 9, the mixture was adjusted to pH 6 again by using 1N hydrochloric acid and extracted with dichloromethane. The combined organic layer was washed with saturated saline solution and dried over anhydrous sodium sulfate (Na₂SO₄), filtered and evaporated under reduced pressure. The residue was purified by column chromatography on silica eluting with a solvent of methanol:dichloromethane=1:20. The fractions containing the product were collected and evaporated to obtain the target compound 34 as white solid (65mg, 50.9%).
¹H-NMR(CD₃OD, 300MHz); δ = 8.17 (s, 1H), 7.88 (d, *J*=5.7Hz, 1H), 7.64 (d, *J*=1.9Hz, 1H), 7.42 (dd, *J*=1.9Hz, 8.8Hz, 1H), 7.33 (d, *J*=5.7Hz, 1H), 6.98 (d, *J*=8.4Hz, 1H), 4.39 (m, 2H), 4.00 (m, 2H).
MS(ESI); 291.1(M⁺+1).

### Example 35)

### Synthesis of 2,6-dibromo-4-(2,3-dihydro-pyrido[4,3-b][1,4]oxaxin-4-sulfonyl)-phenol (compound 35-2)

### a) Synthesis of 4-(4-methoxy-benzenesulfonyl)-3,4-dihydro-2H-pyrido[4,3-b][1.4]oxazine

In a 25ml flask, 3,4-dihydro-2H-pyrido[4,3-b][1,4]oxazine (370mg, 2.72mmol) and 4-methoxybenzenesulfonyl chloride (618mg, 2.99mmol) were dissolved in dichloromethane (10ml). Triethylamine (0.45ml, 3.23mmol) was added thereto and then stirred at room temperature for 2 hours. After neutralizing with 1N hydrochloric acid solution, the mixture was extracted with dichloromethane, and the combined organic layer was dried over anhydrous sodium sulfate (Na₂SO₄), filtered and evaporated under reduced pressure. The residue was purified by column chromatography on silica eluting with a solvent of dichloromethane. The fractions containing the product were collected and evaporated to obtain pale-green solid (700mg, 84.1 %).
¹H-NMR(DMSO-d₆, 300MHz); δ = 8.98 (s, 1H), 8.21 (d, *J*=5.3Hz, 1H), 7.62-7.57 (m, 2H), 6.96-6.91 (m, 2H), 6.75 (d, *J*=5.7Hz, 1H), 3.88-3.79 (m, 7H).
MS(ESI); 307(M⁺+1).

### b) Synthesis of 4-(2,3-dihydro-pyrido[4,3-b][1,4]oxazin-4-sulfonyl)-phenol (compound 35-1)

In a 100ml flask, 4-(4-methoxy-benzenesulfonyl)-3,4-dihydro-2H-pyrido[4,3-b][1,4]oxazine (699mg, 2.282mmol) was dissolved in dichloromethane (30ml) and the mixture was cooled to 0°C. 1.0M Boron tribromide dissolved in dichloromethane (11.4ml, 11.4mmol) was added thereto and stirred at room temperature for 16 hours. After neutralizing with saturated solution of sodium hydrogen carbonate to pH 8 to 9, the mixture was adjusted to pH 6 again by using 1N hydrochloric acid and extracted with dichloromethane. The combined organic layer was washed with saturated saline solution and dried over anhydrous sodium sulfate (Na₂SO₄), filtered and evaporated under reduced pressure. The residue was purified by column chromatography on silica eluting with a solvent of methanol:dichloromethane=1:20. The fractions containing the product were collected and evaporated to obtain white solid (375mg, 56.2%).
¹H-NMR(DMSO-d₆, 300MHz); δ = 10.80 (br s, 1H), 8.73 (s, 1H), 8.15 (d, *J*=5.7Hz, 1H), 7.50-7.45 (m, 2H), 6.91-6.87 (m, 3H), 3.85-3.82 (m, 2H), 3.75-3.72 (m, 2H).
MS(ESI); 293.1(M⁺+1).

### c) Synthesis of 2,6-dibromo-4-(2,3-dihydro-pyrido[4,3-b][1,4]oxazin-4-sulfonyl)-phenol

In a 25ml flask, 4-(2,3-dihydro-pyrido[4,3-b][1,4]oxazin-4-sulfonyl)-phenol (364mg, 1.25mmol) and methanol (7ml) were added and the temperature was set to -25°C. 1,3-dibromo-5,5'-dimethylhydantoin (20.4mg, 0.07mmol) dissolved in methanol (1ml) was added thereto dropwise at -25°C. After stirring at -25°C for 30 minutes, 1,3-dibromo-5,5'-dimethylhydantoin (20.4mg, 0.07mmol) dissolved in methanol (1ml) was further added thereto dropwise at -25°C. After completion of the reaction, water (10ml) was added thereto and the mixture was extracted with ethyl acetate, and the combined organic layer was washed with saturated saline solution and dried over anhydrous sodium sulfate (Na₂SO₄), filtered and evaporated under reduced pressure. The residue was purified by column chromatography on silica eluting with a solvent of methanol :dichloromethane=1:20. The fractions containing the product were collected and evaporated to obtain pale-green solid. The resulting solid was recrystallized from diethyl ether to obtain the target compound 35-2 as white solid (392mg, 69.9%).
H-NMR(DMSO-d₆, 300MHz); δ = 8.78 (s, 1H), 8.25 (d, *J*=5.3Hz, 1H), 7.80 (s, 2H), 7.03 (d, *J*=5.7Hz, 1H), 3.93 (s, 4H).
MS(ESI); 448.7(M⁺+1).

### Example 36)

### Synthesis of (3-chloro-4-hydroxy-5-nitro-phenyl)-(2,3-dihydro-pyrido[3,4-b][1,4]oxazin-1-yl)-methanone (compound 36)

### a) Synthesis of (3-chloro-4-methoxy-5-nitro-phenyl)-(2,3-dihydro-pyrido[3,4-b][1,4]oxazin-1-y)-methanone

In a 10ml flask, 2,3-dihydro-1H-pyrido[3,4-b][1,4]oxazine (96.2mg, 0.706mmol) and 3-chloro-4-methoxy-5-nitro-benzoyl chloride (194mg, 0.777mmol) were dissolved in dichloromethane (3ml). Triethylamine (0.15ml, 1.059mmol) was added thereto and then stirred at room temperature for 2 hours. After neutralizing with 1N hydrochloric acid solution, the mixture was extracted with dichloromethane, and the combined organic layer was dried over anhydrous sodium sulfate (Na₂SO₄), filtered and evaporated under reduced pressure. The residue was purified by column chromatography on silica eluting with a solvent of ethyl acetate:n-hexane=1:1. The fractions containing the product were collected and evaporated to obtain yellow solid (206mg, 83.4%).
¹H-NMR(CDCl₃, 300MHz); δ = 8.33 (s, 1H), 8.03 (d, *J*=5.7Hz, 1H), 7.90 (d, *J*=2.3Hz, 1H), 7.83 (d, *J*=2.3Hz, 1H), 7.11 (d, *J*=5.7Hz, 1H), 4.44-4.40 (m, 2H), 4.10 (s, 3H), 4.01-3.98 (m, 2H).

### b) Synthesis of (3-chloro-4-hydroxy-5-nitro-phenyl)-(2,3-dihydro-pyrido[3,4-b][1,4]oxazin-1-yl)-methanone

In a 25ml flask, (3-chloro-4-methoxy-5-nitro-phenyl)-(2,3-dihydro-pyrido[3,4-b][1,4]oxazin-1-yl)-methanone (176mg, 0.441mmol), lithium chloride (42.7mg, 1.006mmol), piperazine (65mg, 0.755mmol) and N,N-dimethyl formamide (5ml) were added and stirred at 100°C for 30 minutes. Lithium chloride (42.7mg, 1.006mmol) was further added thereto and then stirred at 100°C for 2 hours. After neutralizing with saturated solution of sodium hydrogen carbonate to pH 8 to 9, the mixture was adjusted to pH 6 again by using 1N hydrochloric acid to form solid. The resulting solid was filtered and recrystallized from methanol and tetrahydrofuran to obtain the target compound 36 as yellow solid (153mg, 91%).
¹H-NMR(DMSO-d₆, 300MHz); δ = 8.38 (s, 1H), 8.13 (d, *J*=2.3Hz, 1H), 8.06 (d, *J*=5.7Hz, 1H), 7.85 (d, *J*=2.3Hz, 1H), 7.56 (d, *J*=5.7Hz, 1H), 4.40-4.36 (m, 2H), 3.96-3.93 (m, 2H).
MS(ESI); 336.0(M⁺+1).

### Example 37)

### Synthesis of (3-chloro-4-hydroxy-phenyl)-(2,3-dihydro-pyrido[4,3-b][1,4]oxazin-4-yl)-methanone (compound 37)

In a 25ml flask, (3-chloro-4-methoxy-phenyl)-(2,3-dihydro-pyrido[4,3-b][1,4]oxazin-4-yl)-methanone (223mg, 0.73mmol) was dissolved in dichloromethane, and the mixture was cooled to 0°C. 1.0M Boron tribromide dissolved in dichloromethane (3.65ml, 3.65mmol) was added thereto and stirred at room temperature for 16 hours. n-hexane solvent was added thereto and the formed solid was filtered. The resulting solid was dissolved in methanol and concentrated. After adding and dissolving in water, the mixture was adjusted to pH 6 with saturated solution of sodium hydrogen carbonate and then extracted with dichloromethane. The combined organic layer was dried over anhydrous sodium sulfate (Na₂SO₄), filtered and evaporated under reduced pressure. The residue was recrystallized from dichloromethane and n-hexane to obtain the target compound 37 as white solid (80mg, 38%).
¹H-NMR(DMSO-d₆, 300MHz); δ = 10.92 (br s, 1H), 8.37 (br s, 1H), 8.05 (d, *J*=5.3Hz, 1H), 7.58 (d, *J*=2.3Hz, 1H), 7.38 (dd, *J*=8.4Hz, 2.3Hz, 1H), 7.01 (d, *J*=8.4Hz, 1H), 6.94 (d, *J*=5.3Hz, 1H), 4.38 (m, 2H), 3.91 (m, 2H).
MS(ESI); 291.2(M⁺+1).

### Example 38)

### Synthesis of (3-bromo-4-hydroxy-phenyl)-(2,3-dihydro-pyrido[4,3-b][1,4]oxazin-4-yl)-methanone (compound 38)

By the same method as in Example 3, (3-bromo-4-methoxy-phenyl)-(2,3-dihydro-pyrido[4,3-b][1,4]oxazin-4-yl)-methanone (296mg, 0.85mmol) and 1M solution of boron tribromide (4.25ml, 4.25mmol) were reacted to obtain the target compound 38, i.e., (3-bromo-4-hydroxy-phenyl)-(2,3-dihydro-pyrido[4,3-b][1,4]oxazin-4-yl)-methanone (256mg, 89%).
¹H-NMR(DMSO-d₆, 300MHz); δ = 8.37 (br s, 1H), 8.05 (d, *J*=5.7Hz, 1H), 7.71 (d, *J*=1.9Hz, 1H), 7.41 (dd, *J*=8.0Hz, 1.9Hz, 1H), 6.98 (d, *J*=8.8Hz, 1H), 6.94 (d, *J*=5.7Hz, 1H), 4.56 (m, 2H), 4.01 (m, 2H).
MS(ESI); 335.1(M⁺+1).

### Example 39)

### Synthesis of (3,5-dichloro-4-hydroxy-phenyl)-(2,3-dihydro-pyrido[4,3-b][1,4]oxazin-4-yl)-methanone (compound 39)

In a 25ml flask, (3,5-dichloro-4-methoxy-phenyl)-(2,3-dihydro-pyrido[4,3-b][1,4]oxazin-4-yl)-methanone (344mg, 1.0mmol), lithium chloride (219mg, 6mmol) and piperazine (105mg, 1.2mmol) were dissolved in N,N-dimethyl formamide. After stirring at 100 °C for 2 hours, water was added to quench the reaction. The mixture was adjusted to pH 6 by using 1N hydrochloric acid, and the formed solid was filtered and washed with distilled water and ether. The resulting solid was recrystallized from dichloromethane and n-hexane to obtain the target compound 39, i.e., (3,5-dichloro-4-hydroxy-phenyl)-(2,3-dihydro-pyrido[4,3-b][1,4]oxazin-4-yl)methanone (48mg, 16%).
¹H-NMR(DMSO-d₆, 300MHz); δ = 8.43 (br s, 1H), 8.08 (br s, 1H), 7.58 (s, 2H), 6.95 (d, *J*=5.3Hz, 1H), 4.39 (m, 2H), 3.90 (m, 2H).
MS(ESI); 325.0(M⁺+1).

### Example 40)

### Synthesis of (3-bromo-4-hydroxy-phenyl)-(2,3-dihydro-pyrido[3,4-b][1,4]oxazin-1-yl)-methanone (compound 40)

By the same method as in Example 3, (3-bromo-4-methoxy-phenyl)-(2,3-dihydro-pyrido[3,4-b][1,4]oxazin-1-yl)-methanone (200mg, 0.58mmol) and 1M solution of boron tribromide (2.9ml, 2.9mmol)) were reacted to obtain the target compound 40, i.e., (3-bromo-4-hydroxy-phenyl)-(2,3-dihydro-pyrido[3,4-b][1,4]oxazin-1-yl)-methanone, as beige solid (95mg, 49%).
¹H-NMR(DMSO-d₆, 300MHz); δ = 11.1 (br s, 1H), 8.21 (s, 1H), 7.94 (d, *J*=5.3Hz, 1H), 7.77 (d, *J*=1.9Hz, 1H), 7.47 (dd, *J*=8.4Hz, 1.9Hz, 1H), 7.32 (d, *J*=5.3Hz, 1H), 7.01 (d, *J*=8.4Hz, 1H), 4.33 (m, 2H), 3.90 (m, 2H).
MS(ESI); 335.1 (M⁺+1).

### Example 41)

### Synthesis of (3,5-dichloro-4-hydroxy-phenyl)-(2,3-dihydro-pyrido[3,4-b][1,4]oxazin-1-yl)-methanone (compound 41)

By the same method as in Example 39, (3,5-dichloro-4-methoxy-phenyl)-(2,3-dihydro-pyrido[3,4-b][1,4]oxazin-1-yl)-methanone (100mg, 0.29mmol), lithium chloride (124mg, 2.9mmol) and piperazine (38mg, 0.44mmol) were reacted. The resulting solid was purified by recrystallizing from methanol to obtain the target compound 41 as pale-brown solid (40mg, 47%).
¹H-NMR(DMSO-d₆, 300MHz); 8 = 10.9 (br s, 1H), 8.21 (s, 1H), 7.95 (d, *J*=5.3Hz, 1H), 7.64 (s, 2H), 7.40 (d, *J*=5.3Hz, 1H), 4.33 (m, 2H), 3.89 (m, 2H).
MS(ESI); 325.1 (M⁺+1).

### Example 42)

### Synthesis of 2-(3,5-dibromo-4-hydroxy-phenyl)-1-(2,3-dihydro-pyrido[4,3-b][1,4]oxazin-4-yl)-ethanone (compound 42)

### a) Synthesis of 1-(2,3-dihydro-pyrido[4,3-b][1,4]oxazin-4-yl)-2-(4-methoxy-phenyl)-ethanone

In a 10ml flask, 3,4-dihydro-2H-pyrido[4.3-b][1,4]oxazine (100mg, 0.73mmol) and (4-methoxy-phenyl)-acetyl chloride (163mg, 0.88mmol) were dissolved in dichloromethane. Triethylamine (0.21 ml, 1.46mmol) was added thereto and then stirred at room temperature for 2 hours. After completion of the reaction by adding water, the reaction mixture was extracted with dichloromethane. The combined organic layer was dried over anhydrous sodium sulfate (Na₂SO₄), filtered and evaporated under reduced pressure. The residue was purified by column chromatography on silica eluting with a solvent of dichloromethane:methanol=9:1. The fractions containing the product were collected and evaporated to obtain the target compound, i.e., 1-(2,3-dihydro-pyrido[4,3-b][1,4]oxazin-4-yl)-2-(4-methoxy-phenyl)-ethanone, as yellow liquid (198mg, 95%).
¹H-NMR(CDCl₃, 300MHz); δ = 8.51 (br s, 1H), 8.22 (d, *J*=5.3Hz, 1H), 7.23 (d, 2H, *J*=8.4Hz), 6.85 (d, *J*=8.4Hz, 2H), 6.81 (d, *J*=5.3Hz, 1H), 4.21 (br s, 2H), 3.94 (br s, 2H), 3.91 (s, 2H), 3.79 (s, 3H).
MS(ESI); 285.1(M⁺+1).

### b) Synthesis of 1-(2,1-dihydro-pyrido[4,3-b][1,4]oxazin-4-yl)-2-(4-hydroxy-phenyl)-ethanone

By the same method as in Example 3, 1-(2,3-dihydro-pyrido[4.3-b][1,4]oxazin-4-yl)-2-(4-methoxy-phenyl)-ethanone (130mg, 0.46mmol) and 1M solution of boron tribromide (2.3ml, 2.3mmol) were reacted to obtain the target compound, i.e., 1-(2,3-dihydro-pyrido[4.3-b][1,4]oxazin-4-yl)-2-(4-hydroxy-phenyl)-ethanone, as yellow liquid (100mg, 81%).
¹H-NMR(CDCl₃, 300MHz); δ = 9.29 (br s, 1H), 8.97 (br s, 1H), 8.10 (d, *J*=5.7Hz, 1H), 7.02 (d, 2H, *J*=8.4Hz), 6.90 (d, *J*=5.7Hz, 2H), 6.69 (d, *J*=8.4Hz, 1H), 4.22 (m, 2H), 3.90 (br s, 2H), 3.85 (s, 2H).
MS(ESI); 271.1(M⁺+1).

### c) Synthesis of 2-(3,5-dibromo-4-hydroxy-phenyl)-1-(2,3-dihydro-pyrido[4,3-b][1,4]oxazin-4-yl)-ethanone

In a 10ml flask, 1-(2,3-dihydro-pyrido[4,3-b][1,4]oxazin-4-yl)-2-(4-hydroxy-phenyl)-ethanone (88mg, 0.33mmol) and bromohydantoin (93mg, 0.33mmol) were dissolved in a solvent of methanol:dichloromethane=2:1. The mixture was stirred at -30 °C for 0.5 hour. After completion of the reaction by adding water, the reaction mixture was extracted with dichloromethane, and the combined organic layer was dried over anhydrous sodium sulfate (Na₂SO₄), filtered and evaporated under reduced pressure. The residue was purified by recrystallizing from dichloromethane to obtain the target compound 42, i.e., 2-(3,5-dibromo-4-hydroxy-phenyl)-1-(2,3-dihydro-pyrido[4,3-b][1,4]oxazin-4-yl)-ethanone, as beige solid (13.8mg, 10%).
¹H-NMR(CDCl₃, 300MHz); δ = 9.81 (br s, 1H), 8.94 (br s, 1H), 8.11 (d, *J*=5.3Hz, 1H), 7.41 (s, 2H), 6.93 (d, *J*=5.3Hz, 1H), 4.37 (m, 2H), 3.94 (m, 2H), 3.92 (s, 2H).
MS(ESI); 426.9(M⁺+1).

### Example 43)

### Synthesis of (2,3-dihydro-pyrido[4,3-b][1,4]oxazin-4-yl)-(3-methoxy-isoxazol-5-yl)-methanone compound 43)

In a 10ml flask, 3,4-dihydro-2H-pyrido[4.3-b][1,4]oxazine (100mg, 0.73mmol) and 3-methoxy-isoxazol-5-carbonyl chloride (131mg, 0.80mmol) were dissolved in dichloromethane. Triethylamine (0.51ml, 3.65mmol) was added thereto and then stirred at room temperature for 2 hours. After completion of the reaction by adding water dropwise, the reaction mixture was extracted with dichloromethane, and the combined organic layer was dried over anhydrous sodium sulfate (Na₂SO₄), filtered and evaporated under reduced pressure. The residue was purified by column chromatography on amine silica eluting with a solvent of dichloromethane:methanol=30:1. The fractions containing the product were collected and evaporated to obtain the target compound 43, i.e., (2,3-dihydro-pyrido[4.3-b][1,4]oxazin-4-yl)-(3-methoxy-isoxazol-5-yl)-methanone, as pale-brown solid (149mg, 78%).
¹H-NMR(CDCl₃, 300MHz); δ = 8.77 (br s, 1H), 8.16 (d, 1H, *J*=5.3Hz), 6.98 (d, *J*=5.3Hz, 1H), 6.88 (s, 1H), 4.42 (m, 2H), 4.04 (m, 2H), 3.96 (s, 3H).
MS(ESI); 262.1(M⁺+1).

### Example 44)

### Synthesis of (2,3-dihydro-pyrido[4,3-b][1,4]oxazin-4-yl)-(3-hydroxy-isoxazol-5-yl)-methanone (compound 44)

### a) Synthesis of (3-benzyloxy-isoxazol-5-yl-(2,3-dihydro-pyrido[4,3-b][1,4]oxazin-4-yl)-methanone

To 10 ml of anhydrous tetrahydrofuran, 3-benzyloxy-isoxazol-5-carboxylic acid (50mg, 0.23mmol) and a catalytic amount of N,N-dimethyl formamide were added and dissolved, and then oxalyl chloride (35mg, 0.28mmol) was slowly added thereto and then stirred at room temperature for 4 hours.

The reaction mixture was concentrated under reduced pressure. The residue was dissolved in dichloromethane, triethylamine (115mg, 1.15mmol) and 3,4-dihydro-2H-pyrido[4,3-b][1,4]oxazine (31mg, 0.23mmol) were added thereto and then stirred at room temperature for 2 hours. After completion of the reaction by adding water dropwise, the reaction mixture was extracted with dichloromethane, and the combined organic layer was dried over anhydrous sodium sulfate (Na₂SO₄), filtered and evaporated under reduced pressure. The residue was purified by column chromatography on amine silica eluting with a solvent of dichloromethane:methanol=30:1. The fractions containing the product were collected and evaporated to obtain (3-benzyloxy-isoxazol-5-yl)-(2,3-dihydro-pyrido[4,3-b][1,4]oxazin-4-yl)-methanone as pale-brown liquid (55mg, 54% in two steps).
¹H-NMR(CDCl₃, 300MHz); δ = 8.23 (d, 1H, *J*=5.3Hz), 7.32-7.48 (m, 6H), 6.87 (d, 1H, *J*=5.3Hz), 6.52 (br s, 1H), 5.30 (s, 2H), 4.44 (m, 2H), 4.13 (m, 2H).
MS(ESI); 338.1(M⁺+1).

### b) Synthesis of (2,3-dihydro-pyrido[4,3-b][1,4]oxazin-4-yl)-(3-hydroxy-isoxazol-5-yl)-methanone

In 3ml of 33% hydrobromic acid in acetic acid solution, (3-benzyloxy-isoxazol-5-yl)-(2,3-dihydro-pyrido[4,3-b][1,4]oxazin-4-yl)-methanone (45mg, 0.13mmol) was dissolved and then stirred at room temperature for 24 hours. After completion of the reaction by adding water dropwise, the mixture was adjusted to weak acidic condition (pH=6) by using saturated solution of sodium hydrogen carbonate and extracted with ethyl acetate. The aqueous layer was evaporated under reduced pressure and the residue was purified by using preparative TLC plate to obtain the target compound 44, i.e., (2,3-dihydro-pyrido[4,3-b][1,4]oxazin-4-yl)-(3-hydroxy-isoxazol-5-yl)-methanone, as white solid (8mg, 24%).
¹H-NMR(DMSO-d₆, 300MHz); δ = 8.70 (br s, 1H), 8.13 (d, *J*=5.3Hz, 1H), 6.97 (d, *J*=5.3Hz, 1H), 6.25 (br s, 1H), 4.40 (m, 2H), 4.04 (m, 2H).
MS(ESI); 248.1(M⁺+1).

### Example 45)

### Synthesis of (3,5-dibromo-4-hydroxy-phenyl)-[7-(4-trifluoromethyl-phenyl)-2,3-dihydro-pyrido[2,3-b][1,41oxazin-1-yl]-methanone (compound 45)

### a) Synthesis of 7-bromo-2,3-dihydro-1H-pyrido[2,3-b][1,4]oxazine

7-bromo-1H-pyrido[2,3-b][1,4]oxazin-2-one (1.3g, 5.68mmol) was dissolved in 30ml of anhydrous tetrahydrofuran under nitrogen atmosphere and the mixture was cooled to 0°C. 1.0M Lithium aluminum hydride dissolved in tetrahydrofuran (6.90ml, 6.82mmol) was added thereto dropwise and then stirred for 30 minutes. To the reaction mixture, water (0.3ml), 10% sodium hydroxide (0.6ml) and water (0.9ml) were added dropwise in this order and then stirred vigorously at room temperature for 1 hour. The resulting solid was filtered and washed with excess ethyl acetate. The filtrate was washed with water and saturated saline solution, dried over anhydrous sodium sulfate (Na₂SO₄), filtered and evaporated under reduced pressure to obtain 7-bromo-2,3-dihydro-1H-pyrido[2,3-b][1,4]oxazine as white solid (629mg, 52%).
¹H-NMR(CDCl₃, 300MHz); δ = 7.63 (d, *J*=2.3Hz, 1H), 6.96 (d, *J*=2.3Hz, 1H), 4.39 (m, 2H), 3.93 (br s, 1H), 3.42 (m, 2H).
MS(ESI); 215.0(M⁺+1).

### b) Synthesis of 7-(4-trifluoromethyl-phenyl)-2,3-dihydro-1H-pyrido[2,3-b][1,4]oxazine

To 3ml of tetrahydrofuran and 0.5ml of distilled water, 7-bromo-2,3-dihydro-1H-pyrido[2,3-b][1,4]oxazine (80.0mg, 0.37mmol), 4-fluoromethyl-phenyl boronic acid (78mg, 0.41mmol), potassium carbonate (103 mg, 0.74mmol) and tetrakis triphenylphosphine palladium (21mg, 0.02mmol) were added and then stirred at 80°C for 2 hours. After completion of the reaction, the mixture was extracted with ethyl acetate, and the combined organic layer was dried over anhydrous sodium sulfate (Na₂SO₄), filtered and evaporated under reduced pressure. The residue was purified by column chromatography on amine silica eluting with a solvent of dichloromethane:methanol=50:1. The fractions containing the product were collected and evaporated to obtain 7-(4-trifluoromethyl-phenyl)-2,3-dihydro-1H-pyrido[2,3-b][1,4]oxazine as white solid (77mg, 74%).
¹H-NMR(CDCl₃+CD₃OD, 300MHz); δ = 7.77 (d, *J*=2.3Hz, 1H), 7.67 (d, *J*=8.4Hz, 2H), 7.60 (d, *J*=8.4Hz, 2H), 7.11 (d, *J*=2.3Hz, 1H), 4.46 (m, 2H), 3.46 (m, 2H).
MS(ESI); 281.1(M⁺+1).

### c) Synthesis of (3,5-dibromo-4-methoxy-phenyl)-[7-(4-trifluoromethyl-phenyl1-2,3-dihydro-pyrido[2,3-b][1,4]oxazin-1-yl]-methanone

In a 10ml flask, 7-(4-trifluoromethyl-phenyl)-2,3-dihydro-1H-pyrido[2,3-b][1,4]oxazine (67mg, 0.24mmol) and 3,5-dibromo-4-methoxy-benzoyl chloride (118mg, 0.36mmol) were dissolved in dichloromethane. Triethylamine (0.17ml, 1.20mmol) was added thereto and then stirred at room temperature for 2 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by column chromatography on silica eluting with a solvent of dichloromethane:methanol=19:1. The fractions containing the product were collected and evaporated to obtain (3,5-dibromo-4-methoxy-phenyl)-[7-(4-trifluoromethyl-phenyl)-2,3-dihydro-pyrido[2,3-b][1,4]oxazin-1-yl]-methanone as white solid (122mg, 89%).
¹H-NMR(CDCl₃, 300MHz); δ = 8.29 (d, *J*=2.3Hz, 1H), 8.24 (s, 1H), 7.73 (s, 2H), 7.67 (d, *J*=8.4Hz, 2H), 7.50 (d, *J*=8.4Hz, 2H), 4.53 (m, 2H), 4.02 (m, 2H), 3.95 (s, 3H).
MS(ESI); 570.9(M⁺+1).

### d) Synthesis of (3,5-dibromo-4-hydroxy-phenyl-[7-(4-trifluoromethyl-phenyl)-2,3-dihydro-pyrido[2,3-b][1,4]oxazin-1-yl]-methanone

In a 10ml flask, (3,5-dibromo-4-methoxy-phenyl)-[7-(4-trifluoromethyl-phenyl)-2,3-dihydro-pyrido[2,3-b][1,4]oxazin-1-yl]-methanone (122mg, 0.21mmol), lithium bromide (148mg, 1.71mmol) and piperazine (28mg, 0.32mmol) were dissolved in 3ml of N,N-dimethyl formamide and then stirred at 100°C for 2 hours. After completion of the reaction by adding water dropwise, the mixture was adjusted to weak acidic condition (pH=6) by using 1N hydrochloric acid. The formed solid was filtered and washed with distilled water. The resulting solid was recrystallized from tetrahydrofuran and methanol to obtain the target compound 45, i.e., (3,5-dibromo-4-hydroxy-phenyl)-[7-(4-trifluoromethyl-phenyl)-2,3-dihydro-pyrido[2,3-b][1,4]oxazin-1-yl]-methanone (59 mg, 50%).
¹H-NMR(CDCl₃, 300MHz); δ = 8.28 (d, *J*=1.9Hz, 1H), 8.03 (br s, 1H), 7.73 (s, 2H), 7.67 (d, *J*=8.0Hz, 2H), 7.51 (d, *J*=8.0Hz, 2H), 6.28 (br s, 1H), 4.52 (m, 2H), 4.03 (m, 2H).
MS(ESI); 556.9(M⁺+1).

### Example 46)

### Synthesis of (3,5-dibromo-4-hydroxy-phenyl)-[7-(2-trifluoromethyl-phenyl-2,3-dihydro-pyrido[2,3-b][1,4]oxazin-1-yl]-methanone (compound 46)

### a) Synthesis of 7-(2-trifluoromethyl-phenyl)-2,3-dihydro-1H-pyrido[2,3-b][1,4]oxazine

To 3ml of tetrahydrofuran/0.5ml of distilled water, 7-bromo-2,3-dihydro-1H-pyrido[2,3-b][1,4]oxazine (80.0mg, 0.37mmol), 2-trifluoromethyl-phenyl boronic acid (78mg, 0.41mmol), potassium carbonate(103mg, 0.74mmol) and tetrakis triphenylphosphine palladium (21mg, 0.02mmol) were added and then stirred at 80°C for 2 hours. After completion of the reaction, the mixture was extracted with ethyl acetate, and the combined organic layer was dried over anhydrous sodium sulfate (Na₂SO₄), filtered and evaporated under reduced pressure. The residue was purified by column chromatography on amine silica eluting with a solvent of dichloromethane:methanol=50:1. The fractions containing the product were collected and evaporated to obtain 7-(2-trifluoromethyl-phenyl)-2,3-dihydro-1H-pyrido[2,3-b][1,4]oxazine as white solid (67mg, 64%).
¹H-NMR(CDCl₃, 300MHz); δ = 7.74 (d, *J*=7.2Hz, 1H), 7.60-7.44 (m, 3H), 7.31 (d, *J*=7.6Hz, 1H), 6.85 (d, *J*=1.9Hz, 1H), 4.47 (m, 2H), 3.88 (br s, 1H), 3.48 (m, 2H).
MS(ESI); 281.1(M⁺+1).

### b) Synthesis of (3,5-dibromo-4-methoxy-phenyl)-[7-(2-trifluoromethyl-phenyl)-2,3-dihydro-pyrido[2,3-b][1,4]oxazin-1-yl]-methanone

In a 10ml flask, 7-(2-trifluoromethyl-phenyl)-2,3-dihydro-1H-pyrido[2,3-b][1,4]oxazine (67mg, 0.24mmol) and 3,5-dibromo-4-methoxy-benzoyl chloride (118mg, 0.36mmol) were dissolved in dichloromethane. Triethylamine (0.17ml, 1.20mmol) was added thereto and then stirred at room temperature for 2 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by column chromatography on silica eluting with a solvent of dichloromethane. The fractions containing the product were collected and evaporated to obtain (3,5-dibromo-4-methoxy-phenyl)-[7-(2-trifluoromethyl-phenyl)-2,3-dihydro-pyrido[2,3-b][1,4]oxazin-1-yl]-methanone as white solid (46mg, 34%).
¹H-NMR(CDCl₃, 300MHz); δ = 7.99 (d, *J*=2.3Hz, 1H), 7.72 (d, *J*=7.2Hz, 1H), 7.68 (s, 2H), 7.58-7.48 (m, 3H), 7.20 (d, *J*=7.6Hz, 1H), 4.57 (m, 2H), 4.04 (m, 2H), 3.92 (s, 3H).
MS(ESI); 570.9(M⁺+1).

### c) Synthesis of (3,5-dibromo-4-hydroxy-phenyl)-[7-(2-trifluoromethyl-phenyl)-2,3-dihydro-pyrido[2,3-b][1,4]oxazin-1-yl]-methanone

In a 10ml flask, (3,5-dibromo-4-methoxy-phenyl)-[7-(2-trifluoromethyl-phenyl)-2,3-dihydro-pyrido[2,3-b][1,4]oxazin-1-yl]-methanone (46mg, 0.08mmol), lithium bromide (56mg, 0.64mmol) and piperazine (10mg, 0.04mmol) were dissolved in 3ml of N,N-dimethyl formamide, and then stirred at 100°C for 2 hours. After completion of the reaction by adding water dropwise, the mixture was adjusted to weak acidic condition (pH=6) by using 1N hydrochloric acid. The formed solid was filtered and washed with distilled water. The resulting solid was recrystallized from methanol to obtain the target compound 46, i.e., (3,5-dibromo-4-hydroxy-phenyl)-[7-(2-trifluoromethyl-phenyl)-2,3-dihydro-pyrido[2,3-b][1,4]oxazin-1-yl]-methanone (31 mg, 69%).
¹H-NMR(CDCl₃, 300MHz); δ = 7.99 (d, 1H, *J*=1.9Hz), 7.72 (d, 1H, *J*=7.6Hz), 7.68 (s, 2H), 7.60-7.46 (m, 3H), 7.21 (d, 1H, *J*=6.9Hz), 6.22 (s, 1H), 4.56 (m, 2H), 4.05 (m, 2H).
MS(ESI); 556.9(M⁺+1).

### Example 47)

### Synthesis of 1-(3,5-dibromo-4-hydroxy-benzoyl)-dihydro-1H-pyrido[2,3-b][1,4]oxazin-7-carbonitrile (compound 47-2)

### a) Synthesis of 2,3-dihydro-1H-pyrido[2,3-b][1,4]oxazin-7-carbonitrile

In a 5ml flask, 7-bromo-2,3-dihydro-1H-pyrido[2,3-b][1,4]oxazine (24mg, 0.111mmol) was dissolved in N-methylpyrrolidone (NMP) (20.3ml), and cuprous cyanide (CuCN) (16mg, 0.178mmol) was added thereto and then stirred in microwave at 180°C for 10 minutes using microwave (100W) apparatus. The reaction mixture was cooled to room temperature and water (10ml) was added thereto, and extracted with ethyl acetate (10ml*2). The combined organic layer was dried over anhydrous magnesium sulfate (MgSO₄), filtered and evaporated under reduced pressure. The residue was purified by column chromatography on silica eluting with a solvent of dichloromethane:methanol=20:1. The fractions containing the product were collected and evaporated to obtain pale-yellow solid (17mg, quantitative yield).
¹H-NMR(CD₃OD, 300MHz); δ = 7.97-7.88 (m, 1H), 7.02 (s, 1H), 4.49 (t, *J*=4.3Hz, 2H), 4.19 (br s, 1H), 3.47 (t, *J*=4.3Hz, 2H).
MS(ESI); 162(M⁺+1).

### b) Synthesis of 1-(3,5-dibromo-4-methoxy-benzoyl)-2,3-dihydro-1H-pyrido[2,3-b][1,4]oxazin-7-carbonitrile (compound 47-1)

By the same method as in the step d) of Example 2, 2,3-dihydro-1H-pyrido[2,3-b][1,4]oxazin-7-carbonitrile (7mg, 0.043mmol) was reacted with 3,5-dibromo-4-methoxy-benzoyl chloride (21mg, 0.065mmol) to obtain 1-(3,5-dibromo-4-methoxy-benzoyl)-2,3-dihydro-1H-pyrido[2,3-b][1,4]oxazin-7-carbonitrile as white solid (14.4mg, 74%).
¹H-NMR(CD₃OD, 300MHz); δ = 8.39 (br s, 1H), 8.35 (d, *J*=1.9Hz, 1H), 7.69 (s, 2H), 4.50 (t, *J*=4.5Hz, 2H), 3.95 (m, 4H).
MS(ESI); 451.9(M⁺+1).

### c) Synthesis of 1-(3,5-dibromo-4-hydroxy-benzoyl)-2,3-dihydro-1H-pyrido[2,3-b][1,4]oxazin-7-carbonitrile (compound 47-2)

By the same method as in the step f) of Example 22, 1-(3,5-dibromo-4-methoxy-benzoyl)-2,3-dihydro-1H-pyrido[2,3-b][1,4]oxazin-7-carbonitrile (12mg, 0.026mmol) was reacted with lithium bromide (LiBr) (4.62mg, 0.053mmol) and piperazine (3.4mg, 0.0399mmol) to obtain the target compound 47-2, i.e., 1-(3,5-dibromo-4-hydroxy-benzoyl)-2,3-dihydro-1H-pyrido[2,3-b][1,4]oxazin-7-carbonitrile as white solid (9mg, 78%).
¹H-NMR(CD₃OD, 300MHz); δ = 8.37 (d, *J*=1.9Hz, 1H), 8.32 (d, *J*=1.9Hz, 1H), 7.70 (s, 2H), 4.53 (m, 2H), 4.023 (m, 2H).
MS(ESI); 437.8(M⁺+1).

### Example 48)

### Synthesis of (3,5-dibromo-4-methoxy-phenyl)-[7-(3-dimethylamino-phenyl)-2,3-dihydro-pyrido[2,3-b][1,4]oxazin-1-yl]-methanone (compound 48)

### a) Synthesis of [3-(2,3-dihydro-1H-pyrido[2.3-b][1,4]oxazin-7-yl)-phenyl]-dimethyl-amine

By the same method as in the step b) of Example 45, 7-bromo-2,3-dihydro-1H-pyrido[2,3-b][1,4]oxazine (98mg, 0.45mmol), 3-dimethyl phenyl boronic acid (112mg, 0.68mmol), tetrakis triphenylphosphine palladium (26mg, 0.023mol) and potassium carbonate (125mg, 0.91mmol) were dissolved in tetrahydrofuran/water (2.4ml/0.4ml) and reacted at 80°C for 6 hours to obtain [3-(2,3-dihydro-1H-pyrido[2,3-b][1,4]oxazin-7-yl)-phenyl]-dimethyl-amine as white solid (102mg, 89%).
MS(ESI); 256.1(M⁺+1).

### b) Synthesis of (3,5-dibromo-4-methoxy-phenyl)-[7-(3-nitro-phenyl)-2,3-dihydro-pyrido[2,3-b][1,4]oxazin-1-yl]-methanone

By the same method as in the step d) of Example 2, [3-(2,3-dihydro-1H-pyrido[2,3-b][1,4]oxazin-7-yl)-phenyl]-dimethyl-amine (102mg, 0.39mmol), 3,5-dibromo-4-methoxy-benzoyl chloride (197mg, 0.59mmol) and triethylamine (286µℓ, 1.99mmol) were dissolved in dichloromethane (5ml) and then stirred at room temperature for 3 hours to obtain the target compound 48, i.e., (3,5-dibromo-4-methoxy-phenyl)-[7-(3-dimethylamino-phenyl)-2,3-dihydro-pyrido[2,3-b][1,4]oxazin-1-yl]-methanone, as white solid (135mg, 62%).
¹H-NMR(CDCl₃, 300MHz); δ = 8.25 (d, *J*=2.3Hz, 1H), 7.72 (s, 2H), 7.27-7.20 (m, 2H), 6.70 (dd, *J*=8.4, 2.7Hz, 1H), 6.65 (d, *J*=7.6Hz, 1H), 6.51 (br s, 1H), 4.57-4.51 (m, 2H), 4.07-4.00 (m, 2H), 3.92 (s, 3H), 2.94 (s, 6H).
MS(ESI); 545.9(M⁺+1).

### Example 49)

### Synthesis of (3,5-dibromo-4-methoxy-phenyl)[7-(3-nitro-phenyl)-2,3-dihydro-pyrido[2,3-b][1,4]oxazin-1-yl]-methanone (compound 49)

### a) Synthesis of 7-(3-nitro-phenyl)-2,3-dihydro-1H-pyrido[2,3-b][1,4]oxazine

By the same method as in the step b) of Example 45, 7-bromo-2,3-dihydro-1H-pyrido[2,3-b][1,4]oxazine (99mg, 0.46mmol), 3-nitrophenyl boronic acid (115mg, 0.69mmol), palladium triphenylphosphine (26mg, 0.023mol) and potassium carbonate (127mg, 0.92mmol) were dissolved in tetrahydrofuran/water (2.4ml/0.4ml) and then stirred at 80°C for 6 hours to obtain 7-(3-nitro-phenyl)-2,3-dihydro-1H-pyrido[2,3-b][1,4]oxazine as white solid (90.4mg, 76%).
MS(ESI); 258.0(M⁺+1).

### b) Synthesis of (3,5-dibromo-4-methoxy-phenyl)-[7-(3-nitro-phenyl)-2,3-dihydro-pyrido[2,3-b][1,4]oxazin-1-yl]-methanone

By the same method as in the step d) of Example 2, 7-(3-nitro-phenyl)-2,3-dihydro-1H-pyrido[2,3-b][1,4]oxazine (90mg, 0.35mmol), 3,5-dibromo-4-methoxy-benzoyl chloride (173mg, 0.53mmol), and triethylamine (252µℓ, 1.76mmol) were dissolved in dichloromethane (5ml), and reacted at room temperature for 3 hours to obtain the target compound 49, i.e., (3,5-dibromo-4-methoxy-phenyl)-[7-(3-nitro-phenyl)-2,3-dihydro-pyrido[2,3-b][1,4]oxazin-1-yl]-methanone, as white solid (134mg, 70%).
¹H-NMR(CDCl₃, 300MHz); δ = 8.31 (d, *J*=2.3Hz, 1H), 8.27-8.19 (m, 2H), 8.15 (br s, 1H), 7.78-7.71 (m, 3H), 7.61 (t, *J*=6.8Hz, 1H), 4.56-4.50 (m, 2H), 4.05-3.99 (m, 2H), 3.96 (s, 3H).
MS(ESI); 547.9(M⁺+1).

### Example 50)

### Synthesis of (3,5-dibromo-4-hydroxy-phenyl)-[7-(3-nitro-phenyl)2,3-dihydro-pyrido[2,3-b][1,4]oxazin-1-yl]-methanone (compound 50)

By the same method as in the step f) of Example 22, (3,5-dibromo-4-methoxy-phenyl)-[7-(3-nitro-phenyl)-2,3-dihydro-pyrido[2,3-b][1,4]oxazin-1-yl]-methanone (101mg, 0.18mmol), lithium bromide (32mg, 0.36mmol), and piperazine (24mg, 0.28mmol) were dissolved in N,N-dimethyl formamide (3ml), and reacted at 100°C to obtain the target compound 50, i.e., (3,5-dibromo-4-hydroxy-phenyl)-[7-(3-nitro-phenyl)-2,3-dihydro-pyrido[2,3-b][1,4]oxazin-1-yl]-methanone, as pale-brown solid (24mg, 25%).
¹H-NMR(DMSO-d₆, 300MHz); δ = 8.32 (s, 1H), 8.28 (s, 1H), 8.19 (d, *J*=9.2Hz, 2H), 7.99 (d, *J*=6.9Hz, 1H), 7.80-7.62 (m, 3H), 4.50-4.32 (m, 2H), 4.10-3.80 (m, 2H).
MS(ESI); 533.9(M⁺+1).

### Example 51)

### Synthesis of (3,5-dibromo-4-hydroxy-phenyl)-(2,3-dihydro-pyrido[4,3-b][1,4]oxazin-4-yl)-methanethione (compound 51)

### a) Synthesis of (3,5-dibromo-4-methoxy-phenyl)-(2,3-dihydro-pyrido[4,3-b][1,4]oxazin-4-yl)-methanethione

In a 25ml flask, (3,5-dibromo-4-methoxy-phenyl)-(2,3-dihydro-pyrido[4,3-b][1,4]oxazin-4-yl)-methanone (100mg, 0.23mmol) was suspended in toluene, and Lawesson's reagent (113mg, 1.2 equivalents) was added thereto and then stirred at 120°C for 2 hours. Toluene was evaporated under reduced pressure and the residue was purified on amine silica by using a solvent of dichloromethane:methanol=50:1 to obtain (3,5-dibromo-4-methoxy-phenyl)-(2,3-dihydro-pyrido[4,3-b][1,4]oxazin-4-yl)-methanethione (100mg, 98%).
¹H-NMR(CDCl₃, 300MHz); δ = 8.15 (d, J=5.4Hz, 1H), 7.83 (br s, 1H), 7.55 (s, 2H), 6.88 (d, J=5.4Hz, 1H), 4.60 (m, 4H), 3.89 (s, 3H).
MS(ESI); 442.8(M⁺+1).

### b) Synthesis of (3,5-dibromo-4-hydroxy-phenyl)-(2,3-dihydro-pyrido[4,3-b][1,4]oxazin-4-yl)-methanethione

In a 25ml flask, (3,5-dibromo-4-methoxy-phenyl)-(2,3-dihydro-pyrido[4,3-b][1,4]oxazin-4-yl)-methanethione (100mg, 0.22mmol) was dissolved in N,N-dimethyl formamide, and piperazine (28.4mg, 1.5 equivalents) and lithium bromide (76.4mg, 4 equivalents) were added thereto and then stirred at 100°C for 15 hours. N,N-dimethyl formamide was removed under reduced pressure and water was added the residue. The mixture in suspension was adjusted to pH 6 to 7 by using 1N hydrochloric acid and stirred for 30 minutes. The mixture was filtered and the resulting solid was stirred in methanol solvent and then filtered to give the target compound 51 as yellow solid (100mg, quantitative yield).
¹H-NMR(DMSO-d₆, 300MHz); δ = 8.06 (d, *J*=5.4Hz, 1H), 7.83 (br s, 1H), 7.68 (s, 2H), 7.00 (d, *J*=5.4Hz, 2H), 4.64 (m, 4H), 4.53 (m, 2H).
MS(ESI); 428.8 (M⁺+1).

### Example 52)

### Synthesis of (3,5-dibromo-4-hydroxy-phenyl)-(7-pyridin-3-yl-2,3-dihydro-pyrido[2,3-b][1,4]oxazin-1-yl]-methanone (compound 52)

### a) Synthesis of 7-pyridin-3-yl-1H-pyrido[2,3-b][1,4]oxazin-2-one

To 1.6ml of acetonitrile/0.4ml of distilled water, 7-bromo-1H-pyrido[2,3-b][1,4]oxazin-2-one (100mg, 0.436mmol), 3-pyridine boronic acid (29.5mg, 0.240mmol), palladium acetate (4.9mg, 0.0218mmol), triphenylphosphine (11.4mg, 0.0436mmol) and potassium carbonate (45.2mg, 0.327mmol) were added and then stirred at 100°C for 10 hours. After completion of the reaction, the mixture was extracted with ethyl acetate, and the combined organic layer was dried over anhydrous sodium sulfate (Na₂SO₄), filtered and evaporated under reduced pressure. The residue was purified by column chromatography on silica eluting with a solvent of dichloromethane:methanol=50:1. The fractions containing the product were collected and evaporated to obtain 7-pyridin-3-yl-1H-pyrido[2,3-b][1,4]oxazin-2-one as brown oil (60mg, 60%).
¹H-NMR(DMSO-d₆): δ = 10.99 (br s, 1H), 8.83 (d, *J*=2.4Hz, 1H), 8.58 (dd, 1H, *J*=5.0 Hz, 1.6Hz), 8.15 (d, *J*=2.4Hz, 1H), 8.01 (m, 1H), 7.51-7.46 (m, 2H), 4.84 (s, 2H).
MS(ESI); 228.0(M⁺+1).

### b) Synthesis of 7-pyridin-3-yl-2,3-dihydro-1H-pyrido[2,3-b][1,4]oxazine

7-pyridin-3-yl-1H-pyrido[2,3-b][1,4]oxazin-2-one (60mg, 0.264mmol) was dissolved in 4ml of anhydrous tetrahydrofuran under nitrogen atmosphere, and the mixture was cooled to 0°C, and 1.0M Lithium aluminum hydride dissolved in tetrahydrofuran (0.7ml, 0.704mmol) was added thereto dropwise at room temperature for 5 hours. To the reaction mixture, water (0.3ml), 10% sodium hydroxide (0.6ml) and water (0.9ml) were added dropwise in this order, and then stirred vigorously at room temperature for 1 hour. The formed solid was filtered and washed with excess ethyl acetate. The filtrate was washed with water and saturated saline solution, dried over anhydrous Na₂SO₄, filtered and evaporated under reduced pressure to obtain 7-pyridin-3-yl-2,3-dihydro-1H-pyrido[2,3-b][1,4]oxazine as white solid (30mg, 53%).
MS(ESI); 214.0(M⁺+1).

### c) Synthesis of [3,5-dibromo-4-methoxy-phenyl)-(7-pyridin-3-yl-2,3-dihydro-pyrido[2,3-b][1,4]oxazin-1-yl]-methanone

In a 10ml flask, 7-pyridin-3-yl-2,3-dihydro-1H-pyrido[2,3-b][1,4]oxazine (30mg, 0.140mmol) and 3,5-dibromo-4-methoxy-benzoyl chloride (50.5mg, 0.154mmol) were dissolved in dichloromethane (2ml). Triethylamine (0.058ml, 0.42mmol) was added thereto and then stirred at room temperature for 1 hour. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by preparative TLC eluting a solvent of ethyl acetate:methanol=20:1. Silica gels containing the product were eluted with dichloromethane and the solvent was evaporated to obtain (3,5-dibromo-4-methoxy-phenyl)-(7-pyridin-3-yl-2,3-dihydro-pyrido[2,3-b][1,4]oxazin-1-yl)-methanone as white solid (35mg, 49%).
¹H-NMR(DMSO-d₆, 300MHz); δ = 8.54 (dd, *J*=4.7Hz, 1.6Hz, 2H), 8.32 (d, *J*=2.3Hz, 1H), 7.92 (s, 2H), 7.86 (m, 1H), 7.45 (dd, *J*=4.7Hz, 5.1Hz, 1H), 4.50 (m, 2H), 3.92 (m, 2H), 3.85 (s, 3H). MS(ESI); 503.9(M⁺+1).

### d) Synthesis of (3,5-dibromo-4-hydroxy-phenyl)-(7-pyridin-3-yl-2,3-dihydro-pyrido[2,3-b][1,4]oxazin-1-yl]-methanone

In a 5ml flask, (3,5-dibromo-4-methoxy-phenyl)-(7-pyridin-3-yl-2,3-dihydro-pyrido[2,3-b][1,4]oxazin-1-yl)-methanone (35mg, 0.069mmol) was dissolved in dichloromethane (1ml), and the mixture was cooled to 0°C. 1.0M Boron tribromide dissolved in dichloromethane (0.4ml, 0.415mmol) was added thereto and then stirred at room temperature for 15 hours. n-hexane solvent was added thereto and the formed solid was filtered. The resulting solid was dissolved in methanol and concentrated. After dissolving by adding water, the mixture was adjusted to pH 6 with saturated solution of sodium hydrogen carbonate, and extracted with dichloromethane. The combined organic layer was dried over anhydrous sodium sulfate (Na₂SO₄), filtered and evaporated under reduced pressure. The residue was recrystallized from dichloromethane and n-hexane to obtain the target compound 52 as white solid (18mg, 53%).
¹H-NMR(DMSO-d₆, 300MHz); δ = 10.67 (br s, 1H), 8.64 (s, 1H), 8.54 (dd, *J*=5.1Hz, 1.5Hz, 1H), 8.3 (d, *J*=1.5Hz, 1H) 8.10 (br s, 1H), 7.89 (m, 1H), 7.79 (s, 2H), 7.46 (dd, *J*=4.9Hz, 1.5Hz, 1H), 4.48-4.45 (m, 2H), 3.94-3.91 (m, 2H).
MS(ESI); 489.9(M⁺+1).

### Example 53)

### Synthesis of (3,5-dibromo-4-hydroxy-phenyl)-(7-furan-3-yl-2,3-dihydro-pyrido[2,3-b][1,4]oxazin-1-yl]-methanone (compound 53)

### a) Synthesis of 7-furan-3-yl-2,3-dihydro-1H-pyrido[2,3-b][1,4]oxazine

To 2.0ml of acetonitrile/0.5ml of distilled water, 7-bromo-2,3-dihydro-1H-pyrido[2,3-b][1,4]oxazine (80mg, 0.372mmol), 3-furan boronic acid (45.7mg, 0.409mmol), palladium acetate (8.3mg, 0.0372mmol), triphenylphosphine (19.5mg, 0.0744mmol) and potassium carbonate (102.8mg, 0.744mmol) were added and reacted at 100°C for 10min using microwave (50W) apparatus. After completion of the reaction, the mixture was extracted with ethyl acetate, and the combined organic layer was dried over anhydrous sodium sulfate (Na₂SO₄), filtered and evaporated under reduced pressure. The residue was purified by preparative TLC eluting a solvent of dichloromethane:methanol=20:1. Silica gels containing the product were eluted with dichloromethane and the solvent was evaporated to obtain 7-furan-3-yl-2,3-dihydro-1H-pyrido[2,3-b][1,4]oxazine in oil form (18mg, 24%).
MS(ESI); 203.0(M⁺+1).

### b) Synthesis of (3,5-dibromo-4-methoxy-phenyl)-(7-furan-3-yl-2,3-dihydro-pyrido[2,3-b][1,4]oxazin-1-yl)-methanone

In a 5ml flask, 7-furan-3-yl-2,3-dihydro-1H-pyrido[2,3-b][1,4]oxazine (18mg, 0.089mmol) and 3,5-dibromo-4-methoxy-benzoyl chloride (32.1mg, 0.098mmol) were dissolved in dichloromethane (1ml). Triethylamine (0.037ml, 0.267mmol) was added thereto and then stirred at 0°C for 3 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by preparative TLC eluting a solvent of dichloromethane:methanol=20:1. Silica gels containing the product were eluted with dichloromethane and the solvent was evaporated to obtain (3,5-dibromo-4-methoxy-phenyl)-(7-furan-3-yl-2,3-dihydro-pyrido[2,3-b][1,4] oxazin-1-yl)-methanone as white solid (40mg, 91 %).
¹H-NMR(CDCl₃, 300MHz); δ = 8.17 (d, 1H, *J*=2.2Hz), 7.70 (s, 2H), 7.57 (br s, 1H), 7.46 (dd, 1H, *J*=1.7Hz, 1.8Hz), 7.26 (s, 1H), 6.51 (m, 1H), 4.49-4.46 (m, 2H), 3.99-3.96 (m, 2H). MS(ESI); 492.9(M⁺+1).

### c) Synthesis of (3,5-dibromo-4-hydroxy-phenyl)-(7-furan-3-yl-2,3-dihydro-pyrido[2,3-b][1,4]oxazin-1-yl)-methanone

In a 5ml flask, (3,5-dibromo-4-methoxy-phenyl)-(7-furan-3-yi-2,3-dihydro-pyrido[2,3-b][1,4]oxazin-1-yl)-methanone (40mg, 0.081mmol) was dissolved in dichloromethane (1ml) and then cooled to 0°C. 1.0M Boron tribromide dissolved in dichloromethane (0.8ml, 0.81mmol) was added thereto and then stirred at room temperature for 15 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by preparative TLC eluting a solvent of dichloromethane:methanol=20:1. Silica gels containing the product were eluted with dichloromethane and the solvent was evaporated to obtain the target compound 53, i.e., (3,5-dibromo-4-hydroxy-phenyl)-(7-furan-3-yl-2,3-dihydro-pyrido[2,3-b][1,4]oxazin-1-yl)-methanone as white solid (30mg, 77%).
¹H-NMR(CD₃OD, 300MHz); δ = 8.11 (d, 1H, *J*=2.0Hz), 7.99 (br s, 1H), 7.76 (s, 3H), 7.55 (dd, 1H, *J*=1.8Hz, 1.5Hz), 6.63 (m, 1H), 4.48-4.45 (m, 2H), 4.02-3.99 (m, 2H).
MS(ESI); 478.9(M⁺+1).

### Example 54)

### Synthesis of 1-(3,5-dibromo-4-hydroxy-phenyl)-2-(2,3-dihydro-pyrido[4,3-b][1,4]oxazin-4-yl)ethanone (compound 54)

### a) Synthesis of 2-bromo-1-(3,5-dibromo-4-hydroxy-phenyl)-ethanone

In a 100ml flask, 1-(3,5-dibromo-4-hydroxy-phenyl)-ethanone (1.2g, 4.082mmol) was dissolved in a solvent of anhydrous tetrahydrofuran: anhydrous diethyl ether (1:1, 12ml). Acetic acid (0.4ml) and bromine (2.1ml, 4.082mmol) were added thereto dropwise under nitrogen atmosphere and then stirred at room temperature for 2 hours. After completion of the reaction by adding water dropwise, the mixture was adjusted to pH 8 to 9 with saturated solution of sodium hydrogen carbonate and extracted with ethyl acetate, and the combined organic layer was dried over anhydrous sodium sulfate (Na₂SO₄), filtered and evaporated under reduced pressure. The residue was purified by column chromatography on silica eluting with a solvent of n-hexane:ethyl acetate=2:1. The fractions containing the product were collected and evaporated to obtain 2-bromo-1-(3,5-dibromo-4-hydroxy-phenyl)-ethanone as white solid (785mg, 51.5%).
¹H-NMR(CDCl₃, 300MHz); δ = 8.21 (s, 2H), 6.39 (br s, 1H), 4.36 (s, 2H).
MS(ESI); 370.8(M⁺+1).

### b) Synthesis of 1-(3,5-dibromo-4-hydroxy-phenyl)-2-(2,3-dihydro-pyrido[4,3-b][1,4]oxazin-4-yl)-ethanone

In a 10ml flask, (3,4-dihydro-2H-pyrido[4,3-b][1,4]oxazine (60mg, 0.44mmol) was dissolved in anhydrous acetonitrile (5ml). At room temperature, 2-bromo-1-(3,5-dibromo-4-hydroxy-phenyl)-ethanone (164mg, 0.44mmol) and potassium carbonate (121mg, 0.88mmol) were added thereto and then stirred at room temperature for 16 hours. The reaction mixture was filtered and the resulting white solid was recrystallized from methanol to obtain the target compound 54 as white solid (110mg, 58.5%).
¹H-NMR(CDCl₃, 300MHz); δ = 7.96-8.00 (m, 2H), 7.89 (s, 2H), 7.31 (d, *J*=6.3Hz, 1H), 7.18 (br s, 1H), 5.87 (br s, 2H), 4.45-4.47 (m, 2H), 3.31-3.44 (m, 2H).
MS(ESI); 426.8(M⁺+1).

### Example 55)

### Synthesis of (3,5-dibromo-4-hydroxy-phenyl)-(2-3-dihydro-4-oxa-1,9-diaza-phenanthren-1-yl)-methanone (compound 55)

### a) Synthesis of 3-amino-quinolin-4-ol

In a 100ml flask, 3-nitro-quinolin-4-ol (500mg, 2.629mmol) was dissolved in methanol (50ml) and 10% palladium on active carbon (1.2g, 10w/w%) was added thereto and then stirred for 3 hours at room temperature under hydrogen atmosphere. The reaction mixture was filtered on celite and then evaporated under reduced pressure to obtain brown liquid (445mg, quantitative yield).
¹H-NMR(DMSO-d₆, 300MHz); δ = 11.50 (br s, 1H), 8.09 (d, *J*=8.4Hz, 1H), 7.48 (s, 1H), 7.47 (m, 2H), 7.14-7.20 (m, 1H), 4.38 (br s, 2H).

### b) Synthesis of 1H-4-oxa-1,9-diaza-phenanthren-2-one

In a 50ml flask equipped with a reflux appatus, 3-amino-quinolin-4-ol (421mg, 2.628mmol) was dissolved in N,N-dimethyl formamide (13ml) under nitrogen atmosphere, and then chloroacetyl chloride (0.21ml, 2.76mmol) was added thereto dropwise at 0 °C, and then stirred at 0 °C for 30 minutes. Potassium carbonate (24g, 177mmol) was added thereto and the mixture was heated to 100 °C under reflux. After 16 hours, N,N-dimethyl formamide solvent was evaporated under reduced pressure and methanol was added to the residue, and then the mixture was filtered on celite. The filtrate was evaporated under reduced pressure to obtain the target compound, i.e., 1H-4-oxa-1,9-diaza-phenanthren-2-one, as brown solid (526mg, quantitative yield).
¹H-NMR(DMSO-d₆, 300MHz); δ = 8.28 (s, 1H), 7.72-7.83 (m, 2H), 7.35 (m, 2H), 4.43 (s, 2H). MS(ESI); 201.0(M⁺+1).

### c) Synthesis of 2,3-dihydro-1H-4-oxa-1,9-diaza-phenanthrene

1H-4-oxa-1,9-diaza-phenanthren-2-one (526mg, 2.63mmol) was dissolved in tetrahydrofuran (13ml) and then cooled to 0°C. To the reaction mixture, 1.0M Lithium aluminum hydride dissolved in tetrahydrofuran (5.5ml, 5.26mmol) was added dropwise and stirred at room temperature for 2 hours. After completion of reaction, water (1ml), 10% aqueous solution of sodium hydroxide (2ml) and water (3ml) were added thereto dropwise in this order and then stirred for 1 hour at room temperature. After filtering precipitates, the filtrate was extracted with ethyl acetate. The combined organic layer was dried over anhydrous sodium sulfate (Na₂SO₄), filtered and evaporated under reduced pressure. The residue was purified by column chromatography on amine silica eluting with a solvent of dichloromethane. The fractions containing the product were collected and evaporated to obtain colorless liquid (52mg, 10.6%).
¹H-NMR(CDCl₃, 300MHz); δ = 8.37 (s, 1H), 7.90-7.96 (m, 2H), 7.27-7.48 (m, 2H), 4.50 (m, 2H), 3.56 (m, 2H).
MS(ESI); 187.0(M⁺+1).

### d) Synthesis of (3,5-dibromo-4-methoxy-phenyl)-(2,3-dihydro-4-oxa-1,9-diaza-phenanthren-1-yl)-methanone

In a 10ml flask, 2,3-dihydro-1H-4-oxa-1,9-diaza-phenanthrene (50mg, 0.269mmol) and 3,5-dibromo-4-methoxy-benzoyl chloride (88mg, 0.269mmol) were dissolved in dichloromethane. Triethylamine (0.51ml, 3.7mmol) was added thereto and then stirred at room temperature for 3 hours. After neutralizing with 1N hydrochloric acid solution, the mixture was extracted with dichloromethane, and the combined organic layer was dried over anhydrous sodium sulfate (Na₂SO₄), filtered and evaporated under reduced pressure. The residue was purified by column chromatography on amine silica eluting with a solvent of dichloromethane:methanol=20:1. The fractions containing the product were collected and evaporated to obtain the target compound as yellow solid (110mg, 86%).
¹H-NMR(CDCl₃, 300MHz); δ = 8.57 (br s, 1H), 7.96-8.14 (m, 2H), 7.73 (s, 2H), 7.54-7.73 (m, 2H), 4.65 (m, 2H), 4.11 (m, 2H), 3.94 (s, 3H).
MS(ESI); 475.9(M⁺+1).

### e) Synthesis of (3,5-dibromo-4-hydroxy-phenyl)-(2,3-dihydro-4-oxa-1,9-diaza-phenanthren-1-yl)-methanone

In a 10ml flask, (3,5-dibromo-4-methoxy-phenyl)-(2,3-dihydro-4-oxa-1,9-diaza-phenanthren-1-yl)-methanone (30mg, 0.063mmol), lithium bromide (12mg, 0.134mmol) and piperazine (9mg, 0.101mmol) were dissolved in N,N-dimethyl formamide (3ml), and then stirred at 100°C for 2 hours. After completion of the reaction by adding water dropwise, the mixture was adjusted to weak acidc condition (pH=6) by using 1N hydrochloric acid. The formed solid was filtered and washed with distilled water, and recrystallized from tetrahydrofuran and methanol to obtain the target compound 55, i.e., (3,5-dibromo-4-hydroxy-phenyl)-(2,3-dihydro-4-oxa-1,9-diaza-phenanthren-1-yl)-methanone, as yellow solid (10mg, 34%).
¹H-NMR(DMSO-d₆, 300MHz); δ = 10.66 (br s, 1H), 8.71 (br s, 1H), 8.08 (d, *J*=8.4Hz, 1H), 7.90 (d, *J*=6.9Hz, 1H), 7.83 (s, 2H), 7.70-7.59 (m, 2H), 4.63 (m, 2H), 4.02 (m, 2H).
MS(ESI); 461.9(M⁺+1).

### Example 56)

### Synthesis of 4-[2-(3,5-dibromo-4-hydroxy-phenyl)-2-oxo-ethyl)]-4H-pyrido[4,3-b][1,4]oxazin-3-one (compound 56)

In a 10ml flask, 4H-pyrido[4,3-b][1,4]oxazin-3-one (50mg, 0.33mmol) was dissolved in N,N-dimethyl formamide (2.0ml) and then cooled to 0°C. Sodium hydride (15mg, 0.37mmol) was added thereto at 0°C and then stirred at room temperature for 30 minutes. The reaction mixture was cooled to 0°C, 2-bromo-1-(3,5-dibromo-4-hydroxy-phenyl)-ethanone (124mg, 0.33mmol) was added thereto dropwise and stirred at 0°C for 30 minutes, and then the mixture was stirred at room temperature for 1 hour. The precipitates formed were filtered and washed with acetonitrile. The resulting solid was recrystallized from methanol to obtain the target compound 56, i.e., 4-[2-(3,5-dibromo-4-hydroxy-phenyl)-2-oxo-ethyl]-4H-pyrido[4,3-b][1,4]oxazin-3-one, as white solid (48mg, 33%).
¹H-NMR(DMSO-d₆, 300MHz); δ = 11.66(s, 1H), 8.36 (dd, *J*=6.9Hz, 1.5Hz, 1H), 8.15 (d, *J*=1.5Hz, 1H), 7.88 (s, 2H), 7.60 (d, *J*=6.9Hz, 1H), 5.99 (s, 2H), 5.02 (m, 2H).
MS(ESI); 440.9(M⁺+1).

### Example 57)

### Synthesis of 4-(3,5-dibromo-4-methoxy-benzoyl)-4H-pyrido[4,3-b][1,4]oxazin-3-one (compound 57)

By the same method as in Example 56, 4H-pyrido[4,3-b][1,4]oxazin-3-one (200mg, 1.33mmol), sodium hydride (56mg, 1.40mmol) and 3,5-dibromo-4-methoxy-benzoyl chloride (437mg, 1.33mmol) were reacted, and then water was added thereto and the mixture was extracted with ethyl acetate. The combined organic layer was dried over anhydrous sodium sulfate (Na₂SO₄), filtered and evaporated under reduced pressure. The residue was purified by column chromatography on silica eluting with a solvent of ethyl acetate:methanol=9:1. The fractions containing the product were collected and evaporated to obtain the target compound 57, i.e., 4-(3,5-dibromo-4-methoxy-benzoyl)-4H-pyrido[4,3-b][1,4]oxazin-3-one, as white solid (125mg, 21%).
¹H-NMR(DMSO-d₆, 300MHz); δ = 8.34 (s, 1H), 8.30 (s, 2H), 8.25 (d, *J*=5.4Hz, 1H), 7.18 (d, *J*=5.4Hz, 1H), 4.93 (s, 2H), 3.89 (s, 3H).
MS(ESI); 440.9(M⁺+1).

### Example 58)

### Synthesis of (3,5-dibromo-4-methoxy-phenyl)-(6-methyl-2,3-dihydro-pyrido[2,3-b][1,4]oxazin-1-yl)-methanone (compound 58)

In a 10ml flask, 6-methyl-2,3-dihydro-1H-pyrido[2,3-b][1,4]oxazine (110mg, 0.73mmol) and 3,5-dibromo-4-methoxy-benzoyl chloride (240mg, 0.73mmol) were dissolved in dichloromethane (5ml). Triethylamine (0.26ml, 1.8mmol) was added thereto and then stirred at room temperature for 1 hour. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by column chromatography on amine silica (NH-silica gel) eluting with a solvent of dichloromethane:methanol=19:1. The fractions containing the product were collected and evaporated to obtain the target compound 58, i.e., (3,5-dibromo-4-methoxy-phenyl)-(6-methyl-2,3-dihydro-pyrido[2,3-b][1,4]oxazin-1-yl)-methanone, as white solid (230mg, 71%).
¹H-NMR(DMSO-d₆, 300MHz); δ = 8.20-7.90 (br s, 1H), 7.87 (s, 2H), 6.79-6.82 (m, 1H), 4.37 (m, 2H), 3.84 (s, 3H), 3.82 (m, 2H), 2.31 (s, 3H).
MS(ESI); 440.9(M⁺+1).

### Example 59)

### Synthesis of (2,3-dihydro-pyrido[14,3-b][1,4]oxazin-4-yl)-(2,4-dihydroxy-pyrimidin-5-yl)-methanone (compound 59)

2,4-dihydroxy-pyrimidin-5-carboxylic acid (125mg, 0.80mmol) was dissolved in N,N-dimethyl acetamide (3ml) and then cooled to -15°C. Thionyl chloride (0.066ml, 0.90mmol) was added thereto at -15°C and the reaction mixture was stirred at -5°C for 20 minutes. The reaction mixture was cooled to -15°C, and potassium carbonate (138mg, 1.0mmol) and 3,4-dihydro-2H-pyrido[4,3-b][1,4]oxazine (109mg, 0.80mmol) were added thereto dropwise. The reaction mixture was stirred at -5°C for 30 minutes, and then stirred at room temperature for 3 hours. After the completion of the reaction, water was added thereto and the mixture was extracted with ethyl acetate, and the combined organic layer was dried over anhydrous sodium sulfate (Na₂SO₄), filtered and evaporated under reduced pressure. The residue was purified by column chromatography on silica eluting with a solvent of dichloromethane:methanol=9:1. The fractions containing the product were collected and evaporated to obtain the target compound 59 as white solid (47mg, 21 %).
¹H-NMR(DMSO-d₆, 300MHz); δ = 11.47 (s, 1H), 11.35 (s, 1H), 8.76 (br s, 1H), 8.08 (d, *J*=5.7Hz, 1H), 7.85 (s, 1H), 6.92 (d, *J*=5.7Hz, 1H), 4.34 (m, 2H), 3.84 (m, 2H).
MS(ESI); 275.0(M⁺+1).

### Example 60)

### Synthesis of (2,3-dihydro-pyrido[4,3-b[[1,4]oxazin-4-yl)-(2,6-dihydroxy-pyrimidin-4-yl)-methanone (compound 60)

By the same method as in Example 59, 2,6-dihydroxy-pyrimidin-4-carboxylic acid (125mg, 0.80mmol), thionyl chloride (0.066ml, 0.90mmol), potassium carbonate (138mg, 1.0mmol) and 3,4-dihydro-2H-pyrido[4,3-b][1,4]oxazine (109mg, 0.80mmol) were reacted and the reaction mixture was purified in the same manner to obtain the target compound 60, i.e., (2,3-dihydro-pyrido[4,3-b][1,4]oxazin-4-yl)-(2,6-dihydroxy-pyrimidin-4-yl)-methanone, as white solid (32mg, 15%).
¹H-NMR(DMSO-d₆, 300MHz); δ = 11.35-11.29 (m, 2H), 9.06 (br s, 1H), 8.18 (d, *J*=5.4Hz, 1H), 7.01 (d, *J*=5.4Hz, 1H), 5.81 (s, 1H), 4.42 (m, 2H), 3.95 (m, 2H).
MS(ESI); 275.0 (M⁺+1).

### Example 61)

### Synthesis of (3,5-dibromo-4-hydroxy-phenyl)-(7-isoquinolin-4-yl-2,3-dihydro-pyrido[2,3-b][1,4]oxazin-1-yl)-methanone (compound 61)

### a) Synthesis of 7-isoquinolin-4-yl-2,3-dihydro-1H-pyrido[2,3-b[1,4]oxazine

Under nitrogen ahmosphere, in a 10ml flask, 7-bromo-2,3-dihydro-1H-pyrido[2,3-b][1,4]oxazine (36.3mg, 0.169mmol), isoquinoline boronic acid (44mg, 0.253mmol), tetrakis triphenylphosphine palladium (19.5mg, 0.017mmol) and potassium carbonate (58.3mg, 0.423mmol) were dissolved in a solvent of tetrahydrofuran/water (6/1, 1.4ml/0.3ml). The reaction mixture was stirred for 30 minutes at 80 °C using microwave (40W) device. After cooling to room temperature, the mixture was extracted with ethyl acetate, and the organic layer was washed with saturated saline solution. The combined organic layer was dried over anhydrous magnesium sulfate (MgSO₄), filtered and evaporated under reduced pressure. The residue was purified by column chromatography on amine silica eluting with a solvent of dichloromethane. The fractions containing the product were collected and evaporated to obtain brown oil (22.6mg, 51 %).
¹H-NMR(CDCl₃, 300MHz); δ = 9.23 (s, 1H), 8.43 (s, 1H), 7.59-8.04 (m, 5H), 7.00 (d, *J*=2.1Hz, 1H), 4.48-4.51 (m, 2H), 4.10 (s, 1H), 3.49-3.51 (m, 2H).

### b) Synthesis of (3,5-dibromo-4-methoxy-phenyl)-(7-isoquinolin-4-yl-2,3-dihydro-pyrido[2,3-b][1,4]oxazin-1-yl)-methanone

Under nitrogen ahmosphere, in a 10ml flask, 7-isoquinolin-4-yl-2,3-dihydro-1H-pyrido[2,3-b][1,4]oxazine (22.6mg, 0.085mmol) and 3,5-dibromo-4-methoxy-benzoyl chloride (42.2mg, 0.129mmol) were dissolved in dichloromethane (1.0ml). After cooling to 0°C, triethylamine (0.06ml, 0.429mmol) was added thereto at room temperature for 3 hours. After neutralizing with 1N hydrochloric acid solution, the mixture was extracted with dichloromethane, and the combined organic layer was dried over anhydrous magnesium sulfate (MgSO₄), filtered and evaporated under reduced pressure. The residue was purified by column chromatography on amine silica eluting with a solvent of ethyl acetate and n-hexane. The fractions containing the product were collected and evaporated to obtain white solid (11.0mg, 23%).
¹H-NMR(CDCl₃, 300MHz); δ = 9.25 (s, 1H), 8.34 (s, 1H), 8.19 (d, *J*=2.1Hz, 1H), 8.02 (dd, *J*=6.6Hz, 1.5Hz, 1H), 7.58-7.76 (m, 6H), 4.59-4.63 (m, 2H), 4.06-4.09 (m, 2H), 3.92 (s, 3H).

### c) Synthesis of (3,5-dibromo-4-hydroxy-phenyl)-(7-isoquinolin-4-yl-2,3-dihydro-pyrido[2,3-b][1,4]oxazin-1-yl)-methanone

Under nitrogen ahmosphere, in a 10ml flask, (3,5-dibromo-4-methoxy-phenyl)-(7-isoquinolin-4-yl-2,3-dihydro-pyrido[2,3-b][1,4]oxazin-1-yl)-methanone (11.3mg, 0.020mmol) was dissolved in dichloromethane, and then cooled to 0°C. 1.0M Boron tribromide dissolved in dichloromethane (0.2ml, 0.203mmol) was added thereto and then stirred at room temperature for 18 hours. After adjusting to pH 6 with 1N NaOH solution, the mixture was extracted with dichloromethane, and the combined organic layer was dried over anhydrous sodium sulfate (Na₂SO₄), filtered and evaporated under reduced pressure. Diethyl ether was added to the formed solid and stirred for 30 minutes and then filtered to obtain the target compound 61 as white solid (3.0mg, 27%).
¹H-NMR(CD₃OD, 300MHz); δ = 9.47 (s, 1H), 8.32-8.42 (m, 2H), 8.12 (d, *J*=2.4Hz, 1H), 8.00-7.66 (m, 6H), 4.66-4.63 (m, 2H), 4.09-4.07 (m, 2H).
MS(ESI); 539.8(M⁺+1).

### Example 62)

### Synthesis of (3,5-dibromo-4-hydroxy-phenyl)-(6,7-dihydro-dihydro-pyrimido[4,5-b][1,4]oxazin-5-yl)-methanone(compound 62)

### a) Synthesis of (6-chtoro-5-nitro-pyrimidin-4-yloxy)-acetic acid methyl ester

In a 50ml flask at low temperature, methyl glycolate (0.22ml, 2.8mmol) and sodium hydride (114mg, 2.8mmol) were dissolved in N,N-dimethyl formamide (7ml) and then stirred for 10 minutes. 4,6-dichloro-5-nitro-pyrimidine (500mg, 2.6mmol) dissolved in N,N-dimethyl formamide (6.5ml) was added thereto dropwise and then stirred at the same temperature for additional 10 minutes, and then the temperature was raised to room temperature and stirred for additional 10 hours. After completion of the reaction by adding water, the mixture was extracted with dichloromethane, and the combined organic layer was washed with saturated saline solution, dried over anhydrous sodium sulfate (Na₂SO₄), filtered and evaporated under reduced pressure. The residue was purified by column chromatography eluting with a solvent of dichloromethane to obtain yellow liquid (280mg, 44%).
¹H-NMR(CDCl₃, 300MHz); δ = 8.62 (s, 1H), 5.08 (s, 2H), 3.80 (s, 3H).
MS(ESI); 248.0(M⁺+1).

### b) Synthesis of 5H-pyrimido-[4,5-b][1,4]oxazin 6-one

In a 25ml flask, (6-chloro-5-nitro-pyrimidin-4-yloxy)-acetic acid methyl ester (280mg, 1.1mmol) and tin tetrachloride (849mg, 4.5mmol) were dissolved in hydrochloric acid (7ml) and heated for 1 hour at 80°C. The reaction mixture was cooled to room temperature and neutralized by adding 10% sodium hydroxide solution dropwise, and the formed solid was removed by filtration. The filtrate was extracted with ethyl acetate, and the combined organic layer was washed with saturated saline solution, dried over anhydrous sodium sulfate (Na₂SO₄), filtered and evaporated under reduced pressure. The obtained yellow solid mixture (107mg) was used in the next step without further purification.
¹H-NMR(CDCl₃, 300MHz); δ = 8.58 (s, 1H), 8.19 (s, 1H), 4.95 (s, 2H), 4.33 (br s, 1H).
MS(ESI); 152.0(M⁺+1).

### c) Synthesis of 6,7-dihydro-5H-pyrimido-[4,5-b][1,4]oxazine

In a 10ml flask, the mixture of 5H-pyrimido-[4,5-b][1,4]oxazin-6-one (107mg, 0.58mmol) was dissolved in tetrahydrofuran (3ml) and then cooled to 0°C. 1.0M Lithium aluminum hydride dissolved in tetrahydrofuran (1.15ml, 1.2mmol) was added thereto dropwise and the temperature was raised to room temperature and then stirred for additional 2 hours. After cooling to low temperature again, water, 10% sodium hydroxide solution and water were added in this order to quench the reaction. The mixture was filtered and the filtrate was extracted with ethyl acetate and the combined organic layer was washed with saturated saline solution, dried over anhydrous sodium sulfate (Na₂SO₄), filtered and evaporated under reduced pressure. The residue was purified by column chromatography on amine silica gel eluting with a solvent of dichloromethane to obtain white solid (20mg, 25%).
¹H-NMR(CDCl₃, 300MHz); δ = 8.27 (s, 1H), 7.94 (s, 1H), 4.50-4.47 (m, 2H), 3.87 (br s, 1H), 3.49-3.45 (m, 2H).
MS(ESI); 137.9(M⁺+1).

### d) Synthesis of (3,5-dibromo-4-methoxy-phenyl)-(6,7-dihydro-pyrimido[4,5-b][1,4]oxazin-5-yl)-methanone

In a 10ml flask, 6,7-dihydro-5H-pyrimido-[4,5-b][1,4]oxazine (20mg, 0.15mmol) and triethylamine (0.1ml, 0.73mmol) were dissolved in dichloromethane (1.5ml). To the reaction mixture, 3,5-dibromo-4-methoxy-benzoyl chloride (50mg, 0.15mmol) was added and stirred at room temperature for 12 hours. The solvent was evaporated under reduced pressure and white non-crystalline compound (28mg, 45%) was obtained by column chromatography on amine silica gel eluting with a solvent of dichloromethane:n-hexane=1:1.
¹H-NMR(CDCl₃, 300MHz); δ = 8.80 (s, 1H), 8.61 (s, 1H), 7.69 (s, 2H), 4.55-4.52 (m, 2H), 4.02-3.99 (m, 2H), 3.95 (s, 3H).
MS(ESI); 427.9 (M⁺+1).

### e) Synthesis of (3,5-dibromo-4-hydroxy-phenyl)-(6,7-dihydro-pyrimido[4,5-b][1,4]oxazin-5-yl)-methanone

In a 10ml flask, (3,5-dibromo-4-methoxy-phenyl)-(6,7-dihydro-pyrimido[4,5-b][1,4]oxazin-5-yl)-methanone (26mg, 0.06mmol), lithium bromide (16mg, 0.18mmol) and piperazine (8mg, 0.09mmol) were dissolved in N,N-dimethyl formamide (1.0ml) and heated at 100°C for 6 hours. The reaction mixture was cooled to room temperature, evaporated under reduced pressure, and diluted by adding water. The diluted reaction mixture was neutralized with diluted hydrochloric acid, and the formed white solid was filtered to obtain the target compound 62 (25mg, quantitative yield).
¹H-NMR(DMSO-d₆, 300MHz); δ = 10.6 (br s, 1H), 8.73 (br s, 1H), 8.49 (s, 1H), 7.79 (s, 2H), 4.52-4.49 (m, 2H), 3.93-3.91 (m, 2H).
MS(ESI); 413.9 (M⁺+1).

### Example 63)

### Synthesis of (3,5-dibromo-4-hydroxy-phenyl)-[7-(3-trifluoromethyl-phenyl)-2,3-dihydro-pyrido[2,3-b][1,4]oxazin-1-yl]-methanone (compound 63)

### a) Synthesis of 7-(3-trifluoromethyl-phenyl)-2,3-dihydro-1H-pyrido[2,3-b][1,4]oxazine

By the same method as in the step b) of Example 45, 7-bromo-2,3-dihydro-1H-pyrido[2,3-b[1,4]oxazine (100mg, 0.46mmol), 3-dimethyl phenyl boronic acid (97.2mg, 0.51mmol), palladium triphenylphosphine (26.9mg, 0.023mol) and potassium carbonate (129mg, 0.93mmol) were dissolved in a solvent of tetrahydrofuran/water (3ml/0.5ml), and reacted at 80°C for 6 hours to obtain 7-(3-trifluoromethyl-phenyl)-2,3-dihydro-1H-pyrido[2,3-b][1,4]oxazine as white solid (105mg, 80.6%).
¹H-NMR (CDCl₃, 300MHz); δ = 7.80(d, *J*=1.9Hz, 1H), 7.75-7.65(m, 2H), 7.62-7.50(m, 2H), 7.07(d, *J*=1.9Hz, 1H), 4.50-4.41(m, 2H), 3.98(br s, 1H), 3.52-3.43(m, 2H).
MS(ESI): 281.0 (M⁺+1).

### b) Synthesis of (3,5-dibromo-4-methoxy-phenyl)-[7-(3-trifluoromethyl-phenyl)-2.3-dihydro-pyrido[2,3-b][1,4]oxazin-1-yl-methanone

By the same method as in the step c) of Example 45, 7-(3-trifluoromethyl-phenyl)-2,3-dihydro-1H-pyrido[2,3-b][1,4]oxazine (99mg, 0.35mmol), 3,5-dibromo-4-methoxy-benzoyl chloride (139mg, 0.42mmol) and triethylamine (98.5µℓ, 0.71mmol) were dissolved in dichloromethane (3ml) and reacted at room temperature for 3 hours to obtain (3,5-dibromo-4-methoxy-phenyl)-[7-(3-trifluoromethyl-phenyl)-2,3-dihydro-pyrido[2,3-b][1,4]oxazin-1-yl]-methanone as white solid (192mg, 95%).
¹H-NMR (CDCl₃, 300MHz); 8 = 8.28(d, *J*=2.3Hz, 1H), 8.14-7.95(m, 1H), 7.78-7.72(m, 2H), 7.66-7.50(m, 4H), 4.58-4.48(m, 2H), 4.07-3.98(m, 2H), 3.94(s, 3H).
MS(ESI): 570.9 (M⁺+1).

### c) Synthesis of (3.5-dibromo-4-hydroxy-phenyl)-[7-(3-trifluoromethyl-phenyl)-2.3-dihydro-pyrido[2,3-b][1,4]oxazin-1-yl]-methanone

By the same method as in the step d) of Example 45, (3,5-dibromo-4-methoxy-phenyl)-[7-(3-trifluoromethyl-phenyl)-2,3-dihydro-pyrido[2,3-b][1,4]oxazin-1-yl]-methanone (165mg, 0.29mmol), lithium bromide (100mg, 1.15mmol) and piperazine (37.3mg, 0.43mmol) were dissolved in N,N-dimethyl formamide (2ml) and reacted at 100°C to obtain the target compound 63, i.e., (3,5-dibromo-4-hydroxy-phenyl)-[7-(3-trifluoromethyl-phenyl)-2,3-dihydro-pyrido[2,3-b][1,4]oxazin-1-yl]-methanone, as white solid (90mg, 56%).
¹H-NMR (CD₃OD, 300MHz); 8.21(d, J=2.3Hz, 1H), 8.09-8.01(m, 1H), 7.78(s, 2H), 7.74-7.57(m, 4H), 4.56-4.50(m, 2H), 4.08-4.01(m, 2H).
MS(ESI): 556.9 (M⁺+1).

### Example 64)

### Synthesis of (3,5-dibromo-4-hydroxy-phenyl)-17-(3-fluoromethyl-phenyl)-2,3-dihydro-pyrido[2,3-b][1,4]oxazin-1-yl]-methanone (compound 64)

### a) Synthesis of 7-(3-fluoromethyl-phenyl)-2,3-dihydro-1H-pyrido[2-3-b][1,4]oxazine

By the same method as in the step b) of Example 45, 7-bromo-2,3-dihydro-1H-pyrido[2,3-b[1,4]oxazine (100mg, 0.46mmol), 3-dimethyl phenyl boronic acid (71.6mg, 0.51mmol), palladium triphenylphosphine (26.9mg, 0.023mol) and potassium carbonate (129mg, 0.93mmol) were dissolved in a solvent of tetrahydrofuran/water (3ml/0.5ml) and reacted at 80°C for 6 hours to obtain 7-(3-fluoromethyl-phenyl)-2,3-dihydro-1H-pyrido[2,3-b][1,4]oxazine as white solid (92mg, 85.9%).
¹H-NMR (CDCl₃, 300MHz); δ = 7.83(d, J=1.9Hz, 1H), 7.43-7.32(m, 1H), 7.30-7.28(m, 1H), 7.22-7.16(m, 1H), 7.07-6.98(m, 2H), 4.49-4.41(m, 2H), 3.96(br s, 1H), 3.51-3.42(m, 2H).
MS(ESI): 231.0 (M⁺+1).

### b) Synthesis of (3,5-dibromo-4-methoxy-phenyl-[7-(3-fluoromethyl-phenyl)-2,3-dihydro-pyrido[2,3-b][1,4]oxazin-1-yl]-methanone

By the same method as in the step c) of Example 45, 7-(3-fluoromethyl-phenyl)-2,3-dihydro-1H-pyrido[2,3-b][1,4]oxazine (81mg, 0.35mmol), 3,5-dibromo-4-methoxy-benzoyl chloride (173mg, 0.53mmol) and triethylamine (98µℓ, 0.71mmol) were dissolved in dichloromethane (3ml) and reacted at room temperature for 3 hours to obtain (3,5-dibromo-4-methoxy-phenyl)-[7-(3-fluoromethyl-phenyl)-2,3-dihydro-pyrido[2,3-b][1,4]oxazin-1-yl]-methanone as white solid (180mg, 98%).
¹H-NMR(CDCl₃, 300MHz); δ = 8.25(d, *J*=2.3Hz, 1H), 7.98-7.83(m, 1H), 7.72(s, 2H), 7.42-7.32(m, 1H), 7.15(d, *J*=7.6Hz, 1H), 7.09-6.96(m, 2H), 4.57-4.47(m, 2H), 4.06-3.98(m, 2H), 3.95(s, 3H).
MS(ESI): 520.9 (M⁺+1).

### c) Synthesis of (3,5-dibromo-4-hydroxy-phenyl)-[7-(3-fluoromethyl-phenyl)-2,3-dihydro-pyrido[2,3-b][1,4]oxazin-1-yl]-methanone

By the same method as in the step d) of Example 45, (3,5-dibromo-4-methoxy-phenyl)-[7-(3-fluoromethyl-phenyl)-2,3-dihydro-pyrido[2,3-b][1,4]oxazin-1-yl]-methanone (162mg, 0.31mmol), lithium bromide (108mg, 1.24 mmol) and piperazine (40mg, 0.47mmol) were dissolved in N,N-dimethyl formamide (3ml) and reacted at 100 °C to obtain the target compound 64, i.e., (3,5-dibromo-4-hydroxy-phenyl)-[7-(3-fluoromethyl-phenyl)-2,3-dihydro-pyrido[2,3-b][1,4]oxazin-1-yl]-methanone, as white solid (69.4mg, 44%).
¹H-NMR(CD₃OD, 300MHz); δ = 8.18(d, *J*=2.3Hz, 1H), 8.09-8.01(m, 1H), 7.78(s, 2H), 7.48-7.36(m, 1H), 7.27-7.13(m, 2H), 7.12-7.03(m, 1H), 4.55-4.46(m, 2H), 4.06-3.98(m, 2H).
MS(ESI): 506.9 (M⁺+1).

### Example 65)

### Synthesis of 4-(1-(3,5-dibromo-4-hydroxy-benzoyl)-2,3-dihydro-1H-pyrido(2,3-b][1,4]oxazin-7-yl]-benzonitrile (compound 65)

### a) Synthesis of 4-(2,3-dihydro-1H-pyrido[2,3-b][1,4]oxazin-7-yl]-benzonitrile

By the same method as in the step b) of Example 49, 7-bromo-2,3-dihydro-1H-pyrido[2,3-b][1,4]oxazine (120mg, 0.56mmol), 4-benzonitrile boronic acid (90.2mg, 0.61mmol), palladium triphenylphosphine (32.2mg, 0.028mol) and potassium carbonate (154mg, 1.12mmol) were dissolved in a solvent of tetrahydrofuran/water (3ml/0.5ml) and reacted at 80°C for 6 hours to obtain 4-(2,3-dihydro-1H-pyrido[2,3-b][1,4]oxazin-7-yl]-benzonitrile as pale-green solid (81.7mg, 62%).
¹H-NMR(CDCl₃, 300MHz); δ = 7.85(d, *J*=1.9Hz, 1H), 7.74-7.67(m, 2H), 7.63-7.57(m, 2H), 7.05(d, *J*=2.3Hz, 1H), 4.51-4.42(m, 2H), 4.00(br s,1H), 3.51-3.44(m, 2H).
MS(ESI): 238.0 (M⁺+1).

### b) Synthesis of 4-[1-(3,5-dibromo-4-methoxy-benzoyl)-2,3-dihydro-1H-pyrido[2,3-b][1,4]oxazin-7-yl]-benzonitrile

By the same method as in the step c) of Example 45, 4-(2,3-dihydro-1H-pyrido[2,3-b][1,4]oxazin-7-yl]-benzonitrile (71.4mg, 0.30mmol), 3,5-dibromo-4-methoxy-benzoyl chloride (148mg, 0.45mmol) and triethylamine (84µℓ, 0.60mmol) were dissolved in dichloromethane (3ml) and reacted at room temperature for 3 hours to obtain 4-[1-(3,5-dibromo-4-methoxybenzoyl)-2,3-dihydro-1H-pyrido[2,3-b][1,4]oxazin-7-yl]-benzonitrile as white solid (120mg, 75.6%).
¹H-NMR(CDCl₃, 300MHz); δ = 8.28(d, *J*=2.3Hz, 1H), 8.18-8.08(m, 1H), 7.76-7.67(m, 4H), 7.56-7.46(m, 2H), 4.55-4.48(m, 2H), 4.04-3.97(m, 2H), 3.95(s, 3H).
MS(ESI): 527.9 (M⁺+1).

### c) Synthesis of 4-[1-(3,5-dibromo-4-hydroxy-benzoyl)2,3-dihydro-1H-pyrido[2,3-b][1,4]oxazin-7-yl]-benzonitrile

By the same method as in the step d) of Example 45, 4-[1-(3,5-dibromo-4-methoxy-benzoyl)-2,3-dihydro-1H-pyrido[2,3-b][1,4]oxazin-7-yl]-benzonitrile (116mg, 0.22mmol), lithium bromide (76mg, 0.88mmol) and piperazine (29mg, 0.33mmol) were dissolved in N,N-dimethyl formamide (3ml) and reacted at 100 °C to obtain the target compound 65, i.e., 4-[1-(3,5-dibromo-4-hydroxy-benzoyl)-2,3-dihydro-1H-pyrido[2,3-b][1,4]oxazin-7-yl]-benzonitrile, as white solid (49mg, 43%).
¹H-NMR(DMSO-d₆, 300MHz); δ = 8.36(d, *J*=2.3Hz, 1H), 8.29-8.19(m, 1H), 7.90(d, *J*=8.4Hz, 2H), 7.80(s, 2H), 7.71(d, *J*=8.4Hz, 2H), 4.51-4.42(m, 2H), 3.97-3.87(m, 2H).
MS(ESI): 513.9 (M⁺+1).

### Example 66)

### Synthesis of (3,5-dibromo-4-hydroxy-phenyl)-[7-(4-trifluoromethoxy-phenyl)-2,3-dihydro-pyrido[2,3-b][1,4]ozazin-1-yl]-methanone (compound 66)

### a) Synthesis of 7-(4-trifluoromethoxy-phenyl)-2,3-dihydro-1H-pyrido[2,3-b][1,4]oxazine

By the same method as in the step b) of Example 49, 7-bromo-2,3-dihydro-1H-pyrido[2,3-b[1,4]oxazine (90mg, 0.42mmol), 4-trifluoromethoxy phenyl boronic acid (103mg, 0.50mmol), palladium triphenylphosphine (24.2mg, 0.021 mol) and potassium carbonate (116mg, 0.84mmol) were dissolved in a solvent of tetrahydrofuran/water (3ml/0.5ml) and reacted at 80°C for 6 hours to obtain 7-(4-trifluoromethoxy-phenyl)-2,3-dihydro-1H-pyrido[2,3-b][1,4]oxazine as white solid (86mg, 69.4%).
¹H-NMR(CDCl₃, 300MHz); δ = 7.81(d, *J*=2.3Hz, 1H), 7.54-7.47(m, 2H), 7.31-7.22(m, 2H), 7.03(d, *J*=2.3Hz, 1H), 4.49-4.42(m, 2H), 3.95(br s, 1H), 3.51-3.43(m, 2H).
MS(ESI): 297.0 (M⁺+1).

### b) Synthesis of (3,5-dibromo-4-methoxy-phenyl)-[7-(4-trifluoromethoxy-phenyl)-2,3-dihydro-pyrido[2,3-b][1,4]oxazin-1-yl]-methanone

By the same method as in the step c) of Example 45, 7-(4-trifluoromethoxy-phenyl)-2,3-dihydro-1H-pyrido[2,3-b][1,4]oxazine (83mg, 0.28mmol), 3,5-dibromo-4-methoxy-benzoyl chloride (138mg, 0.42mmol) and triethylamine (78µℓ, 0.56mmol) were dissolved in dichloromethane (3ml) and reacted at room temperature for 3 hours to obtain (3,5-dibromo-4-methoxy-phenyl)-[7-(4-trifluoromethoxy-phenyl)-2,3-dihydro-pyrido[2,3-b][1,4]oxazin-1-yl]-methanone as white solid (115mg, 69.8%).
¹H-NMR(CDCl₃, 300MHz); δ = 8.24(d, *J*=2.3Hz, 1H), 8.02-7.93(m, 1H), 7.72(s, 2H), 7.43-7.39(m, 2H), 7.30-7.22(m, 2H), 4.55-4.48(m, 2H), 4.04-3.98(m, 2H), 3.94(s, 3H).
MS(ESI): 586.9 (M⁺+1).

### c) Synthesis of (3,5-dibromo-4-hydroxy-phenyl)-[7-(4-trfluoromethoxy-phenyl)-2,3-dihydro-pvrido[2.3-b][1,4]oxazin-1-yl]-methanone

By the same method as in the step d) of Example 45, (3,5-dibromo-4-methoxy-phenyl)-[7-(4-trifluoromethoxy-phenyl)-2,3-dihydro-pyrido[2,3-b][1,4]oxazin-1-yl]-methanone (110mg, 0.19mmol), lithium bromide (65mg, 0.75mmol) and piperazine (25mg, 0.29mmol) were dissolved in N,N-dimethyl formamide (3ml) and reacted at 100°C to obtain the target compound 66, i.e., (3,5-dibromo-4-hydroxy-phenyl)-[7-(4-trifluoromethoxy-phenyl)-2,3-dihydro-pyrido[2,3-b][1,4]oxazin-1-yl]-methanone, as white solid (54mg, 48%).
¹H-NMR(CD₃OD, 300MHz); δ = 8.17(d, *J*=2.3Hz, 1H), 8.08(s, 1H), 7.78(s, 2H), 7.50(m, 2H), 7.31(m, 2H), 4.50(t, *J*=4.6Hz, 2H), 4.02(t, J=4.6Hz, 2H).
MS(ESI): 572.9 (M⁺+1).

### Example 67)

### Synthesis of 1-{4-(1-(3,5-dibromo-4-hydroxy-benzoyl)-2,3-dihydro-1H-pyrido[2,3-b][1,4]oxazin-7-yl]-phenyl}-ethanone (compound 67)

### a) Synthesis of 1-[4-(2,3-dihydro-1H-pyrido[2,3-b][1,4]oxazin-7-yl)-phenyl]-ethanone

To 3ml of tetrahydrofuran/0.5ml of distilled water, 7-bromo-2,3-dihydro-1H-pyrido[2,3-b][1,4]oxazine (80.0mg, 0.37mmol), 4-acetylphenyl boronic acid (71mg, 0.55mmol), potassium carbonate (103mg, 0.74mmol) and tetrakis triphenylphosphine palladium (21mg, 0.02mmol) were added and stirred at 80°C for 2 hours. After completion of the reaction, the mixture was extracted ethyl acetate, and the combined organic layer was dried over anhydrous sodium sulfate (Na₂SO₄), filtered and evaporated under reduced pressure. The resulting solid was recrystallized from n-hexane and diethyl ether to obtain 1-[4-(2,3-dihydro-1H-pyrido[2,3-b][1,4]oxazin-7-yl)-phenyl]-ethanone as yellow solid (59mg, 62%).
¹H-NMR(CDCl₃, 300MHz); 8 = 8.01 (d, *J*=8.4Hz, 2H), 7.89 (d, *J*=1.9Hz, 1H), 7.60 (d, *J*=8.4Hz, 2H), 7.09 (d, *J*=1.9Hz, 1H), 4.47 (m, 2H), 3.96 (br s, 1H), 3.48 (m, 2H), 2.64(s, 3H).
MS(ESI); 255.0(M⁺+1).

### b) Synthesis of 1-{4-[1-(3,5-dibromo-4-methoxy-benzoyl)-2,3-dihydro-1H-pyrido[2,3-b][1,4]oxazin-7-yl]phenyl}-ethanone

In a 10ml flask, 1-[4-(2,3-dihydro-1H-pyrido[2,3-b][1,4]oxazin-7-yl)-phenyl]-ethanone (59mg, 0.23mmol) and 3,5-dibromo-4-methoxy-benzoyl chloride (287mg, 0.87mmol) were dissolved in dichloromethane. Triethylamine (0.32ml, 2.3mmol) was added thereto and then stirred at room temperature for 12 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by column chromatography on amine silica eluting with a solvent of dichloromethane. The fractions containing the product were collected and evaporated to obtain 1-{4-[1-(3,5-dibromo-4-methoxy-benzoyl)-2,3-dihydro-1H-pyrido[2,3-b][1,4]oxazin-7-yl]-phenyl}-ethanone as white solid (119mg, 94%).
¹H-NMR(CDCl₃, 300MHz); δ = 8.31 (d, *J*=1.9Hz, 1H), 8.05(br s, 1H), 8.00 (d, *J*=8.4Hz, 2H), 7.73 (s, 2H), 7.48 (d, *J*=8.4Hz, 2H), 4.53 (m, 2H), 4.01 (m, 2H), 3.96 (s, 3H), 2.63 (s, 3H).
MS(ESI); 544.9(M⁺+1).

### c) Synthesis of 1-{4-[1-(3,5-dibromo-4-hydroxy-benzoyl)-2,3-dihydro-1H-pyrido[2,3-b][1,4]oxazin-7-yl]-phenyl}-ethanone

In a 10ml flask, 1-{4-[1-(3,5-dibromo-4-methoxy-benzoyl)-2,3-dihydro-1H-pyrido[2,3-b][1,4]oxazin-7-yl]-phenyl}-ethanone (119mg, 0.22mmol), lithium bromide (189mg, 2.2mmol) and piperazine (28mg, 0.33mmol) were dissolved in 3ml of N,N-dimethyl formamide, and then stirred at 100°C for 2.5 hours. After completion of the reaction by adding water dropwise, the mixture was adjusted to weak acidic condition (pH=6) with 1N hydrochloric acid. The formed solid was filtered and washed with distilled water. The resulting solid was recrystallized from methanol to obtain the target compound 67, i.e., 1-{4-[1-(3,5-dibromo-4-hydroxy-benzoyl)-2,3-dihydro-1H-pyrido[2,3-b][1,4]oxazin-7-yl]-phenyl}-ethanone (65mg, 56%).
¹H-NMR(CDCl₃+CD₃OD, 300MHz); δ = 8.27 (d, *J*=2.3Hz, 1H), 8.01 (br s, 1H), 8.00 (d, *J*=8.0Hz, 2H), 7.71 (s, 2H), 7.49 (d, *J*=8.4Hz, 2H), 4.53 (m, 2H), 4.04 (m, 2H), 2.64 (s, 3H).
MS(ESI); 530.9(M⁺+1).

### Example 68)

### Synthesis of (3,5-dibromo-4-hydroxy-phenyl)-[7-(5-methoxy-pyridin-3-yl)-2,3-dihydro-pyrido[2,3-b][1,4]oxazin-1-yl]-methanone (compound 68)

### a) Synthesis of 7-(5-methoxypyridin-3-yl)-2,3-dihydro-1H-pyrido[2,3-b][1,4]oxazine

To 3ml of tetrahydrofuran/0.5ml of distilled water, 7-bromo-2,3-dihydro-1H-pyrido[2,3-b][1,4]oxazine (80.0mg, 0.37mmol), 3-methoxy-5-(4,4,5,5-tetramethyl-[1,3,2] dioxaborolane-2-yl)-pyridine (105mg, 0.45mmol), potassium carbonate (103mg, 0.74mmol) and tetrakis triphenylphosphine palladium (129mg, 0.11mmol) were added and stirred at 80°C for 4 hours. After completion of the reaction, the mixture was extracted with ethyl acetate, and the combined organic layer was dried over anhydrous sodium sulfate (Na₂SO₄), filtered and evaporated under reduced pressure. The residue was purified by column chromatography on amine silica eluting with a solvent of dichloromethane:methanol=40:1. The fractions containing the product were collected and evaporated to obtain 7-(5-methoxy-pyridin-3-yl)-2,3-dihydro-1H-pyrido[2,3-b][1,4]oxazine as white solid (72mg, 80%).
¹H-NMR(CDCl₃, 300MHz); 8 = 8.37 (d, *J*=1.7Hz, 1H), 8.29 (d, *J*=2.7Hz, 1H), 7.83 (d, *J*=2.3Hz, 1H), 7.29 (m, 1H), 7.05 (d, *J*=2.3Hz, 1H), 4.47 (m, 2H), 3.98 (br s, 1H), 3.92 (s, 3H), 3.48 (m, 2H).
MS(ESI); 244.0(M⁺+1).

### b) Synthesis of (3,5-dibromo-4-methoxy-phenyl)-[7-(5-methoxy-pyridin-3-yl)-2,3-dihydro-pyrido[2,3-b][1,4]oxazin-1-yl]-methanone

In a 10ml flask, 7-(5-methoxy-pyridin-3-yl)-2,3-dihydro-1H-pyrido[2,3-b][1,4]oxazine (72mg, 0.30mmol) and 3,5-dibromo-4-methoxy-benzoyl chloride (146mg, 0.45mmol) were dissolved in dichloromethane. Triethylamine (0.21ml, 1.50mmol) was added thereto and then stirred at room temperature for 3 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by column chromatography on amine silica eluting with a solvent of dichloromethane. The fractions containing the product were collected and evaporated to obtain (3,5-dibromo-4-methoxy-phenyl)-[7-(5-methoxy-pyridin-3-yl)-2,3-dihydro-pyrido[2,3-b][1,4]oxazin-1-yl]-methanone as white solid (116 mg, 73%).
¹H-NMR(CDCl₃, 300MHz); δ = 8.30 (d, *J*=2.7Hz, 1H), 8.26 (d, *J*=2.3Hz, 1H), 8.25 (br s, 1H), 7.90 (br s, 1H), 7.72 (s, 2H), 7.07 (br s, 1H), 4.54 (m, 2H), 4.03 (m, 2H), 3.95 (s, 3H), 3.88 (s, 3H).
MS(ESI); 533.9(M⁺+1).

### c) Synthesis of (3,5-dibromo-4-hydroxy-phenyl)-[7-(5-methoxy-pyridin-3-yl)-2,3-dihydro-pvrido[2,3-b][1,4]oxazin-1-yl]-methanone

In a 10ml flask, (3,5-dibromo-4-methoxy-phenyl)-[7-(5-methoxy-pyridin-3-yl)-2,3-dihydro-pyrido[2,3-b][1,4]oxazin-1-yl]-methanone (110mg, 0.21mmol), lithium bromide (56mg, 1.58mmol) and piperazine (27mg, 0.32mmol) were dissolved in 3ml of N,N-dimethyl formamide, and then stirred at 100°C for 2 hours. After completion of the reaction by adding water dropwise, the mixture was adjusted to weak acidic condition (pH=6) with 1N hydrochloric acid. The formed solid was filtered and washed with distilled water. The resulting solid was recrystallized from methanol to obtain the target compound 68, i.e., (3,5-dibromo-4-hydroxy-phenyl)-[7-(5-methoxy-pyridin-3-yl)-2,3-dihydro-pyrido[2,3-b][1,4]oxazin-1-yl]-methanone (40mg, 37%).
¹H-NMR(CDCl₃+CD₃OD, 300MHz); δ = 8.22 (d, *J*=2.7Hz, 1H), 8.19 (d, *J*=2.3Hz, 1H), 8.10 (br s, 1H), 7.80 (br s, 1H), 7.70 (s, 2H), 7.16 (br s, 1H), 4.48 (m, 2H), 3.97 (m, 2H), 3.81 (s, 3H).
MS(ESI); 519.9(M⁺+1).

### Example 69)

### Synthesis of (4-hydroxy-3-trifluoromethyl-phenyl)-(7-methyl-2,3-dihydro-pyrido[2,3-b][1,4]oxazin-1-yl)-methanone (compound 69)

4-hydroxy-3-trifluoromethyl-benzoic acid (151mg, 0.73mmol) was dissolved in N,N-dimethyl acetamide (2.0ml) and then cooled to -20°C. Thionyl chloride (0.073ml, 1.00mmol) was added thereto and stirred at -20°C for 30 minutes. 5-methyl-2,3-dihydro-1H-pyrido[2,3-b][1,4]oxazine dissolved in N,N-dimethyl acetamide (1.0ml) was added thereto dropwise and then stirred at -20°C for 20 minutes, and then stirred at room temperature for 15 hours. Water was added thereto and the mixture was extracted with dichloromethane, and the combined organic layer was dried over anhydrous sodium sulfate (Na₂SO₄), filtered and evaporated under reduced pressure. The resulting solid resudue was recrystallized from dichloromethane to obtain the target compound 69, i.e., (4-hydroxy-3-trifluoromethyl-phenyl)-(7-methyl-2,3-dihydro-pyrido[2,3-b][1,4]oxazin-1-yl)-methanone, as white solid (176mg, 78%).
¹H-NMR(DMSO-d₆, 300MHz); δ = 11.27 (br s, 1H), 7.67-7.75 (m, 4H), 7.08 (d, *J*=8.4Hz, 1H), 4.34 (m, 2H), 3.84 (m, 2H), 2.13 (s, 3H).
MS(ESI); 339.1(M⁺+1).

### Example 70)

### Synthesis of (3,5-dibromo-4-hydroxy-phenyl)-(7-(1H-indol-4-yl)-2,3-dihydro-pyrido(2,3-b][1,4]oxazin-1-yl]-methanone (compound 70)

### a) Synthesis of 7-(1H-indol-4-yl)-2,3-dihydro-1H-pyrido[2,3-b][1,4]oxazine

To 3 ml of tetrahydrofuran(THF)/0.5ml of distilled water, 7-bromo-2,3-dihydro-1H-pyrido[2,3-b][1,4]oxazine (80.0mg, 0.37mmol), 4-(4,4,5,5,-tetramethyl-[1,3,2] dioxaborolane-2-yl)-1H-indole (109mg, 0.45mmol), potassium carbonate (103mg, 0.74mmol) and tetrakis triphenylphosphine palladium (172mg, 0.15mmol) were added and stirred at 80°C for 4 hours. After completion of the reaction, the mixture was extracted with ethyl acetate, and the combined organic layer was dried over anhydrous sodium sulfate (Na₂SO₄), filtered and evaporated under reduced pressure. The residue was purified by column chromatography on amine silica eluting with a solvent of dichloromethane:methanol=40:1. The fractions containing the product were collected and evaporated to obtain 7-(1H-indol-4-yl)-2,3-dihydro-1H-pyrido[2,3-b][1,4]oxazine as white solid (130mg).
¹H-NMR (CDCl₃, 300MHz); 8 = 8.41 (br s, 1H), 7.96 (d, *J*=1.9 Hz, 1H), 7.39 (m, 1H), 7.27-7.25 (m, 2H), 7.19 (d, *J*=2.3 Hz, 1H), 7.11 (dd, *J*=7.3 Hz, 1.4 Hz, 1H), 6.69 (m, 1H), 4.47 (m, 2H), 3.91 (br s, 1H), 3.47 (m, 2H).
MS(ESI): 252.1 (M⁺+1).

### b) Synthesis of (3,5-dibromo-4-methoxy-phenyl)-[7-(1H-indol-4-yl)-2,3-dihydro-pyrido[2,3-b][1,4]oxazin-1-yl]-methanone

In a 10ml flask, 7-(1H-indol-4-yl)-2,3-dihydro-1H-pyrido[2,3-b][1,4]oxazine (130mg, 0.52mmol) and 3,5-dibromo-4-methoxy-benzoyl chloride (204mg, 0.62mmol) were dissolved in dichloromethane. Triethylamine (0.36ml, 2.59mmol) was added thereto and then stirred at room temperature for 3 hours. After completion of the reaction by adding water dropwise, the mixture was extracted with dichloromethane, and the combined organic layer was dried over anhydrous sodium sulfate (Na₂SO₄), filtered and evaporated under reduced pressure. The residue was purified by column chromatography on amine silica eluting with a solvent of dichloromethane. The fractions containing the product were collected and evaporated to obtain (3,5-dibromo-4-methoxy-phenyl)-[7-(1H-indol-4-yl)-2,3-dihydro-pyrido[2,3-b][1,4]oxazin-1-yl]-methanone as white solid (129mg, 64% in two steps).
¹H-NMR (CDCl₃, 300MHz); δ = 8.37 (d, *J*=2.3 Hz, 1H), 8.30 (br s, 1H), 7.92 (br s, 1H), 7.74 (s, 2H), 7.39 (m, 1H), 7.24 (m, 1H), 7.20 (d, J=8.0 Hz, 1H), 6.98 (d, *J*=7.2 Hz, 1H), 6.32 (br s, 1H), 4.57 (m, 2H), 4.05 (m, 2H), 3.94 (s, 3H).
MS(ESI): 541.9 (M⁺+1).

### c) Synthesis of (3,5-dibromo-4-hydroxy-phenyl)-[7-(1H-indol-4-yl)-2,3-dihydro-pyrido[2,3-b][1,4]oxazin-1-yl]-methanone

In a 10ml flask, (3,5-dibromo-4-methoxy-phenyl)-[7-(1H-indol-4-yl)-2,3-dihydro-pyrido[2,3-b][1,4]oxazin-1-yl]-methanone (124mg, 0.23mmol), lithium bromide (246mg, 2.83mmol) and piperazine (29mg, 0.34mmol) were dissolved in 5 ml of N,N-dimethyl formamide (DMF), and then stirred at 100°C for 2 hours. After completion of the reaction by adding water dropwise, the mixture was adjusted to weak acidic condition (pH=6) with 1N hydrochloric acid. The formed solid was filtered and washed with distilled water. The resulting solid was recrystallized from methanol to obtain the target compound 70, i.e., (3,5-dibromo-4-hydroxy-phenyl)-[7-(1H-indol-4-yl)-2,3-dihydro-pyrido[2,3-b][1,4]oxazin-1-yl]-methanone (77mg, 64%).
¹H-NMR (DMSO-d₆, 300MHz); δ = 11.26 (br s, 1H), 10.62(br s, 1H), 8.18 (d, *J*=1.9 Hz, 1H), 8.06 (br s, 1H), 7.82 (s, 2H), 7.39 (d, *J*=8.4 Hz, 1H), 7.32 (m, 1H), 7.11 (m, 1H), 6.98 (d, *J*=6.9 Hz, 1H), 6.17 (br s, 1H), 4.49 (m, 2H), 3.94 (m, 2H).
MS(ESI): 527.9 (M⁺+1).

### Example 71)

### Synthesis of (3,5-dibromo-4-hydroxy-phenyl)-(2,3-dihydro-pyrido[4,3-b][1,4]oxazin-4-yl)-methanone sulfuric acid salt (compound 71)

In a 25ml flask, (3,5-dibromo-4-hydroxy-phenyl)-(2,3-dihydro-pyrido[4,3-b][1,4]oxazin-4-yl)-methanone (100mg, 0.24mmol) was suspended in tetrahydrofuran (8ml) and the mixture was dissolved under heating. After cooling to room temperature, 1M sulfuric acid solution was added thereto dropwise. The formed solid was filtered to obtain the target compound 71 as white solid (78mg, 69%).
¹H-NMR(DMSO-d₆, 300MHz); δ = 9.01 (s, 1H), 8.44(d, *J*=6.6Hz, 1H), 7.82(s, 2H), 7.53(d, *J*=6.3Hz, 1H), 4.60-4.50 (m, 2H), 4.05-3.96(m, 2H).
MS(ESI); 412.9 (M⁺+1).

### Example 72)

### Synthesis of (2,6-dibromo-4-(2,3-dihydro-pyrido[4,3-b][1,4]oxazin-4-carbonyl)-phenolate sodium salt (compound 72)

In a 100ml flask, (3,5-dibromo-4-hydroxy-phenyl)-(2,3-dihydro-pyrido[4,3-b][1,4]oxazin-4-yl)-methanone (500mg, 1.20mmol) was suspended in tetrahydrofuran (40ml) and the mixture was dissolved under heating. After cooling to room temperature, 10N sodium hydroxide solution was added thereto dropwise. The solvent was evaporated under reduced pressure, and the residue was dissolved in a solvent of acetonitrile and water (1:1 ratio, 80ml) to obtain the target compound 72 as white non-crystalline fom (240mg, 48%).
¹H-NMR(DMSO-d₆, 300MHz); δ = 8.31(s, 1H), 7.99(d, *J*=5.7Hz, 1H), 7.49(s, 2H), 6.94(d, *J*=5.7Hz 1H), 4.38-4.21(m, 2H), 3.97-3.85(m, 2H).
MS(ESI); 412.8 (M⁺+1).

### Example 73)

### Synthesis of (2,6-dibromo-4-(2,3-dihydro-pyrido[4,3-b][1,4]oxazin-4-carbonyl)-phenolate potassium salt (compound 73)

In a 100ml flask, (3,5-dibromo-4-hydroxy-phenyl)-(2,3-dihydro-pyrido[4,3-b][1,4]oxazin-4-yl)-methanone (500mg, 1.20mmol) was suspended in tetrahydrofuran (40ml) and the mixture was dissolved under heating. After cooling to room temperature, 10N potassium hydroxide solution was added thereto dropwise. The solvent was evaporated under reduced pressure, and the residue was dissolved in a solvent of acetonitrile and water (1:1 ratio, 80ml), and freeze-dried to obtain the target compound 73 as white non-crystalline fom (480mg, 92%).
¹H-NMR(DMSO-d₆, 300MHz); δ = 8.30(s, 1H), 7.98(d, *J*=5.4Hz, 1H), 7.47(s, 2H), 6.89(d, *J*=5.1Hz 1H), 4.35-4.25(m, 2H), 3.95-3.85(m, 2H).
MS(ESI); 412.9 (M⁺+1).

### Example 74)

### Synthesis of (3,5-dibromo-4-hydroxy-phenyl)-[2,3-dihydro-pyrido(4,3-b][1,4]oxazin-4-yl)-methanethione trifluoroacetic acid salt (compound 74)

In a 25ml flask, (3,5-dibromo-4-methoxy-phenyl)-(2,3-dihydro-pyrido[4,3-b][1,4]oxazin-4-yl)-methanethione (100mg, 0.22mmol) was dissolved in N,N-dimethyl formamide, and piperazine (28.4mg, 1.5 equivalents) and lithium bromide (76.4mg, 4 equivalents) were added thereto, and then stirred at 100 °C for 15 hours. Under reduced pressure, N,N-dimethyl formamide was removed and water was added, and then the suspension was adjusted to pH 6 to 7 with 1N hydrochloric acid and stirred for 30 minutes. The resulting solid was purified by medium pressure liquid chromatography (MPLC) eluting with a solvent of acetonitrile:water=90:1 containing 0.1% of trifluoroacetic acid. The fractions containing the product were collected and freeze-dried to obtain the target compound 74 as yellow solid (50mg, 49%).
¹H-NMR(DMSO-d₆, 300MHz); 8.22(d, *J*=5.2Hz,1H), 8.10(br s, 1H), 7.73(s, 2H), 7.24(d, *J*=5.2Hz, 1H), 4.71(m, 2H), 4.51(m, 2H).
MS(ESI): 428.8(M⁺+1).

### Example 75)

### Synthesis of 1-[1-(3,5-bromo-4-hydroxy-benzoyl)-2,3-dihydro-1H-pyrido[2,3-b][1,4]oxazin-7-yl]-pyrrolidin-2-one (compound 75)

### a) Synthesis of 1-(2,3-dihydro-1H-pyrido[2,3-b][1,4]oxazin-7-yl)-pyrrolidin-2-one

In a 5ml flask, 7-bromo-2,3-dihydro-1H-pyrido[2,3-b][1,4]oxazine (120mg, 0.558mmol), pyrrolidin-2-one (142mg, 1.668mmol), tris(dibenzylidene acetone)dipalladium(0) (Pd₂dba₃) (51mg, 0.056mmol) and xantphos (96mg, 0.166mmol) were dissolved in N,N-dimethyl acetamide (1ml). Cesium carbonate (Cs₂CO₃) (364mg, 1.117mmol) was added thereto and then stirred at room temperature for 10 minutes, and further reacted at 150 °C for 20 minutes using microwave (50W) apparatus. The reaction mixture was filtered on celite 545 and evaporated under reduced pressure. The resulting residue was purified by column chromatography on silica eluting with a solvent of dichloromethane:methanol=19:1. The fractions containing the product were collected and evaporated to obtain pale-yellow solid (120mg, 98%).
MS(ESI); 220.2 (M⁺+1).

### b) Synthesis of 1-[1-(3,5-dibromo-4-methoxy-benzoyl)-2,3-dihydro-1H-pyrido[2,3-b][1,4]oxazin-7-yl]-pyrrolidin-2-one

In a 10ml flask, 1-[1-(3,5-dibromo-4-methoxy-benzoyl)-2,3-dihydro-1H-pyrido[2,3-b][1,4]oxazin-7-yl]-pyrrolidin-2-one (120mg, 0.547mmol) was dissolved in dichloromethane (3ml). Diisopropylamine (160µl, 0.919mmol) was added thereto and then stirred at 0°C for 10 minutes, and then 3,5-dibromo-4-methoxy-benzoyl chloride (180mg, 0.264mmol) was added and stirred at room temperature for 1 hour. To the reaction mixture, water (20ml) was added and stirred at room temperature 10 minutes. The reaction mixture was extracted with ethyl acetate (30ml) and evaporated under reduced pressure. The residue was purified by column chromatography on silica eluting with a solvent of dichloromethane:ethyl acetate=1:2. The fractions containing the product were collected and evaporated to obtain white solid (6mg, 2.1 %).
¹H-NMR(CD₃OD, 300MHz); δ = 8.23(s, 1H), 8.17(s, 1H), 7.68(s, 2H), 4.46(t, *J*=4.6Hz, 2H), 3.99-3.92(m, 2H), 3.93 (s, 3H), 3.77 (t, *J*=6.9Hz, 2H), 2.55 (m, 2H), 2.22-2.12 (m, 2H).

### c) Synthesis of 1-[1-(3,5-dibromo-4-hydroxy-benzoyl)-2,3-dihydro-1H-pyrido[2,3-b][1,4]oxazin-7-yl]-pyrrolidin-2-one

In a 10ml flask, 1-[1-(3,5-dibromo-4-methoxy-benzoyl)-2,3-dihydro-1H-pyrido[2,3-b][1,4]oxazin-7-yl]-pyrrolidin-2-one (6mg, 0.0117mmol) was dissolved in dichloromethane (2ml), and then cooled to 0°C. 1.0M Boron tribromide dissolved in dichloromethane (0.2ml, 0.2mmol) was added thereto and then stirred at room temperature for 24 hours. Dichloromethane (3 ml) and methanol (0.1ml) were added and then stirred for 10 minutes, filtered and evaporated under reduced pressure. The residue was purified by column chromatography on silica eluting with a solvent of dichloromethane:ethyl acetate:methanol=20:1:1. The fractions containing the product were collected and evaporated to obtain the target compound 75 as brown solid (4.6mg, 79%).
¹H-NMR(CD₃OD, 300MHz); δ = 8.22(s, 1H), 8.20(s, 1H), 7.68(s, 2H), 4.44(t, *J*=4.6Hz, 2H), 3.95(t, *J*=4.6Hz, 2H), 3.77 (t, *J*=7.6Hz, 2H), 2.55 (m, 2H), 2.22-2.12 (m, 2H).
MS(ESI); 495.9(M⁺+1).

The structures of the compounds prepared in the above Examples are shown in the following table.

| **Structure** | **Compound No.** | **Structure** | **Compound No.** |
|---|---|---|---|
| 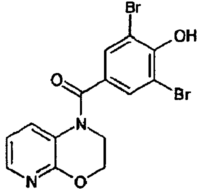 | 1 | 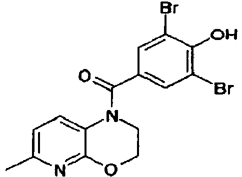 | 7 |
| 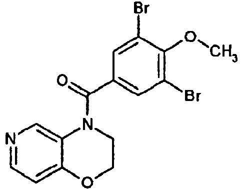 | 2 | 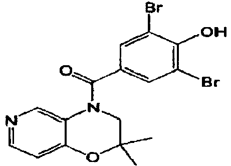 | 8 |
| 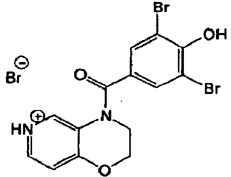 | 3 | 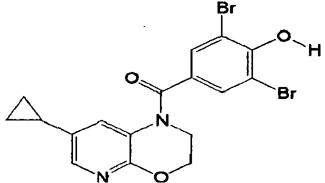 | 9 |
| 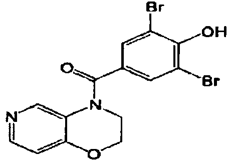 | 4 | 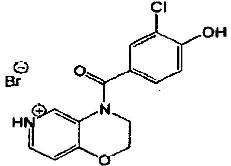 | 10 |
| 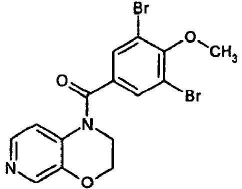 | 5 | 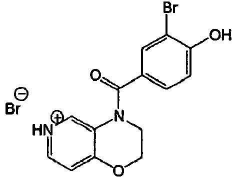 | 11 |
| 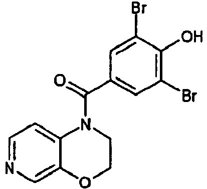 | 6 | 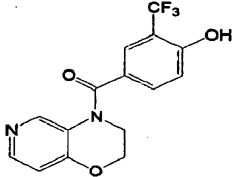 | 12 |
| 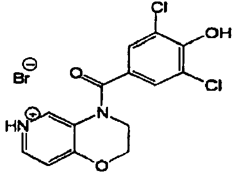 | 13 | 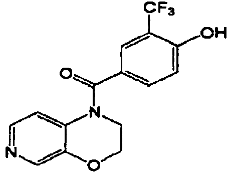 | 19 |
| 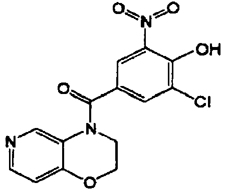 | 14 | 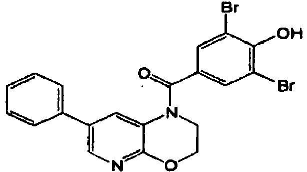 | 20 |
| 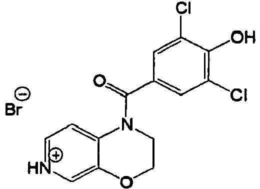 | 15 | 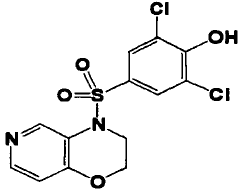 | 21 |
| 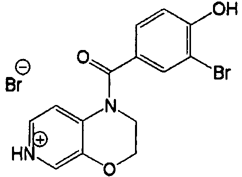 | 16 | 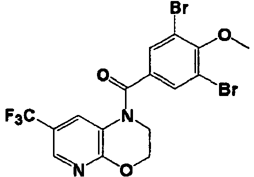 | 22-1 |
| 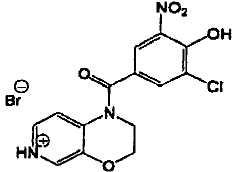 | 17 | 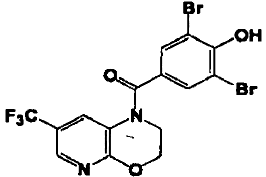 | 22-2 |
| 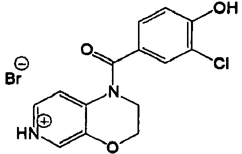 | 18 | 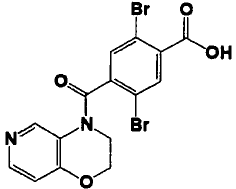 | 23 |
| 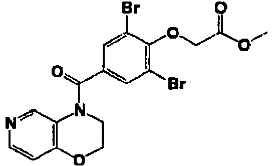 | 24 | 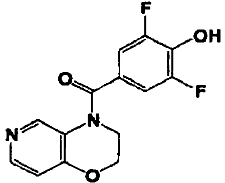 | 29 |
| 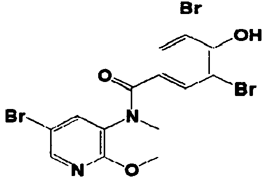 | 25 | 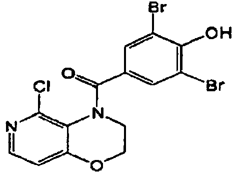 | 30 |
| 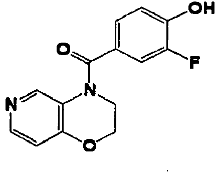 | 26 | 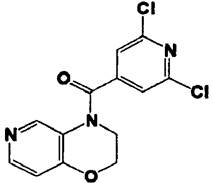 | 31 |
| 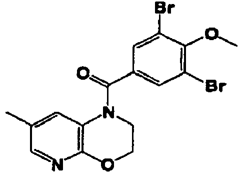 | 27-1 | 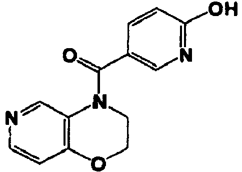 | 32 |
| 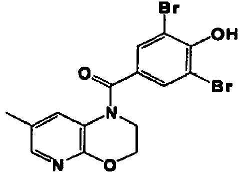 | 27-2 | 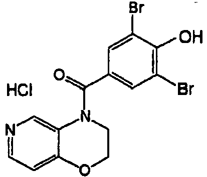 | 33 |
| 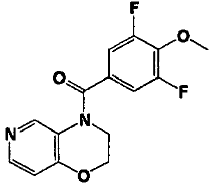 | 28 | 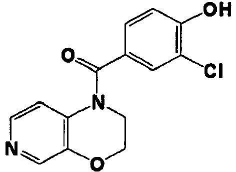 | 34 |
| 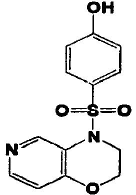 | 35-1 | 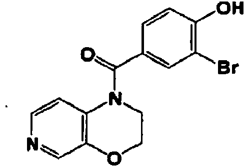 | 40 |
| 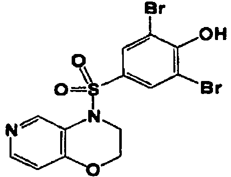 | 35-2 | 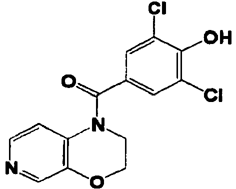 | 41 |
| 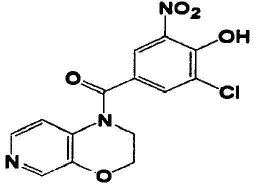 | 36 | 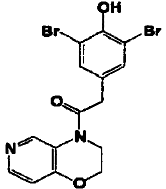 | 42 |
| 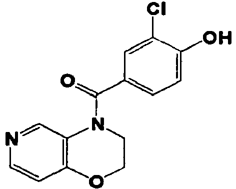 | 37 | 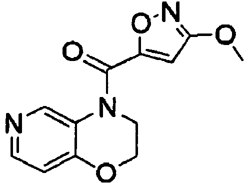 | 43 |
| 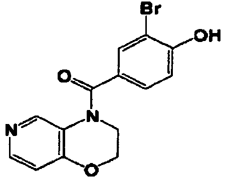 | 38 | 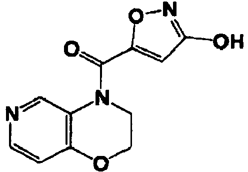 | 44 |
| 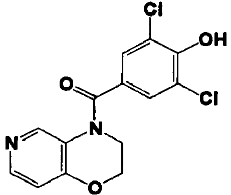 | 39 | 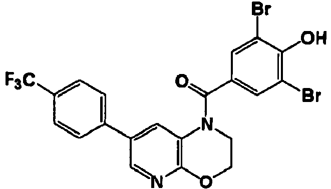 | 45 |
| 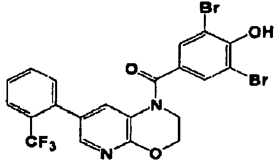 | 46 | 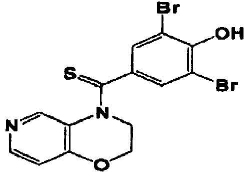 | 51 |
| 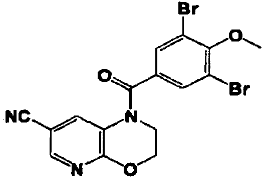 | 47-1 | 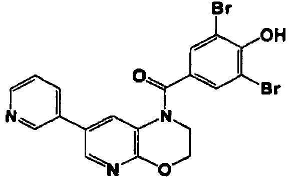 | 52 |
| 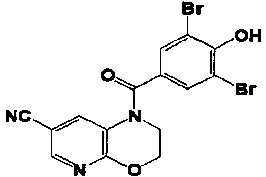 | 47-2 | 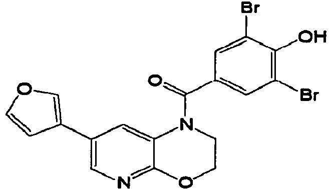 | 53 |
| 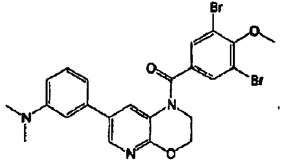 | 48 | 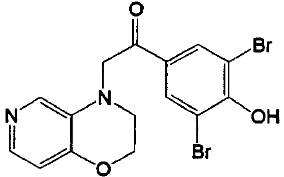 | 54 |
| 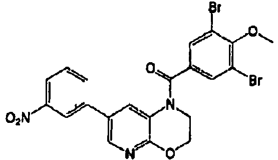 | 49 | 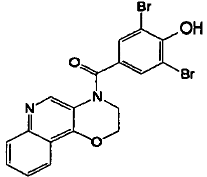 | 55 |
| 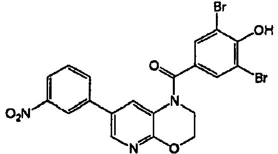 | 50 | 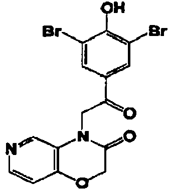 | 56 |
| 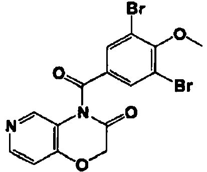 | 57 | 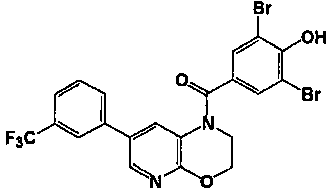 | 63 |
| 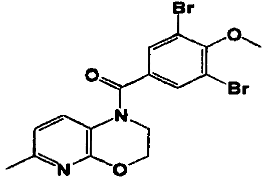 | 58 | 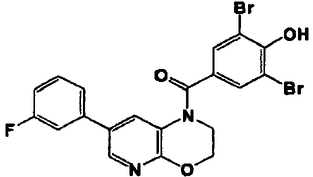 | 64 |
| 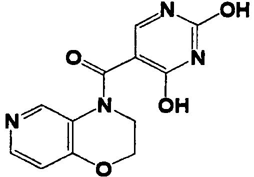 | 59 | 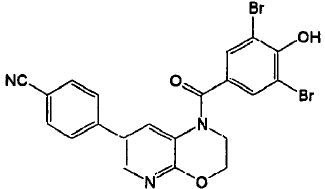 | 65 |
| 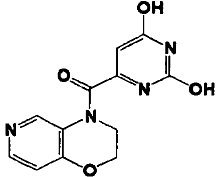 | 60 | 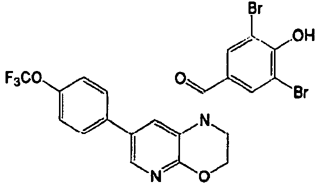 | 66 |
| 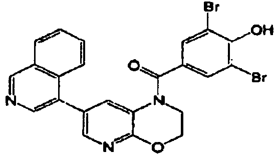 | 61 | 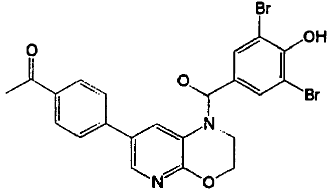 | 67 |
| 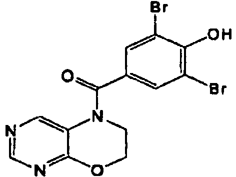 | 62 | 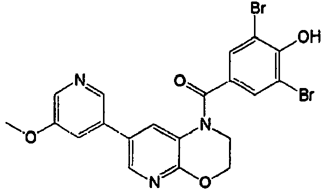 | 68 |
| 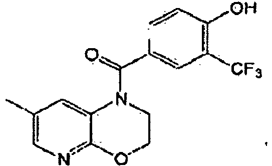 | 69 | 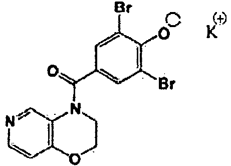 | 73 |
| 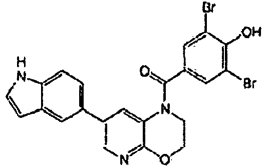 | 70 | 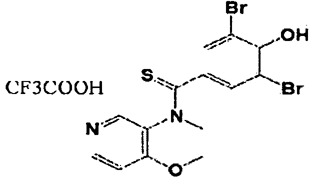 | 74 |
| 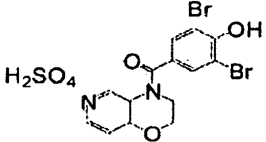 | 71 | 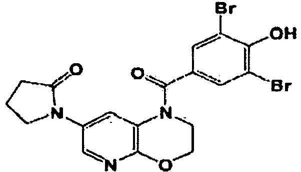 | 75 |
| 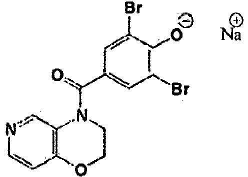 | 72 | | |

### Intermediate Synthesis Example (1)

### Synthesis of 5-methyl-2,3-dihydro-1H-pyrido[2,3-b][1,4]oxazine

### a) Synthesis of 5-methyl-1H-pyrido[2,3-b][1,4]oxazin-2-one

3-amino-5-methyl-pyridin-2-ol (1.32g, 10.6mmol) was dissolved in 50ml of anhydrous N,N-dimethyl formamide under nitrogen atmosphere, and chloroacetyl chloride solution (1.02ml, 12.72mmol) was added thereto dropwise at room temperature. After stirring for 30 minutes, potassium carbonate (3.7g, 26.5mmol) was added thereto dropwise at room temperature, and the reaction mixture was heated to 100°C and then stirred for 18 hours. The reaction mixture was cooled to room temperature and the reaction was quenched by adding water. The organic layer was extracted with ethyl acetate, and the combined organic layer was washed with water and saturated saline solution, dried over anhydrous sodium sulfate (Na₂SO₄), filtered and evaporated under reduced pressure to obtain straw yellow solid (424mg). The resulting solid was used in the next step without further purification.
¹H-NMR (DMSO-d₆, 300MHz); δ = 10.2 (br s, 1H), 8.00 (s, 1H), 7.30 (s, 1H), 4.74 (s, 2H), 2.00 (s, 3H).
MS(ESI); 165.0(M⁺+1).

### b) Synthesis of 5-methyl-2,3-dihydro-1H-pyrido[2,3-b][1,4]oxazine

5-methyl-1H-pyrido[2,3-b][1,4]oxazin-2-one (424mg, 2.58mmol) was dissolved in 20ml of anhydrous tetrahydrofuran under nitrogen atmosphere, and then cooled to 0°C. 1.0M Lithium aluminum hydride dissolved in tetrahydrofuran (5.16ml, 5.16mmol) was added thereto dropwise and then stirred for 30 minutes. The reaction tempreature was raised to room temperature and then stirred for 1 hour. The reaction mixture was cooled to 0°C again and water (0.2ml), 10% sodium hydroxide solution (0.4ml) and water (0.6ml) were added thereto dropwise in this order, and stirred vogirously at room temperature for 1 hour. The formed precipitate was filtered and washed with excess ethyl acetate. The filtrate was washed with water and saturated saline solution, dried over anhydrous sodium sulfate (Na₂SO₄), filtered and evaporated under reduced pressure to obtain 5-methyl-2,3-dihydro-1H-pyrido[2,3-b][1,4]oxazine as white solid(100mg, 26% in two steps).
¹H-NMR(CDCl₃, 300MHz); δ = 7.42 (m, 1H), 6.68 (m, 1H), 4.37 (m, 2H), 3.74 (br s, 1H), 3.39 (m, 2H), 2.17 (s, 3H).
MS(ESI); 151.0(M⁺+1).

### Intermediate Synthesis Example (2)

### Synthesis of 7-methyl-2,3-dihydro-1H-pyrido[2,3-b][1,4]oxazine

### a) Synthesis of 2-chloro-5-methyl-3-nitro-pyridine

5-methyl-3-nitro-pyridin-2-ol (5g, 32.4mmol) and benzyltrimethyl ammonium chloride (3.01g, 16.2mmol) were dissolved in acetonitrile, and phosphorus oxychloride (9.0ml, 97.2mmol) was added thereto and stirred at 80°C for 6 hours. The reaction mixture was cooled and poured into ice water to quench the reaction, and extracted with dichloromethane. The combined organic layer was washed with saturated saline solution, dried over anhydrous sodium sulfate (Na₂SO₄), filtered and evaporated under reduced pressure. The residue was purified by column chromatography on silica eluting with a solvent of dichloromethane. The fractions containing the product were collected and evaporated to obtain 2-chloro-5-methyl-3-nitro-pyridine as yellow solid (5.39g, 97%).
¹H-NMR(CDCl₃, 300MHz); δ = 8.45 (d, 1H, *J*=2.3Hz), 8.04 (d, 1H, *J*=2.3Hz), 2.46 (s, 3H).
MS(ESI); 173.0(M⁺+1).

### b) Synthesis of (5-methyl-3-nitro-pyridin-2-yloxy)-acetic acid methyl ester

Hydroxy-acetic acid methyl ester (3.96g, 47.0mmol) was dissolved in 50ml of anhydrous tetrahydrofuran under nitrogen atmosphere, and sodium hydride (2.01g, 53.2mmol) was added thereto at room temperature. After stirring for 30 minuntes, 2-chloro-5-methyl-3-nitropyridine (5.39g, 31.3mmol) dissolved in 15ml of anhydrous tetrahydrofuran was added dropwise at room temperature, and the reaction mixture was stirred at room temperature for 18 hours. After completion of the reaction by adding water, the mixture was extracted with ethyl acetate, and the combined organic layer was washed with water and saturated saline solution, dried over anhydrous sodium sulfate (Na₂SO₄), filtered and evaporated under reduced pressure. The resulting solid was recrystallized from dichloromethane and ether to obtain (5-methyl-3-nitro-pyridin-2-yloxy)-acetic acid methyl ester as yellow solid (5.47g, 77%).
¹H-NMR(CDCl₃, 300MHz); δ = 8.16 (s, 2H), 5.05 (s, 2H), 3.76 (s, 3H), 2.36(s, 3H).
MS(ESI); 227.0(M⁺+1).

### c) Synthesis of 7-methyl-1H-pyrido[2,3-b][1,4]oxazin-2-one

In a 250ml round flask, (5-methyl-3-nitro-pyridin-2-yloxy)-acetic acid methyl ester (5.47g, 23.9mmol) and tin tetrachloride (18.1g, 95.6mmol) were dissolved in 24ml of concentrated hydrochloric acid, and then stirred at 80°C for 1 hour. The reaction mixture was cooled to room temperature, neutralized with 10% NaOH solution, and extracted with ethyl acetate. The combined organic layer was washed with water and saturated saline solution, dried over anhydrous sodium sulfate (Na₂SO₄), filtered and evaporated under reduced pressure. The residue was purified by column chromatography on silica eluting with a solvent of dichloromethane:methanol=19:1. The resulting solid was recrystallized from dichloromethane/methanol/tetrahydrofuran to obtain 7-methyl-1H-pyrido[2,3-b][1,4]oxazin-2-one as pale-brown solid (2.65g, 67%).
¹H-NMR(CDCl₃, 300MHz); δ = 8.17 (br s, 1H), 7.75 (s, 1H), 6.94 (d, *J*=1.5Hz, 1H), 4.81 (s, 2H), 2.29 (s, 3H).
MS(ESI); 165.0(M⁺+1).

### d) Synthesis of 5-methyl-2,3-dihydro-1H-pyrido[2 3-b][1,4]oxazine

5-methyl-1H-pyrido[2,3-b][1,4]oxazin-2-one (1.5g, 9.1mmol) was dissolved in 20ml of anhydrous tetrahydrofuran under nitrogen atmosphere, and then cooled to 0°C. 1.0M Lithium aluminum hydride dissolved in tetrahydrofuran (11ml, 10.9mmol) was added thereto dropwise and then stirred for 30 minutes. The reaction tempreature was raised to room temperature and stirred for 1 hour. The reaction mixture was cooled to 0°C again and water (0.3ml), 10% sodium hydroxide solution (0.6ml) and water (0.9ml) were added thereto dropwise in this order, and then stirred vigorously at room temperature for 1 hour. The formed solid was filtered and washed with excess ethyl acetate. The filtrate was washed with water and saturated saline solution, dried over anhydrous sodium sulfate (Na₂SO₄), filtered and evaporated under reduced pressure to obtain 5-methyl-2,3-dihydro-1H-pyrido[2,3-b][1,4]oxazine as white solid (680mg, 50%).
¹H-NMR(CDCl₃, 300MHz); δ =7.42 (m, 1H), 6.68 (m, 1H), 4. 37 (m, 2H), 3.74 (br s, 1H), 3.39 (m, 2H), 2.17 (s, 3H).
MS(ESI); 151.0(M⁺+1).

### Intermediate Synthesis Example (3)

### Synthesis of 7-bromo-1H-pyrido(2,3-b][1,4]oxazin-2-one

### a) Synthesis of (5-bromo-3-nitro-pyridin-2-yloxy)-acetic acid methyl ester

Hydroxy-acetic acid methyl ester (46mg, 0.50mmol) was dissolved in 2ml of anhydrous tetrahydrofuran under nitrogen atmosphere, and sodium hydride (40mg, 1.0mmol) was added thereto at room temperature. After stirring for 30 minuntes, 5-bromo-2-chloro-3-nitro-pyridine (100mg, 0.42mmol) dissolved in 1ml of anhydrous tetrahydrofuran was added dropwise at room temperature, and the reaction mixture was stirred at room temperature for 18 hours. After completion of the reaction by adding water, the mixture was extracted with dichloromethane, and the organic layer was washed with water and saturated saline solution, dried over anhydrous sodium sulfate (Na₂SO₄), filtered and evaporated under reduced pressure. The residue was purified by column chromatography on silica eluting with a solvent of n-hexane:ethyl acetate=9:1. The fractions containing the product were collected and evaporated to obtain (5-bromo-3-nitro-pyridin-2-yloxy)-acetic acid methyl ester as yellow solid (144mg, 99%).
¹H-NMR(CDCl₃, 300MHz); δ = 8.47 (d, *J*=2.3Hz,1H), 8.40 (d, *J*=2.3Hz,¹H), 5.07 (s, 2H), 3.78 (s, 3H).
MS(ESI); 290.9(M⁺+1).

### b) Synthesis of 7-bromo-1H-pyrido[2,3-b][1,4]oxazin-2-one

In a 10ml round flask, (5-bromo-3-nitro-pyridin-2-yloxy)-acetic acid methyl ester (144mg, 0.49mmol) and tin tetrachloride (375mg, 1.96mmol) were dissolved in 3ml of concentrated hydrochloric acid, and then stirred at 80°C for 1 hour. The reaction mixture was cooled to room temperature, neutralized with 10% sodium hydroxide solution, and extracted with dichloromethane. The combined organic layer was washed with water and saturated saline solution, dried over anhydrous sodium sulfate (Na₂SO₄), filtered and evaporated under reduced pressure. The resulting solid was recrystallized from tetrahydrofuran and methanol to obtain 7-bromo-1H-pyrido[2,3-b][1,4]oxazin-2-one as white solid (59mg, 52%).
¹H-NMR(DMSO-d₆, 300MHz); δ = 11.0 (br s, 1H), 7.88 (d, *J*=2.3Hz, 1H), 7.33 (d, *J*=2.3Hz, 1H), 4.81 (s, 2H).
MS(ESI); 228.9(M⁺+1).

### Intermediate Synthesis Example (4)

### Synthesis of 7-fluoro-1H-pyrido[2,3-b][1,4]oxazin-2-one

### a) Synthesis of 2-chloro-5-fluoro-3-nitro-pyridine

5-fluoro-3-nitro-pyridin-2-ol (2g, 12.7mmol) and benzyltrimethyl ammonium chloride (1.17g, 6.35mmol) were dissolved in acetonitrile, and phosphorus oxychloride (3.5ml, 38.1mmol) was added thereto and stirred at 80°C for 6 hours. The reaction mixture was cooled and poured into ice water to quench the reaction, and extracted with dichloromethane. The combined organic layer was washed with saturated saline solution, dried over anhydrous sodium sulfate (Na₂SO₄), filtered and evaporated under reduced pressure. The residue was purified by column chromatography on silica eluting with a solvent of dichloromethane:methanol=30:1. The fractions containing the product were collected and evaporated to obtain yellow liquid (1.57g, 70%).
¹H-NMR(CDCl₃, 300MHz); δ = 8.56 (d, J=2.7Hz, 1H), 8.40 (dd, *J*=6.5Hz, 2.7Hz, 1H).
MS(ESI); 176.9(M⁺+1).

### b) Synthesis of (5-fluoro-3-nitro-pyridin-2-yloxy)-acetic acid methyl ester

Hydroxy-acetic acid methyl ester (0.96g, 10.7mmol) was dissolved in 2ml of anhydrous tetrahydrofuran under nitrogen atmosphere, and sodium hydride (0.42g, 10.7mmol) was added thereto at room temperature. After stirring for 30 minuntes, 2-chloro-5-fluoro-3-nitro-pyridine (1.57g, 8.89mmol) dissolved in 15ml of anhydrous tetrahydrofuran was added dropwise at room temperature, and the reaction mixture was stirred at room temperature for 18 hours. After completion of the reaction by adding water, the mixture was extracted with dichloromethane, and the combined organic layer was washed with water and saturated saline solution, dried over anhydrous sodium sulfate (Na₂SO₄), filtered and evaporated under reduced pressure. The residue was purified by column chromatography on silica eluting with a solvent of n-hexane:ethyl acetate=9:1. The fractions containing the product were collected and evaporated to obtain (5-fluoro-3-nitro-pyridin-2-yloxy)-acetic acid methyl ester as yellow liquid (1.35g, 66%).
¹H-NMR(CDCl₃, 300MHz); δ = 8.25 (d, *J*=2.7Hz, 1H), 8.40 (dd, *J*=6.9Hz, 2.7H, 1H z), 5.06 (s, 2H), 3.78 (s, 3H).
MS(ESI); 231.1(M⁺+1).

### c) Synthesis of 7-fluoro-1H-pyrido[2,3-b][1,4]oxazin-2-one

In a 250ml round flask, (5-fluoro-3-nitro-pyridin-2-yloxy)-acetic acid methyl ester (1.35g, 5.87mmol) and tin tetrachloride (4.45g, 23.48mmol) were dissolved in 15ml of concentrated hydrochloric acid, and then stirred at 80°C for 1 hour. The reaction mixture was cooled to room temperature, neutralized with 10% sodium hydroxide solution, and extracted with ethyl acetate. The combined organic layer was washed with water and saturated saline solution, dried over anhydrous sodium sulfate (Na₂SO₄), filtered and evaporated under reduced pressure. The resulting solid was recrystallized from tetrahydrofuran and methanol to obtain 7-fluoro-1H-pyrido[2,3-b][1,4]oxazin-2-one as beige solid (0.70g, 71%).
¹H-NMR(DMSO-d₆, 300MHz); δ = 11.0 (br s, 1H), 7.77 (d, *J*=2.7Hz, 1H), 7.10 (dd, *J*=8.4Hz, 2.7Hz, 1H), 4.78 (s, 2H).
MS(ESI); 168.9(M⁺+1).

### Intermediate Synthesis Example (5)

### Synthesis of 8-chloro-2,3-dihydro-1H-pyrido[2,3-b][1,4]oxazine

### a) Synthesis of 2,4-dichloro-3-nitro-pyridine

3-nitro-pyridin-2,4-diol (5.0g, 0.032mmol) was dissolved in phosphorus oxychloride (30ml) and the reaction mixture was heated to 100°C and stirred for 18 hours. The reaction mixture was cooled to 0°C and the reaction was quenched by adding water slowly, and ammonia water (NH₄OH) was added therto dropwise to neutralize the mixture. The organic layer was extracted with dichloromethane, and the combined organic layer was washed with water and saturated saline solution, dried over anhydrous sodium sulfate (Na₂SO₄), filtered and evaporated under reduced pressure. The residue was purified by column chromatography on silica eluting with a solvent of dichloromethane. The fractions containing the product were collected and evaporated under reduced pressure to obtain pale-brown solid (4.01g, 65%).
¹H-NMR(CDCl₃, 300MHz); δ = 8.44 (d, *J*=5.4Hz, 1H), 7.46 (d, *J*=5.4Hz, 1H).

### b) Synthesis of 4-chloro-3-nitro-pyridin-2-ol

2,4-dichloro-3-nitro-pyridine (4.01 g, 20.8mmol) and cesium acetate (8.0g, 41.6mmol) were dissolved in anhydrous N,N-dimethyl formamide (104ml) under nitrogen atmosphere, and the reaction mixture was heated to 80°C and stirred for 18 hours. The reaction mixture was cooled to room temperature the reaction was quenched by adding water. The organic layer was Extracted with ethyl acetate, and the combined organic layer was washed with water and saturated saline solution, dried over anhydrous sodium sulfate (Na₂SO₄), filtered and evaporated under reduced pressure to obtain pale-yellow solid (1.69g, 47%)
¹H-NMR(DMSO-d₆, 300MHz); δ = 8.24 (d, *J*=5.7Hz, 1H), 7.09 (d, *J*=5.7Hz, 1H).

### c) Synthesis of 3-amino-4-chloro-pyridin-2-ol

4-chloro-3-nitro-pyridin-2-ol (1.69g, 9.72mmol) and tin tetrachloride dihydrate (SnCl₂·2H₂O) (10.97g, 48.62mmol) was dissolved in ethanol (32ml), heated to 70°C and stirred for 2 hours. The reaction mixture was cooled to room temperature and the ethanol was evaporated under reduced pressure. The residue was neutralized with saturated aqueous solution of sodium hydrogen carbonate, and extracted with ethyl acetate. The combined organic layer was washed with water and saturated saline solution, dried over anhydrous sodium sulfate (Na₂SO₄), filtered and evaporated under reduced pressure to obtain pale-yellow solid (650mg, 46%).
¹H-NMR(DMSO-d₆, 300MHz); δ = 10.8 (br s, 1H), 7.45 (d, *J*=5.4Hz, 1H), 6.69 (d, *J*=5.1Hz, 1H), 4.73 (br s, 2H).

### d) Synthesis of 8-chloro-1H-pyrido[2,3-b][1,4]oxazin-2-one

3-amino-4-chloro-pyridin-2-ol (650mg, 4.49mmol) was dissolved in anhydrous N,N-dimethyl formamide (22ml) under nitrogen atmosphere, and chloroacetyl chloride solution (0.394ml, 4.94mmol) was added thereto dropwise at room temperature and stirred for 30 minuntes. Potassium carbonate (1.55g, 11.24mmol) was added thereto dropwise at room temperature, and the reaction mixture was heated to 100°C and stirred for 18 hours. The reaction mixture was cooled to room temperature and water was added to quench the reaction, and the organic layer was extracted with ethyl acetate. The combined organic layer was washed with water and saturated saline solution, dried over anhydrous sodium sulfate (Na₂SO₄), filtered and evaporated under reduced pressure. The residue was recrystallized from dichloromethane and diisopropyl ether to obtain white solid (608mg, 73%).
¹H-NMR(DMSO-d₆, 300MHz); δ = 10.6 (s, 1H), 7.91 (d, *J*=5.7Hz, 1H), 7.04 (d, *J*=5.4Hz, 1H), 4.75 (s, 2H).

### e) Synthesis of 8-chloro-2,3-dihydro-1H-pyrido[2,3-b][1,4]oxazine

8-chloro-1H-pyrido[2,3-b][1,4]oxazin-2-one (600mg, 3.25mmol) was dissolved in anhydrous tetrahydrofuran (16ml) under nitrogen atmosphere, and then cooled to 0°C. 1.0M Lithium aluminum hydride dissolved in tetrahydrofuran (6.5ml, 6.50mmol) was added thereto dropwise, and then stirred for 30 minutes. The reaction temperature was raised to room temperature and stirred for 1 hour. The reaction mixture was cooled to 0°C again, and water (0.26ml), 10% sodium hydroxide solution (0.52ml) and water (0.78ml) were added thereto dropwise in this order, and then stirred vigorously at room temperature for 1 hour. The formed solid was filtered and washed with excess ethyl acetate.

The filtrate was washed with water and saturated saline solution, dried over anhydrous sodium sulfate (Na₂SO₄), filtered and evaporated under reduced pressure. The residue was purified by column chromatography on silica eluting with a solvent of dichloromethane:methanol=50:1. The fractions containing the product were collected and evaporated to obtain yellow solid (385mg, 70%).
¹H-NMR(DMSO-d₆, 300MHz); δ = 7.47 (d, *J*=5.4Hz, 1H), 6.72 (d, *J*=5.1Hz, 1H), 5.81 (s, 1H), 4.19 (m, 2H), 6.72 (m, 2H).

### Experimental Example 1: Test for inhibiting urate reuptake activity via hURAT1

### [hURAT1 overexpression by transient transfection in human kidney cell line]

A human kidney cell line, HEK293 was incubated by using OptiMEM medium in 100mm dish at (3*10^6 cells) for 24 hours. In order for the expression of hURAT1, a transfection mixture solution (Fugene6 18µℓ:hURAT1 DNA 6µg=3:1, in 600µℓ OptiMEM medium/dish) was prepared and kept for 15 minutes at room temperature. To each dish, 600µℓ of the mixture solution was injected and then incubated in 37°C cell incubator for 24 hours. The hURAT1-overexpressed HEK293 cell line was trypsinized and cells were harvested. Cells were resuspended in medium and seeded at 6*10^5 cells/well and incubated for 24 hours in 24-well plate which was surface-coated with Poly-D-Lysine.

### [Uric acid transport assay in hURAT1-overexpressed HEK293 cell line]

Each compound of the present invention prepared in the examples was dissolved in DMSO as 1000 fold concentrated stock sample solution with various concentration to examine. For each concentration, the stock sample solution was diluted in PBS (1µℓ in 800µℓ of PBS) as 1.25 fold concentrated working sample solution to obtain final concentrations of 0.01, 0.1, 1 and 10µM. The hURAT1-overexpressed cell line was washed with 1ml of PBS. After washing, PBS was discarded and the sample solution was diluted by 1.25 fold with PBS. Preincubation of cells with 200µℓ/well of sample solution made above was conducted for 30 minutes in 37°C cell incubator. After the incubation, each well was treated with 50µℓ of PBS containing 500µM [¹⁴C]-uric acid (final 100µM of uric acid/well). Cells were incubated for 2 minutes. The cells were washed twice with 1ml of PBS.

To measure the uric acid uptake, each well was treated with 150µℓ of M-per buffer (PIERCE) and kept in 37°C cell incubator for 30 minutes to make cell lysates. 150µℓ of cell lysates was transferred into test tube and 4ml of scintillation cocktail was added. CPM value of [¹⁴C]-uric acid absorbed into cells was detected using liquid scintillation counter. IC₅₀ for the inhibition of uric acid uptake by each test sample was analyzed by computational numerical processing. The results are shown in Table 1.

**[Table 1] Level of uric acid reuptake inhibition**

| **Compound No.** | **IC₅₀ (µM) hURAT** | **Compound No.** | **IC₅₀ (µM) hURAT** | **Compound No.** | **IC₅₀ (µM) hURAT** |
|---|---|---|---|---|---|
| **1** | 0.079 | **22-1** | 0.0888 | **49** | 0.311 |
| **2** | 0.1365 | **22-2** | 0.0043 | **50** | 0.0044 |
| **3** | 0.0165 | **24** | 0.3635 | **51** | 0.0115 |
| **4** | 0.023 | **25** | 0.0073 | **52** | 0.0103 |
| **5** | 0.1745 | **27-1** | 0.036 | **53** | 0.0562 |
| **6** | 0.0565 | **27-2** | 0.012 | **55** | 0.0488 |
| **7** | 0.3 | **29** | 0.2346 | **58** | 0.7417 |
| 8 | 0.353 | **30** | 0.028 | **61** | 0.002 |
| **9** | 0.02 | **35-2** | 0.277 | **62** | 0.1284 |
| **10** | 0.334 | **36** | 0.1745 | **63** | 0.0123 |
| **11** | 0.175 | **37** | 0.2117 | **64** | 0.007 |
| **12** | 0.0408 | **38** | 0.0964 | **65** | 0.003 |
| **13** | 0.337 | **39** | 0.0884 | **66** | 0.0358 |
| **14** | 0.1255 | **41** | 0.3755 | **67** | 0.0078 |
| **15** | 0.458 | **42** | 0.2 | **68** | 0.0232 |
| **16** | 0.93 | **45** | 0.0197 | **69** | 0.0331 |
| **17** | 1 | **46** | 0.0047 | **70** | 0.0048 |
| **19** | 0.2687 | **47-1** | 0.5162 | **74** | 0.006 |
| **20** | 0.034 | **47-2** | 0.0179 | | |
| **21** | 0.954 | **48** | 0.0933 | | |

### Expreimental Example 2: CYP2C9 enzyme inhibition assay

Each compound of the present invention prepared in the examples was dissolved in DMSO and adjust the concentration into 10mM stock sample solution. Make serial dilution by 10 fold scale in DMSO starting from 10mM to 0.01mM (500 fold concentrated stock). After fully agitated for 10 seconds, sample solution was sonicated for 1 hour. The stock sample solution was diluted in distilled water by 125 fold (e.g. add 2µℓ stock sample solution into 248µℓ of distilled water, 4 fold concentrated working sample solution) and agitated for 15 minutes to obtain homogenous sample suspension. Plating the working sample solution into 96 well plate to make final concentrations of 0.02, 0.2, 2 and 20µM. Each reagent used this assay was prepared following the manufacturer's instructions (Promega #V8792: V4790+V9790).

### [Preparation of the 4 fold concentrated CYP2C9 reaction mixture]

According to the number of test samples, 1.25µℓ of 1M potassium phosphate aqueous solution, 1µℓ of Luciferin-H (5mM) solution and 0.5µℓ of CYP2C9 membrane were mixed and prepared in 9.75µℓ of Luciferin-free, distilled water. For the calibration to the same amount of protein with CYP2C9 membrane, CYP2C9 membrane was substituted for 0.26µℓ of control membrane and added to a mixture having the same composition above in 9.99µℓ of Luciferin-free, distilled water to make total 12.5µℓ of reaction mixture.

### [Preparation of 2 fold concentrated CYP2C9 NADPH Regeneration system]

According to the number of test samples, 2.5µℓ of Solution A and 0.5µℓ of Solution B provided by the kit were mixed and prepared in 22µℓ of Luciferin-free, distilled water.

### [Preparation of Luciferin detection buffer]

To a known luciferin indicator provided as powder, 50ml of P450-Glo^{™} buffer solution was added and mixed, and then the resulting mixture was kept at -20□ under shading the light.

### [Experiment]

Each of the test samples was allocated in a light-shaded, 96 well white plate with a volume of 12.5 µℓ, and as a blank, 12.5 µℓ of a solvent having the same condition as that of the test samples was added. The pre-formulated 4 fold concentrated CYP2C9 mixture solution was allocated to each test sample and the blank with a volume of 12.5 µℓ. As a control, 12.5 µℓ of distilled water was added to a well and 12.5 µℓ of control membrane was added thereto and mixed together. After keeping the resulting mixtures in 37°C thermostatic device for 10 minutes, the pre-formulated 2 times concentrated NADPH mixture solution was allocated to each test sample and the control with a volume of 25 µℓ, mixed well, and kept in 37°C thermostatic device for 30 minutes. After keeping the resulting mixtures, 50 µℓ of luciferin indicator was allocated to each test sample and control, mixed well, and kept in 37°C thermostatic device for 20 minutes. The activity of the test sample to CYP2C9 enzyme was measured by phosphorescence detector. The level of enzyme inhibition (IC₅₀) was analyzed with using a formula provided by the kit.

The results are shown in Table 2. The reference IC₅₀ value of Benzbromaron was reported as 40nM for inhibiting CYP2C9 enzyme [Dermot F. McGinnity et al., Drug Metabolism and Disposition, 33, p1700-1707 (2005)]. In this experiment, IC₅₀ value of Benzbromarone was 51nM and each result of test compounds were shown in Table 2.

**[Table 2] Level of enzyme inhibition**

| **Compound No.** | **IC₅₀ (µM) CYP2C9** | **Compound No.** | **IC₅₀ (µM) CYP2C9** | **Compound No.** | **IC₅₀ (µM) CYP2C9** |
|---|---|---|---|---|---|
| **1** | 12.08 | **27-2** | >20 | **50** | 1.26 |
| **2** | >12 | **29** | >20 | **51** | 8.8 |
| **3** | 24.5 | **30** | >40 | **52** | >20 |
| **4** | 24.3 | **31** | >20 | **53** | 10.27 |
| **6** | 3.05 | **32** | >20 | **54** | >6 |
| **7** | >20 | **34** | 19.58 | **55** | 8.87 |
| **8** | >20 | **35-1** | >20 | **56** | >20 |
| **9** | >20 | **35-2** | >20 | **57** | >20 |
| **10** | >60 | **36** | 1.93 | **58** | >20 |
| **11** | >60 | **37** | >20 | **59** | >20 |
| **12** | >60 | **38** | >20 | **60** | >20 |
| **13** | >60 | **39** | >20 | **61** | 1.23 |
| **14** | 16.56 | **40** | 15.09 | **62** | >20 |
| **15** | 4.5 | **41** | >20 | **63** | 1.11 |
| **16** | 3.5 | **42** | 18.39 | **64** | 5.54 |
| **18** | 9.7 | **43** | >20 | **65** | 12.43 |
| **19** | 6.8 | **44** | >20 | **66** | 1.34 |
| **20** | 5.2 | **45** | 1.18 | **68** | 1.39 |
| **21** | 1.11 | **46** | 1.09 | **69** | >20 |
| **22-1** | 13.42 | **47-1** | >20 | **70** | 10.79 |
| **22-2** | 9.45 | **47-2** | >20 | **71** | >20 |
| **24** | >20 | **48** | 1.68 | **74** | 16.71 |
| **25** | >20 | **49** | >20 | **75** | 11.58 |
| **27-1** | >20 | | | | |

### Experimental Example 3: Test for solubility according to pH

### [First solution]

2.0g of sodium chloride was dissolved in 7.0mL of hydrochloric acid and water to make the total volume of 1000mL. This solution was colorless and clear. Its pH was about 1.2.

### [Second solution]

To 250mL of 0.2mol/L potassium dihydrogen phosphate solution, 118mL of 0.2mol/L sodium hydroxide solution and water were added to make the total volume of 1000mL. This solution was colorless and clear. Its pH was about 6.8.

### [Experiment]

For the compounds of present invention prepared in the examples, the test samples were prepared in each of the first and second solutions with 2mg/mL concentration. This test sample solution was agitated for 10 seconds, sonicated for 1 minute and heated at 37°C for 2 hours. The heated test sample solution was filtered to remove undissolved compounds therein. From filtered solution, 100µℓ of the completely dissolved solution was sampled and 100µℓ of acetonitrile was added thereto to make test sample solution. For each analysis of test sample solution, the solubility was measured by using liquid chromatography. The results of solubility test in the first and second solutions are shown in Table 3.

**[Table 3] Solubility**

| **Compound No.** | **Solubility (µg/mL)** | | **Compound No.** | **Solubility (µg/mL)** | |
|---|---|---|---|---|---|
| | **pH 1.2** | **pH 6.8** | | **pH 1.2** | **pH 6.8** |
| **1** | 56.1 | 276.8 | **33** | 1576.5 | 809.5 |
| **3** | 1420.1 | 886.5 | **35-2** | 1458.1 | 233.7 |
| **4** | 1927 | 970 | **36** | 1561.7 | 743.1 |
| **7** | 105.8 | 109.1 | **37** | 1931.1 | 114.2 |
| **8** | 986 | 344 | **39** | 1339.3 | 480.5 |
| **12** | 1882 | 132 | **40** | 1317.92 | 473.19 |
| **13** | 1395 | 847 | **41** | 808.87 | 156.54 |
| **14** | 1479 | 747.2 | **42** | 1310.4 | 142.6 |
| **15** | 1511 | 453 | **43** | 1260.4 | 1007.6 |
| **17** | 1612 | 1890 | **44** | 1960 | 2095.4 |
| **22-2** | <0.1 | 153 | **47-2** | 6.4 | 329.6 |
| **24** | 1254.6 | 133.6 | **59** | 2242.5 | 676.2 |
| **27-2** | 207.3 | 578.9 | **60** | 2125.3 | 599.6 |
| **29** | 1085.7 | 381.9 | **62** | 34.8 | 624 |
| **30** | 7.2 | 332 | **71** | 2138.2 | 344.7 |
| **31** | 1846.64 | 198.66 | **72** | 1873.1 | 375.4 |
| **32** | 1960.9 | 1474 | **73** | 1153.1 | 565.1 |

### Experimental Example 4: Test for uricosuric effect in Cebus monkey

For the example 4 compound which showed superior activity as described above, its uricosuric activity was tested by measuring uric acid level in plasma and urine in Cebus monkey. Placebo control (0.5% MC solution) and Benzbromarone was administered as a negative and positive control, respectively.

The example 4 compound was administered orally according to the clinical administration pathway. The administered dose was 15mg/kg for Benzbromarone, and 15mg/kg (same dose), 7.5mg/kg and 3.75mg/kg (lower doses) for the example 4 compound. The example 4 compound was prepared by suspending in vehicle (0.5% MC solution). The prepared sample was stirred sufficiently to make homogenous suspension, filled in an injector and administered through oral catheter at once. The catheter was washed again with 0.5% MC solution to remove remaining suspension in catheter, thereby completely administered the total samples into Cebus monkey. The test samples prepared above were administered once a day.

After the administration, blood and urine were collected as indicated time points (0, 0.5, 1, 2, 4, 8, and 24 hours). The concentrations of uric acid, creatinine and lactic acid in plasma and urine were measured to analyze the lowered plasma uric acid as well as its enhanced urine excretion.

Fractional excretion rates of uric acid (FEua) by each test group (vehicle, Benzbromarone, example 4 compound) during 0~8 hours FEua of example 4 compound (3.75mg/kg, 7.5mg/kg and 15mg/kg) were 24.1±13.3%, 31.6±13.3% and 33.2±19.8%, respectively, which showed superior effects compared with 8.5±1.5% of the placebo control group. The lowering effect of uric aicd in plasma which is followed by enhanced urine excretion was measured at 4 hours after the administration of 3.75mg/kg, 7.5mg/kg and 15mg/kg of example 4 compound. Plasma uric acid level was 3.88mg/dL, 3.66mg/dL and 3.40mg/dL, respectively, suggesting its superior effects to 5.10mg/dL of the placebo control group.

FEua of the Benzbromarone administered group during 0~8 hours of was 20.0±10.5%, and the uric acid level in plasma was 3.80mg/dL at 4 hours after the administration.

From the above results, the uricosruic effect of example 4 compound showed a remarkably superior effect than Benzbromarone at 15mg/kg showing 160% enhanced uricosuric effect during 0~8 hours in Cebus monkey. Furthermore, example 4 compound showed equal potency of uricosuric effect (24.1±13.3% of FEua) to 15mg/kg of Benzbromarone (20.0±10.5% of FEua) at 3.75mg/kg which was a quarter amount of Benzbromarone.

In summary, as a result of the test for the effect of enhancing uric acid excretion in Cebus monkey, the example 4 compound showed a superior uricosuric effect to the comparative drug, Benzbromarone.

Meanwhile, according to the results from comparison test of the example 3 compound with the compound disclosed in WO2006/057460, the example 3 compound showed 180% enhanced superior uricosuric activity compared to the compound disclosed in WO2006/057460.

All the study described above in Cebus monkey was performed by Japan Bioscience Center (JBS) which is one of the entrusted CRO in Japan.

## Claims

1. A heterocycle derivative compound having Formula I: 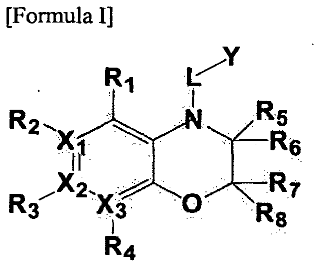 wherein, in Formula I,
each of X₁, X₂, and X₃ is independently carbon or nitrogen, provided that at least one of X₁, X₂, and X₃ is nitrogen,
each of R₁, R₂, R₃ and R₄ may be same or different and is independently selected from the group consisting of hydrogen; hydroxy; C₁-C₆ alkyl; C₂-C₇ alkenyl; C₂-C₇ alkynyl; C₁-C₆ hydroxyalkyl; C₁-C₆ haloalkyl; C₁-C₆ alkoxy; C₁-C₆ haloalkoxy; halogen; phenyl; cyano; nitro; amino; carboxylic acid group; phosphoric acid group; N-oxide; amide; C₁-C₆ alkylamide; aldehyde; hydroxamic acid; C₁-C₆ alkylsulfide; C₁-C₆ alkylthioxo; C₁-C₆ alkylsulfonyl; C₁-C₆ oximealkyl; C₁-C₆ aminoalkyl; C₃-C₈ alkylcarbonylalkyl; C₂-C₇ alkanoyl; C₂-C₇ alkoxycarbonyl; C₂-C₇ alkanoyloxy; C₃-C₁₂ mono or bicycloalkyl; C₄-C₁₂ cycloalkylalkyl; C₆-C₁₂ aryl; , saturated or unsaturated C₃-C₁₂ mono or polycarbocyclyl; and, saturated or unsaturated 3- to 12-membered mono or polyheterocyclyl containing 1 to 3 heteroatoms, provided that when X₁ is nitrogen, R₂ does not exist; when X₂ is nitrogen, R₃ does not exist; and when X₃ is nitrogen, R₄ does not exist, or each of R₁-R₂, R₂-R₃ and R₃-R₄ pairs may be independently fused to form a saturated or unsaturated 5- to 11-membered carbocycle or heterocycle,
each of R₅, R₆, R₇ and R₈ may be same or different and is independently selected from the group consisting of hydrogen; hydroxy; C₁-C₆ alkyl; C₂-C₇ alkenyl; C₂-C₇ alkynyl; C₁-C₆ hydroxyalkyl; C₁-C₆ haloalkyl; C₁-C₆ alkoxy; C₁-C₆ haloalkoxy; C₂-C₇ alkanoyl; phosphoric acid group; N-oxide; amide; C₁-C₆ alkylamide; aldehyde; hydroxamic acid; C₁-C₆ alkylsulfide; C₁-C₆ alkylthioxo; C₁-C₆ alkylsulfonyl; C₁-C₆ oximealkyl; C₁-C₆ aminoalkyl; C₃-C₈ alkylcarbonylalkyl; halogen; phenyl; cyano; nitro; amino; and carboxylic acid group, or R₅ and R₆ together with a carbon atom to which they are attached may form a carbonyl group (C=O) or thioxo group (C=S), or R₇ and R₈ together with a carbon atom to which they are attached may form a carbonyl group (C=O) or thioxo group (C=S),
L may form a carbonyl group (-C(=O)-), sulfonyl group (-S(=O)₂-), C₁-C₆ alkyl carbonyl, carbonyl C₁-C₆ alkyl or thioxo group (-C(=S)-), and
Y is selected from the group consisting of saturated or unsaturated C₃-C₁₂ mono or polycarbocyclyl substituted with R₉, R₁₀, and R₁₁; and saturated or unsaturated 3- to 12-membered mono or polyheterocyclyl containing 1 to 3 heteroatoms and being substituted with R₉, R₁₀, and R₁₁,
wherein each of R₉, R₁₀ and R₁₁ is independently selected from the group consisting of hydrogen; hydroxy; C₁-C₆alkyl; C₂-C₇ alkenyl; C₂-C₇ alkynyl; C₁-C₆ hydroxyalkyl; C₁-C₆ haloalkyl; C₁-C₆ alkoxy; C₁-C₆ haloalkoxy; halogen; C₁-C₆ alkylsulfide; C₁-C₆ alkylthioxo; hydroxamic acid; phenyl; cyano; nitro; amino; carboxylic acid group; amide; C₁-C₆ alkylamide; C₂-C₇ alkanoyl; aldehyde; C₃-C₈ ester; C₃-C₈ esteroxy; C₁-C₆ alkylsulfonyl; C₁-C₆ oximealkyl; C₁-C₆ aminoalkyl; C₃-C₈ alkylcarbonylalkyl; phosphoric acid group; and N-oxide,
provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen,
or racemate, or pharmaceutically acceptable salt thereof.

2. The compound according to claim 1, wherein L is a carbonyl group (-C(=O)-), sulfonyl group(-S(=O)₂), or thioxo group (-C(=S)-),
or racemate, or pharmaceutically acceptable salt thereof.

3. The compound according to claim 1, wherein each of R₅, R₆, R₇ and R₈ may be same or different and is independently selected from the group consisting of hydrogen; hydroxy; C₁-C₄ alkyl; C₁-C₄ haloalkyl; C₁-C₄ alkoxy; C₁-C₄ haloalkoxy; C₂-C₅ alkanoyl; halogen; phenyl; cyano; nitro; amino; and carboxylic acid group; or R₅ and R₆ together with a carbon atom to which they are attached may form a carbonyl group (C=O) or thioxo group (C=S), or R₇ and R₈ together with a carbon atom to which they are attached may form a carbonyl group (C=O) or thioxo group (C=S),
or racemate, or pharmaceutically acceptable salt thereof.

4. The compound according to claim 1, wherein Y is selected from the group consisting of saturated or unsaturated C₅-C₆ carbocyclyl substituted with R₉, R₁₀ and R₁₁; and saturated or unsaturated 5- to 6-membered heterocyclyl containing 1 to 3 heteroatoms and being substituted with R₉, R₁₀ and R₁₁, wherein R₉, R₁₀ and R₁₁ are the same as defined in claim 1,
or racemate, or pharmaceutically acceptable salt thereof.

5. The compound according to claim 1, wherein each of R₉, R₁₀ and R₁₁ is independently selected from the group consisting of hydrogen; hydroxy; C₁-C₄ alkyl; C₁-C₄ haloalkyl; C₁-C₄ alkoxy; C₁-C₄ haloalkoxy; halogen; phenyl; cyano; nitro; amino; carboxylic acid group; amide; C₁-C₆ alkylamide; C₂-C₅ alkanoyl; aldehyde; C₃-C₇ ester; C₃-C₇ esteroxy; C₁-C₄ alkylsulfonyl; C₁-C₄ oximealkyl; C₁-C₄ aminoalkyl; C₃-C₇ alkylcarbonylalkyl; phosphoric acid group; and N-oxide,
provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen,
or racemate, or pharmaceutically acceptable salt thereof.

6. The compound according to claim 1, wherein each of R₁-R₂, R₂-R₃ and R₃-R₄ pairs is independently fused to form a saturated or unsaturated 5- to 6-membered carbocycle or heterocycle, wherein the heterocycle preferably contains 1 to 3 heteroatoms selected from N, O and S,
or racemate, or pharmaceutically acceptable salt thereof.

7. The compound according to claim 1, wherein each of R₁, R₂, R₃ and R₄ may be same or different and is independently selected from the group consisting of hydrogen; hydroxy; C₁-C₄ alkyl; C₁-C₄ haloalkyl; unsubstituted or substituted C₁-C₄ alkoxy; C₁-C₄ haloalkoxy; halogen; phenyl; cyano; nitro; amino; carboxylic acid group; C₂-C₅ alkanoyl; C₂-C₅ alkoxycarbonyl; C₂-C₅ alkanoyloxy; C₃-C₁₀ mono or bicycloalkyl; C₄-C₁₁ cycloalkylalkyl; C₆-C₁₀ aryl; , saturated or unsaturated C₃-C₁₀ mono or polycarbocyclyl; and, saturated or unsaturated 3- to 10-membered mono or polyheterocyclyl containing 1 to 3 heteroatoms, provided that when X₁ is nitrogen, R₂ does not exist; when X₂ is nitrogen, R₃ does not exist; and when X₃ is nitrogen, R₄ does not exist,
or racemate, or pharmaceutically acceptable salt thereof.

8. The compound according to claim 1, wherein the C₃-C₁₂ mono or bicycloalkyl is a monocycloalkyl selected from cyclopropyl, cyclobutyl, cyclopentyl, cyclohexyl, cycloheptyl, cyclooctyl and cyclononyl, or a bicycloalkyl obtained by the fusion of the same or different two of said monocycloalkyls,
or racemate, or pharmaceutically acceptable salt thereof.

9. The compound according to claim 1, wherein the C₃-C₁₂ mono or polycarbocyclyl is a monocycloalkyl selected from cyclopropyl, cyclobutyl, cyclopentyl, cyclohexyl, cycloheptyl, cyclooctyl and cyclononyl, or a polycycloalkyl obtained by the fusion of the same or different two or more of said monocycloalkyls, or carboaryl,
or racemate, or pharmaceutically acceptable salt thereof.

10. The compound according to claim 1, wherein the saturated or unsaturated 3- to 12-membered mono or polyheterocyclyl containing 1 to 3 heteroatoms is thienyl, thiazolyl, imidazolyl, benzimidazolyl, triazolyl, tetrahydropyranyl, pyridinyl, furanyl, pyranyl, pyrrolyl, pyrazolyl, pyrazinyl, pyrimidinyl, isothiazolyl, isoxazolyl, pyridazinyl, isobenzopyranyl, chromenyl, indolyl, indazolyl, quinolinyl, purinyl, pyrrolinyl, chromanyl, pyrazolidinyl, piperidinyl or piperazinyl,
or racemate, or pharmaceutically acceptable salt thereof.

11. The compound according to claim 1, wherein X₂ is carbon, and each of X₁ and X₃ is independently carbon or nitrogen, provided that at least one of X₁ and X₃ is nitrogen,
or racemate, or pharmaceutically acceptable salt thereof.

12. The compound according to claim 1, wherein each of R₁, R₂, R₃ and R₄ may be same or different and is independently selected from the group consisting of hydrogen; C₁-C₄ alkyl; C₁-C₄ haloalkyl; halogen; phenyl; cyano; and , saturated or unsaturated 3- to 10-membered mono or polyheterocyclyl containing 1 to 3 heteroatoms; or each of R₁-R₂, R₂-R₃ and R₃-R₄ pairs may be independently fused to form a saturated or unsaturated 5- to 6-membered carbocycle,
provided that when X₁ is nitrogen, R₂ does not exist; when X₂ is nitrogen, R₃ does not exist; and when X₃ is nitrogen, R₄ does not exist,
or racemate, or pharmaceutically acceptable salt thereof.

13. The compound according to claim 1, wherein Y is an aromatic 5- to 6-membered carbocycle or heterocycle substituted with R₉, R₁₀ and R₁₁, wherein R₉, R₁₀ and R₁₁ are the same as defined in claim 1, or racemate, or pharmaceutically acceptable salt thereof.

14. The compound according to claim 1, wherein each of R₉, R₁₀ and R₁₁ is independently selected from the group consisting of hydrogen; hydroxy; C₁-C₄ alkyl; C₁-C₄ haloalkyl; C₁-C₄ alkoxy; C₁-C₄ haloalkoxy; halogen; nitro; carboxylic acid group; and C₃-C₇ esteroxy,
provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen,
or racemate, or pharmaceutically acceptable salt thereof.

15. The compound according to claim 1 wherein
each of X₁, X₂ and X₃ is independently carbon or nitrogen, provided that at least one of X₁, X₂ and X₃ is nitrogen,
each of R₁, R₂, R₃ and R₄ may be same or different and is independently selected from the group consisting of hydrogen; hydroxy; C₁-C₄ alkyl; C₁-C₄ haloalkyl; C₁-C₄ alkoxy; C₁-C₄ haloalkoxy; halogen; cyano; nitro; amino; carboxylic acid group; C₂-C₅ alkanoyl; C₂-C₅ alkoxycarbonyl; C₂-C₅ alkanoyloxy; C₆-C₁₂ aryl; and , saturated or unsaturated C₃-C₁₂ mono or polycarbocyclyl or heterocyclyl, provided that when X₁ is nitrogen, R₂ does not exist; when X₂ is nitrogen, R₃ does not exist; and when X₃ is nitrogen, R₄ does not exist, or each of R₁-R₂, R₂-R₃ and R₃-R₄ pairs may be independently fused to form a saturated or unsaturated 5- to 6-membered carbocycle or heterocycle,
each of R₅, R₆, R₇ and R₈ may be same or different and is independently selected from the group consisting of hydrogen; hydroxy; C₁-C₄ alkyl; C₁-C₄ haloalkyl; C₁-C₄ alkoxy; C₁-C₄ haloalkoxy; C₂-C₅ alkanoyl; halogen; cyano; nitro; amino; and carboxylic acid group, or R₅ and R₆ together with a carbon atom to which they are attached may form a carbonyl group (C=O), or R₇ and R₈ together with a carbon atom to which they are attached may form a carbonyl group (C=O),
L may form a carbonyl group (-C(=O)-), sulfonyl group (-S(=O)₂-) or thioxo group (-C(=S)-), and
Y is selected from the group consisting of saturated or unsaturated C₃-C₁₂ mono or polycarbocyclyl substituted with R₉, R₁₀ and R₁₁; and saturated or unsaturated 3- to 12-membered mono or polyheterocyclyl containing 1 to 3 heteroatoms and being substituted with R₉, R₁₀ and R₁₁, wherein the heterocycle preferably contains 1 to 3 heteroatoms selected from N, O and S,
wherein each of R₉, R₁₀ and R₁₁ is independently selected from the group consisting of hydrogen; hydroxy; C₁-C₄ alkyl; C₁-C₄ haloalkyl; C₁-C₄ alkoxy; C₁-C₄ haloalkoxy; halogen; cyano; nitro; amino; carboxylic acid group; and C₃-C₇ esteroxy,
provided that when Y is phenyl, i) at least one of R₉, R₁₀ and R₁₁ is hydroxy or ii) all of R₉, R₁₀ and R₁₁ are other than hydrogen if none of R₉, R₁₀ and R₁₁ is hydroxy, and when Y is pyridinyl, at least one of R₉, R₁₀ and R₁₁ is not hydrogen,
or racemate, or pharmaceutically acceptable salt thereof.

16. The compound according to claim 1 wherein
each of X₁, X₂ and X₃ is independently carbon or nitrogen, provided that at least one of X₁, X₂ and X₃ is nitrogen,
each of R₁, R₂, R₃ and R₄ may be same or different and is independently selected from the group consisting of hydrogen; C₁-C₄ alkyl; C₁-C₄ haloalkyl; C₁-C₄ alkoxy; halogen; cyano; nitro; amino; and saturated or unsaturated C₅-C₆ carbocyclyl or heterocyclyl, provided that when X₁ is nitrogen, R₂ does not exist; when X₂ is nitrogen, R₃ does not exist; and when X₃ is nitrogen, R₄ does not exist, or each of R₁-R₂, R₂-R₃ and R₃-R₄ pairs may be independently fused to form a phenyl or a 6-membered heterocycle containing 1 to 2 atoms of nitrogen or oxygen,
each of R₅, R₆, R₇ and R₈ may be same or different and is independently selected from the group consisting of hydrogen; unsubstituted or substituted C₁-C₄ alkyl; C₁-C₄ alkoxy; halogen; cyano; nitro; and amino,
L is a carbonyl group (-C(=O)-) or thioxo group (-C(=S)-), and
Y is a phenyl which has a hydroxy group in para-position to the attachment position of L and is further substituted with 1 to 3 substituents independently selected from halogen and nitro,
or racemate, or pharmaceutically acceptable salt thereof.

17. The compound according to claim 1 which is selected from the group consisting of:
(3,5-dibromo-4-hydroxy-phenyl)-(2,3-dihydro-pyrido[2,3-b][1,4]oxazin-1-yl)-methanone (compound 1);
(3,5-dibromo-4-methoxy-phenyl)-(2,3-dihydro-pyrido[4,3-b][1,4]oxazin-4-yl)-methanone (compound 2);
(3,5-dibromo-4-hydroxy-phenyl)-(2,3-dihydro-pyrido[4,3-b][1,4]oxazin-4-yl)-methanone hydrobromic acid salt (compound 3);
(3,5-dibromo-4-hydroxy-phenyl)-(2,3-dihydro-pyrido[4,3-b][1,4]oxazin-4-yl)-methanone (compound 4);
(3,5-dibromo-4-methoxy-phenyl)-(2,3-dihydro-pyrido[3,4-b][1,4]oxazin-1-yl)-methanone (compound 5);
(3,5-dibromo-4-hydroxy-phenyl)-(2,3-dihydro-pyrido[3,4-b][1,4]oxazin-1-yl)-methanone (compound 6);
(3,5-dibromo-4-hydroxy-phenyl)-(6-methyl-2,3-dihydro-pyrido[2,3-b][1,4]oxazin-1-yl)-methanone (compound 7);
(3,5-dibromo-4-hydroxy-phenyl)-(2,2-dimethyl-2,3-dihydro-pyrido[4,3-b][1,4]oxazin-4-yl)-methanone (compound 8);
(3,5-dibromo-4-hydroxy-phenyl)-(7-cyclopropyl-2,3-dihydro-pyrido[2,3-b][1,4]oxazin-1-yl)-methanone (compound 9);
(3-chloro-4-hydroxy-phenyl)-(2,3-dihydro-pyrido[4,3-b][1,4]oxazin-4-yl)-methanone hydrobromic acid salt (compound 10);
(3-bromo-4-hydroxy-phenyl)-(2,3-dihydro-pyrido[4,3-b][1,4]oxazin-4-yl)-methanone hydrobromic acid salt (compound 11);
(2,3-dihydro-pyrido[4,3-b][1,4]oxazin-4-yl)-(4-hydroxy-3-trifluoromethyl-phenyl)-methanone (compound 12);
(3,5-dichloro-4-hydroxy-phenyl)-(2,3-dihydro-pyrido[4,3-b][1,4]oxazin-4-yl)-methanone hydrobromic acid salt (compound 13);
(3-chloro-4-hydroxy-5-nitro-phenyl)-(2,3-dihydro-pyrido[4,3-b][1,4]oxazin-4-yl)-methanone (compound 14);
(3,5-dichloro-4-hydroxy-phenyl)-(2,3-dihydro-pyrido[3,4-b][1,4]oxazin-1-yl)-methanone hydrobromic acid salt (compound 15); (3-bromo-4-hydroxy-phenyl)-(2,3-dihydro-pyrido[3,4-b][1,4]oxazin-1-yl)-methanone hydrobromic acid salt (compound 16);
(3-chloro-4-hydroxy-5-nitro-phenyl)-(2,3-dihydro-pyrido[3,4-b][1,4]oxazin-1-yl)-methanone hydrobromic acid salt (compound 17);
(3-chloro-4-hydroxy-phenyl)-(2,3-dihydro-pyrido[3,4-b][1,4]oxazin-1-yl)-methanone hydrobromic acid salt (compound 18);
(2,3-dihydro-pyrido[3,4-b][1,4]oxazin-1-yl)-(4-hydroxy-3-trifluoromethyl-phenyl)-methanone (compound 19);
(3,5-dibromo-4-hydroxy-phenyl)-(7-phenyl-2,3-dihydro-pyrido[2,3-b] [1,4]oxazin-1-yl)-methanone (compound 20);
2,6-dichloro-4-(2,3-dihydro-pyrido[4,3-b][1,4]oxazin-4-sulfonyl)-phenol (compound 21);
(3,5-dibromo-4-methoxy-phenyl)-(7-trifluoromethyl-2,3-dihydro-pyrido[2,3-b][1,4]oxazin-1-yl)-methanone (compound 22-1);
(3,5-dibromo-4-hydroxy-phenyl)-(7-trifluoromethyl-2,3-dihydro-pyrido[2,3-b][1,4]oxazin-1-yl)-methanone (compound 22-2);
2,5-dibromo-4-(2,3-dihydro-pyrido[4,3-b][1,4]oxazin-4-carbonyl)-benzoic acid (compound 23);
[2,6-dibromo-4-(2,3-dihydro-pyrido[4,3-b][1,4]oxazin-4-carbonyl)-phenoxy]-acetic acid methyl ester (compound 24);
(7-bromo-2,3-dihydro-pyrido[2,3-b][1,4]oxazin-1-yl)-(3,5-dibromo-4-hydroxy-phenyl)-methanone (compound 25);
(2,3-dihydro-pyrido[4,3-b][1,4]oxazin-4-yl)-(3-fluoro-4-hydroxy-phenyl)-methanone (compound 26);
(3,5-dibromo-4-methoxy-phenyl)-(7-methyl-2,3-dihydro-pyrido[2,3-b][1,4]oxazin-1-yl)-methanone (compound 27-1);
(3,5-dibromo-4-hydroxy-phenyl)-(7-methyl-2,3-dihydro-pyrido[2,3-b][1,4]oxazin-1-yl)-methanone (compound 27-2);
(3,5-difluoro-4-methoxy-phenyl)-(2,3-dihydro-pyrido[4,3-b][1,4]oxazin-4-yl)-methanone (compound 28);
(3,5-difluoro-4-hydroxy-phenyl)-(2,3-dihydro-pyrido[4,3-b][1,4]oxazin-4-yl)-methanone (compound 29);
(5-chloro-2,3-dihydro-pyrido[4,3-b][1,4]oxazin-4-yl)-(3,5-dibromo-4-hydroxy-phenyl)-methanone (compound 30);
(2,6-dichloro-pyridin-4-yl)-(2,3-dihydro-pyrido[4,3-b][1,4]oxazin-4-yl)-methanone (compound 31);
(2,3-dihydro-pyrido[4,3-b][1,4]oxazin-4-yl)-(6-hydroxy-pyridin-3-yl)-methanone (compound 32);
(3,5-dibromo-4-hydroxy-phenyl)-(2,3-dihydro-pyrido[4,3-b][1,4]oxazin-4-yl)-methanone hydrochloric acid salt (compound 33);
(3-chloro-4-hydroxy-phenyl)-(2,3-dihydro-pyrido[3,4-b][1,4]oxazin-1-yl)-methanone (compound 34);
4-(2,3-dihydro-pyrido[4,3-b][1,4]oxazin-4-sulfonyl)-phenol (compound 35-1);
2,6-dibromo-4-(2,3-dihydro-pyrido[4,3-b][1,4]oxazin-4-sulfonyl)-phenol (compound 35-2);
(3-chloro-4-hydroxy-5-nitro-phenyl)-(2,3-dihydro-pyrido[3,4-b][1,4]oxazin-1-yl)-methanone (compound 36);
(3-chloro-4-hydroxy-phenyl)-(2,3-dihydro-pyrido[4,3-b][1,4]oxazin-4-yl)-methanone (compound 37);
(3-bromo-4-hydroxy-phenyl)-(2,3-dihydro-pyrido[4,3-b][1,4]oxazin-4-yl)-methanone (compound 38);
(3,5-dichloro-4-hydroxy-phenyl)-(2,3-dihydro-pyrido[4,3-b][1,4]oxazin-4-yl)-methanone (compound 39);
(3-bromo-4-hydroxy-phenyl)-(2,3-dihydro-pyrido[3,4-b][1,4]oxazin-1-yl)-methanone (compound 40);
(3,5-dichloro-4-hydroxy-phenyl)-(2,3-dihydro-pyrido[3,4-b][1,4]oxazin-1-yl)-methanone (compound 41);
2-(3,5-dibromo-4-hydroxy-phenyl)-1-(2,3-dihydro-pyrido[4,3-b][1,4]oxazin-4-yl)-ethanone (compound 42);
(2,3-dihydro-pyrido[4,3-b][1,4]oxazin-4-yl)-(3-methoxy-isoxazol-5-yl)-methanone (compound 43);
(2,3-dihydro-pyrido[4,3-b][1,4]oxazin-4-yl)-(3-hydroxy-isoxazol-5-yl)-methanone (compound 44);
(3,5-dibromo-4-hydroxy-phenyl)-[7-(4-trifluoromethyl-phenyl)-2,3-dihydro-pyrido[2,3-b][1,4]oxazin-1-yl]-methanone (compound 45);
(3,5-dibromo-4-hydroxy-phenyl)-[7-(2-trifluoromethyl-phenyl)-2,3-dihydro-pyrido[2,3-b][1,4]oxazin-1-yl]-methanone (compound 46);
1-(3,5-dibromo-4-methoxy-benzoyl)-2,3-dihydro-1H-pyrido[2,3-b][1,4]oxazin-7-carbonitrile (compound 47-1);
1-(3,5-dibromo-4-hydroxy-benzoyl)-2,3-dihydro-1H-pyrido[2,3-b][1,4]oxazin-7-carbonitrile (compound 47-2);
(3,5-dibromo-4-methoxy-phenyl)-[7-(3-nitro-phenyl)-2,3-dihydro-pyrido[2,3-b][1,4]oxazin-1-yl]-methanone (compound 48);
(3,5-dibromo-4-methoxy-phenyl)-[7-(3-nitro-phenyl)-2,3-dihydro-pyrido[2,3-b][1,4]oxazin-1-yl]-methanone (compound 49);
(3,5-dibromo-4-hydroxy-phenyl)-[7-(3-dimethylamino-phenyl)-2,3-dihydropyrido[2,3-b][1,4]oxazin-1-yl]-methanone (compound 50);
(3,5-dibromo-4-hydroxy-phenyl)-(2,3-dihydro-pyrido[4,3-b][1,4]oxazin-4-yl)-methanethione (compound 51);
(3,5-dibromo-4-hydroxy-phenyl)-(7-pyridin-3-yl-2,3-dihydro-pyrido[2,3-b][1,4]oxazin-1-yl]-methanone (compound 52);
(3,5-dibromo-4-hydroxy-phenyl)-(7-furan-3-yl-2,3-dihydro-pyrido[2,3-b][1,4]oxazin-1-yl]-methanone (compound 53);
1-(3,5-dibromo-4-hydroxy-phenyl)-2-(2,3-dihydro-pyrido[4,3-b][1,4]oxazin-4-yl)-ethanone (compound 54);
(3,5-dibromo-4-hydroxy-phenyl)-(2,3-dihydro-4-oxa-1,9-diaza-phenanthren-1-yl)-methanone (compound 55);
4-[2-(3,5-dibromo-4-hydroxy-phenyl)-2-oxo-ethyl)]-4H-pyrido[4,3-b][1,4]oxazin-3-one (compound 56);
4-(3,5-dibromo-4-methoxy-benzoyl)-4H-pyrido[4,3-b][1,4]oxazin-3-one (compound 57);
(3,5-dibromo-4-methoxy-phenyl)-(6-methyl-2,3-dihydro-pyrido[2,3-b][1,4]oxazin-1-yl)-methanone (compound 58);
(2,3-dihydro-pyrido[4,3-b][1,4]oxazin-4-yl)-(2,4-dihydroxy-pyrimidin-5-yl)-methanone (compound 59);
(2,3-dihydro-pyrido[4,3-b][1,4]oxazin-4-yl)-(2,6-dihydroxy-pyrimidin-4-yl)-methanone (compound 60);
(3,5-dibromo-4-hydroxy-phenyl)-(7-isoquinolin-4-yl-2,3-dihydro-pyrido[2,3-b][1,4]oxazin-1-yl)-methanone (compound 61);
(3,5-dibromo-4-hydroxy-phenyl)-(6,7-dihydro-pyrimido[4,5-b][1,4]oxazin-5-yl)-methanone (compound 62);
(3,5-dibromo-4-hydroxy-phenyl)-[7-(3-trifluoromethyl-phenyl)-2,3-dihydro-pyrido[2,3-b][1,4]oxazin-1-yl]-methanone (compound 63);
(3,5-dibromo-4-hydroxy-phenyl)-[7-(3-fluoromethyl-phenyl)-2,3-dihydro-pyrido[2,3-b][1,4]oxazin-1-yl]-methanone (compound 64);
4-[1-(3,5-dibromo-4-hydroxy-benzoyl)-2,3-dihydro-1H-pyrido[2,3-b][1,4]oxazin-7-yl]-benzonitrile (compound 65);
(3,5-dibromo-4-hydroxy-phenyl)-[7-(4-trifluoromethoxy-phenyl)-2,3-dihydro-pyrido[2,3-b][1,4]oxazin-1-yl]-methanone (compound 66);
1- {4-[1-(3,5-dibromo-4-hydroxy-benzoyl)-2,3-dihydro-1H-pyrido[2,3-b][1,4]oxazin-7-yl]-phenyl}-ethanone (compound 67);
(3,5-dibromo-4-hydroxy-phenyl)-[7-(5-methoxy-pyridin-3-yl)-2,3-dihydro-pyrido[2,3-b][1,4]oxazin-1-yl]-methanone (compound 68);
(4-hydroxy-3-trifluoromethyl-phenyl)-(7-methyl-2,3-dihydro-pyrido[2,3-b][1,4]oxazin-1-yl)-methanone (compound 69);
(3,5-dibromo-4-hydroxy-phenyl)-[7-(1H-indol-4-yl)-2,3-dihydro-pyrido[2,3-b][1,4]oxazin-1-yl]-methanone (compound 70);
(3,5-dibromo-4-hydroxy-phenyl)-(2,3-dihydro-pyrido[4,3-b][1,4]oxazin-4-yl)-methanone sulfuric acid salt (compound 71);
(2,6-dibromo-4-(2,3-dihydro-pyrido[4,3-b][1,4]oxazin-4-carbonyl)-phenolate sodium salt (compound 72);
(2,6-dibromo-4-(2,3-dihydro-pyrido[4,3-b][1,4]oxazin-4-carbonyl)-phenolate potassium salt (compound 73);
(3,5-dibromo-4-hydroxy-phenyl)-(2,3-dihydro-pyrido[4,3-b][1,4]oxazin-4-yl)-methanethione trifluoroacetic acid salt (compound 74); and
1-[1-(3,5-dibromo-4-hydroxy-benzoyl)-2,3-dihydro-1H-pyrido[2,3-b][1,4]oxazin-7-yl]-pyrrolidin-2-one (compound 75),
or racemate, or pharmaceutically acceptable salt thereof.

18. A method for preparing a compound of Formula II, or racemate, or pharmaceutically acceptable salt thereof, comprising the steps of:
a) reducing Formula VII compound to obtain Formula VI compound,
b) cyclizing the obtained Formula VI compound with Formula V compound to obtain Formula IV compound, and
c) peptide-bonding the obtained Formula IV compound with Formula III compound to obtain Formula II compound: 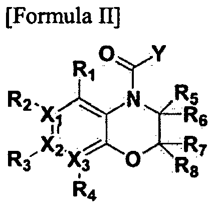 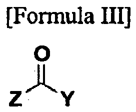 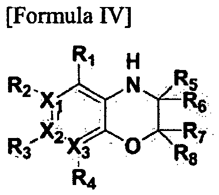 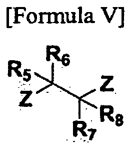 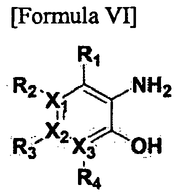 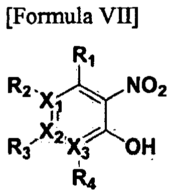 wherein, in Formulae II to VII, X₁, X₂, X₃, R₁, R₂, R₃, R₄, R₅, R₆, R₇, R₈ and Y are the same as defined in claim 1, and
Z represents a reactive leaving group.

19. A method for preparing a compound of Formula II, or racemate, or pharmaceutically acceptable salt thereof, comprising the steps of:
a) halogenating Formula VII compound to obtain Formula X compound and then reacting the obtained Formula X compound with Formula IX compound to obtain Formula VIII compound, or conducting a Mitsunobu reaction of Formula VII compound and Formula IX compound to obtain Formula VIII compound,
b) cyclizing the obtained Formula VIII compound to obtain Formula IV compound, and
c) peptide-bonding the obtained Formula IV compound with Formula III compound to obtain Formula II compound: 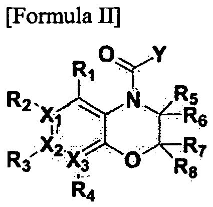 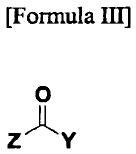 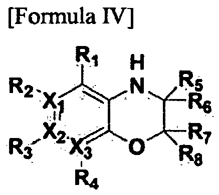 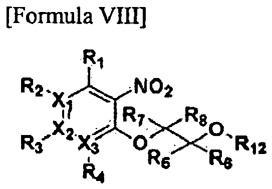 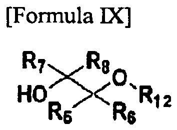 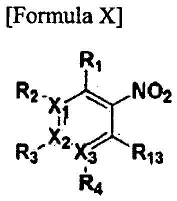 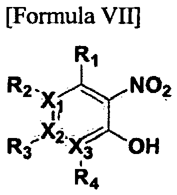 wherein, in Formulae II to X,
X₁, X₂, X₃, R₁, R₂, R₃, R₄, R₅, R₆, R₇, R₈ and Y are the same as defined in claim 1,
R₁₂ is a non-hydrogen substituent, and
R₁₃ represents a reactive leaving group.

20. A method for preparing a compound of Formula XI, or racemate, or pharmaceutically acceptable salt thereof, comprising the step of reacting Formula II compound with Lawesson's reagent: 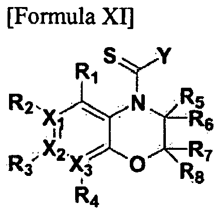 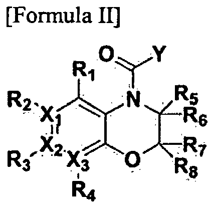 wherein, in Formulae II and XI,
X₁, X₂, X₃, R₁, R₂, R₃, R₄, R₅, R₆, R₇, R₈ and Y are the same as defined in claim 1.

21. A method for preparing a compound of Formula XII, or racemate, or pharmaceutically acceptable salt thereof, comprising the step of amide-bonding Formula IV compound with Formula XIII compound in the presence of base: 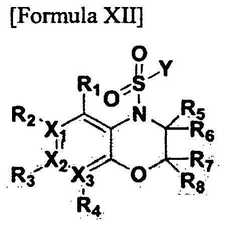 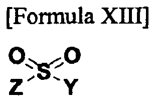 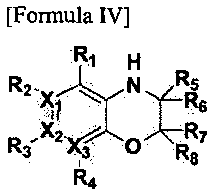 wherein, in Formulae IV, XII and XIII,
X₁, X₂, X₃, R₁, R₂, R₃, R₄, R₅, R₆, R₇, R₈ and Y are the same as defined in claim 1,
Z represents a reactive leaving group.

22. A method for preparing a compound of Formula XIV, or racemate, or pharmaceutically acceptable salt thereof, comprising the step of alkylating Formula IV compound with Formula XV compound in the presence of base: 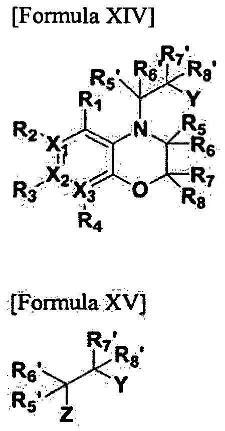 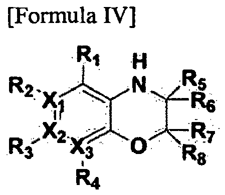 wherein, in Formulae IV to XV,
X₁, X₂, X₃, R₁, R₂, R₃, R₄, R₅, R₆, R₇, R₈ and Y are the same as defined in claim 1,
Z represents a reactive leaving group, and
each of R₅, R₆, R₇ and R₈ may be same or different and is independently selected from the group consisting of hydrogen; hydroxy; C₁-C₆ alkyl; C₁-C₆ haloalkyl; C₁-C₆ alkoxy; C₁-C₆ haloalkoxy; C₂-C₇ alkanoyl; halogen; phenyl; cyano; nitro; amino; and carboxylic acid group; or R₅' and R₆ together with a carbon atom to which they are attached may form a carbonyl group (C=O) or thioxo group (C=S), or R₇' and R₈' together with a carbon atom to which they are attached may form a carbonyl group (C=O) or thioxo group (C=S).

23. A pharmaceutical composition comprising an effective amount of the compound defined in any one of claims 1 to 17 or racemate, or pharmaceutically acceptable salt thereof as active ingredient, and pharmaceutically acceptable carrier.

24. The pharmaceutical composition according to claim 23 which is for treatment or prophylaxis of hyperuricemia, gout disease, nephritis, chronic renal failure,
nephrolithiasis, uremia, urolithiasis, or disease associated with uric acid.

25. The pharmaceutical composition according to claim 24, wherein the gout disease is acute gouty arthritis, chronic gouty arthritis, tophus or gout nephrosis.

26. The pharmaceutical composition according to claim 24, wherein the disease associated with uric acid is hyperlipidemia, ischemic heart disease, myocardial infarction, cerebral infarction, cerebrovascular disease, diabetes or hypertension.

27. The pharmaceutical composition according to claim 23 which is formulated for oral administration.

## Patentansprüche

1. Heterocyclusderivatverbindung, welche die Formel I hat: 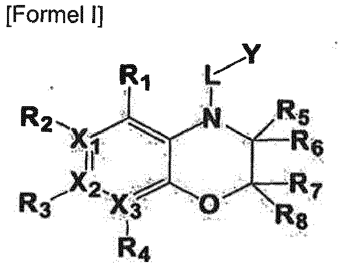 worin in der Formel I
jeder von X₁, X₂ und X₃ unabhängig Kohlenstoff oder Stickstoff ist, mit der Maßgabe, dass mindestens einer von X₁, X₂ und X₃ Stickstoff ist,
jeder von R₁, R₂, R₃ und R₄ gleich oder verschieden sein kann und unabhängig aus der Gruppe, bestehend aus Wasserstoff, Hydroxy, C₁-C₆-Alkyl, C₂-C₇-Alkenyl, C₂-C₇-Alkinyl, C₁-C₆-Hydroxyalkyl, C₁-C₆-Halogenalkyl, C₁-C₆-Alkoxy, C₁-C₆-Halogenalkoxy, Halogen, Phenyl, Cyano, Nitro, Amino, einer Carbonsäuregruppe, einer Phosphorsäuregruppe, N-Oxid, Amid, C₁-C₆-Alkylamid, Aldehyd, Hydroxamsäure, C₁-C₆-Alkylsulfid, C₁-C₆-Alkylthioxo, C₁-C₆-Alkylsulfonyl, C₁-C₆-Oximalkyl, C₁-C₆-Aminoalkyl, C₃-C₈-Alkylcarbonylalkyl, C₂-C₇-Alkanoyl, C₂-C₇-Alkoxycarbonyl, C₂-C₇-Alkanoyloxy, C₃-C₁₂-Mono- oder Bicycloalkyl, C₄-C₁₂-Cycloalkylalkyl, C₆-C₁₂-Aryl, gesättigtem oder ungesättigtem C₃-C₁₂-Mono- oder Polycarbocyclyl und gesättigtem oder ungesättigtem 3- bis 12-gliedrigen Mono- oder Polyheterocyclyl, das 1 bis 3 Heteroatome enthält, ausgewählt ist, mit der Maßgabe, dass dann, wenn X₁ Stickstoff ist, R₂ nicht vorliegt, wenn X₂ Stickstoff ist, R₃ nicht vorliegt, und wenn X₃ Stickstoff ist, R₄ nicht vorliegt, oder jedes von R₁-R₂-, R₂-R₃- und R₃-R₄-Paaren unabhängig anelliert sein kann, so dass ein gesättigter oder ungesättigter 5- bis 11-gliedriger Carbocyclus oder Heterocyclus gebildet wird,
jeder von R₅, R₆, R₇ und R₈ gleich oder verschieden sein kann und unabhängig aus der Gruppe, bestehend aus Wasserstoff, Hydroxy, C₁-C₆-Alkyl, C₂-C₇-Alkenyl, C₂-C₇-Alkinyl, C₁-C₆-Hydroxyalkyl, C₁-C₆-Halogenalkyl, C₁-C₆-Alkoxy, C₁-C₆-Halogenalkoxy, C₂-C₇-Alkanoyl, einer Phosphorsäuregruppe, N-Oxid, Amid, C₁-C₆-Alkylamid, Aldehyd, Hydroxamsäure, C₁-C₆-Alkylsulfid, C₁-C₆-Alkylthioxo, C₁-C₆-Alkylsulfonyl, C₁-C₆-Oximalkyl, C₁-C₆-Aminoalkyl, C₃-C₈-Alkylcarbonylalkyl, Halogen, Phenyl, Cyano, Nitro, Amino und einer Carbonsäuregruppe, ausgewählt ist, oder R₅ und R₆ zusammen mit einem Kohlenstoffatom, an das sie gebunden sind, eine Carbonylgruppe (C=O) oder eine Thioxogruppe (C=S) bilden können, oder R₇ und R₈ zusammen mit einem Kohlenstoffatom, an das sie gebunden sind, eine Carbonylgruppe (C=O) oder eine Thioxogruppe (C=S) bilden können,
L eine Carbonylgruppe (-C(=O)-), Sulfonylgruppe (-S(=O)₂-), C₁-C₆-Alkylcarbonyl-, Carbonyl-C₁-C₆-alkyl- oder Thioxogruppe (-C(=S)-) bilden kann und
Y aus der Gruppe, bestehend aus gesättigtem oder ungesättigtem C₃-C₁₂-Mono- oder Polycarbocyclyl, das mit R₉, R₁₀ und R₁₁ substituiert ist, und gesättigtem oder ungesättigtem 3- bis 12-gliedrigen Mono- oder Polyheterocyclyl, das 1 bis 3 Heteroatome enthält und mit R₉, R₁₀ und R₁₁ substituiert ist, ausgewählt ist,
wobei jeder von R₉, R₁₀ und R₁₁ unabhängig aus der Gruppe, bestehend aus Wasserstoff, Hydroxy, C₁-C₆-Alkyl, C₂-C₇-Alkenyl, C₂-C₇-Alkinyl, C₁-C₆-Hydroxyalkyl, C₁-C₆-Halogenalkyl, C₁-C₆-Alkoxy, C₁-C₆-Halogenalkoxy, Halogen, C₁-C₆-Alkylsulfid, C₁-C₆-Alkylthioxo, Hydroxamsäure, Phenyl, Cyano, Nitro, Amino, einer Carbonsäuregruppe, Amid, C₁-C₆-Alkylamid, C₂-C₇-Alkanoyl, Aldehyd, C₃-C₈-Ester, C₃-C₈-Esteroxy, C₁-C₆-Alkylsulfonyl, C₁-C₆- Oximalkyl, C₁-C₆-Aminoalkyl, C₃-C₈-Alkylcarbonylalkyl, einer Phosphorsäuregruppe und N-Oxid, ausgewählt ist,
mit der Maßgabe, dass dann, wenn Y Phenyl ist, i) mindestens einer von R₉, R₁₀ und R₁₁ Hydroxy ist, oder ii) alle von R₉, R₁₀ und R₁₁ von Wasserstoff verschieden sind, wenn keiner von R₉, R₁₀ und R₁₁ Hydroxy ist, und wenn Y Pyridinyl ist, mindestens einer von R₉, R₁₀ und R₁₁ nicht Wasserstoff ist,
oder ein Racemat oder ein pharmazeutisch verträgliches Salz davon.

2. Verbindung nach Anspruch 1, bei der L eine Carbonylgruppe (-C(=O)-), Sulfonylgruppe (-S(=O)₂-) oder Thioxogruppe (-C(=S)-) ist,
oder ein Racemat oder ein pharmazeutisch verträgliches Salz davon.

3. Verbindung nach Anspruch 1, bei der jeder von R₅, R₆, R₇ und R₈ gleich oder verschieden sein kann und unabhängig aus der Gruppe, bestehend aus Wasserstoff, Hydroxy, C₁-C₄-Alkyl, C₁-C₄-Halogenalkyl, C₁-C₄-Alkoxy, C₁-C₄-Halogenalkoxy, C₂-C₅-Alkanoyl, Halogen, Phenyl, Cyano, Nitro, Amino und einer Carbonsäuregruppe, ausgewählt ist, oder R₅ und R₆ zusammen mit einem Kohlenstoffatom, an das sie gebunden sind, eine Carbonylgruppe (C=O) oder eine Thioxogruppe (C=S) bilden können, oder R₇ und R₈ zusammen mit einem Kohlenstoffatom, an das sie gebunden sind, eine Carbonylgruppe (C=O) oder eine Thioxogruppe (C=S) bilden können,
oder ein Racemat oder ein pharmazeutisch verträgliches Salz davon.

4. Verbindung nach Anspruch 1, bei der Y aus der Gruppe, bestehend aus gesättigtem oder ungesättigtem C₅-C₆-Carbocyclyl, das mit R₉, R₁₀ und R₁₁ substituiert ist, und gesättigtem oder ungesättigtem 5- bis 6-gliedrigen Heterocyclyl, das 1 bis 3 Heteroatome enthält und mit R₉, R₁₀ und R₁₁ substituiert ist, wobei R₉, R₁₀ und R₁₁ mit denjenigen identisch sind, die im Anspruch 1 definiert sind, ausgewählt ist,
oder ein Racemat oder ein pharmazeutisch verträgliches Salz davon.

5. Verbindung nach Anspruch 1, bei der jeder von R₉, R₁₀ und R₁₁ unabhängig aus der Gruppe, bestehend aus Wasserstoff, Hydroxy, C₁-C₄-Alkyl, C₁-C₄-Halogenalkyl, C₁-C₄-Alk-oxy, C₁-C₄-Halogenalkoxy, Halogen, Phenyl, Cyano, Nitro, Amino, einer Carbonsäuregruppe, Amid, C₁-C₆-Alkylamid, C₂-C₅-Alkanoyl, Aldehyd, C₃-C₇-Ester, C₃-C₇-Esteroxy, C₁-C₄-Alkylsulfonyl, C₁-C₄-Oximalkyl, C₁-C₄-Aminoalkyl, C₃-C₇-Alkylcarbonylalkyl, einer Phosphorsäuregruppe und N-Oxid, ausgewählt ist,
mit der Maßgabe, dass dann, wenn Y Phenyl ist, i) mindestens einer von R₉, R₁₀ und R₁₁ Hydroxy ist, oder ii) alle von R₉, R₁₀ und R₁₁ von Wasserstoff verschieden sind, wenn keiner von R₉, R₁₀ und R₁₁ Hydroxy ist, und wenn Y Pyridinyl ist, mindestens einer von R₉, R₁₀ und R₁₁ nicht Wasserstoff ist,
oder ein Racemat oder ein pharmazeutisch verträgliches Salz davon.

6. Verbindung nach Anspruch 1, bei der jedes von R₁-R₂-, R₂-R₃- und R₃-R₄-Paaren unabhängig anelliert ist, so dass ein gesättigter oder ungesättigter 5- bis 6-gliedriger Carbocyclus oder Heterocyclus gebildet wird, wobei der Heterocyclus vorzugsweise 1 bis 3 Heteroatome enthält, die aus N, O und S ausgewählt sind,
oder ein Racemat oder ein pharmazeutisch verträgliches Salz davon.

7. Verbindung nach Anspruch 1, bei der jeder von R₁, R₂, R₃ und R₄ gleich oder verschieden sein kann und unabhängig aus der Gruppe, bestehend aus Wasserstoff, Hydroxy, C₁-C₄-Alkyl, C₁-C₄-Halogenalkyl, unsubstituiertem oder substituiertem C₁-C₄-Alkoxy, C₁-C₄-Halogenalkoxy, Halogen, Phenyl, Cyano, Nitro, Amino, einer Carbonsäuregruppe, C₂-C₅-Alkanoyl, C₂-C₅-Alkoxycarbonyl, C₂-C₅-Alkanoyloxy, C₃-C₁₀-Mono- oder Bicycloalkyl, C₄-C₁₁-Cycloalkylalkyl, C₆-C₁₀-Aryl, gesättigtem oder ungesättigtem C₃-C₁₀-Mono- oder Polycarbocyclyl und gesättigtem oder ungesättigtem 3- bis 10-gliedrigen Mono- oder Polyheterocyclyl, das 1 bis 3 Heteroatome enthält, ausgewählt ist, mit der Maßgabe, dass dann, wenn X₁ Stickstoff ist, R₂ nicht vorliegt, wenn X₂ Stickstoff ist, R₃ nicht vorliegt, und wenn X₃ Stickstoff ist, R₄ nicht vorliegt,
oder ein Racemat oder ein pharmazeutisch verträgliches Salz davon.

8. Verbindung nach Anspruch 1, bei der das C₃-C₁₂-Mono- oder Bicycloalkyl ein Monocycloalkyl ist, das aus Cyclopropyl, Cyclobutyl, Cyclopentyl, Cyclohexyl, Cycloheptyl, Cyclooctyl und Cyclononyl oder einem Bicycloalkyl ausgewählt ist, das durch die Vereinigung der gleichen oder von verschiedenen zwei der Monocycloalkyle erhalten wird,
oder ein Racemat oder ein pharmazeutisch verträgliches Salz davon.

9. Verbindung nach Anspruch 1, bei der das C₃-C₁₂-Mono- oder Polycarbocyclyl ein Monocycloalkyl ist, das aus Cyclopropyl, Cyclobutyl, Cyclopentyl, Cyclohexyl, Cycloheptyl, Cyclooctyl und Cyclononyl oder einem Polycycloalkyl, das durch die Vereinigung der gleichen oder von verschiedenen zwei oder mehr der Monocycloalkyle erhalten wird, oder Carboaryl ausgewählt ist,
oder ein Racemat oder ein pharmazeutisch verträgliches Salz davon.

10. Verbindung nach Anspruch 1, bei der das gesättigte oder ungesättigte 3- bis 12-gliedrige Mono- oder Polyheterocyclyl, das 1 bis 3 Heteroatome enthält, Thienyl, Thiazolyl, Imidazolyl, Benzimidazolyl, Triazolyl, Tetrahydropyranyl, Pyridinyl, Furanyl, Pyranyl, Pyrrolyl, Pyrazolyl, Pyrazinyl, Pyrimidinyl, Isothiazolyl, Isoxazolyl, Pyridazinyl, Isobenzopyranyl, Chromenyl, Indolyl, Indazolyl, Chinolinyl, Purinyl, Pyrrolinyl, Chromanyl, Pyrazolidinyl, Piperidinyl oder Piperazinyl ist,
oder ein Racemat oder ein pharmazeutisch verträgliches Salz davon.

11. Verbindung nach Anspruch 1, bei der X₂ Kohlenstoff ist und jeder von X₁ und X₃ unabhängig aus Kohlenstoff oder Stickstoff ausgewählt ist, mit der Maßgabe, dass mindestens einer von X₁ und X₃ Stickstoff ist,
oder ein Racemat oder ein pharmazeutisch verträgliches Salz davon.

12. Verbindung nach Anspruch 1, bei der jeder von R₁, R₂, R₃ und R₄ gleich oder verschieden sein kann und unabhängig aus der Gruppe, bestehend aus Wasserstoff, C₁-C₄-Alkyl, C₁-C₄-Halogenalkyl, Halogen, Phenyl, Cyano und einem gesättigtem oder ungesättigtem 3- bis 10-gliedrigen Mono- oder Polyheterocyclyl, das 1 bis 3 Heteroatome enthält, ausgewählt ist, oder jedes von R₁-R₂-, R₂-R₃- und R₃-R₄-Paaren unabhängig anelliert ist, so dass ein gesättigter oder ungesättigter 5- bis 6-gliedriger Carbocyclus gebildet wird,
mit der Maßgabe, dass dann, wenn X₁ Stickstoff ist, R₂ nicht vorliegt, wenn X₂ Stickstoff ist, R₃ nicht vorliegt, und wenn X₃ Stickstoff ist, R₄ nicht vorliegt,
oder ein Racemat oder ein pharmazeutisch verträgliches Salz davon.

13. Verbindung nach Anspruch 1, bei der Y ein aromatischer 5- bis 6-gliedriger Carbocyclus oder Heterocyclus ist, der mit R₉, R₁₀ und R₁₁ substituiert ist, wobei R₉, R₁₀ und R₁₁ mit denjenigen identisch sind, die im Anspruch 1 definiert sind,
oder ein Racemat oder ein pharmazeutisch verträgliches Salz davon.

14. Verbindung nach Anspruch 1, bei der jeder von R₉, R₁₀ und R₁₁ unabhängig aus der Gruppe, bestehend aus Wasserstoff, Hydroxy, C₁-C₄-Alkyl, C₁-C₄-Halogenalkyl, C₁-C₄-Alkoxy, C₁-C₄-Halogenalkoxy, Halogen, Nitro, einer Carbonsäuregruppe und C₃-C₇-Esteroxy, ausgewählt ist,
mit der Maßgabe, dass dann, wenn Y Phenyl ist, i) mindestens einer von R₉, R₁₀ und R₁₁ Hydroxy ist, oder ii) alle von R₉, R₁₀ und R₁₁ von Wasserstoff verschieden sind, wenn keiner von R₉, R₁₀ und R₁₁ Hydroxy ist, und wenn Y Pyridinyl ist, mindestens einer von R₉, R₁₀ und R₁₁ nicht Wasserstoff ist,
oder ein Racemat oder ein pharmazeutisch verträgliches Salz davon.

15. Verbindung nach Anspruch 1, bei der
jeder von X₁, X₂ und X₃ unabhängig Kohlenstoff oder Stickstoff ist, mit der Maßgabe, dass mindestens einer von X₁, X₂ und X₃ Stickstoff ist,
jeder von R₁, R₂, R₃ und R₄ gleich oder verschieden sein kann und unabhängig aus der Gruppe, bestehend aus Wasserstoff, Hydroxy, C₁-C₄-Alkyl, C₁-C₄-Halogenalkyl, C₁-C₄-Alkoxy, C₁-C₄-Halogenalkoxy, Halogen, Cyano, Nitro, Amino, einer Carbonsäuregruppe, C₂-C₅-Alkanoyl, C₂-C₅-Alkoxycarbonyl, C₂-C₅-Alkanoyloxy, C₆-C₁₂-Aryl und gesättigtem oder ungesättigtem C₃-C₁₂-Mono- oder Polycarbocyclyl oder heterocyclyl, ausgewählt ist, mit der Maßgabe, dass dann, wenn X₁ Stickstoff ist, R₂ nicht vorliegt, wenn X₂ Stickstoff ist, R₃ nicht vorliegt, und wenn X₃ Stickstoff ist, R₄ nicht vorliegt, oder jedes von R₁-R₂-, R₂-R₃- und R₃-R₄-Paaren unabhängig anelliert sein kann, so dass ein gesättigter oder ungesättigter 5- bis 6-gliedriger Carbocyclus oder Heterocyclus gebildet wird,
jeder von R₅, R₆, R₇ und R₈ gleich oder verschieden sein kann und unabhängig aus der Gruppe, bestehend aus Wasserstoff, Hydroxy, C₁-C₄-Alkyl, C₁-C₄-Halogenalkyl, C₁-C₄-Alkoxy, C₁-C₄-Halogenalkoxy, C₂-C₅-Alkanoyl, Halogen, Cyano, Nitro, Amino und einer Carbonsäuregruppe, ausgewählt ist, oder R₅ und R₆ zusammen mit einem Kohlenstoffatom, an das sie gebunden sind, eine Carbonylgruppe (C=O) bilden können, oder R₇ und R₈ zusammen mit einem Kohlenstoffatom, an das sie gebunden sind, eine Carbonylgruppe (C=O) bilden können,
L eine Carbonylgruppe (-C(=O)-), Sulfonylgruppe (-S(=O)₂-) oder Thioxogruppe (-C(=S)-) bilden kann und
Y aus der Gruppe, bestehend aus gesättigtem oder ungesättigtem C₃-C₁₂-Mono- oder Polycarbocyclyl, das mit R₉, R₁₀ und R₁₁ substituiert ist, und gesättigtem oder ungesättigtem 3- bis 12-gliedrigen Mono- oder Polyheterocyclyl, das 1 bis 3 Heteroatome enthält und mit R₉, R₁₀ und R₁₁ substituiert ist, wobei der Heterocyclus vorzugsweise 1 bis 3 Heteroatome enthält, die aus N, O und S ausgewählt sind, ausgewählt ist,
wobei jeder von R₉, R₁₀ und R₁₁ unabhängig aus der Gruppe, bestehend aus Wasserstoff, Hydroxy, C₁-C₄-Alkyl, C₁-C₄-Halogenalkyl, C₁-C₄-Alkoxy, C₁-C₄-Halogenalkoxy, Halogen, Cyano, Nitro, Amino, einer Carbonsäuregruppe und C₃-C₇-Esteroxy, ausgewählt ist,
mit der Maßgabe, dass dann, wenn Y Phenyl ist, i) mindestens einer von R₉, R₁₀ und R₁₁ Hydroxy ist, oder ii) alle von R₉, R₁₀ und R₁₁ von Wasserstoff verschieden sind, wenn keiner von R₉, R₁₀ und R₁₁ Hydroxy ist, und wenn Y Pyridinyl ist, mindestens einer von R₉, R₁₀ und R₁₁ nicht Wasserstoff ist,
oder ein Racemat oder ein pharmazeutisch verträgliches Salz davon.

16. Verbindung nach Anspruch 1, bei der
jeder von X₁, X₂ und X₃ unabhängig Kohlenstoff oder Stickstoff ist, mit der Maßgabe, dass mindestens einer von X₁, X₂ und X₃ Stickstoff ist,
jeder von R₁, R₂, R₃ und R₄ gleich oder verschieden sein kann und unabhängig aus der Gruppe, bestehend aus Wasserstoff, C₁-C₄-Alkyl, C₁-C₄-Halogenalkyl, C₁-C₄-Alkoxy, Halogen, Cyano, Nitro, Amino, und gesättigtem oder ungesättigtem C₅-C₆-Carbocyclyl oder - Heterocyclyl, ausgewählt ist, mit der Maßgabe, dass dann, wenn X₁ Stickstoff ist, R₂ nicht vorliegt, wenn X₂ Stickstoff ist, R₃ nicht vorliegt, und wenn X₃ Stickstoff ist, R₄ nicht vorliegt, oder jedes von R₁-R₂-, R₂-R₃- und R₃-R₄-Paaren unabhängig anelliert sein kann, so dass ein Phenyl oder ein 6-gliedriger Heterocyclus gebildet wird, der 1 bis 2 Atome von Stickstoff oder Sauerstoff enthält,
jeder von R₅, R₆, R₇ und R₈ gleich oder verschieden sein kann und unabhängig aus der Gruppe, bestehend aus Wasserstoff, unsubstituiertem oder substituiertem C₁-C₄-Alkyl, C₁-C₄-Alkoxy, Halogen, Cyano, Nitro und Amino, ausgewählt ist,
L eine Carbonylgruppe (-C(=O)-) oder Thioxogruppe (-C(=S)-) ist und
Y ein Phenyl ist, das eine Hydroxygruppe in der para-Position zu der Bindungsposition von L aufweist und ferner mit 1 bis 3 Substituenten substituiert ist, die unabhängig aus Halogen und Nitro ausgewählt sind,
oder ein Racemat oder ein pharmazeutisch verträgliches Salz davon.

17. Verbindung nach Anspruch 1, die aus der Gruppe, bestehend aus:
(3,5-Dibrom-4-hydroxyphenyl)-(2,3-dihydropyrido[2,3-b][1,4]oxazin-1-yl)-methanon (Verbindung 1),
(3,5-Dibrom-4-methoxyphenyl)-(2,3-dihydropyrido[4,3-b][1,4]oxazin-4-yl)-methanon (Verbindung 2),
(3,5-Dibrom-4-hydroxyphenyl)-(2,3-dihydropyrido[4,3-b][1,4]oxazin-4-yl)-methanon-Bromwasserstoffsäuresalz (Verbindung 3),
(3,5-Dibrom-4-hydroxyphenyl)-(2,3-dihydropyrido[4,3-b][1,4]oxazin-4-yl)-methanon (Verbindung 4),
(3,5-Dibrom-4-methoxyphenyl)-(2,3-dihydropyrido[3,4-b][1,4]oxazin-1-yl)-methanon (Verbindung 5),
(3,5-Dibrom-4-hydroxyphenyl)-(2,3-dihydropyrido[3,4-b][1,4]oxazin-1-yl)-methanon (Verbindung 6),
(3,5-Dibrom-4-hydroxyphenyl)-(6-methyl-2,3-dihydropyrido[2,3-b][1,4]oxazin-1-yl)-methanon (Verbindung 7),
(3,5-Dibrom-4-hydroxyphenyl)-(2,2-dimethyl-2,3-dihydropyrido[4,3-b][1,4]oxazin-4-yl)-methanon (Verbindung 8),
(3,5-Dibrom-4-hydroxyphenyl)-(7-cyclopropyl-2,3-dihydropyrido[2,3-b][1,4]oxazin-1-yl)-methanon (Verbindung 9),
(3-Chlor-4-hydroxyphenyl)-(2,3-dihydropyrido[4,3-b][1,4]oxazin-4-yl)-methanon-Bromwasserstoffsäuresalz (Verbindung 10),
(3-Brom-4-hydroxyphenyl)-(2,3-dihydropyrido[4,3-b][1,4]oxazin-4-yl)-methanon-Bromwasserstoffsäuresalz (Verbindung 11),
(2,3-Dihydropyrido[4,3-b][1,4]oxazin-4-yl)-(4-hydroxy-3-trifluormethylphenyl)-methanon (Verbindung 12),
(3,5-Dichlor-4-hydroxyphenyl)-(2,3-dihydropyrido[4,3-b][1,4]oxazin-4-yl)-methanon-Bromwasserstoffsäuresalz (Verbindung 13),
(3-Chlor-4-hydroxy-5-nitrophenyl)-(2,3-dihydropyrido[4,3-b][1,4]oxazin-4-yl)-methanon (Verbindung 14),
(3,5-Dichlor-4-hydroxyphenyl)-(2,3-dihydropyrido[3,4-b][1,4]oxazin-1-yl)-methanon-Bromwasserstoffsäuresalz (Verbindung 15),
(3-Brom-4-hydroxyphenyl)-(2, 3-dihydropyrido[3,4-b][1,4]oxazin-1-yl)-methanon-Bromwasserstoffsäuresalz (Verbindung 16),
(3-Chlor-4-hydroxy-5-nitrophenyl)-(2,3-dihydropyrido[3,4-b][1,4]oxazin-1-yl)-methanon-Bromwasserstoffsäuresalz (Verbindung 17),
(3-Chlor-4-hydroxyphenyl)-(2,3-dihydropyrido[3,4-b][1,4]oxazin-1-yl)-methanon-Bromwasserstoffsäuresalz (Verbindung 18),
(2,3-Dihydropyrido[3,4-b][1,4]oxazin-1-yl)-(4-hydroxy-3-trifluormethylphenyl)-methanon (Verbindung 19),
(3,5-Dibrom-4-hydroxyphenyl)-(7-phenyl-2,3-dihydropyrido[2,3-b][1,4]oxazin-1-yl)-methanon (Verbindung 20),
2,6-Dichlor-4-(2,3-dihydropyrido[4,3-b][1,4]oxazin-4-sulfonyl)-phenol (Verbindung 21),
(3,5-Dibrom-4-methoxyphenyl)-(7-trifluormethyl-2, 3-dihydropyrido[2,3-b][1,4]oxazin-1-yl)-methanon (Verbindung 22-1),
(3,5-Dibrom-4-hydroxyphenyl)-(7-trifluormethyl-2,3-dihydropyrido[2,3-b][1,4]oxazin-1-yl)-methanon (Verbindung 22-2),
2,5-Dibrom-4-(2,3-dihydropyrido[4,3-b][1,4]oxazin-4-carbonyl)-benzoesäure (Verbindung 23),
[2,6-Dibrom-4-(2,3-dihydropyrido[4,3-b][1,4]oxazin-4-carbonyl)-phenoxy]-essigsäuremethylester (Verbindung 24),
(7-Brom-2,3-dihydropyrido[2,3-b][1,4]oxazin-1-yl)-(3,5-dibrom-4-hydroxyphenyl)-methanon (Verbindung 25),
(2,3-Dihydropyrido[4,3-b][1,4]oxazin-4-yl)-(3-fluor-4-hydroxyphenyl)-methanon (Verbindung 26),
(3,5-Dibrom-4-methoxyphenyl)-(7-methyl-2,3-dihydropyrido[2,3-b][1,4]oxazin-1-yl)-methanon (Verbindung 27-1),
(3,5-Dibrom-4-hydroxyphenyl)-(7-methyl-2,3-dihydropyrido[2,3-b][1,4]oxazin-1-yl)-methanon (Verbindung 27-2),
(3,5-Difluor-4-methoxyphenyl)-(2,3-dihydropyrido[4,3-b][1,4]oxazin-4-yl)-methanon (Verbindung 28),
(3,5-Difluor-4-hydroxyphenyl)-(2,3-dihydropyrido[4,3-b][1,4]oxazin-4-yl)-methanon (Verbindung 29),
(5-Chlor-2,3-dihydropyrido[4,3-b][1,4]oxazin-4-yl)-(3,5-dibrom-4-hydroxyphenyl)-methanon (Verbindung 30),
(2,6-Dichlorpyridin-4-yl)-(2,3-dihydropyrido[4,3-b][1,4]oxazin-4-yl)-methanon (Verbindung 31),
(2,3-Dihydropyrido[4,3-b][1,4]oxazin-4-yl)-(6-hydroxypyridin-3-yl)-methanon (Verbindung 32),
(3,5-Dibrom-4-hydroxyphenyl)-(2,3-dihydropyrido[4,3-b][1,4]oxazin-4-yl)-methanon-Chlorwasserstoffsäuresalz (Verbindung 33),
(3-Chlor-4-hydroxyphenyl)-(2,3-dihydropyrido[3,4-b][1,4]oxazin-1-yl)-methanon (Verbindung 34),
4-(2,3-Dihydropyrido[4,3-b][1,4]oxazin-4-sulfonyl)-phenol (Verbindung 35-1),
2,6-Dibrom-4-(2,3-dihydropyrido[4,3-b][1,4]oxazin-4-sulfonyl)-phenol (Verbindung 35-2),
(3-Chlor-4-hydroxy-5-nitrophenyl)-(2,3-dihydropyrido[3,4-b][1,4]oxazin-1-yl)-methanon (Verbindung 36),
(3-Chlor-4-hydroxyphenyl)-(2,3-dihydropyrido[4,3-b][1,4]oxazin-4-yl)-methanon (Verbindung 37),
(3-Brom-4-hydroxyphenyl)-(2,3-dihydropyrido[4,3-b][1,4]oxazin-4-yl)-methanon (Verbindung 38),
(3,5-Dichlor-4-hydroxyphenyl)-(2,3-dihydropyrido[4,3-b][1,4]oxazin-4-yl)-methanon (Verbindung 39),
(3-Brom-4-hydroxyphenyl)-(2,3-dihydropyrido[3,4-b][1,4]oxazin-1-yl)-methanon (Verbindung 40),
(3,5-Dichlor-4-hydroxyphenyl)-(2,3-dihydropyrido[3,4-b][1,4]oxazin-1-yl)-methanon (Verbindung 41),
2-(3,5-Dibrom-4-hydroxyphenyl)-1-(2,3-dihydropyrido[4,3-b][1,4]oxazin-4-yl)-ethanon (Verbindung 42),
(2,3-Dihydropyrido[4,3-b][1,4]oxazin-4-yl)-(3-methoxyisoxazol-5-yl)-methanon (Verbindung 43),
(2,3-Dihydropyrido[4,3-b][1,4]oxazin-4-yl)-(3-hydroxyisoxazol-5-yl)-methanon (Verbindung 44),
(3,5-Dibrom-4-hydroxyphenyl)-[7-(4-trifluormethylphenyl)-2,3-dihydropyrido[2,3-b][1,4]oxazin-1-yl]-methanon (Verbindung 45),
(3,5-Dibrom-4-hydroxyphenyl)-[7-(2-trifluormethylphenyl)-2,3-dihydropyrido[2,3-b][1,4]oxazin-1-yl]-methanon (Verbindung 46),
1-(3,5-Dibrom-4-methoxybenzoyl)-2,3-dihydro-1 H-pyrido[2,3-b][1,4]oxazin-7-carbonitril (Verbindung 47-1),
1-(3,5-Dibrom-4-hydroxybenzoyl)-2,3-dihydro-1H-pyrido[2,3-b][1,4]oxazin-7-carbonitril (Verbindung 47-2),
(3,5-Dibrom-4-methoxyphenyl)-[7-(3-nitrophenyl)-2,3-dihydropyrido[2,3-b][1,4]oxazin-1-yl]-methanon (Verbindung 48),
(3,5-Dibrom-4-methoxyphenyl)-[7-(3-nitrophenyl)-2,3-dihydropyrido[2,3-b][1,4]oxazin-1-yl]-methanon (Verbindung 49),
(3,5-Dibrom-4-hydroxyphenyl)-[7-(3-dimethylaminophenyl)-2,3-dihydropyrido[2,3-b][1,4]oxazin-1-yl]-methanon (Verbindung 50),
(3,5-Dibrom-4-hydroxyphenyl)-2,3-dihydropyrido[4,3-b][1,4]oxazin-4-yl)-methanthion (Verbindung 51),
(3,5-Dibrom-4-hydroxyphenyl)-(7-pyridin-3-yl-2,3-dihydropyrido[2,3-b][1,4]oxazin-1-yl]-methanon (Verbindung 52),
(3,5-Dibrom-4-hydroxyphenyl)-(7-furan-3-yl-2,3-dihydropyrido[2,3-b][1,4]oxazin-1-yl]-methanon (Verbindung 53),
1-(3,5-Dibrom-4-hydroxyphenyl)-2-(2,3-dihydropyrido[4,3-b][1,4]oxazin-4-yl)-ethanon (Verbindung 54),
(3,5-Dibrom-4-hydroxyphenyl)-(2,3-dihydro-4-oxa-1,9-diazaphenanthren-1-yl)-methanon (Verbindung 55),
4-[2-(3,5-Dibrom-4-hydroxyphenyl)-2-oxoethyl)]-4H-pyrido[4,3-b][1,4]oxazin-3-on (Verbindung 56),
4-(3,5-Dibrom-4-methoxybenzoyl)-4H-pyrido[4,3-b][1,4]oxazin-3-on (Verbindung 57),
(3,5-Dibrom-4-methoxyphenyl)-(6-methyl-2,3-dihydropyrido[2,3-b][1,4]oxazin-1-yl)-methanon (Verbindung 58),
(2,3-Dihydropyrido[4,3-b][1,4]oxazin-4-yl)-(2,4-dihydroxypyrimidin-5-yl)-methanon (Verbindung 59),
(2,3-Dihydropyrido[4,3-b][1,4]oxazin-4-yl)-(2,6-dihydroxypyrimidin-4-yl)-methanon (Verbindung 60),
(3,5-Dibrom-4-hydroxyphenyl)-(7-isochinolin-4-yl-2,3-dihydropyrido[2,3-b][1,4]oxazin-1-yl)-methanon (Verbindung 61),
(3,5-Dibrom-4-hydroxyphenyl)-(6,7-dihydropyrimido[4,5-b][1,4]oxazin-5-yl)-methanon (Verbindung 62),
(3,5-Dibrom-4-hydroxyphenyl)-[7-(3-trifluormethylphenyl)-2,3-dihydropyrido[2,3-b][1,4]oxazin-1-yl]-methanon (Verbindung 63),
(3,5-Dibrom-4-hydroxyphenyl)-[7-(3-fluormethylphenyl)-2,3-dihydropyrido[2,3-b][1,4]oxazin-1-yl]-methanon (Verbindung 64),
4-[1-(3,5-Dibrom-4-hydroxybenzoyl)-2,3-dihydro-1H-pyrido[2,3-b][1,4]oxazin-7-yl]-benzonitril (Verbindung 65),
(3,5-Dibrom-4-hydroxyphenyl)-[7-(4-trifluormethoxyphenyl)-2,3-dihydropyrido[2,3-b][1,4]oxazin-1-yl]-methanon (Verbindung 66),
1-{4-[1-(3,5-Dibrom-4-hydroxybenzoyl)-2,3-dihydro-1H-pyrido[2,3-b][1,4]oxazin-7-yl]-phenyl}-ethanon (Verbindung 67),
(3,5-Dibrom-4-hydroxyphenyl)-[7-(5-methoxypyridin-3-yl)-2,3-dihydropyrido[2,3-b][1,4]oxazin-1-yl]-methanon (Verbindung 68),
(4-Hydroxy-3-trifluormethylphenyl)-(7-methyl-2,3-dihydropyrido[2,3-b][1,4]oxazin-1-yl)-methanon (Verbindung 69),
(3,5-Dibrom-4-hydroxyphenyl)-[7-(1H-indol-4-yl)-2,3-dihydropyrido[2,3-b][1,4]oxazin-1-yl]-methanon (Verbindung 70),
(3,5-Dibrom-4-hydroxyphenyl)-(2,3-dihydropyrido[4,3-b][1,4]oxazin-4-yl)-methanon-Schwefelsäuresalz (Verbindung 71),
(2,6-Dibrom-4-(2,3-dihydropyrido[4,3-b][1,4]oxazin-4-carbonyl)-phenolat-Natriumsalz (Verbindung 72),
(2,6-Dibrom-4-(2,3-dihydropyrido[4,3-b][1,4]oxazin-4-carbonyl)-phenolat-Kaliumsalz (Verbindung 73),
(3,5-Dibrom-4-hydroxyphenyl)-(2,3-dihydropyrido[4,3-b][1,4]oxazin-4-yl)-methanthion-Trifluoressigsäuresalz (Verbindung 74) und
1-[1-(3,5-Dibrom-4-hydroxybenzoyl)-2,3-dihydro-1H-pyrido[2,3-b][1,4]oxazin-7-yl]-pyrrolidin-2-on (Verbindung 75),
oder einem Racemat oder einem pharmazeutisch verträglichen Salz davon, ausgewählt ist.

18. Verfahren zur Herstellung einer Verbindung mit der Formel II oder eines Racemats oder eines pharmazeutisch verträglichen Salzes davon, umfassend die Schritte:
a) Reduzieren der Verbindung mit der Formel VII, so dass eine Verbindung mit der Formel VI erhalten wird,
b) Cyclisieren der erhaltenen Verbindung mit der Formel VI mit der Verbindung mit der Formel V, so dass eine Verbindung mit der Formel IV erhalten wird, und
c) Peptidbinden der erhaltenen Verbindung mit der Formel IV mit der Verbindung mit der Formel III, so dass eine Verbindung mit der Formel II erhalten wird: 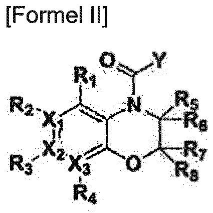 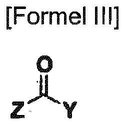 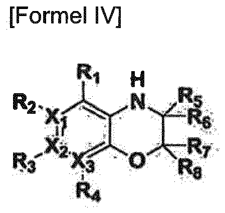 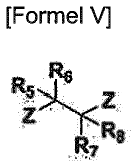 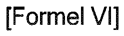 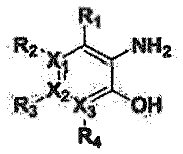 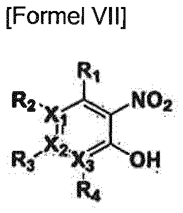 worin in den Formeln II bis VII X₁ X₂, X₃, R₁, R₂, R₃, R₄, R₅, R₆, R₇, R₈ und Y mit denjenigen identisch sind, die im Anspruch 1 festgelegt sind und
Z eine reaktive Abgangsgruppe darstellt.

19. Verfahren zur Herstellung einer Verbindung mit der Formel II oder eines Racemats oder eines pharmazeutisch verträglichen Salzes davon, umfassend die Schritte:
a) Halogenieren der Verbindung mit der Formel VII, so dass eine Verbindung mit der Formel X erhalten wird, und dann Umsetzen der erhaltenen Verbindung mit der Formel X mit der Verbindung mit der Formel IX, so dass eine Verbindung mit der Formel VIII erhalten wird, oder Durchführen einer Mitsunobu-Reaktion mit der Verbindung mit der Formel VII und der Verbindung mit der Formel IX, so dass eine Verbindung mit der Formel VIII erhalten wird,
b) Cyclisieren der erhaltenen Verbindung mit der Formel VIII, so dass eine Verbindung mit der Formel IV erhalten wird, und
c) Peptidbinden der erhaltenen Verbindung mit der Formel IV mit der Verbindung mit der Formel III, so dass eine Verbindung mit der Formel II erhalten wird: 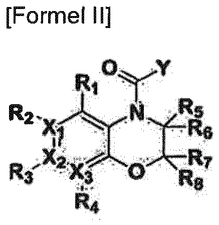 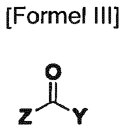 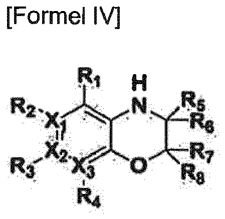 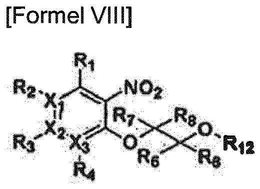 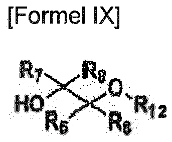 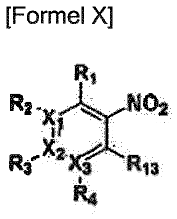 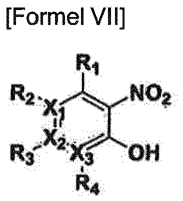 worin in den Formeln II bis X X₁, X₂, X₃, R₁, R₂, R₃, R₄, R₅, R₆, R₇, R₈ und Y mit denjenigen identisch sind, die im Anspruch 1 festgelegt sind,
R₁₂ ein von Wasserstoff verschiedener Substituent ist und
R₁₃ eine reaktive Abgangsgruppe darstellt.

20. Verfahren zur Herstellung einer Verbindung mit der Formel XI oder eines Racemats oder eines pharmazeutisch verträglichen Salzes davon, umfassend die Schritte des Umsetzens einer Verbindung mit der Formel II mit Lawessons's Reagenz, 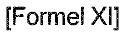 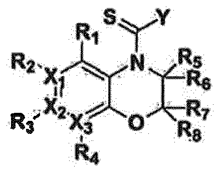 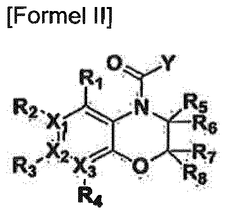 worin in den Formeln II und XI X₁, X₂, X₃, R₁, R₂, R₃, R₄, R₅, R₆, R₇, R₈ und Y mit denjenigen identisch sind, die im Anspruch 1 festgelegt sind.

21. Verfahren zur Herstellung einer Verbindung mit der Formel XII oder eines Racemats oder eines pharmazeutisch verträglichen Salzes davon, umfassend die Schritte des Amidbindens einer Verbindung mit der Formel IV mit einer Verbindung mit der Formel XIII in der Gegenwart einer Base: 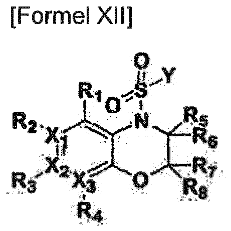 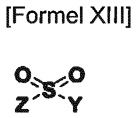 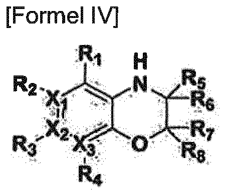 worin in den Formeln IV, XII und XIII X₁, X₂, X₃, R₁, R₂, R₃, R₄, R₅, R₆, R₇, R₈ und Y mit denjenigen identisch sind, die im Anspruch 1 festgelegt sind,
Z eine reaktive Abgangsgruppe darstellt.

22. Verfahren zur Herstellung einer Verbindung mit der Formel XIV oder eines Racemats oder eines pharmazeutisch verträglichen Salzes davon, umfassend die Schritte des Alkylierens einer Verbindung mit der Formel IV mit einer Verbindung mit der Formel XV in der Gegenwart einer Base: 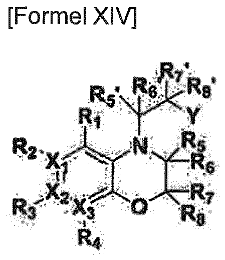 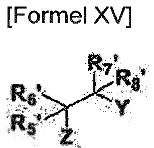 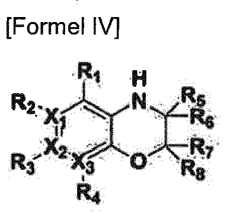 worin in den Formeln IV bis XV X₁, X₂, X₃, R₁, R₂, R₃, R₄, R₅, R₆, R₇, R₈ und Y mit denjenigen identisch sind, die im Anspruch 1 festgelegt sind,
Z eine reaktive Abgangsgruppe darstellt und
jeder von R₅', R₆', R₇' und R₈' gleich oder verschieden sein kann und unabhängig aus der Gruppe, bestehend aus Wasserstoff, Hydroxy, C₁-C₆-Alkyl, C₁-C₆-Halogenalkyl, C₁-C₆-Alkoxy, C₁-C₆-Halogenalkoxy, C₂-C₇-Alkanoyl, Halogen, Phenyl, Cyano, Nitro, Amino und einer Carbonsäuregruppe, ausgewählt ist, oder R₅' und R₆' zusammen mit einem Kohlenstoffatom, an das sie gebunden sind, eine Carbonylgruppe (C=O) oder eine Thioxogruppe (C=S) bilden können, oder R₇' und R₈' zusammen mit einem Kohlenstoffatom, an das sie gebunden sind, eine Carbonylgruppe (C=O) oder eine Thioxogruppe (C=S) bilden können.

23. Pharmazeutische Zusammensetzung, die eine wirksame Menge der Verbindung, die in einem der Ansprüche 1 bis 17 definiert ist, oder eines Racemats oder eines pharmazeutisch verträglichen Salzes davon als Wirkstoff und einen pharmazeutisch verträglichen Träger umfasst.

24. Pharmazeutische Zusammensetzung nach Anspruch 23 zur Behandlung oder Prophylaxe von Hyperurikämie, Gicht, Nephritis, chronischem Nierenversagen, Nierensteinkrankheit, Urämie, Harnsteinleiden oder einer Krankheit, die mit Harnsäure zusammenhängt.

25. Pharmazeutische Zusammensetzung nach Anspruch 24, wobei es sich bei der Gicht um akute Gichtarthritis, chronische Gichtarthritis, Gichtknoten oder Gichtnephropathie handelt.

26. Pharmazeutische Zusammensetzung nach Anspruch 24, bei der die Krankheit, die mit Harnsäure zusammenhängt, Hyperlipidämie, ischämische Herzkrankheit, Myokardinfarkt, Hirninfarkt, zerebrovaskuläre Verschlusskrankheit, Diabetes oder Hypertonie ist.

27. Pharmazeutische Zusammensetzung nach Anspruch 23, die für eine orale Verabreichung formuliert ist.

## Revendications

1. Composé dérivé hétérocyclique de formule I : 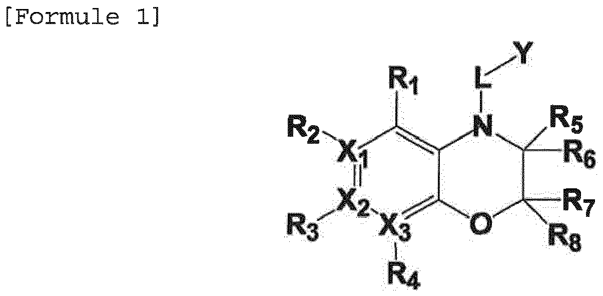 où, dans la formule I,
chacun de X₁, X₂ et X₃ est indépendamment le carbone ou l'azote, sous réserve qu'au moins l'un de X₁, X₂ et X₃ soit l'azote,
chacun de R₁, R₂, R₃ et R₄, qui peuvent être identiques ou différents, est indépendamment choisi dans l'ensemble constitué par l'hydrogène ; les halogènes ; hydroxy ; alkyle en C₁ à C₆ ; alcényle en C₂ à C₇ ; alcynyle en C₂ à C₇ ; hydroxyalkyle en C₁ à C₆ ; halogénoalkyle en C₁ à C₆ ; alcoxy en C₁ à C₆ ; halogénoalcoxy en C₁ à C₆ ; phényle ; cyano ; nitro ; amino ; un groupe acide carboxylique ; un groupe acide phosphorique ; N-oxyde ; amide ; alkylamide en C₁ à C₆ ; aldéhyde ; l'acide hydroxamique ; alkylsulfure en C₁ à C₆ ; alkylthioxo en C₁ à C₆ ; alkylsulfonyle en C₁ à C₆ ; oxime-alkyle en C₁ à C₆ ; aminoalkyle en C₁ à C₆ ; alkylcarbonylalkyle en C₃ à C₈ ; alcanoyle en C₂ à C₇ ; alcoxycarbonyle en C₂ à C₇ ; alcanoyloxy en C₂ à C₇ ; mono- ou bi-cycloalkyle en C₃ à C₁₂ ; cycloalkylalkyle en C₄ à C₁₂ ; aryle en C₆ à C₁₂ ; mono- ou poly-carbocyclyle en C₃ à C₁₂ saturé ou insaturé ; et mono- ou poly-hétérocyclyle à 3 à 12 chaînons, saturé ou insaturé, contenant 1 à 3 hétéroatomes, sous réserve que, lorsque X₁ est l'azote, R₂ n'existe pas ; lorsque X₂ est l'azote, R₃ n'existe pas ; et lorsque X₃ est l'azote, R₄ n'existe pas, ou bien chacune des paires R₁-R₂, R₂-R₃ et R₃-R₄ puisse être indépendamment condensée pour former un carbocycle ou hétérocycle à 5 à 11 chaînons, saturé ou insaturé,
chacun de R₅, R₆, R₇ et R₈, qui peuvent être identiques ou différents, est indépendamment choisi dans l'ensemble constitué par l'hydrogène ; les halogènes ; hydroxy ; alkyle en C₁ à C₆ ; alcényle en C₂ à C₇ ; alcynyle en C₂ à C₇ ; hydroxyalkyle en C₁ à C₆ ; halogénoalkyle en C₁ à C₆ ; alcoxy en C₁ à C₆ ; halogénoalcoxy en C₁ à C₆ ; alcanoyle en C₂ à C₇ ; un groupe acide phosphorique ; N-oxyde ; amide ; alkylamide en C₁ à C₆ ; aldéhyde ; l'acide hydroxamique ; alkylsulfure en C₁ à C₆ ; alkylthioxo en C₁ à C₆ ; alkylsulfonyle en C₁ à C₆ ; oxime-alkyle en C₁ à C₆ ; aminoalkyle en C₁ à C₆ ; alkylcarbonylalkyle en C₃ à C₈ ; phényle ; cyano ; nitro ; amino ; et un groupe acide carboxylique, ou bien R₅ et R₆, conjointement avec un atome de carbone auquel ils sont rattachés, peuvent former un groupe carbonyle (C=O) ou un groupe thioxo (C=S), ou bien R₇ et R₈, conjointement avec un atome de carbone auquel ils sont rattachés, peuvent former un groupe carbonyle (C=O) ou un groupe thioxo (C=S),
L peut former un groupe carbonyle (-C(=O)-), un groupe sulfonyle (-S(=O)₂-), un groupe (alkyle en C₁ à C₆)-carbonyle, un groupe carbonyl-alkyle en C₁ à C₆ ou un groupe thioxo (-C(=S)-), et
Y est choisi dans l'ensemble constitué par un mono- ou poly-carbocyclyle en C₃ à C₁₂, saturé ou insaturé, substitué par R₉, R₁₀ et R₁₁ ; et un mono- ou poly-hétérocyclyle à 3 à 12 chaînons, saturé ou insaturé, contenant 1 à 3 hétéroatomes et substitué par R₉, R₁₀ et R₁₁,
où chacun de R₉, R₁₀ et R₁₁ est indépendamment choisi dans l'ensemble constitué par l'hydrogène ; les halogènes ; hydroxy ; alkyle en C₁ à C₆ ; alcényle en C₂ à C₇ ; alcynyle en C₂ à C₇ ; hydroxyalkyle en C₁ à C₆ ; halogénoalkyle en C₁ à C₆ ; alcoxy en C₁ à C₆ ; halogénoalcoxy en C₁ à C₆ ; alkylsulfure en C₁ à C₆ ; alkylthioxo en C₁ à C₆ ; l'acide hydroxamique ; phényle ; cyano ; nitro ; amino ; un groupe acide carboxylique ; amide ; alkylamide en C₁ à C₆ ; alcanoyle en C₂ à C₇ ; aldéhyde ; ester en C₃ à C₈ ; esteroxy en C₃ à C₈ ; alkylsulfonyle en C₁ à C₆ ; oxime-alkyle en C₁ à C₆ ; aminoalkyle en C₁ à C₆ ; alkylcarbonylalkyle en C₃ à C₈ ; un groupe acide phosphorique ; et N-oxyde,
sous réserve que, lorsque Y est phényle, i) au moins l'un de R₉, R₁₀ et R₁₁ soit hydroxy ou ii) R₉, R₁₀ et R₁₁ soient tous autres que l'hydrogène si aucun de R₉, R₁₀ et R₁₁ n'est hydroxy, et lorsque Y est pyridinyle, au moins l'un de R₉, R₁₀ et R₁₁ ne soit pas l' hydrogène,
ou un racémate, ou un sel pharmaceutiquement acceptable d'un tel composé.

2. Composé selon la revendication 1, dans lequel L est un groupe carbonyle (-C(=O)-), un groupe sulfonyle (-S(=O)₂), ou un groupe thioxo (-C(=S)-), ou un racémate ou un sel pharmaceutiquement acceptable d'un tel composé.

3. Composé selon la revendication 1, dans lequel chacun de R₅, R₆, R₇ et R₈, qui peuvent être identiques ou différents, est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ ; halogénoalcoxy en C₁ à C₄ ; alcanoyle en C₂ à C₅ ; phényle ; cyano ; nitro ; amino ; et un groupe acide carboxylique ; ou bien R₅ et R₆, conjointement avec un atome de carbone auquel ils sont rattachés, peuvent former un groupe carbonyle (C=O) ou un groupe thioxo (C=S), ou bien R₇ et R₈, conjointement avec un atome de carbone auquel ils sont rattachés, peuvent former un groupe carbonyle (C=O) ou un groupe thioxo (C=S), ou un racémate ou un sel pharmaceutiquement acceptable d'un tel composé.

4. Composé selon la revendication 1, dans lequel Y est choisi dans l'ensemble constitué par un carbocyclyle en C₅ à C₆ saturé ou insaturé, substitué par R₉, R₁₀ et R₁₁ ; et un hétérocyclyle à 5 ou 6 chaînons, saturé ou insaturé, contenant 1 à 3 hétéroatomes et substitué par R₉, R₁₀ et R₁₁, où R₉, R₁₀ et R₁₁ sont tels que définis dans la revendication 1, ou un racémate ou un sel pharmaceutiquement acceptable d'un tel composé.

5. Composé selon la revendication 1, dans lequel chacun de R₉, R₁₀ et R₁₁ est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ ; halogénoalcoxy en C₁ à C₄ ; phényle ; cyano ; nitro ; amino ; un groupe acide carboxylique ; amide ; alkylamide en C₁ à C₆ ; alcanoyle en C₂ à C₅ ; aldéhyde ; ester en C₃ à C₇ ; esteroxy en C₃ à C₇ ; alkylsulfonyle en C₁ à C₄ ; oxime-alkyle en C₁ à C₄ ; aminoalkyle en C₁ à C₄ ; alkylcarbonylalkyle en C₃ à C₇ ; un groupe acide phosphorique ; et N-oxyde,
sous réserve que, lorsque Y est phényle, i) au moins l'un de R₉, R₁₀ et R₁₁ soit hydroxy ou ii) R₉, R₁₀ et R₁₁ soient tous autres que l'hydrogène si aucun de R₉, R₁₀ et R₁₁ n'est hydroxy, et lorsque Y est pyridinyle, au moins l'un de R₉, R₁₀ et R₁₁ ne soit pas l'hydrogène,
ou un racémate, ou un sel pharmaceutiquement acceptable d'un tel composé.

6. Composé selon la revendication 1, dans lequel chacune des paires R₁-R_{2,} R₂-R₃ et R₃-R₄ est indépendamment condensée pour former un carbocycle ou hétérocycle à 5 ou 6 chaînons, saturé ou insaturé, l'hétérocycle contenant de préférence 1 à 3 hétéroatomes choisis parmi N, O et S, ou un racémate ou un sel pharmaceutiquement acceptable d'un tel composé.

7. Composé selon la revendication 1, dans lequel chacun de R₁, R₂, R₃ et R₄, qui peuvent être identiques ou différents, est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ substitué ou non substitué ; halogénoalcoxy en C₁ à C₄ ; phényle ; cyano ; nitro ; amino ; un groupe acide carboxylique ; alcanoyle en C₂ à C₅ ; alcoxycarbonyle en C₂ à C₅ ; alcanoyloxy en C₂ à C₅ ; mono- ou bi-cycloalkyle en C₃ à C₁₀ ; cycloalkylalkyle en C₄ à C₁₁ ; aryle en C₆ à C₁₀ ; mono- ou poly-carbocyclyle en C₃ à C₁₀ saturé ou insaturé ; et mono- ou poly-hétérocyclyle à 3 à 10 chaînons, saturé ou insaturé, contenant 1 à 3 hétéroatomes, sous réserve que, lorsque X₁ est l'azote, R₂ n'existe pas ; lorsque X₂ est l'azote, R₃ n'existe pas ; et lorsque X₃ est l'azote, R₄ n'existe pas ; ou un racémate ou un sel pharmaceutiquement acceptable d'un tel composé.

8. Composé selon la revendication 1, dans lequel le mono- ou bi-cycloalkyle en C₃ à C₁₂ est un monocycloalkyle choisi parmi cyclopropyle, cyclobutyle, cyclopentyle, cyclohexyle, cycloheptyle, cyclooctyle et cyclononyle, ou un bicycloalkyle obtenu par condensation de deux desdits monocycloalkyles identiques ou différents, ou un racémate ou un sel pharmaceutiquement acceptable d'un tel composé.

9. Composé selon la revendication 1, dans lequel le mono- ou poly-carbocyclyle en C₃ à C₁₂ est un monocycloalkyle choisi parmi cyclopropyle, cyclobutyle, cyclopentyle, cyclohexyle, cycloheptyle, cyclooctyle et cyclononyle, ou un polycycloalkyle obtenu par condensation de deux ou plus desdits monocycloalkyles identiques ou différents, ou carboaryle, ou un racémate ou un sel pharmaceutiquement acceptable d'un tel composé.

10. Composé selon la revendication 1, dans lequel le mono- ou poly-hétérocyclyle à 3 à 12 chaînons, saturé ou insaturé, contenant 1 à 3 hétéroatomes, est thiényle, thiazolyle, imidazolyle, benzimidazolyle, triazolyle, tétrahydropyranyle, pyridinyle, furanyle, pyranyle, pyrrolyle, pyrazolyle, pyrazinyle, pyrimidinyle, isothiazolyle, isoxazolyle, pyridazinyle, isobenzopyranyle, chroményle, indolyle, indazolyle, quinolinyle, purinyle, pyrrolinyle, chromanyle, pyrazolidinyle, pipéridinyle ou pipérazinyle, ou un racémate ou un sel pharmaceutiquement acceptable d'un tel composé.

11. Composé selon la revendication 1, dans lequel X₂ est le carbone, et chacun de X₁ et X₃ est indépendamment le carbone ou l'azote, sous réserve qu'au moins l'un de X₁ et X₃ soit l'azote, ou un racémate ou un sel pharmaceutiquement acceptable d'un tel composé.

12. Composé selon la revendication 1, dans lequel chacun de R₁, R₂, R₃ et R₄, qui peuvent être identiques ou différents, est indépendamment choisi dans l'ensemble constitué par l'hydrogène ; les halogènes ; alkyle en C₁ à C₄ ; halogénoalkyle en C₁ à C₄ ; phényle ; cyano ; et mono-ou poly-hétérocyclyle à 3 à 10 chaînons, saturé ou insaturé, contenant 1 à 3 hétéroatomes ; ou bien chacune des paires R₁-R₂, R₂-R₃ et R₃-R₄ peut être indépendamment condensée pour former un carbocycle à 5 ou 6 chaînons, saturé ou insaturé,
sous réserve que, lorsque X₁ est l'azote, R₂ n'existe pas ; lorsque X₂ est l'azote, R₃ n'existe pas ; et lorsque X₃ est l'azote, R₄ n'existe pas,
ou un racémate ou un sel pharmaceutiquement acceptable d'un tel composé.

13. Composé selon la revendication 1, dans lequel Y est un carbocycle ou hétérocycle aromatique à 5 ou 6 chaînons substitué par R₉, R₁₀ et R₁₁, où R₉, R₁₀ et R₁₁ sont tels que définis dans la revendication 1, ou un racémate ou un sel pharmaceutiquement acceptable d'un tel composé.

14. Composé selon la revendication 1, dans lequel chacun de R₉, R₁₀ et R₁₁ est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ ; halogénoalcoxy en C₁ à C₄ ; nitro ; un groupe acide carboxylique ; et esteroxy en C₃ à C₇,
sous réserve que, lorsque Y est phényle, i) au moins l'un de R₉, R₁₀ et R₁₁ soit hydroxy ou ii) R₉, R₁₀ et R₁₁ soient tous autres que l'hydrogène si aucun de R₉, R₁₀ et R₁₁ n'est hydroxy, et lorsque Y est pyridinyle, au moins l'un de R₉, R₁₀ et R₁₁ ne soit pas l'hydrogène,
ou un racémate, ou un sel pharmaceutiquement acceptable d'un tel composé.

15. Composé selon la revendication 1, dans lequel chacun de X₁, X₂ et X₃ est indépendamment le carbone ou l'azote, sous réserve qu'au moins l'un de X₁, X₂ et X₃ soit l'azote,
chacun de R₁, R₂, R₃ et R₄, qui peuvent être identiques ou différents, est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ ; halogénoalcoxy en C₁ à C₄ ; cyano ; nitro ; amino ; un groupe acide carboxylique ; alcanoyle en C₂ à C₅ ; alcoxycarbonyle en C₂ à C₅ ; alcanoyloxy en C₂ à C₅ ; aryle en C₆ à C₁₂ ; et mono- ou poly-carbocyclyle ou hétérocyclyle en C₃ à C₁₂, saturé ou insaturé, sous réserve que, lorsque X₁ est l'azote, R₂ n'existe pas ; lorsque X₂ est l'azote, R₃ n'existe pas ; et lorsque X₃ est l'azote, R₄ n'existe pas, ou bien chacune des paires R₁-R₂, R₂-R₃ et R₃-R₄ puisse être indépendamment condensée pour former un carbocycle ou hétérocycle à 5 ou 6 chaînons, saturé ou insaturé,
chacun de R₅, R₆, R₇ et R₈, qui peuvent être identiques ou différents, est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ ; halogénoalcoxy en C₁ à C₄ ; alcanoyle en C₂ à C₅ ; cyano ; nitro ; amino ; et un groupe acide carboxylique, ou bien R₅ et R₆, conjointement avec un atpme de carbone auquel ils sont rattachés, peuvent former un groupe carbonyle (C=O), ou bien R₇ et R₈, conjointement avec un atome de carbone auquel ils sont rattachés, peuvent former un groupe carbonyle (C=O),
L peut former un groupe carbonyle (-C(=O)-), un groupe sulfonyle (-S(=O)₂-), ou un groupe thioxo (-C(=S)-), et
Y est choisi dans l'ensemble constitué par un mono- ou poly-carbocyclyle en C₃ à C₁₂, saturé ou insaturé, substitué par R₉, R₁₀ et R₁₁ ; et un mono- ou poly-hétérocyclyle à 3 à 12 chaînons, saturé ou insaturé, contenant 1 à 3 hétéroatomes et substitué par R₉, R₁₀ et R₁₁, l'hétérocycle contenant de préférence 1 à 3 hétéroatomes choisis parmi N, O et S,
où chacun de R₉, R₁₀ et R₁₁ est indépendamment choisi dans l'ensemble constitué par l'hydrogène ; les halogènes ; hydroxy ; alkyle en C₁ à C₄ ; halogénoalkyle en C₁ à C₄ ; alcoxy en C₁ à C₄ ; halogénoalcoxy en C₁ à C₄ ; cyano ; nitro ; amino ; un groupe acide carboxylique ; et esteroxy en C₃ à C₇,
sous réserve que, lorsque Y est phényle, i) au moins l'un de R₉, R₁₀ et R₁₁ soit hydroxy ou ii) R₉, R₁₀ et R₁₁ soient tous autres que l'hydrogène si aucun de R₉, R₁₀ et R₁₁ n'est hydroxy, et lorsque Y est pyridinyle, au moins l'un de R₉, R₁₀ et R₁₁ ne soit pas l' hydrogène,
ou un racémate, ou un sel pharmaceutiquement acceptable d'un tel composé.

16. Composé selon la revendication 1, dans lequel
chacun de X₁, X₂ et X₃ est indépendamment le carbone ou l'azote, sous réserve qu'au moins l'un de X₁, X₂ et X₃ soit l'azote,
chacun de R₁, R₂, R₃ et R₄, qui peuvent être identiques ou différents, est indépendamment choisi dans l'ensemble constitué par l'hydrogène ; les halogènes ; alkyle en C₁ à C₄ ; halogénoalkyle en C₁ à C₄ ; alcoxy en C₁ à C₄ ; cyano ; nitro ; amino ; et carbocyclyle ou hétérocyclyle en C₅ à C₆, saturé ou insaturé, sous réserve que, lorsque X₁ est l'azote, R₂ n'existe pas ; lorsque X₂ est l'azote, R₃ n'existe pas ; et lorsque X₃ est l'azote, R₄ n'existe pas, ou chacune des paires R₁-R₂, R₂-R₃ et R₃-R₄ puisse être indépendamment condensée pour former un phényle ou un hétérocycle à 6 chaînons contenant 1 ou 2 atomes d'azote ou d'oxygène,
chacun de R₅, R₆, R₇ et R₈, qui peuvent être identiques ou différents, est indépendamment choisi dans l'ensemble constitué par l'hydrogène ; les halogènes ; alkyle en C₁ à C₄ substitué ou non substitué ; alcoxy en C₁ à C₄ ; cyano ; nitro ; et amino,
L est un groupe carbonyle (-C(=O)-) ou un groupe thioxo (-C(=S)-), et
Y est un phényle qui porte un groupe hydroxy en position para vis-à-vis de la position de rattachement de L et est encore substitué par 1 à 3 substituants indépendamment choisis parmi les halogènes et nitro,
ou un racémate, ou un sel pharmaceutiquement acceptable d'un tel composé.

17. Composé selon la revendication 1, qui est choisi dans l'ensemble constitué par les suivants :
(3,5-dibromo-4-hydroxyphényl)-(2,3-dihydropyrido[2,3-b][1,4]oxazin-1-yl)méthanone (composé 1) ;
(3,5-dibromo-4-méthoxyphényl)-(2,3-dihydropyrido[4,3-b][1,4]oxazin-4-yl)méthanone (composé 2) ;
sel d'acide bromhydrique et de (3,5-dibromo-4-hydroxyphényl)-(2,3-dihydropyrido[4,3-b][1,4]oxazin-4-yl)méthanone (composé 3) ;
(3,5-dibromo-4-hydroxyphényl)-(2,3-dihydropyrido[4,3-b] [1,4]oxazin-4-yl)méthanone (composé 4) ;
(3,5-dibromo-4-méthoxyphényl)-(2,3-dihydropyrido[3,4-b] [1,4]oxazin-1-yl)méthanone (composé 5) ;
(3,5-dibromo-4-hydroxyphényl)-(2,3-dihydropyrido[3,4-b][1,4]oxazin-1-yl)méthanone (composé 6) ;
(3,5-dibromo-4-hydroxyphényl)-(6-méthyl-2,3-dihydropyrido-[2,3-b][1,4]oxazin-1-yl)méthanone (composé 7) ;
(3,5-dibromo-4-hydroxyphényl)-(2,2-diméthyl-2,3-dihydro-pyrido[4,3-b][1,4]oxazin-4-yl)méthanone (composé 8) ;
(3,5-dibromo-4-hydroxyphényl)-(7-cyclopropyl-2,3-dihydro-pyrido[2,3-b][1,4]oxazin-1-yl)méthanone (composé 9) ;
sel d'acide bromhydrique et de (3-chloro-4-hydroxyphényl)-(2,3-dihydropyrido[4,3-b][1,4]oxazin-4-yl)méthanone (composé 10) ;
sel d'acide bromhydrique et de (3-bromo-4-hydroxyphényl)-(2,3-dihydropyrido[4,3-b][1,4]oxazin-4-yl)méthanone (composé 11) ;
(2,3-dihydropyrido[4,3-b][1,4]oxazin-4-yl)-(4-hydroxy-3-trifluorométhylphényl)méthanone (composé 12) ;
sel d'acide bromhydrique et de (3,5-dichloro-4-hydroxyphényl)-(2,3-dihydropyrido[4,3-b][1,4]oxazin-4-yl)méthanone (composé 13) ;
(3-chloro-4-hydroxy-5-nitrophényl)-(2,3-dihydropyrido[4,3-b][1,4]oxazin-4-yl)méthanone (composé 14) ;
sel d'acide bromhydrique et de (3,5-dichloro-4-hydroxyphényl)-(2,3-dihydropyrido[3,4-b][1,4]oxazin-1-yl)méthanone (composé 15) ;
sel d'acide bromhydrique et de (3-bromo-4-hydroxyphényl)-(2,3-dihydropyrido[3,4-b][1,4]oxazin-1-yl)méthanone (composé 16) ;
sel d'acide bromhydrique et de (3-chloro-4-hydroxy-5-nitrophényl)-(2,3-dihydropyrido[3,4-b][1,4]oxazin-1-yl)méthanone (composé 17) ;
sel d'acide bromhydrique et de (3-chloro-4-hydroxyphényl)-(2,3-dihydropyrido[3,4-b][1,4]oxazin-1-yl)méthanone (composé 18) ;
(2,3-dihydropyrido[3,4-b][1,4]oxazin-1-yl)-(4-hydroxy-3-trifluorométhylphényl)méthanone (composé 19) ;
(3,5-dibromo-4-hydroxyphényl)-(7-phényl-2,3-dihydropyrido[2,3-b][1,4]oxazin-1-yl)méthanone (composé 20) ;
2,6-dichloro-4-(2,3-dihydropyrido[4,3-b][1,4]oxazin-4-sulfonyl)phénol (composé 21) ;
(3,5-dibromo-4-méthoxyphényl)-(7-trifluorométhyl-2,3-dihydropyrido[2,3-b][1,4]oxazin-1-yl)méthanone (composé 22-1) ;
(3,5-dibromo-4-hydroxyphényl)-(7-trifluorométhyl-2,3-dihydropyrido[2,3-b][1,4]oxazin-1-yl)méthanone (composé 22-2) ;
acide 2,5-dibromo-4-(2,3-dihydropyrido[4,3-b][1,4]oxazin-4-carbonyl)benzoïque (composé 23) ;
ester méthylique d'acide (2,6-dibromo-4-(2,3-dihydropyrido[4,3-b][1,4]oxazin-4-carbonyl)phénoxy]acétique (composé 24) ;
(7-bromo-2,3-dihydropyrido[2,3-b][1,4]oxazin-1-yl)-(3,5-dibromo-4-hydroxyphényl)méthanone (composé 25) ;
(2,3-dihydropyrido[4,3-b][1,4]oxazin-4-yl)-(3-fluoro-4-hydroxyphényl)méthanone (composé 26) ;
(3,5-dibromo-4-méthoxyphényl)-(7-méthyl-2,3-dihydropyrido[2,3-b][1,4]oxazin-1-yl)méthanone (composé 27-1) ;
(3,5-dibromo-4-hydroxyphényl)-(7-méthyl-2,3-dihydropyrido[2,3-b][1,4]oxazin-1-yl)méthanone (composé 27-2) ;
(3,5-difluoro-4-méthoxyphényl)-(2,3-dihydropyrido[4,3-b][1,4]oxazin-4-yl)méthanone (composé 28) ;
(3,5-difluoro-4-hydroxyphényl)-(2,3-dihydropyrido[4,3-b][1,4]oxazin-4-yl)méthanone (composé 29) ;
(5-chloro-2,3-dihydropyrido[4,3-b][1,4]oxazin-4-yl)-(3,5-dibromo-4-hydroxyphényl)méthanone (composé 30) ;
(2,6-dichloropyridin-4-yl)-(2,3-dihydropyrido[4,3-b][1,4]oxazin-4-yl)méthanone (composé 31) ;
(2,3-dihydropyrido[4,3-b][1,4]oxazin-4-yl)-(6-hydroxypyridin-3-yl)méthanone (composé 32) ;
sel d'acide chlorhydrique et de (3,5-dibromo-4-hydroxyphényl)-(2,3-dihydropyrido[4,3-b][1,4]oxazin-4-yl)méthanone (composé 33) ;
(3-chloro-4-hydroxyphényl)-(2,3-dihydropyrido[3,4-b][1,4]oxazin-1-yl)méthanone (composé 34) ;
4-(2,3-dihydropyrido[4,3-b] [1,4]oxazin-4-sulfonyl)phénol (composé 35-1) ;
2,6-dibromo-4-(2,3-dihydropyrido[4,3-b][1,4]oxazin-4-sulfonyl)phénol (composé 35-2) ;
(3-chloro-4-hydroxy-5-nitrophényl)-(2,3-dihydropyrido[3,4-b][1,4]oxazin-1-yl)méthanone (composé 36) ;
(3-chloro-4-hydroxyphényl)-(2,3-dihydropyrido[4,3-b][1,4]oxazin-4-yl)méthanone (composé 37) ;
(3-bromo-4-hydroxyphényl)-(2,3-dihydropyrido[4,3-b][1,4]oxazin-4-yl)méthanone (composé 38) ;
(3,5-dichloro-4-hydroxyphényl)-(2,3-dihydropyrido[4,3-b][1,4]oxazin-4-yl)méthanone (composé 39) ;
(3-bromo-4-hydroxyphényl)-(2,3-dihydropyrido[3,4-b][1,4]oxazin-1-yl)méthanone (composé 40) ;
(3,5-dichloro-4-hydroxyphényl)-(2,3-dihydropyrido[3,4-b][1,4]oxazin-1-yl)méthanone (composé 41) ;
2-(3,5-dibromo-4-hydroxyphényl)-1-(2,3-dihydropyrido[4.3-b][1,4]oxazin-4-yl)éthanone (composé 42) ;
(2,3-dihydropyrido[4,3-b][1,4]oxazin-4-yl)-(3-méthoxy-isoxazol-5-yl)méthanone (composé 43) ;
(2,3-dihydropyrido[4,3-b][1,4]oxazin-4-yl)-(3-hydroxy-isoxazol-5-yl)méthanone (composé 44) ;
(3,5-dibromo-4-hydroxyphényl)-[7-(4-trifluorométhylphényl)-2,3-dihydropyrido[2,3-b][1,4]oxazin-1-yl]méthanone (composé 45) ;
(3,5-dibromo-4-hydroxyphényl)-[7-(2-trifluorométhylphényl)-2,3-dihydropyrido[2,3-b][1,4]oxazin-1-yl]méthanone (composé 46) ;
1-(3,5-dibromo-4-méthoxybenzoyl)-2,3-dihydro-1H-pyrido[2,3-b][1,4]oxazin-7-carbonitrile (composé 47-1) ;
1-(3,5-dibromo-4-hydroxybenzoyl)-2,3-dihydro-1H-pyrido[2,3-b][1,4]oxazin-7-carbonitrile (composé 47-2) ;
(3,5-dibromo-4-méthoxyphényl)-[7-(3-nitrophényl)-2,3-dihydropyrido[2,3-b][1,4]oxazin-1-yl]méthanone (composé 48) ;
(3,5-dibromo-4-méthoxyphényl)-[7-(3-nitrophényl)-2,3-dihydropyrido[2,3-b][1,4]oxazin-1-yl]méthanone (composé 49) ;
(3,5-dibromo-4-hydroxyphényl)-[7-(3-diméthylaminophényl)-2,3-dihydropyrido[2,3-b][1,4]oxazin-1-yl]méthanone (composé 50) ;
(3,5-dibromo-4-hydroxyphényl)-(2,3-dihydropyrido[4,3-b][1,4]oxazin-4-yl)méthanethione (composé 51) ;
(3,5-dibromo-4-hydroxyphényl)-(7-pyridin-3-yl-2,3-dihydropyrido[2,3-b][1,4]oxazin-1-yl]méthanone (composé 52) ;
(3,5-dibromo-4-hydroxyphényl)-(7-furan-3-yl-2,3-dihydropyrido[2,3-b][1,4]oxazin-1-yl]méthanone (composé 53) ;
1-(3,5-dibromo-4-hydroxyphényl)-2-(2,3-dihydropyrido[4.3-b][1,4]oxazin-4-yl)éthanone (composé 54) ;
(3,5-dibromo-4-hydroxyphényl)-(2,3-dihydro-4-oxa-1,9-diazaphénanthrén-1-yl)méthanone (composé 55) ;
4-[2-(3,5-dibromo-4-hydroxyphényl)-2-oxoéthyl)]-4H-pyrido[4,3-b][1,4]oxazin-3-one (composé 56) ;
4-(3,5-dibromo-4-méthoxybenzoyl)-4H-pyrido[4,3-b] [1,4]oxazin-3-one (composé 57) ;
(3,5-dibromo-4-méthoxyphényl)-(6-méthyl-2,3-dihydropyrido[2,3-b][1,4]oxazin-1-yl)méthanone (composé 58) ;
(2,3-dihydropyrido[4,3-b][1,4]oxazin-4-yl)-(2,4-dihydroxypyrimidin-5-yl)méthanone (composé 59) ;
(2,3-dihydropyrido[4,3-b][1,4]oxazin-4-yl)-(2,6-dihydroxypyrimidin-4-yl)méthanone (composé 60) ;
(3,5-dibromo-4-hydroxyphényl)-(7-isoquinolin-4-yl-2,3-dihydropyrido[2,3-b][1,4]oxazin-1-yl)méthanone (composé 61) ;
(3,5-dibromo-4-hydroxyphényl)-(6,7-dihydropyrimido[4,5-b][1,4]oxazin-5-yl)méthanone (composé 62) ;
(3,5-dibromo-4-hydroxyphényl)-[7-(3-trifluorométhylphényl)-2,3-dihydropyrido[2,3-b][1,4]oxazin-1-yl]méthanone (composé 63) ;
(3,5-dibromo-4-hydroxyphényl)-[7-(3-fluorométhylphényl)-2,3-dihydropyrido[2,3-b][1,4]oxazin-1-yl]-méthanone (composé 64) ;
4-[1-(3,5-dibromo-4-hydroxybenzoyl)-2,3-dihydro-1H-pyrido[2,3-b][1,4]oxazin-7-yl]benzonitrile (composé 65) ;
(3,5-dibromo-4-hydroxyphényl)-[7-(4-trifluorométhoxyphényl)-2,3-dihydropyrido[2,3-b][1,4]oxazin-1-yl]-méthanone (composé 66) ;
1-{4-[1-(3,5-dibromo-4-hydroxybenzoyl)-2,3-dihydro-1H-pyrido[2,3-b][1,4]oxazin-7-yl]phényl}éthanone (composé 67) ;
(3,5-dibromo-4-hydroxyphényl)-[7-(5-méthoxypyridin-3-yl)-2,3-dihydropyrido[2,3-b][1,4]oxazin-1-yl]méthanone (composé 68) ;
(4-hydroxy-3-trifluorométhylphényl)-(7-méthyl-2,3-dihydropyrido[2,3-b][1,4]oxazin-1-yl)méthanone (composé 69) ;
(3,5-dibromo-4-hydroxyphényl)-[7-(1H-indol-4-yl)-2,3-dihydropyrido[2,3-b][1,4]oxazin-1-yl]méthanone (composé 70) ;
sel d'acide sulfurique et de (3,5-dibromo-4-hydroxyphényl)-(2,3-dihydropyrido[4,3-b][1,4]oxazin-4-yl)méthanone (composé 71) ;
sel (2,6-dibromo-4-(2,3-dihydropyrido[4,3-b][1,4]oxazin-4-carbonyl)-phénolate de sodium (composé 72) ;
sel (2,6-dibromo-4-(2,3-dihydropyrido[4,3-b][1,4]oxazin-4-carbonyl)phénolate de potassium (composé 73) ;
sel d'acide trifluoroacétique et de (3,5-dibromo-4-hydroxyphényl)-(2,3-dihydropyrido[4,3-b][1,4]oxazin-4-yl)méthanethione (composé 74) ; et
1-[1-(3,5-dibromo-4-hydroxybenzoyl)-2,3-dihydro-1H-pyrido[2,3-b][1,4]oxazin-7-yl]-pyrrolidin-2-one (composé 75),
ou un racémate, ou un sel pharmaceutiquement acceptable d'un tel composé.

18. Procédé pour préparer un composé de formule II, ou un racémate, ou un sel pharmaceutiquement acceptable d'un tel composé, comprenant les étapes de :
a) réduction d'un composé de formule VII pour obtenir un composé de formule VI,
b) cyclisation du composé de formule VI obtenu avec un composé de formule V pour obtenir un composé de formule IV, et
c) liaison peptidique du composé de formule IV obtenu avec un composé de formule III pour obtenir un composé de formule II : 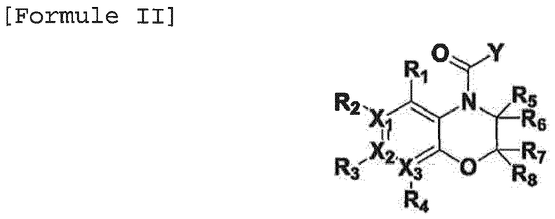 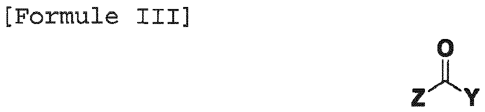 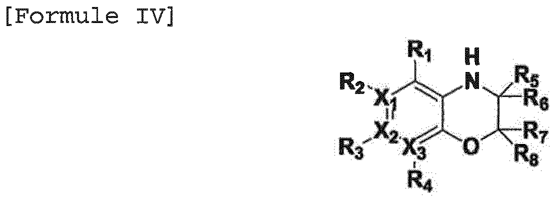 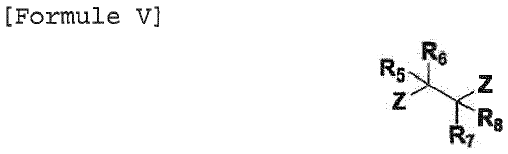 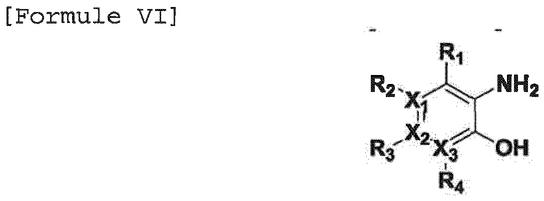 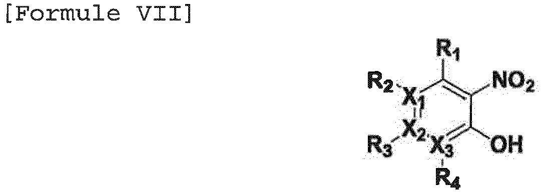 où, dans les formules II à VII,
X₁, X₂, X₃, R₁, R₂, R₃, R₄, R₅, R₆, R₇, R₈ et Y sont tels que définis dans la revendication 1, et Z représente un groupe partant réactif.

19. Procédé pour préparer un composé de formule II, ou un racémate, ou un sel pharmaceutiquement acceptable d'un tel composé, comprenant les étapes de :
a) halogénation d'un composé de formule VII pour obtenir un composé de formule X et ensuite réaction du composé de formule X obtenu avec un composé de formule IX pour obtenir un composé de formule VIII, ou mise en oeuvre d'une réaction de Mitsunobu avec le composé de formule VII et le composé de formule IX pour obtenir un composé de formule VIII,
b) cyclisation du composé de formule VIII obtenu pour obtenir un composé de formule IV, et
c) liaison peptidique du composé de formule IV obtenu avec un composé de formule III pour obtenir un composé de formule II : 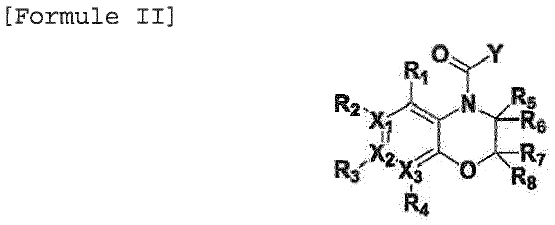 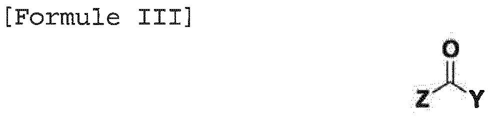 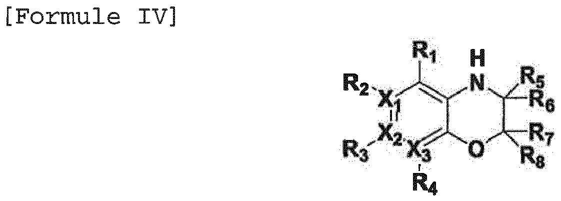 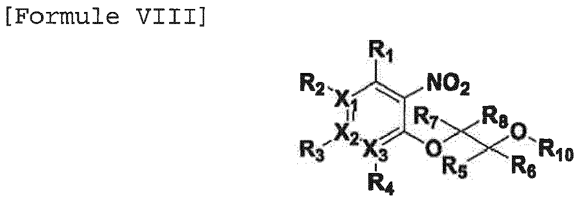 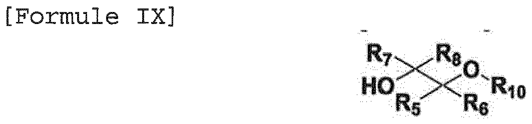 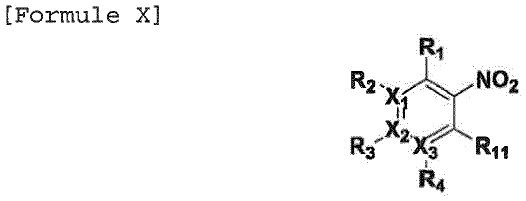 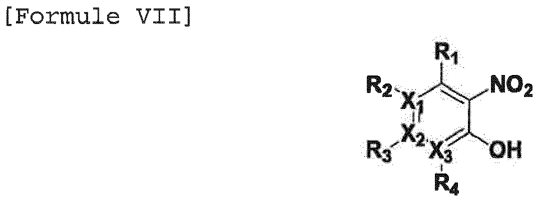 où, dans les formules II à X,
X₁, X₂, X₃, R₁, R₂, R₃, R₄, R₅, R₆, R₇, R₈ et Y sont tels que définis dans la revendication 1, R₁₂ est un substituant non hydrogène, et R₁₃ représente un groupe partant réactif.

20. Procédé pour préparer un composé de formule XI, ou un racémate ou un sel pharmaceutiquement acceptable d'un tel composé, comprenant l'étape de réaction d'un composé de formule II avec un réactif de Lawesson : 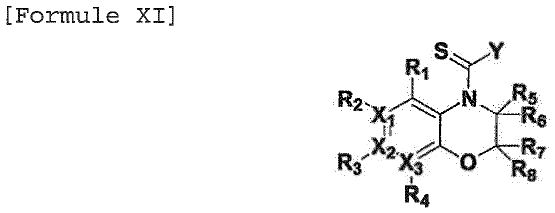 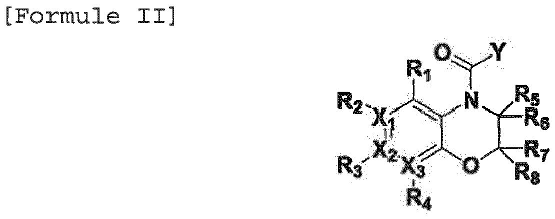 où, dans les formules II et XI,
X₁, X₂, X₃, R₁, R₂, R₃, R₄, R₅, R₆, R₇, R₈ et Y sont tels que définis dans la revendication 1.

21. Procédé pour préparer un composé de formule XII, ou un racémate ou un sel pharmaceutiquement acceptable d'un tel composé, 1 comprenant l'étape de liaison amide d'un composé de formule IV avec un composé de formule XIII en présence d'une base : 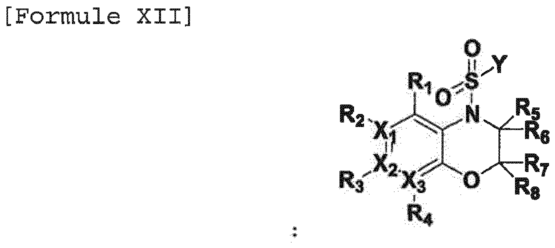 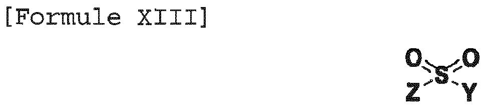 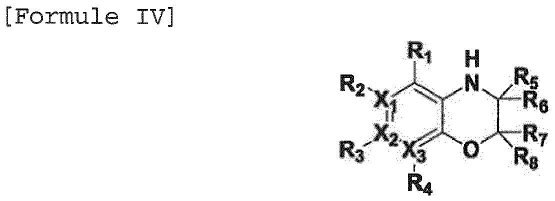 où, dans les formules IV, XII et XIII,
X₁, X₂, X₃, R₁, R₂, R₃, R₄, R₅, R₆, R₇, R₈ et Y sont tels que définis dans la revendication 1, et Z représente un groupe partant réactif.

22. Procédé pour préparer un composé de formule XIV, ou un racémate ou un sel pharmaceutiquement acceptable d'un tel composé, comprenant l'étape d'alkylation d'un composé de formule IV avec un composé de formule XV en présence d'une base : 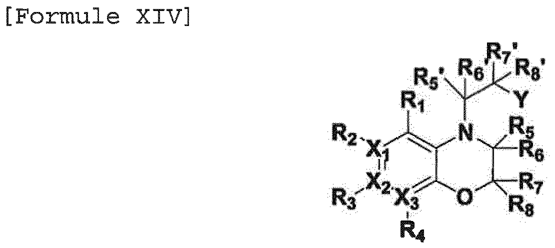 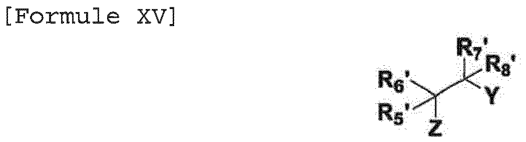 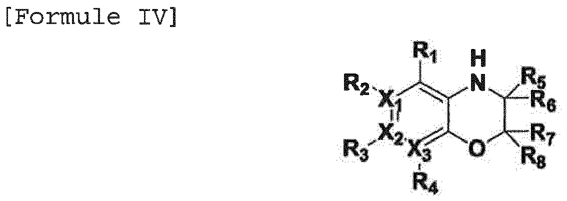 où, dans les formules IV à XV,
X₁, X₂, X₃, R₁, R₂, R₃, R₄, R₅, R₆, R₇, R₈ et Y sont tels que définis dans la revendication 1,
Z représente un groupe partant réactif, et
chacun de R₅', R₆', R₇' et R₈', qui peuvent être identiques ou différents, est indépendamment choisi dans l'ensemble constitué par l'hydrogène ; les halogènes ; hydroxy ; alkyle en C₁ à C₆ ; halogénoalkyle en C₁ à C₆ ; alcoxy en C₁ à C₆ ; halogénoalcoxy en C₁ à C₆ ; alcanoyle en C₂ à C₇ ; phényle ; cyano ; nitro ; amino ; et un groupe acide carboxylique ; ou bien R₅' et R₆', conjointement avec un atome de carbone auquel ils sont rattachés, peuvent former un groupe carbonyle (C=O) ou un groupe thioxo (C=S), ou bien R₇' et R₈', conjointement avec un atome de carbone auquel ils sont rattachés, peuvent former un groupe carbonyle (C=O) ou un groupe thioxo (C=S).

23. Composition pharmaceutique comprenant une quantité efficace du composé défini dans l'une quelconque des revendications 1 à 17 ou un racémate ou un sel pharmaceutiquement acceptable d'un tel composé, à titre d'agent actif, et un véhicule pharmaceutiquement acceptable.

24. Composition pharmaceutique selon la revendication 23, qui est destinée au traitement ou à la prophylaxie de l'hyperuricémie, de la goutte, d'une néphrite, d'une insuffisance rénale chronique, d'une lithiase rénale, d'une urémie, d'une lithiase urinaire, ou d'une maladie associée à l'acide urique.

25. Composition pharmaceutique selon la revendication 24, dans laquelle la goutte est l'arthrite goutteuse aiguë, l'arthrite goutteuse chronique, le tophus ou le syndrome néphrotique associé à la goutte.

26. Composition pharmaceutique selon la revendication 24, dans laquelle la maladie associée à l'acide urique est une hyperlipidémie, une maladie cardiaque ischémique, un infarctus du myocarde, un infarctus cérébral, une maladie cérébrovasculaire, le diabète ou l'hypertension.

27. Composition pharmaceutique selon la revendication 23, qui est formulée pour une administration par voie orale.
